(19) Europäisches Patentamt / European Patent Office / Office européen des brevets

(11) **EP 4 610 049 A1**

(12) **EUROPEAN PATENT APPLICATION**

(43) Date of publication:
**03.09.2025 Bulletin 2025/36**

(21) Application number: **25159050.1**

(22) Date of filing: **20.02.2025**

(51) International Patent Classification (IPC):
***B41C 1/10*** *(2006.01)*

(52) Cooperative Patent Classification (CPC):
**B41C 1/1008;** B41C 2210/04; B41C 2210/08; B41C 2210/22

(84) Designated Contracting States:
**AL AT BE BG CH CY CZ DE DK EE ES FI FR GB GR HR HU IE IS IT LI LT LU LV MC ME MK MT NL NO PL PT RO RS SE SI SK SM TR**
Designated Extension States:
**BA**
Designated Validation States:
**GE KH MA MD TN**

(30) Priority: **29.02.2024 JP 2024030661**

(71) Applicant: **FUJIFILM Corporation**
**Tokyo 106-8620 (JP)**

(72) Inventors:
- **MIZUNO, Akio**
  **4000,Kawashiri,Yoshida-cho,Haibara-gun,Shizuoka,**
  **421-0396 (JP)**
- **UEMURA, Minoru**
  **4000,Kawashiri,Yoshida-cho,Haibara-gun,Shizuoka,**
  **421-0396 (JP)**
- **SAKURAGI, Seiya**
  **4000,Kawashiri,Yoshida-cho,Haibara-gun,Shizuoka,**
  **421-0396 (JP)**
- **MIYAGAWA, Yuya**
  **4000,Kawashiri,Yoshida-cho,Haibara-gun,Shizuoka,**
  **421-0396 (JP)**
- **WATANABE, Shumpei**
  **4000,Kawashiri,Yoshida-cho,Haibara-gun,Shizuoka,**
  **421-0396 (JP)**
- **NISHIMOTO, Koichiro**
  **4000,Kawashiri,Yoshida-cho,Haibara-gun,Shizuoka,**
  **421-0396 (JP)**

(74) Representative: **HGF**
**HGF Limited**
**1 City Walk**
**Leeds LS11 9DX (GB)**

(54) **ON-PRESS DEVELOPMENT TYPE LITHOGRAPHIC PRINTING PLATE PRECURSOR, METHOD OF PREPARING LITHOGRAPHIC PRINTING PLATE, LITHOGRAPHIC PRINTING METHOD, AND COMPOUND**

(57) Provided is an on-press development type lithographic printing plate precursor having excellent printing durability.

An on-press development type lithographic printing plate precursor includes: a support; and an image-recording layer on the support, in which the image-recording layer contains a compound represented by Formula (1), a method of preparing a lithographic printing plate using the on-press development type lithographic printing plate precursor, a lithographic printing method, and a novel compound. In the formula, $R^1$ represents a group represented by Formula (2) or Formula (3), $R^{10}$ represents a monovalent organic group having an aryl group, $R^{11}$ to $R^{14}$ and $R^{17}$ to $R^{20}$ each independently represent a hydrogen atom, an alkyl group, or an aryl group, $R^{15}$ and $R^{16}$ each independently represent a hydrogen atom, an alkyl group, or an aryl group, and at least two of $R^{11}$ to $R^{20}$ may be bonded to each other to form a ring structure.

EP 4 610 049 A1

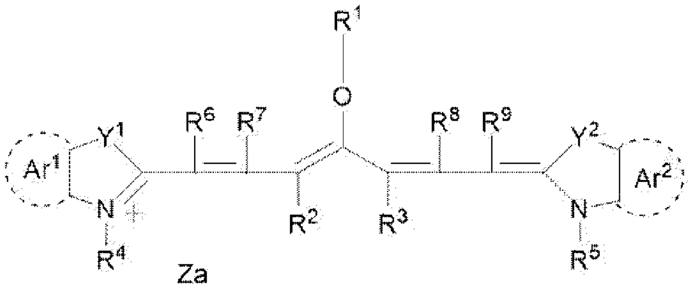

( 1 )

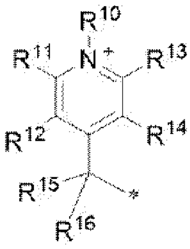

( 2 )

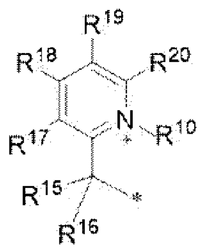

( 3 )

## Description

### BACKGROUND OF THE INVENTION

#### 1. Field of the Invention

The present disclosure relates to an on-press development type lithographic printing plate precursor, a method of preparing a lithographic printing plate, a lithographic printing method, and a compound.

#### 2. Description of the Related Art

In general, a lithographic printing plate is composed of lipophilic image areas that receive ink during a printing process and hydrophilic non-image areas that receive a dampening water. Lithographic printing is a printing method that utilizes the property that water and oil-based ink repel each other to cause differences in ink attachment behavior on a surface of the lithographic printing plate by using lipophilic image areas on the lithographic printing plate as an ink-receiving area and using hydrophilic non-image areas on the lithographic printing plate as dampening water-receiving areas (non-ink-receiving areas), thereby depositing the ink only to the image areas, and then transferring the ink to a printing material, such as paper.

In the related art, in order to prepare this lithographic printing plate, a lithographic printing plate precursor (PS plate) has been widely used which is obtained by providing a lipophilic photosensitive resin layer (image-recording layer) on a hydrophilic support. Usually, a lithographic printing plate is obtained by a plate making method of exposing a lithographic printing plate precursor through an original picture such as a lith film, then keeping a portion of an image-recording layer that will be an image area while removing other unnecessary portions of the image-recording layer by dissolving such portions in an alkaline developer or an organic solvent, and forming a non-image area by exposing a surface of the hydrophilic support.

In response to the intensifying interest in the global environment, an environmental issue of waste liquid generated by wet treatments such as a development treatment has gathered more attention.

Regarding the environmental issue described above, an attempt is made to simplify development or plate making or to remove treatments. As one of simple preparation methods, a method called "on-press development" is being carried out. That is, the on-press development is a method of exposing a lithographic printing plate precursor, then immediately mounting the precursor on a printer without performing development of the related art, and removing an unnecessary portion of the image-recording layer at an initial stage of the ordinary printing step.

In the present disclosure, a lithographic printing plate precursor that can be used for such on-press development is called "on-press development type lithographic printing plate precursor".

Examples of the lithographic printing plate precursors in the related art include those described in WO2017/141882A or WO2016/027886A.

WO2017/141882A and WO2016/027886A disclose a color-forming composition containing a decomposition-type infrared absorber having a specific structure.

### SUMMARY OF THE INVENTION

An object of an embodiment of the present disclosure is to provide an on-press development type lithographic printing plate precursor having excellent printing durability.

An object of another embodiment of the present disclosure is to provide a method of preparing a lithographic printing plate and a lithographic printing method in which the on-press development type lithographic printing plate precursor is used.

An object of still another embodiment of the present disclosure is to provide a novel compound.

Means for solving the above issues include the following aspects.

<1> An on-press development type lithographic printing plate precursor having a support and an image-recording layer on the support, in which the image-recording layer contains a compound represented by Formula (1).

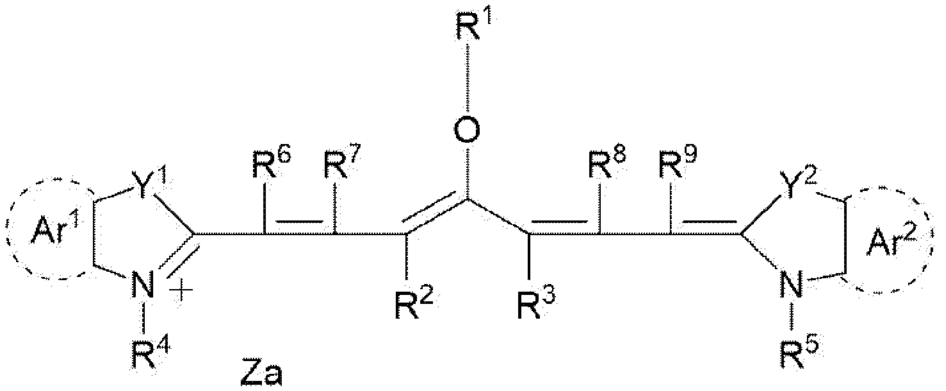

(1)

In Formula (1), $R^1$ represents a group represented by Formula (2) or Formula (3), $R^2$ and $R^3$ each independently represent a hydrogen atom or an alkyl group, $R^2$ and $R^3$ may be linked to each other to form a ring, $Ar^1$ and $Ar^2$ each independently represent a benzene ring or a naphthalene ring, $Y^1$ and $Y^2$ each independently represent an oxygen atom, a sulfur atom, -$NR^0$-, or a dialkylmethylene group, $R^4$ and $R^5$ each independently represent an alkyl group, $R^6$ to $R^9$ each independently represent a hydrogen atom or an alkyl group, $R^0$ represents a hydrogen atom, an alkyl group, or an aryl group, and Za represents a counterion that neutralizes a charge.

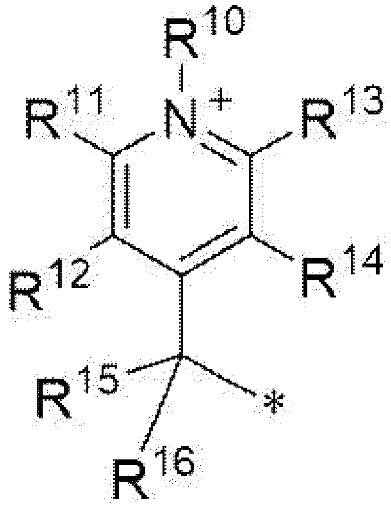

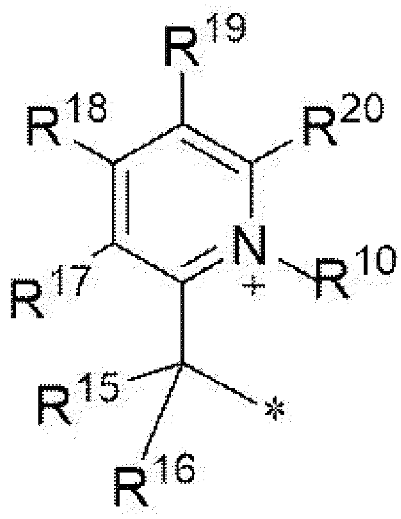

(2) (3)

In Formulae (2) and (3), $R^{10}$ represents a monovalent organic group having an aryl group, $R^{11}$ to $R^{14}$ and $R^{17}$ to $R^{20}$ each independently represent a hydrogen atom, an alkyl group, an aryl group, a hydroxy group, an alkoxy group, or a halogen atom, $R^{15}$ and $R^{16}$ each independently represent a hydrogen atom, an alkyl group, or an aryl group, at least two of $R^{11}$ to $R^{20}$ may be bonded to each other to form a ring structure, and * represents a single bond that is bonded to an oxygen atom in Formula (1).

<2> The on-press development type lithographic printing plate precursor according to <1>, in which the $R^{10}$ is a substituted benzyl group.

<3> The on-press development type lithographic printing plate precursor according to <2>, in which $R^{10}$ is a benzyl group having an alkyl group on a benzene ring.

<4> The on-press development type lithographic printing plate precursor according to any one of <1> to <3>, in which $R^{15}$ is a hydrogen atom and $R^{16}$ is a branched alkyl group.

<5> The on-press development type lithographic printing plate precursor according to any one of <1> to <4>, in which $R^{15}$ is a hydrogen atom and $R^{16}$ is a t-butyl group.

<6> The on-press development type lithographic printing plate precursor according to any one of <1> to <5>, in which $R^{11}$ to $R^{14}$ and $R^{17}$ to $R^{20}$ are hydrogen atoms.

<7> The on-press development type lithographic printing plate precursor according to any one of <1> to <6>, in which the image-recording layer further contains a borate compound.

<8> The on-press development type lithographic printing plate precursor according to any one of <1> to <7>, in which the image-recording layer further contains a polymerizable compound.

<9> The on-press development type lithographic printing plate precursor according to any one of <1> to <8>, in which the image-recording layer further contains an onium salt compound.

<10> The on-press development type lithographic printing plate precursor according to <9>, in which the onium salt compound includes an iodonium salt compound.

<11> The on-press development type lithographic printing plate precursor according to any one of <1> to <10>, in which the image-recording layer further contains a phenol compound.

<12> A method of preparing a lithographic printing plate, comprising: a step of exposing the on-press development type lithographic printing plate precursor according to any one of <1> to <11> in a shape of an image; and a step of supplying at least one selected from the group consisting of a printing ink and dampening water on a printer to remove an image-recording layer in a non-image area.

<13> A lithographic printing method comprising: a step of exposing the on-press development type lithographic printing plate precursor according to any one of <1> to <11> in a shape of an image; a step of supplying at least one selected from the group consisting of a printing ink and dampening water on a printer to remove an image-recording layer in a non-image area and to prepare a lithographic printing plate; and a step of performing printing using the obtained lithographic printing plate.

<14> A compound which is represented by Formula (1A).

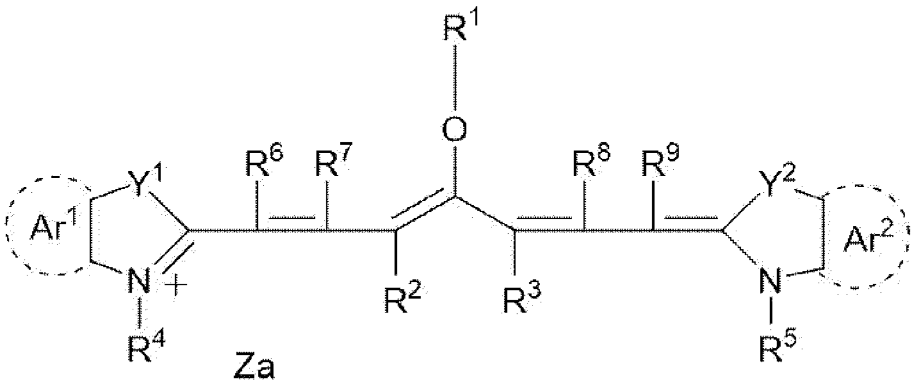

Za

(1A)

In Formula (1A), $R^1$ represents a group represented by Formula (2A) or Formula (3A), $R^2$ and $R^3$ each independently represent a hydrogen atom or an alkyl group, $R^2$ and $R^3$ may be linked to each other to form a ring, $Ar^1$ and $Ar^2$ each independently represent a benzene ring or a naphthalene ring, $Y^1$ and $Y^2$ each independently represent an oxygen atom, a sulfur atom, $-NR^0-$, or a dialkylmethylene group, $R^4$ and $R^5$ each independently represent an alkyl group, $R^6$ to $R^9$ each independently represent a hydrogen atom or an alkyl group, $R^0$ represents a hydrogen atom, an alkyl group, or an aryl group, and Za represents a counterion that neutralizes a charge.

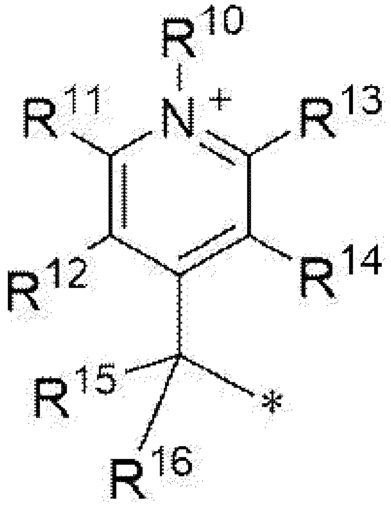

( 2A )

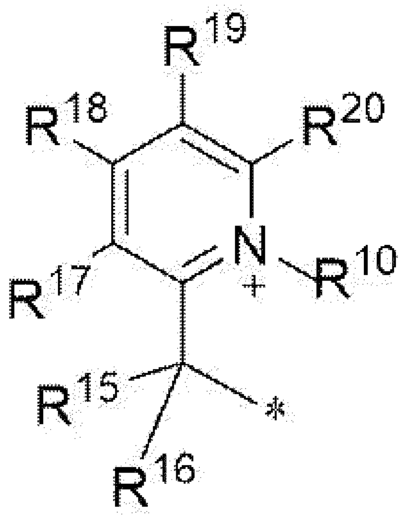

( 3A )

In Formulae (2A) and (3A), $R^{10}$ represents a group having one or more substituents on an aromatic ring of a benzyl group, $R^{11}$ to $R^{14}$ and $R^{17}$ to $R^{20}$ each independently represent a hydrogen atom, an alkyl group, an aryl group, a hydroxy group, an alkoxy group, or a halogen atom, $R^{15}$ and $R^{16}$ each independently represent a hydrogen atom, an alkyl group, or an aryl group, at least two of $R^{11}$ to $R^{20}$ may be bonded to each other to form a ring structure, and * represents a single bond that is bonded to an oxygen atom in Formula (1A).

<15> The compound according to <14>, in which the compound is a compound represented by Formula (7) or Formula (8).

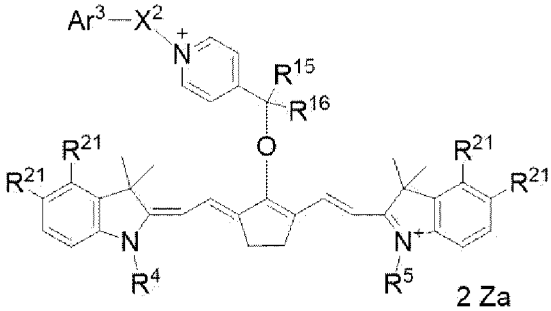

2 Za

( 7 )

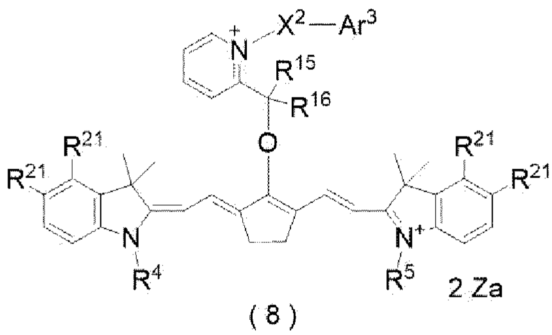

2 Za

( 8 )

In Formula (7) and Formula (8), $X^2$ represents a single bond or an alkylene group, $Ar^3$ represents an aryl group having one or more substituents on an aromatic ring, $R^4$ and $R^5$ each independently represent an alkyl group, $R^{15}$ and $R^{16}$ each independently represent a hydrogen atom, an alkyl group, or an aryl group, $R^{15}$ and $R^{16}$ may be bonded to each other to form a ring structure, $R^{21}$'s each independently represent an alkyl group having 1 to 12 carbon atoms, an alkoxy group, an aryloxy group, an amino group, an alkoxythio group, or a halogen atom, $R^{21}$'s may be linked to each other to form a ring, and Za represents a counterion that neutralizes a charge.

<16> The compound according to <14>, in which the compound is a compound represented by Formula (9),

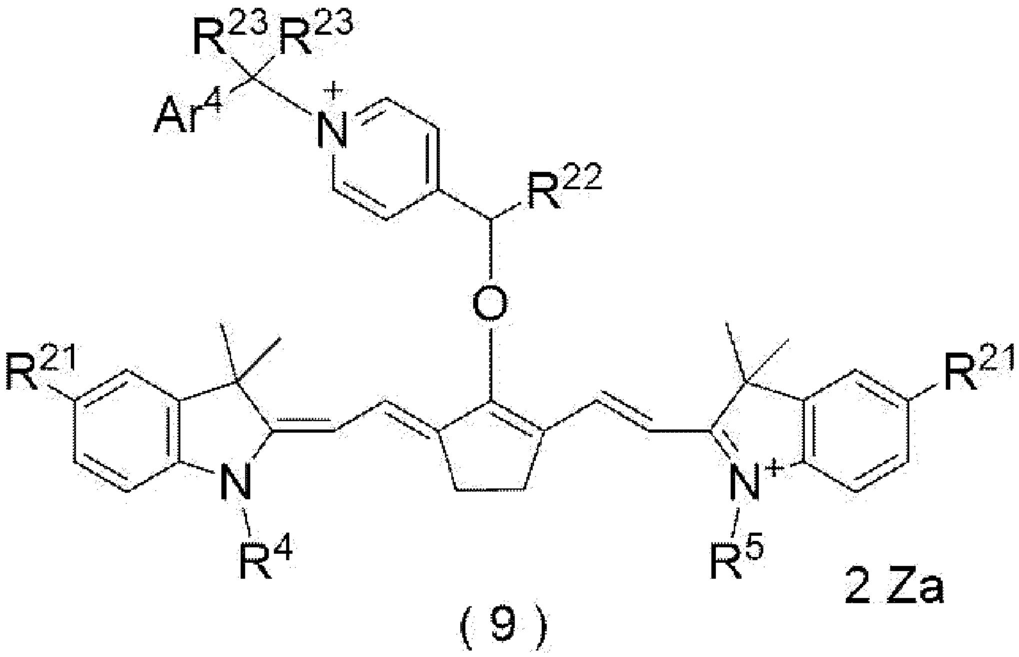

(9)

In Formula (9), $Ar^4$ represents an aryl group having one or more substituents on an aromatic ring, $R^4$ and $R^5$ each independently represent an alkyl group, $R^{21}$'s each independently represent an alkyl group having 1 to 12 carbon atoms, an alkoxy group, an aryloxy group, an amino group, an alkoxythio group, or a halogen atom, $R^{22}$ represents a hydrogen atom, an alkyl group, or an aryl group, $R^{23}$'s each independently represent a hydrogen atom or an alkyl group, and Za represents a counterion that neutralizes charge.

According to an embodiment of the present disclosure, it is possible to provide an on-press development type lithographic printing plate precursor having excellent printing durability.

In addition, according to another embodiment of the present disclosure, it is possible to provide a method of preparing a lithographic printing plate and a lithographic printing method in which the on-press development type lithographic printing plate precursor is used.

Furthermore, according to still another embodiment of the present disclosure, it is possible to provide a novel compound.

## BRIEF DESCRIPTION OF THE DRAWINGS

Fig. 1 is a graph of an example of a waveform diagram of alternating waveform current used for an electrochemical roughening treatment.

Fig. 2 is a lateral view showing an example of a radial cell in an electrochemical roughening treatment using alternating current.

Fig. 3 is a schematic view showing a cross-sectional shape of an end part of a lithographic printing plate precursor.

Fig. 4 is a conceptual view showing an example of a cutting portion of a slitter device.

Fig. 5 is a schematic view of an anodization treatment device used for an anodization treatment in a manufacturing method of a support having an anodic oxide film.

## DESCRIPTION OF THE PREFERRED EMBODIMENTS

Hereinafter, the contents of the present disclosure will be described in detail. The description of configuration requirements below is made based on representative embodiments of the present disclosure in some cases, but the present disclosure is not limited to such embodiments.

In the present disclosure, a numerical range expressed using "to" includes numerical values listed before and after "to" as the lower limit value and the upper limit value.

In addition, in the present disclosure, in a case where there is no description regarding whether a group (atomic group) is substituted or unsubstituted, such a group includes both a group having no substituent and a group having a

substituent. For example, "alkyl group" includes not only an alkyl group having no substituent (unsubstituted alkyl group) but also an alkyl group having a substituent (substituted alkyl group).

In the present disclosure, "(meth)acryl" is a term used to explain a concept including both the acryl and methacryl, and "(meth)acryloyl" is a term used to explain a concept including both the acryloyl and methacryloyl.

The term "step" in the present disclosure means not only an independent step but also a step that cannot be clearly differentiated from other steps as long as the intended goal of the step is achieved.

In the present disclosure, "% by mass" and "% by weight" have the same meaning, and "part(s) by mass" and "part(s) by weight" have the same meaning.

In the present disclosure, unless otherwise specified, as each component contained in a composition or each constitutional unit contained in a polymer, one component or one constitutional unit may be used alone, or two or more components or two or more constitutional units may be used in combination.

In the present disclosure, in a case where there is a plurality of substances corresponding to each component in a composition, or in a case where there is a plurality of constitutional units corresponding to each constitutional unit in a polymer, unless otherwise specified, the amount of each component in the composition or the amount of each constitutional unit in the polymer means the total amount of the plurality of corresponding substances present in the composition or the total amount of the plurality of corresponding constitutional units present in the polymer.

In the present disclosure, a combination of two or more preferred aspects is a more preferred aspect.

In the present disclosure, unless otherwise specified, each of the weight-average molecular weight (Mw) and number-average molecular weight (Mn) is a molecular weight that is detected using a gel permeation chromatography (GPC) analysis device using TSKgel GMHxL, TSKgel G4000HxL, and TSKgel G2000HxL (trade names, manufactured by Tosoh Corporation) as columns, tetrahydrofuran (THF) as a solvent, and a differential refractometer, and expressed in terms of polystyrene as a standard substance.

In the present disclosure, the term "lithographic printing plate precursor" refers not only to a lithographic printing plate precursor but also to a key plate precursor. The term "lithographic printing plate" refers not only to a lithographic printing plate prepared by performing operations such as exposure and development as necessary on a lithographic printing plate precursor but also to a key plate. The key plate precursor is not necessarily subjected to the operations such as exposure and development. The key plate refers to a lithographic printing plate precursor to be mounted on a plate cylinder that is not used, in a case where monochromatic or dichromatic printing is carried out on a part of paper during, for example, color newspaper printing.

In the present disclosure, "*" in a chemical structural formula represents a bonding position with other structures.

On-press development type lithographic printing plate precursor

An on-press development type lithographic printing plate precursor (simply also referred to as a "lithographic printing plate precursor") according to the present disclosure includes a support and an image-recording layer on the support, in which the image-recording layer contains a compound represented by Formula (1).

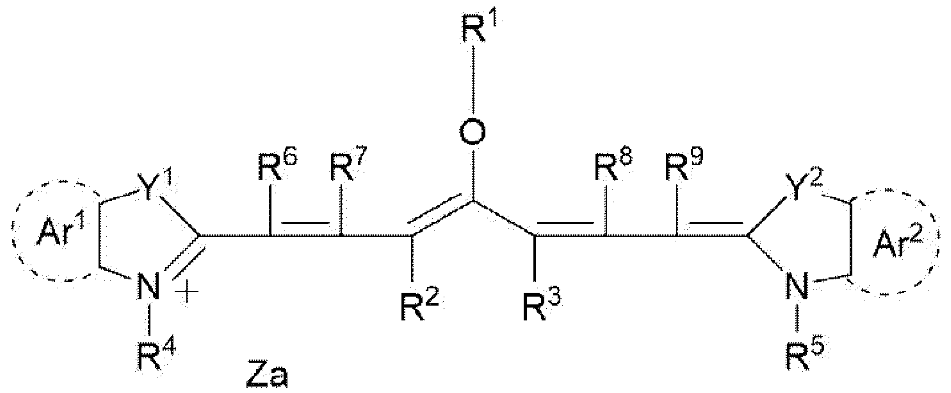

( 1 )

In Formula (1), $R^1$ represents a group represented by Formula (2) or Formula (3), $R^2$ and $R^3$ each independently represent a hydrogen atom or an alkyl group, $R^2$ and $R^3$ may be linked to each other to form a ring, $Ar^1$ and $Ar^2$ each independently represent a benzene ring or a naphthalene ring, $Y^1$ and $Y^2$ each independently represent an oxygen atom, a sulfur atom, $-NR^0-$, or a dialkylmethylene group, $R^4$ and $R^5$ each independently represent an alkyl group, $R^6$ to $R^9$ each independently represent a hydrogen atom or an alkyl group, $R^0$ represents a hydrogen atom, an alkyl group, or an aryl group, and Za represents a counterion that neutralizes a charge.

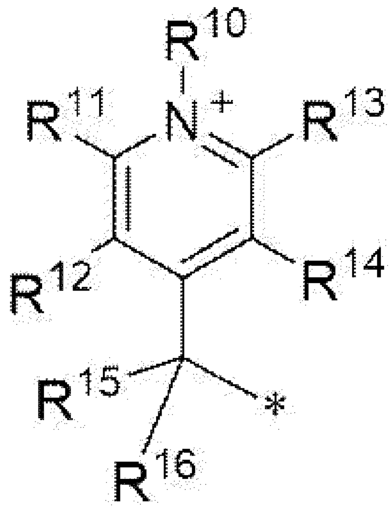

(2)

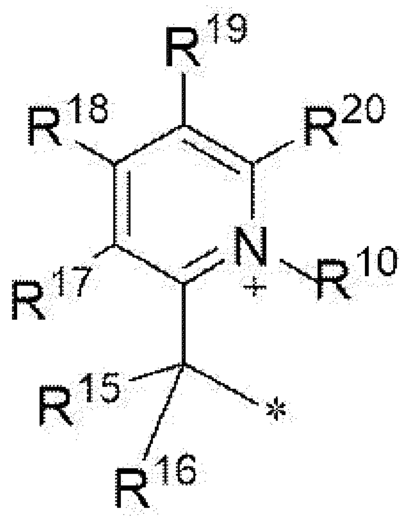

(3)

In Formulae (2) and (3), $R^{10}$ represents a monovalent organic group having an aryl group, $R^{11}$ to $R^{14}$ and $R^{17}$ to $R^{20}$ each independently represent a hydrogen atom, an alkyl group, an aryl group, a hydroxy group, an alkoxy group, or a halogen atom, $R^{15}$ and $R^{16}$ each independently represent a hydrogen atom, an alkyl group, or an aryl group, at least two of $R^{11}$ to $R^{20}$ may be bonded to each other to form a ring structure, and * represents a single bond that is bonded to an oxygen atom in Formula (1).

The lithographic printing plate precursor according to the present disclosure is preferably a negative tone lithographic printing plate precursor in which an exposed portion is polymerized by exposure to infrared.

The present inventors have found that it is difficult to obtain a lithographic printing plate precursor having excellent printing durability in a lithographic printing plate precursor having an image recording layer containing a decomposition-type infrared absorber as described in WO2017/141882A or WO2016/027886A.

It is presumed that since the image-recording layer of the lithographic printing plate precursor according to the present disclosure contains the compound represented by Formula (1), both the stability and the decomposability of the group represented by Formula (2) or Formula (3) are high, and an on-press development type lithographic printing plate precursor having excellent printing durability can be provided, although the details are unknown.

In addition, since the lithographic printing plate precursor according to the present disclosure has the group represented by Formula (2) or Formula (3) in which the compound represented by Formula (1) has both excellent stability and excellent decomposability, the lithographic printing plate precursor is excellent in color formability, on-press developability, and on-press developability after aging.

Hereinafter, each of the configuration requirements in the lithographic printing plate precursor according to the present disclosure will be specifically described.

Image-recording layer

The image-recording layer in the lithographic printing plate precursor according to the present disclosure is preferably a water-soluble or water-dispersible negative tone image-recording layer.

**In** addition, in the image-recording layer in the lithographic printing plate precursor according to the present disclosure, from the viewpoint of on-press developability, a non-exposed portion of the image-recording layer is preferably removable by at least any of dampening water or printing ink.

Hereinafter, each of the components to be contained in the image-recording layer will be specifically described.

Compound represented by Formula (1)

**In** the lithographic printing plate precursor according to the present disclosure, the image-recording layer contains the compound represented by Formula (1).

The compound represented by Formula (1) has a characteristic of decomposing by heat or infrared energy to generate a color forming substance having high visibility. **In** the present specification, the color forming means that the

coloring is stronger after heating or exposure to infrared than before heating or exposure to infrared, or the absorption is shifted to a shorter wavelength and absorption in the visible light region is exhibited. That is, the compound represented by Formula (1) is a compound that is decomposed by heat or exposure to infrared, and has an increased absorption in the visible light region or has an absorption in the visible light region due to a shift of the absorption to a short wavelength side, as compared with the absorption before heating or exposure to infrared. The compound represented by Formula (1) is preferably a compound that is decomposed by heat or exposure to infrared to generate a compound having a maximal absorption wavelength at 500 nm to 600 nm.

As described below, it is considered that in the color forming mechanism of the compound represented by Formula (1), the $R^1$-O bond is cleaved by heat or exposure to infrared, and the cleaved oxygen atom forms a carbonyl group, thereby generating a merocyanine coloring agent which is a color forming substance having high visibility.

In addition, it is considered that it is also an important factor that the above-described group represented by Formula (2) or Formula (3), in which the $R^1$-O bond is cleaved by heat or exposure to infrared, is contained as $R^1$, and $R^1$ and the cyanine coloring agent structure are bonded to each other through an oxygen atom in order to generate the merocyanine coloring agent.

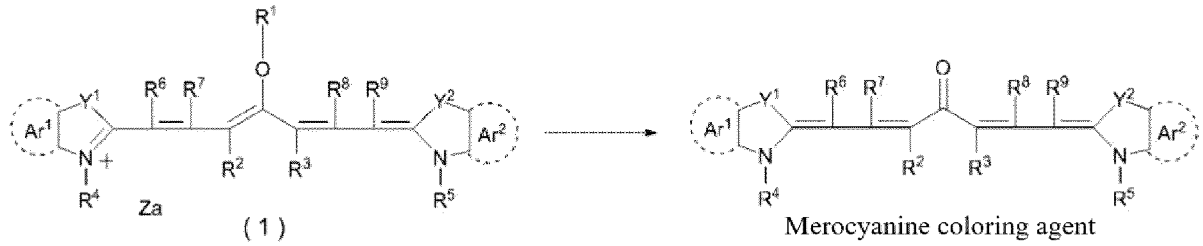

(1) Merocyanine coloring agent

In Formula (1), $R^1$ represents the group represented by Formula (2) or Formula (3) described above, in which the $R^1$-O bond is cleaved by heat or exposure to infrared. Specific examples thereof include a group that absorbs thermal energy or infrared rays given from the outside and causes the compound represented by Formula (1) to undergo decomposition or isomerization reaction by energy generated in a case of returning from an excited state to a ground state or a chemical reaction that proceeds from an excited state, thereby cleaving an $R^1$-O bond.

In a case where $R^1$ is an aryl group or a linear alkyl group, cleavage of the $R^1$-O bond does not occur by heat or exposure to infrared.

$R^1$ represents a group represented by Formula (2) or Formula (3), and from the viewpoint of visibility, printing durability, synthetic suitability, and raw material supply property, $R^1$ is preferably a group represented by Formula (2).

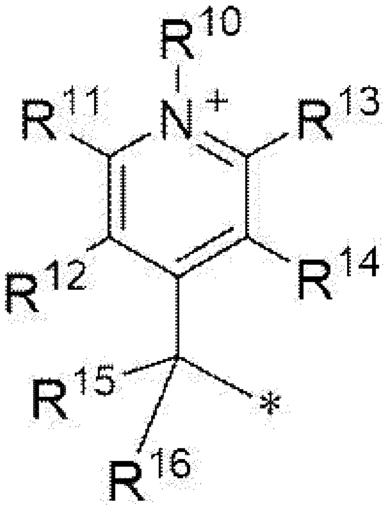

(2)

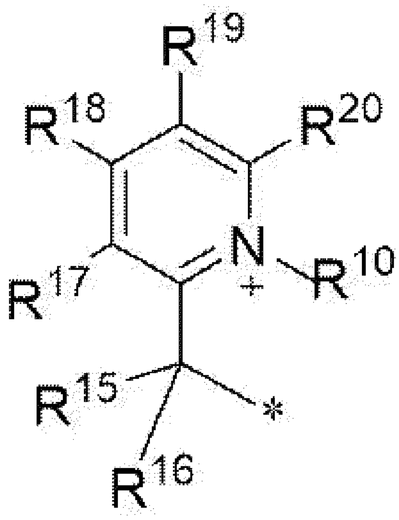

(3)

In Formulae (2) and (3), $R^{10}$ represents a monovalent organic group having an aryl group, $R^{11}$ to $R^{14}$ and $R^{17}$ to $R^{20}$ each independently represent a hydrogen atom, an alkyl group, an aryl group, a hydroxy group, an alkoxy group, or a

halogen atom, $R^{15}$ and $R^{16}$ each independently represent a hydrogen atom, an alkyl group, or an aryl group, at least two of $R^{11}$ to $R^{20}$ may be bonded to each other to form a ring structure, and * represents a single bond that is bonded to an oxygen atom in Formula (1).

From the viewpoint of visibility, printing durability, on-press developability, and on-press developability after aging, the aryl group in $R^{10}$ is preferably an aryl group having 6 to 30 carbon atoms (also referred to as "number of carbon atoms"), more preferably an aryl group having 6 to 20 carbon atoms, and still more preferably an aryl group having 6 to 12 carbon atoms.

In addition, from the viewpoint of visibility, printing durability, on-press developability, and on-press developability after aging, the aryl group as $R^{10}$ is preferably an aryl group having one or more substituents on an aromatic ring, more preferably a phenyl group having one or more substituents on an aromatic ring, still more preferably a phenyl group having one or more alkyl groups on an aromatic ring, and particularly preferably an alkylphenyl group.

From the viewpoint of visibility, printing durability, on-press developability, and on-press developability after aging, $R^{10}$ is preferably a group having one or more substituents on an aromatic ring of an arylalkyl group, more preferably a group having one or more substituents on an aromatic ring of a benzyl group, still more preferably a benzyl group having one or more substituents (substituted benzyl group), and particularly preferably a benzyl group having an alkyl group on a benzene ring.

In addition, from the viewpoint of visibility, printing durability, on-press developability, and on-press developability after aging, $R^{10}$ is preferably a group represented by Formula 4.

$$-X^2-Ar^3 \qquad (4)$$

In Formula (4), $X^2$ represents a single bond or an alkylene group, and $Ar^3$ represents an aryl group.

From the viewpoint of visibility, printing durability, and synthetic suitability, $X^2$ is preferably an alkylene group, more preferably an alkylene group having 1 to 6 carbon atoms, still more preferably an alkylene group having 1 to 3 carbon atoms, particularly preferably an alkylene group having 1 or 2 carbon atoms, and most preferably a methylene group.

From the viewpoint of visibility, printing durability, and synthetic suitability, $Ar^3$ is preferably an aryl group having 6 to 30 carbon atoms, more preferably an aryl group having 6 to 20 carbon atoms, and still more preferably an aryl group having 6 to 12 carbon atoms.

The aryl group may have a substituent, and preferably has one or more substituents. Examples of the substituent include an alkyl group, an alkoxy group, an aryloxy group, an amino group, an alkylthio group, an arylthio group, a halogen atom, a carboxy group, a carboxylate group, a sulfo group, a sulfonate group, an alkyloxycarbonyl group, an aryloxycarbonyl group, and a group obtained by combining these groups.

Specific examples thereof include a phenyl group, a naphthyl group, a p-tolyl group, a p-ethylphenyl group, a p-t-butylphenyl group, a p-chlorophenyl group, a p-fluorophenyl group, a p-methoxyphenyl group, a p-dimethylaminophenyl group, a p-methylthiophenyl group, a p-phenylthiophenyl group, a p-phenylphenyl group, an o-tolyl group, an o-ethylphenyl group, an o-tBu phenyl group, an o-chlorophenyl group, an o-fluorophenyl group, an o-methoxyphenyl group, an o-dimethylaminophenyl group, an o-methylthiophenyl group, and an o-phenylthiophenyl group.

In addition, from the viewpoint of visibility, printing durability, on-press developability, and on-press developability after aging, $Ar^3$ is preferably an aryl group having one or more substituents on an aromatic ring, more preferably a phenyl group (substituted phenyl group) having one or more substituents on an aromatic ring, still more preferably an alkylphenyl group, particularly preferably a phenyl group having a branched alkyl group, and most preferably a t-butylphenyl group.

In addition, from the viewpoint of visibility, printing durability, on-press developability, and on-press developability after aging, the alkylphenyl group is preferably a p-alkylphenyl group.

$R^{11}$ to $R^{14}$ and $R^{17}$ to $R^{20}$ represent a hydrogen atom, an alkyl group, an aryl group, a hydroxy group, an alkoxy group, or a halogen atom, and the same group may be used or different groups may be used. In addition, a plurality of $R^{11}$'s to $R^{14}$'s or a plurality of $R^{17}$'s to $R^{20}$'s may be linked to each other to form a ring.

The alkyl group represented by $R^{11}$ to $R^{14}$ and $R^{17}$ to $R^{20}$ may be linear or branched, or may have a ring structure.

Specific examples of the alkyl group include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a hexadecyl group, an octadecyl group, an eicosyl group, an isopropyl group, an isobutyl group, an s-butyl group, a t-butyl group, an isopentyl group, a neopentyl group, a 1-methylbutyl group, an isohexyl group, a 2-ethylhexyl group, a 2-methylhexyl group, a cyclohexyl group, a cyclopentyl group, and a 2-norbornyl group.

Among these alkyl groups, a methyl group, an ethyl group, a propyl group, or a butyl group is preferable.

The alkyl group in $R^{11}$ to $R^{14}$ and $R^{17}$ to $R^{20}$ may have a substituent. Examples of the substituent include an aryl group, an alkoxy group, an aryloxy group, an amino group, an alkylthio group, an arylthio group, a halogen atom, a carboxy group, a carboxylate group, a sulfo group, a sulfonate group, an alkyloxycarbonyl group, an aryloxycarbonyl group, and a group obtained by combining these groups.

The aryl group in $R^{11}$ to $R^{14}$ and $R^{17}$ to $R^{20}$ is preferably an aryl group having 6 to 30 carbon atoms, more

preferably an aryl group having 6 to 20 carbon atoms, and still more preferably an aryl group having 6 to 12 carbon atoms.

The above-described aryl group may have a substituent. Examples of the substituent include an alkyl group, an alkoxy group, an aryloxy group, an amino group, an alkylthio group, an arylthio group, a halogen atom, a carboxy group, a carboxylate group, a sulfo group, a sulfonate group, an alkyloxycarbonyl group, an aryloxycarbonyl group, and a group obtained by combining these groups.

Specific examples thereof include a phenyl group, a naphthyl group, a p-tolyl group, a p-chlorophenyl group, a p-fluorophenyl group, a p-methoxyphenyl group, a p-dimethylaminophenyl group, a p-methylthiophenyl group, and a p-phenylthiophenyl group.

Among these aryl groups, a phenyl group, a p-methoxyphenyl group, a p-dimethylaminophenyl group, or a naphthyl group is preferable.

From the viewpoint of visibility and printing durability, $R^{11}$ to $R^{14}$ and $R^{17}$ to $R^{20}$ each independently preferably represent a hydrogen atom, an alkyl group, an alkoxy group, or a halogen atom, and particularly preferably represent a hydrogen atom.

$R^{15}$ and $R^{16}$ represent a hydrogen atom, an alkyl group, or an aryl group, and may be the same group or different groups.

From the viewpoint of visibility, printing durability, and synthetic suitability, $R^{15}$ is preferably a hydrogen atom.

From the viewpoint of visibility and printing durability, $R^{16}$ is preferably an alkyl group or an aryl group, more preferably an alkyl group, still more preferably a branched alkyl group, and particularly preferably a t-butyl group.

From the viewpoint of visibility and printing durability, preferred examples of the branched alkyl group in $R^{16}$ include an isopropyl group, a t-butyl group, an isobutyl group, a cyclopentyl group, and a cyclohexyl group, and from the viewpoint of visibility and printing durability, preferred examples of the aryl group in $R^{16}$ include a phenyl group.

The alkyl group represented by $R^{15}$ and $R^{16}$ is preferably an alkyl group having 1 to 20 carbon atoms, more preferably an alkyl group having 1 to 10 carbon atoms, and still more preferably an alkyl group having 1 to 6 carbon atoms.

The alkyl group represented by $R^{15}$ and $R^{16}$ may be linear or branched, or may have a ring structure.

Specific examples of the alkyl group include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a hexadecyl group, an octadecyl group, an eicosyl group, an isopropyl group, an isobutyl group, an s-butyl group, a t-butyl group, an isopentyl group, a neopentyl group, a 1-methylbutyl group, an isohexyl group, a 2-ethylhexyl group, a 2-methylhexyl group, a cyclohexyl group, a cyclopentyl group, and a 2-norbornyl group.

Among the alkyl groups, a methyl group, an ethyl group, a propyl group, an isopropyl group, a butyl group, a t-butyl group, an isobutyl group, a cyclopentyl group, or a cyclohexyl group is preferable.

The alkyl group in $R^{15}$ and $R^{16}$ may have a substituent. Examples of the substituent include an aryl group, an alkoxy group, an aryloxy group, an amino group, an alkylthio group, an arylthio group, a halogen atom, a carboxy group, a carboxylate group, a sulfo group, a sulfonate group, an alkyloxycarbonyl group, an aryloxycarbonyl group, and a group obtained by combining these groups.

The preferred aspect of the aryl group in $R^{15}$ and $R^{16}$ is the same as the preferred aspect of the aryl group in $R^{11}$ to $R^{14}$ and $R^{17}$ to $R^{20}$.

Preferred aspects of the alkyl group in $R^2$, $R^3$, $R^6$, $R^7$, $R^8$, $R^9$, and $R^0$ are the same as the preferred aspects of the alkyl group in $R^{11}$ to $R^{14}$ and $R^{17}$ to $R^{20}$.

The preferred aspect of the aryl group in $R^0$ is the same as the preferred aspect of the aryl group in $R^{11}$ to $R^{14}$ and $R^{17}$ to $R^{20}$.

It is preferable that $R^2$ and $R^3$ be linked to each other to form a ring.

In a case where $R^2$ and $R^3$ are linked to each other to form a ring, a 5-membered ring or a 6-membered ring is preferable, and a 5-membered ring is particularly preferable.

$Y^1$ and $Y^2$ each independently represent an oxygen atom, a sulfur atom, $-NR^0-$, or a dialkylmethylene group, preferably $-NR^0-$ or a dialkylmethylene group, and more preferably a dialkylmethylene group.

$R^0$ represents a hydrogen atom, an alkyl group, or an aryl group and preferably an alkyl group.

The alkyl group represented by $R^4$ or $R^5$ is preferably an alkyl group having 1 to 30 carbon atoms, more preferably an alkyl group having 1 to 15 carbon atoms, and still more preferably an alkyl group having 1 to 10 carbon atoms. The alkyl group represented by $R^4$ and $R^5$ may be linear or branched, or may have a ring structure.

Specific examples of the alkyl group include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a hexadecyl group, an octadecyl group, an eicosyl group, an isopropyl group, an isobutyl group, an s-butyl group, a t-butyl group, an isopentyl group, a neopentyl group, a 1-methylbutyl group, an isohexyl group, a 2-ethylhexyl group, a 2-methylhexyl group, a cyclohexyl group, a cyclopentyl group, and a 2-norbornyl group.

The alkyl group in $R^4$ or $R^5$ may have a substituent. Examples of the substituent include an aryl group, an alkoxy group, an aryloxy group, an amino group, an alkylthio group, an arylthio group, a halogen atom, a carboxy group, a carboxylate group, a sulfo group, a sulfonate group, an alkyloxycarbonyl group, an aryloxycarbonyl group, and a group

obtained by combining these groups.

Among the alkyl groups in $R^4$ or $R^5$, a methyl group, an ethyl group, a propyl group, or a butyl group is preferable.

As the substituent on the alkyl group, a methoxy group, a phenoxy group, a phenyl group, or a naphthyl group is preferable.

From the viewpoint of solubility and developability, the alkyl group in $R^4$ or $R^5$ is particularly preferably an ethyl group or a propyl group having a substituent (for example, a substituent of Dye-12 described later).

In addition, $R^4$ and $R^5$ are preferably the same group.

$R^6$ to $R^9$ each independently represent a hydrogen atom or an alkyl group, and preferably each independently represent a hydrogen atom.

$Ar^1$ and $Ar^2$ each independently represent a group forming a benzene ring or a naphthalene ring. The benzene ring and the naphthalene ring may have a substituent. Examples of the substituent include an alkyl group, an alkoxy group, an aryloxy group, an amino group, an alkylthio group, an arylthio group, a halogen atom, a carboxy group, a carboxylate group, a sulfo group, a sulfonate group, an alkyloxycarbonyl group, an aryloxycarbonyl group, an acyloxy group, a phosphonic acid group, groups obtained by combining these, and the like. As the substituent, an alkyl group or an alkoxy group is preferable.

Further, from the viewpoints of increasing the maximum absorption wavelength of the compound represented by Formula (1) and improving the color formability and the printing durability of the lithographic printing plate, $Ar^1$ and $Ar^2$ each independently represent preferably a naphthalene ring or a group that forms a benzene ring containing an alkyl group or an alkoxy group as a substituent, more preferably a naphthalene ring or a group that forms a benzene ring containing an alkoxy group as a substituent, and particularly preferably a group that forms a benzene ring containing a methoxy group as a substituent.

In Formula (1), it is preferable that $Ar^1$ or $Ar^2$ is a group forming a group represented by the following formula.

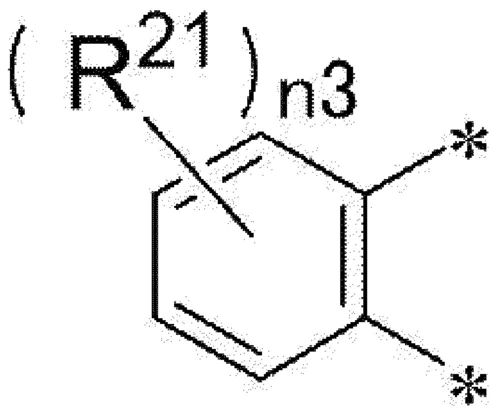

In the formulae, $R^{21}$'s each independently represent an alkyl group having 1 to 12 carbon atoms, an alkoxy group, an aryloxy group, an amino group, an alkoxythio group, or a halogen atom, n3 represents an integer of 1 to 4, a plurality of $R^{21}$'s may be bonded to each other to form a ring, and * represents a bonding site.

Za represents a counterion for neutralizing the electric charge. The counterion may be one counterion or two or more counterions depending on the neutralizing valence, and may have two or more counterions of the same or different kinds. Here, in a case where the compound represented by Formula (1) has a corresponding ionic substituent in the structure and charge neutralization is not required, Za is unnecessary. In a case of indicating an anionic species, examples thereof include a sulfonate ion, a carboxylate ion, a tetrafluoroborate ion, a hexafluorophosphate ion, a p-toluenesulfonate ion, and a perchlorate ion, and a tetrafluoroborate ion, a hexafluorophosphate ion, or a p-toluenesulfonate ion is preferable. In a case of indicating a cationic species, examples thereof include an alkali metal ion, an alkaline earth metal ion, an ammonium ion, a pyridinium ion, and a sulfonium ion. Among these, a sodium ion, a potassium ion, an ammonium ion, a pyridinium ion, or a sulfonium ion is preferable, and a sodium ion, a potassium ion, or an ammonium ion is more preferable.

$R^2$ to $R^9$, $R^0$, $Ar^1$, $Ar^2$, $Y^1$ and $Y^2$ may have an anion structure or a cation structure, and in a case where all $R^2$ to $R^9$, $R^0$, $Ar^1$, $Ar^2$, $Y^1$ and $Y^2$ represent a charge-neutral group, Za represents a monovalent counter anion. However, for example, in a case where $R^2$ to $R^9$, $R^0$, $Ar^1$, $Ar^2$, $Y^1$, and $Y^2$ have two or more anion structures, Za may represent a counter cation.

From the viewpoint of visibility, printing durability, on-press developability, and on-press developability after aging, the compound represented by Formula (1) is preferably a compound represented by Formula (5) or Formula (6), more preferably a compound represented by Formula (7) or Formula (8), and particularly preferably a compound represented by Formula (9).

In addition, from the viewpoint of visibility, printing durability, on-press developability, and on-press developability after aging, the compound represented by Formula (1) is preferably a compound represented by Formula (1A) described later.

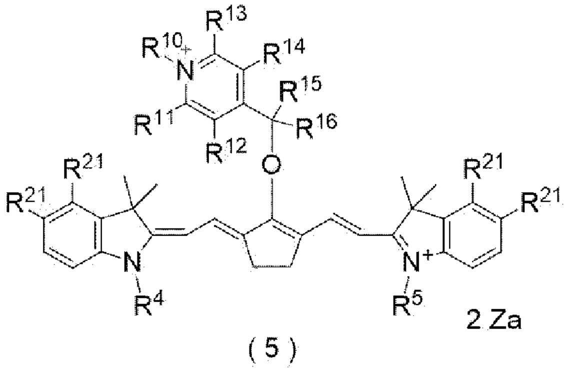

(5)

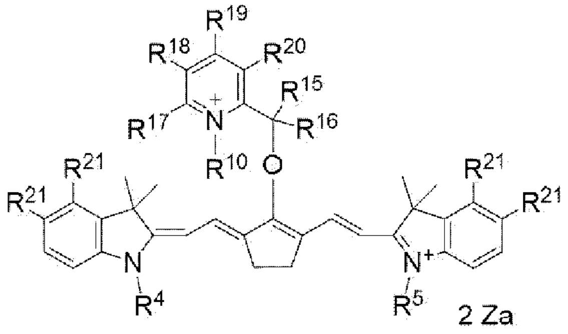

(6)

In Formulae (5) and (6), $R^4$ and $R^5$ each independently represent an alkyl group, $R^{10}$ represents a monovalent organic group having an aryl group, $R^{11}$ to $R^{14}$ and $R^{17}$ to $R^{20}$ each independently represent a hydrogen atom, an alkyl group, an aryl group, a hydroxy group, an alkoxy group, or a halogen atom, $R^{15}$ and $R^{16}$ each independently represent a hydrogen atom, an alkyl group, or an aryl group, at least two of $R^{11}$ to $R^{20}$ may be bonded to each other to form a ring structure, $R^{21}$'s each independently represent an alkyl group having 1 to 12 carbon atoms, an alkoxy group, an aryloxy group, an amino group, an alkoxythio group, or a halogen atom, $R^{21}$'s may be linked to each other to form a ring, and Za represents a counterion that neutralizes a charge.

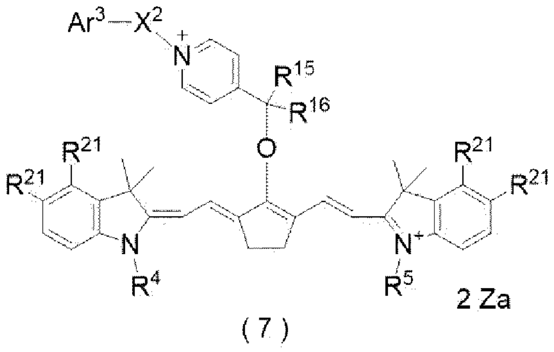

(7)

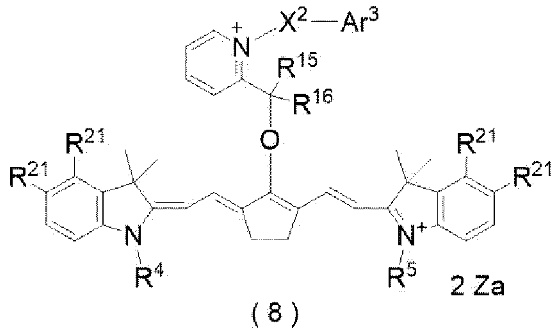

(8)

In Formula (7) and Formula (8), $X^2$ represents a single bond or an alkylene group, $Ar^3$ represents an aryl group, $R^4$ and $R^5$ each independently represent an alkyl group, $R^{15}$ and $R^{16}$ each independently represent a hydrogen atom, an alkyl group, or an aryl group, $R^{15}$ and $R^{16}$ may be bonded to each other to form a ring structure, $R^{21}$'s each independently represent an alkyl group having 1 to 12 carbon atoms, an alkoxy group, an aryloxy group, an amino group, an alkoxythio group, or a halogen atom, $R^{21}$'s may be linked to each other to form a ring, and Za represents a counterion that neutralizes a charge.

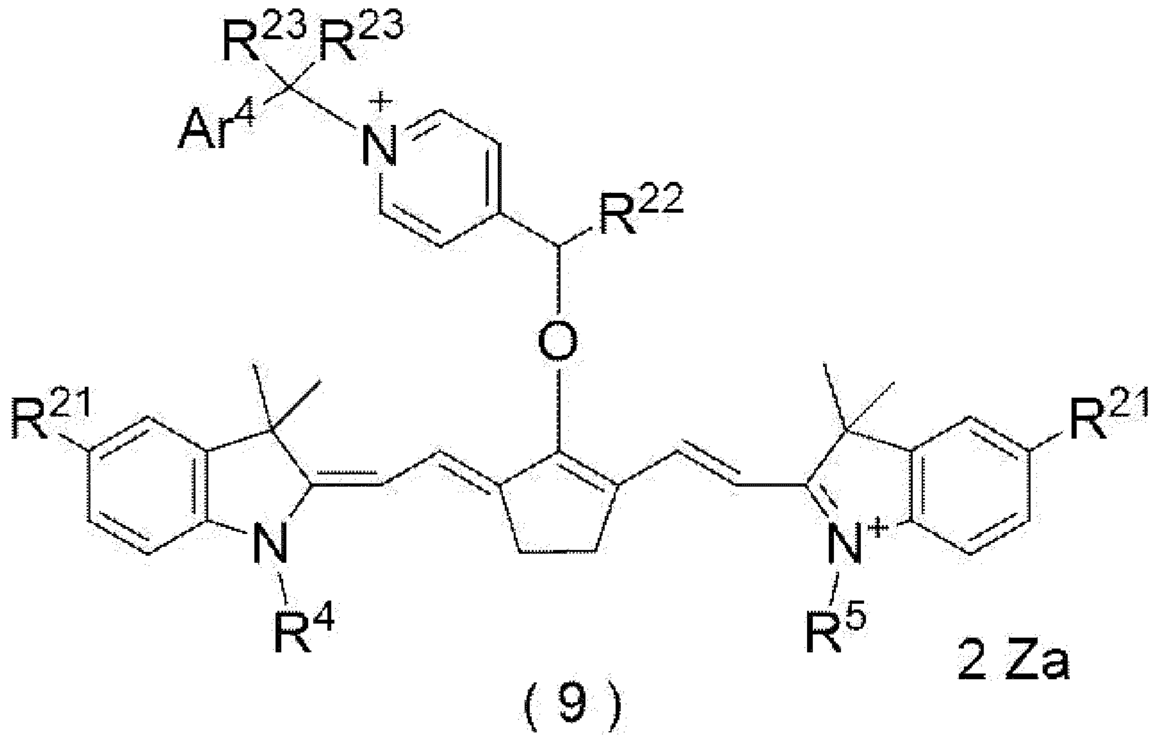

(9)

In Formula (9), $Ar^4$ represents an aryl group, $R^4$ and $R^5$ each independently represent an alkyl group, $R^{21}$'s each independently represent an alkyl group having 1 to 12 carbon atoms, an alkoxy group, an aryloxy group, an amino group, an alkoxythio group, or a halogen atom, $R^{22}$ represents a hydrogen atom, an alkyl group, or an aryl group, $R^{23}$'s each independently represent a hydrogen atom or an alkyl group, and Za represents a counterion that neutralizes charge.
The preferred aspects of $R^4$, $R^5$, $R^{11}$ to $R^{20}$, and Za in Formulae (5) to (9) are respectively the same as the preferred aspects of $R^4$, $R^5$, $R^{11}$ to $R^{20}$, and Za in Formulae (1) to (3).
The preferred aspects of $R^{21}$ in Formulae (5) to (9) are respectively the same as the preferred aspects of $R^{21}$ described above.
Preferred aspects of $X^2$ and $Ar^3$ in Formula (7) and Formula (8) are respectively the same as the preferred aspects of $X^2$ and $Ar^3$ in Formula (4).
$Ar^4$ in Formula (9) is preferably an aryl group having 6 to 30 carbon atoms, more preferably an aryl group having 6 to 20 carbon atoms, and still more preferably an aryl group having 6 to 12 carbon atoms.
The above-described aryl group may have a substituent. Examples of the substituent include an alkyl group, an alkoxy group, an aryloxy group, and a group obtained by combining these groups.
Among these, an alkyl group is preferable as the substituent.
Specific examples of $Ar^4$ in Formula (9) include a phenyl group, a naphthyl group, a p-tolyl group, a p-ethylphenyl group, a p-t-butylphenyl group, a p-fluorophenyl group, and a p-methoxyphenyl group.
Among these, as $Ar^4$, from the viewpoint of visibility, printing durability, on-press developability, and on-press developability after aging, a phenyl group, a p-methoxyphenyl group, a p-tBu phenyl group, or a naphthyl group is preferable, a phenyl group, a p-methoxyphenyl group, or a p-t-butylphenyl group is more preferable, and a p-t-butylphenyl group is particularly preferable.
$R^{22}$ in Formula (9) is preferably an alkyl group, more preferably an alkyl group having 1 to 8 carbon atoms, and may be divided by an oxygen atom or the like.
From the viewpoints of visibility, printing durability, and synthetic suitability, the number of carbon atoms in the alkyl group is more preferably 1 to 6, still more preferably 3 to 5, and particularly preferably 3 or 4.
$R^{23}$ in Formula (9) is preferably a hydrogen atom from the viewpoint of manufacturing suitability.
Specific preferred examples of the compound represented by Formula (1) are shown below, but the present invention is not limited thereto. In the following structural formulae, Me represents a methyl group, and $Ts^{O-}$ represents a tosylate anion.

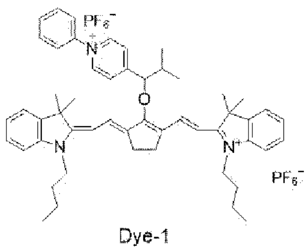
Dye-1

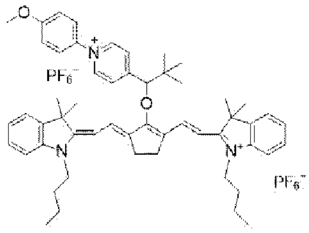
Dye-2

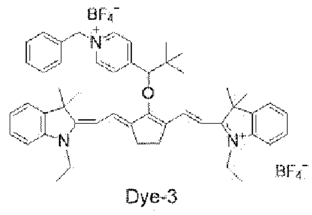
Dye-3

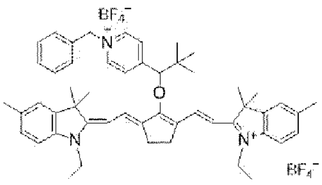
Dye-4

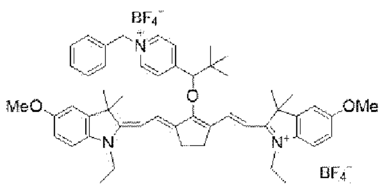
Dye-5

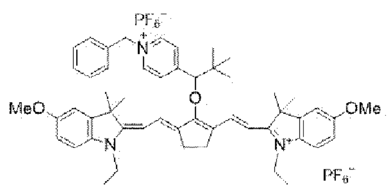
Dye-6

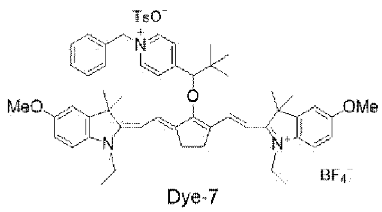
Dye-7

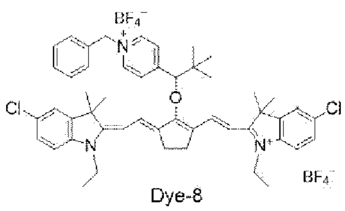
Dye-8

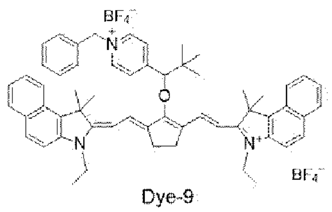
Dye-9

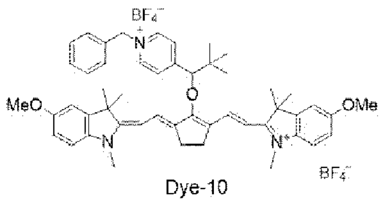
Dye-10

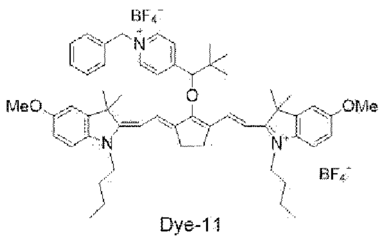
Dye-11

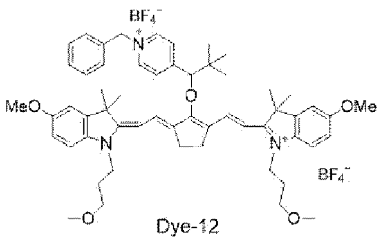
Dye-12

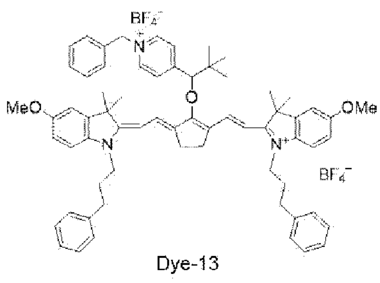
Dye-13

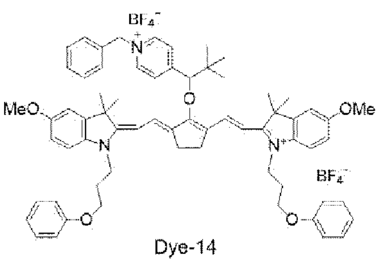
Dye-14

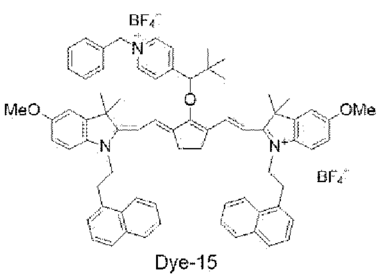
Dye-15

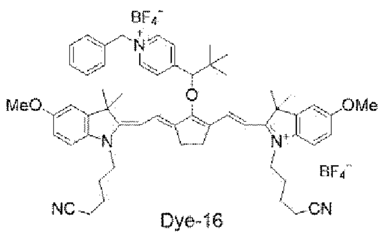
Dye-16

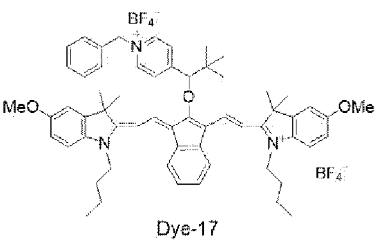
Dye-17

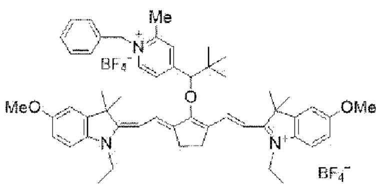
Dye-18

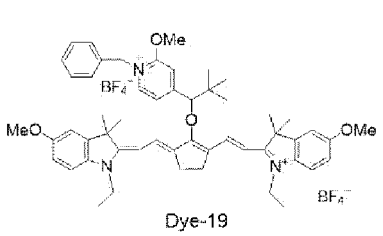
Dye-19

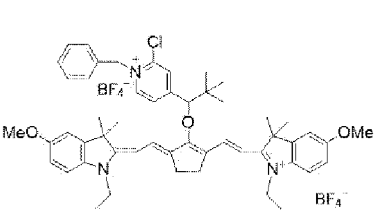
Dye-20

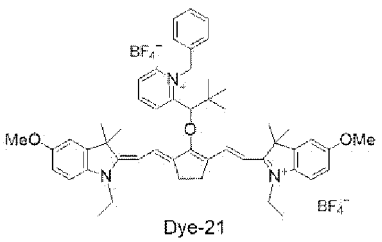
Dye-21

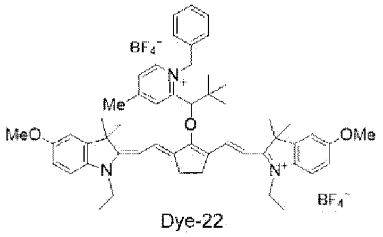
Dye-22

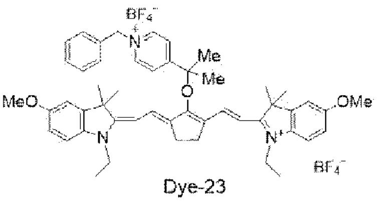
Dye-23

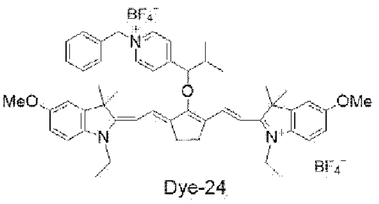
Dye-24

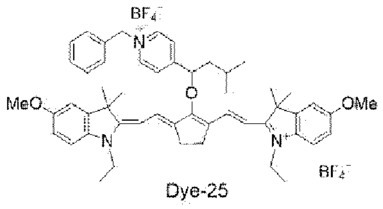
Dye-25

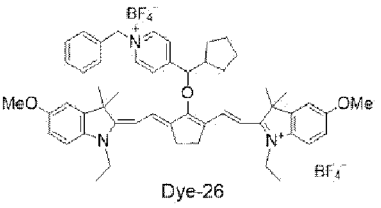
Dye-26

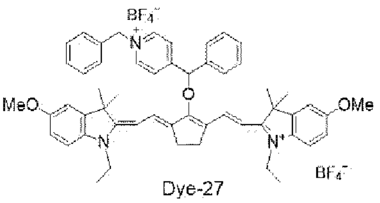
Dye-27

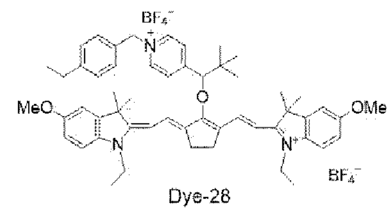
Dye-28

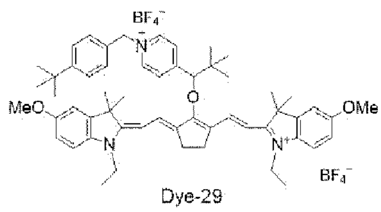
Dye-29

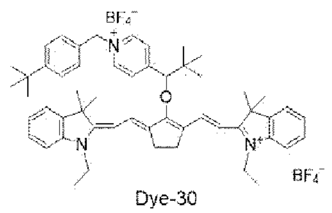
Dye-30

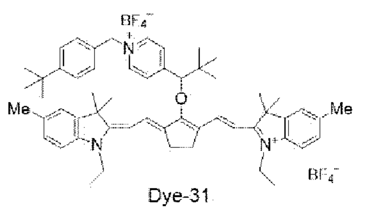
Dye-31

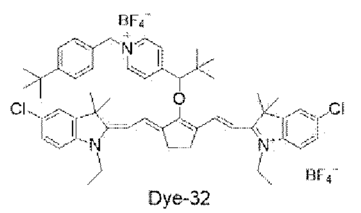
Dye-32

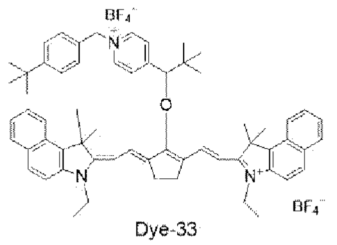
Dye-33

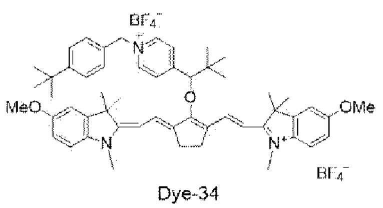
Dye-34

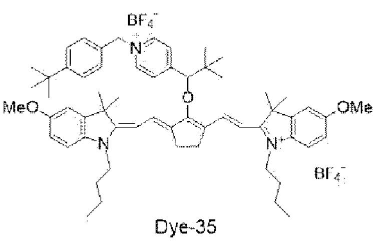
Dye-35

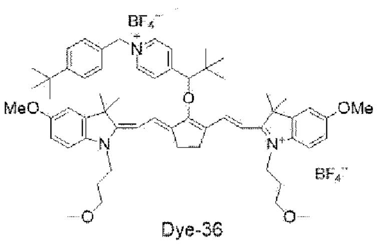
Dye-36

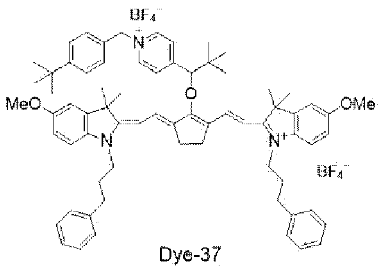
Dye-37

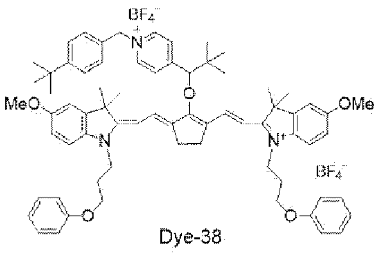
Dye-38

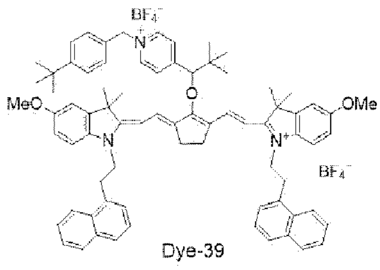
Dye-39

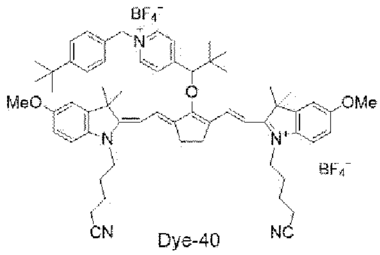
Dye-40

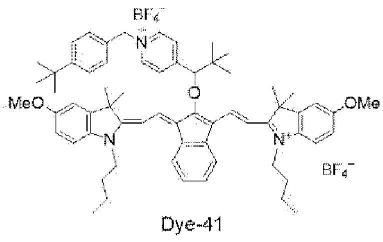
Dye-41

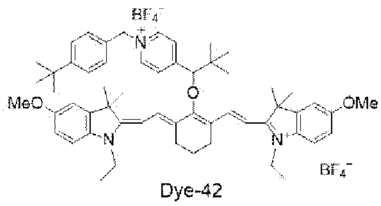
Dye-42

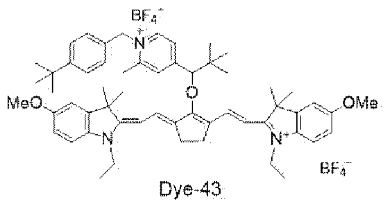
Dye-43

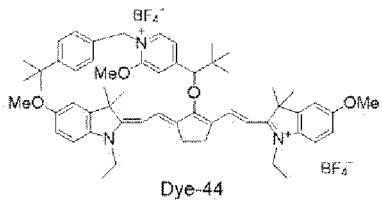
Dye-44

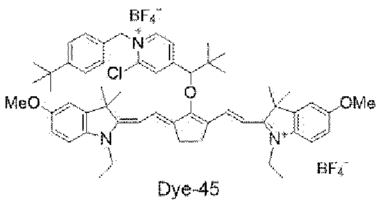
Dye-45

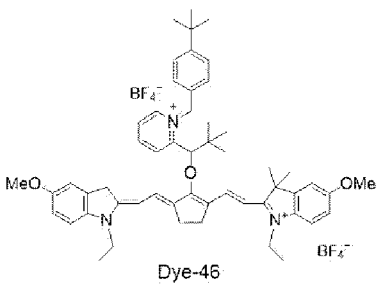
Dye-46

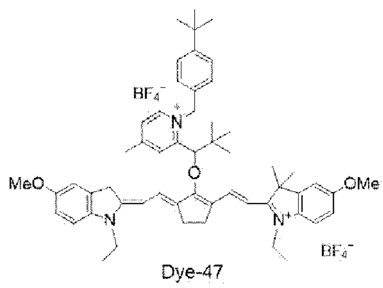
Dye-47

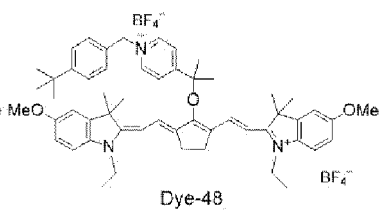
Dye-48

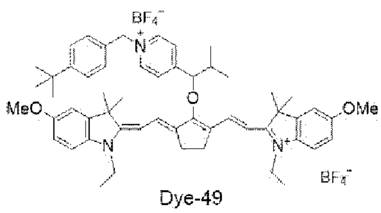
Dye-49

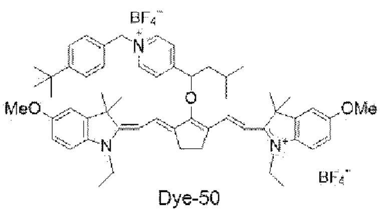
Dye-50

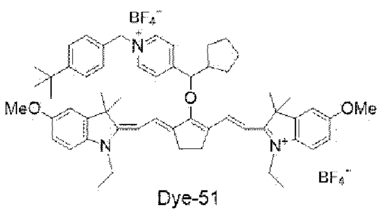
Dye-51

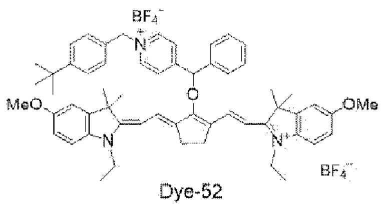
Dye-52

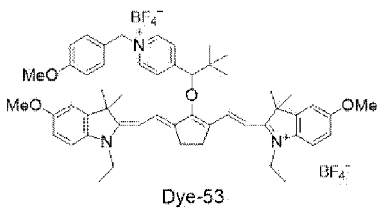
Dye-53

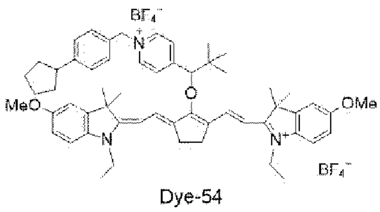
Dye-54

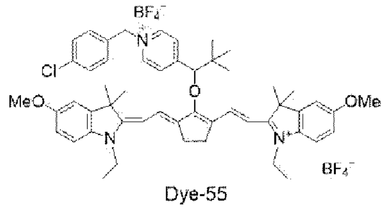

Dye-55

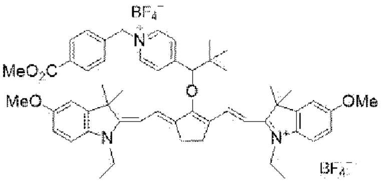

Dye-56

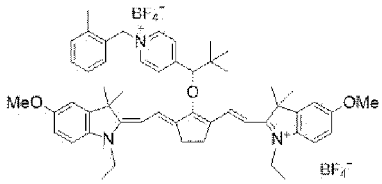

Dye-57

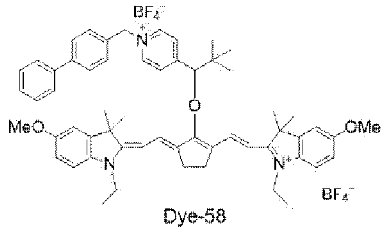

Dye-58

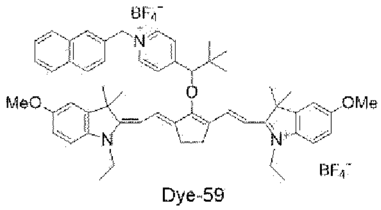

Dye-59

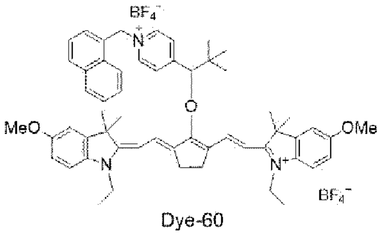

Dye-60

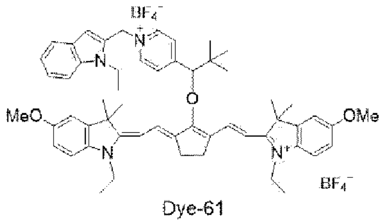

Dye-61

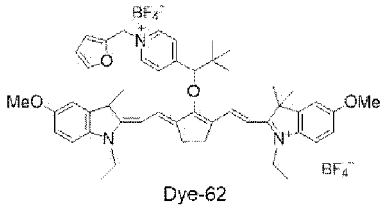

Dye-62

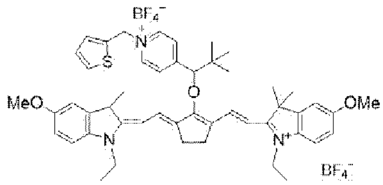

Dye-63

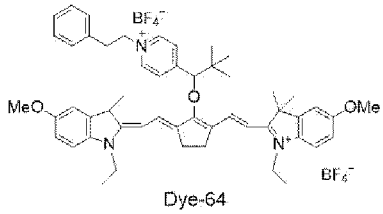

Dye-64

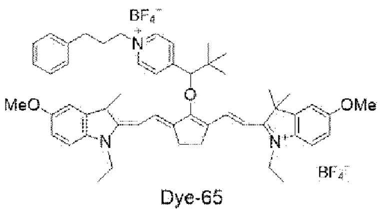

Dye-65

The compound represented by Formula (1) may be used alone or in combination of two or more kinds thereof.

The content of the compound represented by Formula (1) in the image-recording layer is preferably in a range of 0.1% by mass to 10.0% by mass and more preferably in a range of 0.5% by mass to 5.0% by mass with respect to the total mass of the image-recording layer.

Electron-accepting polymerization initiator

The image-recording layer preferably contains an electron-accepting polymerization initiator.

The electron-accepting polymerization initiator is a compound that generates a polymerization initiation species such as a radical by accepting one electron through intermolecular electron transfer in a case where electrons of the infrared absorber are excited by exposure to infrared in one aspect.

In addition, the electron-accepting polymerization initiator used in the present disclosure is a compound that generates a polymerization initiation species such as a radical or a cation by either or both of light energy and heat energy, and can be appropriately selected from known thermal polymerization initiators, compounds having a bond that requires low bond dissociation energy, photopolymerization initiators, and the like.

The electron-accepting polymerization initiator is preferably a radical polymerization initiator and more preferably an onium salt compound.

In addition, as the electron-accepting polymerization initiator, an infrared-ray-sensitive polymerization initiator is preferable.

Examples of the electron-accepting radical polymerization initiator include (a) organic halide, (b) carbonyl compound, (c) azo compound, (d) organic peroxide, (e) metallocene compound, (f) azide compound, (g) hexaarylbiimidazole compound, (i) disulfone compound, (j) oxime ester compound, and (k) onium salt compound.

As (a) organic halide, for example, the compounds described in paragraphs "0022" and "0023" of JP2008-195018A are preferable.

As (b) carbonyl compound, for example, the compounds described in paragraph "0024" of JP2008-195018A are preferable.

As (c) azo compound, for example, the azo compounds described in JP1996-108621A (JP-H8-108621A) and the like can be used.

As (d) organic peroxide, for example, the compounds described in paragraph "0025" of JP2008-195018A are preferable.

As (e) metallocene compound, for example, the compounds described in paragraph "0026" of JP2008-195018A are preferable.

Examples of (f) azide compound can include compounds such as 2,6-bis(4-azidobenzylidene)-4-methylcyclohexanone.

As (g) hexaarylbiimidazole compound, for example, the compounds described in paragraph "0027" of JP2008-195018A are preferable.

Examples of (i) disulfone compound include the compounds described in JP1986-166544A (JP-S61-166544A) and JP2002-328465A.

As (j) oxime ester compound, for example, the compounds described in paragraphs "0028" to "0030" of JP2008-195018A are preferable.

Among the above electron-accepting polymerization initiators, from the viewpoint of curing properties, an oxime ester compound and an onium salt compound are preferable. Particularly, from the viewpoint of printing durability, an iodonium salt compound, a sulfonium salt compound, or an azinium salt compound is preferable, an iodonium salt compound or a sulfonium salt compound is more preferable, and an iodonium salt compound is particularly preferable.

Specific examples of these compounds include the compounds described in paragraphs "0043" to "0046" of WO2020/262685A.

As the electron-accepting polymerization initiator, a compound A represented by Formula (Ia) and one or more compounds B selected from the group consisting of a compound represented by Formula (Ib) and a compound represented by Formula (Ic).

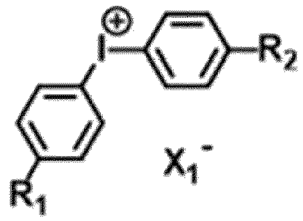

Formula (Ia)

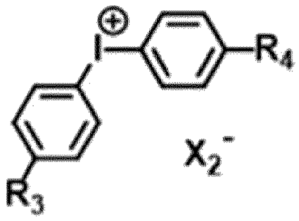

Formula (Ib)

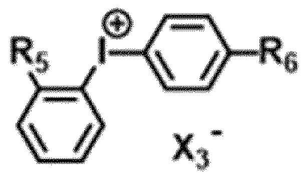

Formula (Ic)

In Formulae (Ia) to (Ic), $R_1$, $R_2$, $R_3$, $R_4$, $R_5$, and $R_6$ are each independently a substituted or unsubstituted alkyl group having 2 to 9 carbon atoms or a substituted or unsubstituted alkoxy group having 2 to 9 carbon atoms, at least one of $R_3$ or $R_4$ is different from $R_1$ or $R_2$, a difference between a total number of carbon atoms in $R_1$ and $R_2$ and a total number of carbon atoms in $R_3$ and $R_4$ is 0 to 4 (that is, 0, 1, 2, 3, or 4), a difference between the total number of carbon atoms in $R_1$ and $R_2$ and a total number of carbon atoms in $R_5$ and $R_6$ is 0 to 4, and $X_1$, $X_2$, and $X_3$ are the same or different anions.

$R_1$, $R_2$, $R_3$, $R_4$, $R_5$, and $R_6$ are each independently preferably a substituted or unsubstituted alkyl group having 2 to 9 carbon atoms or a substituted or unsubstituted alkoxy group having 2 to 9 carbon atoms, more preferably a substituted or unsubstituted alkyl group having 3 to 6 carbon atoms or a substituted or unsubstituted alkoxy group having 3 to 6 carbon atoms, and still more preferably a substituted or unsubstituted alkyl group having 3 to 6 carbon atoms. The alkyl group and the alkoxy group all may be linear or branched, but are preferably branched.

Examples of the substituted or unsubstituted alkyl group include an ethyl group, an n-propyl group, an iso-propyl group, an n-butyl group, an iso-butyl group, a sec-butyl group, a t-butyl group, an n-pentyl group, a t-pentyl group, a sec-pentyl group, a neopentyl group, a n-hexyl group, an iso-hexyl group, a sec-hexyl group, a t-hexyl group, an n-heptyl group, an n-octyl group, an iso-octyl group, a 2-ethyl hexyl group, and an n-nonyl group.

Examples of the substituted or unsubstituted alkoxy group include an ethoxy group, an n-propoxy group, an iso-propoxy group, a t-butoxy group, an n-butoxy group, and an n-octyloxy group.

As $X_1$, $X_2$, and $X_3$, $ClO_4^-$, $PF_6^-$, $BF_4^-$, $SbF_6^-$, $CH_3SO_3^-$, $CF_3SO_3^-$, $C_6H_5SO_3^-$, $CH_3C_6H_4SO_3^-$, $HOC_6H_4SO_3^-$, $ClC_6H_4SO_3^-$, and a borate anion represented by the following Formula (Id) are preferable.

$$B^-(R^1)(R^2)(R^3)(R^4) \qquad \text{Formula (Id)}$$

In Formula (Id), $R^1$, $R^2$, $R^3$, and $R^4$ each independently represent a substituted or unsubstituted alkyl group, a

substituted or unsubstituted aryl group (including a halogen-substituted aryl group), a substituted or unsubstituted alkenyl group, a substituted or unsubstituted alkynyl group, a substituted or unsubstituted cycloalkyl group, or a substituted or unsubstituted heterocyclic group. Two or more of $R^1$, $R^2$, $R^3$, and $R^4$ may be linked to each other to form a substituted or unsubstituted heterocyclic ring containing a boron atom. The heterocyclic ring to be obtained has a maximum of 7 carbon atoms, nitrogen atoms, oxygen atoms, or nitrogen atoms.

Examples of the substituent in $R^1$, $R^2$, $R^3$, and $R^4$ include a chlorine atom, a fluorine atom, a nitro group, an alkyl group, an alkoxy group, and an acetoxy group.

All of $R^1$, $R^2$, $R^3$, and $R^4$ are preferably a substituted or unsubstituted aryl group, and more preferably an unsubstituted phenyl group.

It is preferable that at least one of $X_1$, $X_2$, or $X_3$ is a tetraaryl borate anion including the same or different aryl groups, it is more preferable that one or more of $X_1$, $X_2$, and $X_3$ are tetraphenyl borate anions, and it is still more preferable that each of $X_1$, $X_2$, and $X_3$ is a tetraphenyl borate anion.

Examples of a preferable aspect include an aspect in which the compound B includes a compound represented by Formula (Ic), $R_1$ is the same as $R_5$, and $R_2$ is the same as $R_6$. In particular, it is preferable that $R_1$ is the same as $R_2$, and for example, both $R_1$ and $R_2$ are an isopropyl group, an iso-butyl group, or a t-butyl group.

In addition, examples of another preferable aspect include an aspect in which the compound B includes a compound represented by Formula (Ib), $R_1$ is the same as $R_2$, and $R_3$ is the same as $R_4$. In this case, it is preferable that both $R_1$ and $R_2$ are an iso-propyl group, an iso-butyl group, or a t-butyl group. In addition, the difference in the number of carbon atoms between $R_1$ and $R_3$ is preferably 1 or 2.

The compound B may be a mixture of a compound represented by Formula (Ib) and a compound represented by Formula (Ic).

The compounds represented by Formulae (Ia) to (Ic) may be obtained from Sigma-Aldrich Co., LLC or the like, or may be synthesized by a known synthesis method using easily available starting materials.

The molar ratio between the compound A and the compound B is preferably 10:90 to 90: 10, more preferably 20:80 to 80:20, and still more preferably 30:70 to 70:30.

From the viewpoints of developability and printing durability of the lithographic printing plate to be obtained, the electron-accepting polymerization initiator may include a compound represented by Formula (II) described in paragraphs "0186" to "0197" of WO2022/019217A.

From the viewpoint of improving sensitivity and making it difficult for plate missing to occur, the lowest unoccupied molecular orbital (LUMO) of the electron-accepting polymerization initiator is preferably -3.00 eV or less, and more preferably -3.02 eV or less.

The lower limit of LUMO is preferably -3.80 eV or more and more preferably -3.60 eV or more.

One electron-accepting polymerization initiator may be used alone, or two or more electron-accepting polymerization initiators may be used in combination.

The content of the electron-accepting polymerization initiator with respect to the total mass of the image-recording layer is preferably 0.1% by mass to 50% by mass, more preferably 0.5% by mass to 30% by mass, and particularly preferably 0.8% by mass to 20% by mass.

Borate compound

The image-recording layer preferably contains a borate compound. It is presumed that the borate compound, preferably the tetraaryl borate compound, and the portion of the group represented by Formula (2) or Formula (3) in the compound represented by Formula (1) interact with each other, and the decomposition of the compound represented by Formula (1) is promoted, and the visibility, the printing durability, the on-press developability, and the on-press developability after aging are more excellent.

The borate compound is used as an electron-donating polymerization initiator in the image-recording layer.

The borate compound is a compound that generates a polymerization initiation species such as a radical or a cation by light, heat, or energy of both.

The borate compound is preferably a tetraaryl borate compound or a monoalkyltriaryl borate compound, from the viewpoint of compound stability, more preferably a tetraaryl borate compound, and particularly preferably a tetraphenyl borate compound.

The countercation that the borate compound has is not particularly limited, but is preferably an alkali metal ion or a tetraalkyl ammonium ion and more preferably a sodium ion, a potassium ion, or a tetrabutylammonium ion.

From the viewpoint of visibility, printing durability, on-press developability, and on-press developability after aging, the borate compound is preferably a compound represented by Formula (B1).

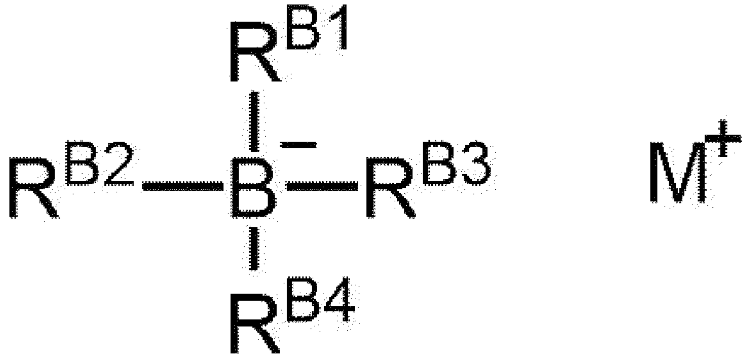

(B1)

In Formula (B 1), $R^{B1}$ to $R^{B4}$ each independently represent an unsubstituted or substituted alkyl group, an unsubstituted or substituted aryl group, an unsubstituted or substituted alkenyl group, or an unsubstituted or substituted alkynyl group, and $R^{B1}$ to $R^{B4}$ may each independently have a ring structure. However, at least one of $R^{B1}$ to $R^{B4}$ is different from the others. $M^+$ represents a cation.

Examples of the alkyl group represented by $R^{B1}$ to $R^{B4}$, which include those with 1 to 20 carbon atoms, include a methyl group, an ethyl group, a propyl group, a butyl group, a pentyl group, a hexyl group, a heptyl group, an octyl group, a nonyl group, a decyl group, an undecyl group, a dodecyl group, a tridecyl group, a hexadecyl group, an octadecyl group, an eicosyl group, an isopropyl group, an isobutyl group, an s-butyl group, a t-butyl group, an isopentyl group, a neopentyl group, a 1-methylbutyl group, an isohexyl group, a 2-ethylhexyl group, a 2-methylhexyl group, a cyclohexyl group, a cyclopentyl group, and a 2-norbornyl group. The alkyl group described above may be linear or branched, or may have a ring structure.

In addition, the alkyl group represented by $R^{B1}$ to $R^{B4}$ may have a substituent. Examples of the substituent of the alkyl group include a halogen atom, an alkyl group, an aryl group, an alkenyl group, an alkoxy group, an ester group, a carbonyl group, a sulfonyl group, an amino group, an amide group, and a group obtained by combining these groups.

As the alkyl group represented by each of $R^{B1}$ to $R^{B4}$, an unsubstituted linear or branched alkyl group having 1 to 12 carbon atoms is preferable, an unsubstituted linear or branched alkyl group having 1 to 10 carbon atoms is more preferable, and an unsubstituted linear or branched alkyl group having 1 to 6 carbon atoms is still more preferable. Among these, from the viewpoint of suppressing deterioration in development over time, an unsubstituted alkyl group having a branch is particularly preferable.

Examples of the aryl group represented by $R^{B1}$ to $R^{B4}$ include an aryl group having 6 to 20 carbon atoms, and examples thereof include a phenyl group, a naphthyl group, an anthryl group, a phenanthryl group, an indenyl group, an acenaphthenyl group, and a fluorenyl group.

Examples of the substituent of the aryl group include the substituent of the above-described alkyl group.

As the aryl group represented by each of $R^{B1}$ to $R^{B4}$, an unsubstituted or substituted phenyl group is preferable.

Examples of the alkenyl group represented by $R^{B1}$ to $R^{B4}$ include an alkenyl group having 2 to 20 carbon atoms, and examples thereof include a vinyl group, a 1-propenyl group, a 1-butenyl group, a cinnamyl group, and a 2-chloro-1-etenyl group.

The alkenyl group represented by $R^{B1}$ to $R^{B4}$ may be linear, branched, or have a ring structure.

Examples of the substituent of the alkenyl group include the substituent of the above-described alkyl group.

As the alkenyl group represented by each of $R^{B1}$ to $R^{B4}$, an unsubstituted alkenyl group having 2 to 20 carbon atoms is preferable.

Examples of the alkynyl group represented by $R^{B1}$ to $R^{B4}$ include an alkynyl group having 2 to 20 carbon atoms, and examples thereof include an ethynyl group, a 1-propynyl group, a 1-butynyl group, a trimethylsilyl ethynyl group, and a phenyl ethynyl group.

The alkynyl group may be linear or branched.

Among the above-described functional groups, $R^{B1}$ to $R^{B4}$ are each independently preferably a substituted or unsubstituted aryl group.

Since $R^{B1}$ to $R^{B4}$ are each independently a substituted or unsubstituted aryl group, the HOMO potential of the borate compound is lowered, and the film stability of the image-recording layer is improved. In this manner, the life of the lithographic printing plate can be extended.

In the present disclosure, at least one of $R^{B1}$, ..., or $R^{B4}$ is different from the others. Among these, it is preferable that $R^{B1}$ to $R^{B3}$ are the same and $R^{B4}$ is different from $R^{B1}$ to $R^{B3}$ in $R^{B1}$ to $R^{B4}$. In this manner, a high-purity borate compound can be obtained. In addition, the generation of radicals can be suppressed, and side reactions are less likely to occur.

In the above case, it is more preferable that $R^{B1}$ to $R^{B3}$ are each a phenyl group.

In addition, it is also preferable that at least two of $R^{B1}$ to $R^{B4}$ are a phenyl group, and at least one of them is a substituted aryl group.

Furthermore, it is more preferable that $R^{B1}$ to $R^{B3}$ are a phenyl group, and $R^{B4}$ is an aryl group having a substituent (that is, a substituted aryl group).

In a case where $R^{B1}$ to $R^{B3}$ are each a phenyl group and $R^{B4}$ is an aryl group having a substituent, the total number of carbon atoms and oxygen atoms in the substituent of the aryl group is preferably 2 or more and more preferably 3 or more. The upper limit thereof is, for example, 8 or less.

The compound represented by Formula (B1) is preferably a compound represented by Formula (B2).

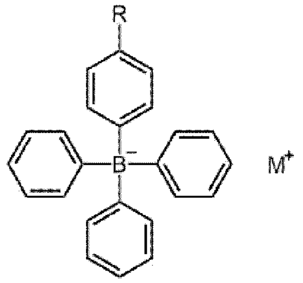

(B2)

In Formula (B2), R represents an alkyl group having 2 or more carbon atoms or an alkoxy group having a total number of carbon atoms and oxygen atoms of 2 or more. $M^+$ represents an iodonium cation or an infrared absorbing colorant cation.

In Formula (B2), the alkyl group represented by R preferably has 2 to 8 carbon atoms, more preferably has 2 to 6 carbon atoms, and still more preferably has 2 to 4 carbon atoms.

Examples of the above-described alkyl group include an ethyl group, a propyl group, an n-butyl group, a tert-butyl group, an isobutyl group, a sec-butyl group, a pentyl group, a hexyl group, a heptyl group, an octyl group, a neopentyl group, and an isopropyl group.

The alkyl group described above may be linear or branched, or may have a ring structure.

In Formula (B2), the alkoxy group of R preferably has a total number of carbon atoms and oxygen atoms of 2 to 4.

Examples of the above-described alkoxy group include a methoxy group, an n-propoxy group, an isopropoxy group, and an n-butoxy group.

The alkoxy group may be linear or branched.

In addition, in Formula (B1), the phenyl group represented by $R^{B1}$ to $R^{B3}$ is preferably a phenyl group substituted with an electron-withdrawing group.

Examples of the electron-withdrawing group include a halogen atom and a fluoroalkyl group. Among these, a fluorine atom, a chlorine atom, and a fluoroalkyl group having 1 to 3 carbon atoms are preferable.

In Formula (B1), the substituent contained in the aryl group represented by $R^{B4}$ is preferably an alkyl group, an aryl group, an alkenyl group, an alkoxy group, an ester group, a carbonyl group, or an amide group, more preferably an alkyl group, an alkenyl group, or an alkoxy group, and still more preferably an alkyl group or an alkoxy group.

In the present disclosure, the compound represented by Formula (B1) may be a compound represented by Formula (B3).

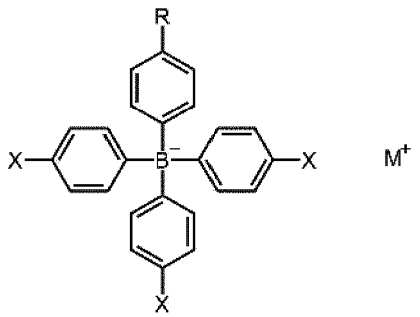

(B3)

In Formula (B3), R represents a group having a total number of carbon atoms and oxygen atoms of 2 or more, X represents a halogen atom, an alkyl group, or an alkoxy group, the sum of Hammett values of R and X is -0.09 to 0.09, and $M^+$ represents $Li^+$, $Na^+$, $K^+$, an iodonium cation, or an infrared absorbing colorant cation.

Examples of R in Formula (B3) include an alkyl group, an aryl group, an alkenyl group, an alkoxy group, an ester group, a carbonyl group, a sulfonyl group, an amide group, and a group obtained by combining these groups.

In Formula (B3), examples of the halogen atom represented by X include a fluorine atom, a chlorine atom, and a bromine atom.

In Formula (B3), the alkyl group and the alkoxy group represented by X are the same as the alkyl group and the alkoxy group in Formula (B2), and the same applies to the preferred aspects thereof.

Among these, R in Formula (B3) is preferably an alkyl group having 2 or more and 4 or less carbon atoms or an alkoxy group having 2 or more and 4 or less carbon atoms and oxygen atoms in total, and more preferably an alkyl group having 2 or more and 4 or less carbon atoms. In the above case, X is preferably a halogen atom. Here, the halogen atoms may be different halogen atoms from each other, but it is more preferable that all the halogen atoms are the same halogen atom.

In Formula (B3), the sum of Hammett σ values of the substituents (R and X) introduced into the aryl skeleton is preferably in a range of -0.2 to 0.2, and more preferably in a range of - 0.09 to 0.09. In a case where the HOMO potential is within the above-described range, the HOMO potential can be adjusted to a desired range, and the balance between the film stability and the printing durability of the image-recording layer is excellent.

In the present disclosure, for the calculation of the total Hammett σ value of the substituents introduced into the aryl skeleton, the numerical values described in the reference "Chemical Seminar 10 Hammett Side-Structure and Reactivity-" (written by Naoki Inamoto, published by Maruzen Co., Ltd., June 1983) were used.

$M^+$ in Formula (B1) represents a cation. That is, $M^+$ is a counter cation of a boron anion. In the present disclosure, $M^+$ is not particularly limited as long as it is a cation capable of neutralizing a boron anion, but from the viewpoint of suppressing contamination during development, at least one selected from the group consisting of an inorganic cation, an iodonium cation, and an infrared absorbing colorant cation is preferable. The counter cations may be used alone or in combination of two or more kinds thereof.

In Formula (B3), $M^+$ represents $Li^+$, $Na^+$, $K^+$, an iodonium cation, or an infrared absorbing colorant cation. In Formula (B2), $M^+$ represents an iodonium cation or an infrared absorbing colorant cation.

The combination of two or more kinds of counter cations is not particularly limited, but is preferably a combination of inorganic cations, a combination of iodonium cations, or a combination of infrared absorbing coloring agent cations, and more preferably a combination of iodonium cations or a combination of infrared absorbing colorant cations. In this manner, the suppressing property of the temporal development defect and the printing durability are further improved.

The iodonium cation may be a cationic moiety of an electron-accepting polymerization initiator described later, and the infrared absorbing colorant cation may be a cationic moiety of an infrared absorber described later.

In the image-recording layer, the cationic moiety of the electron-donating polymerization initiator and the cationic moiety of the infrared absorber can be bonded to the anionic moiety of the borate compound represented by Formula (B1) to form a salt.

Examples of the inorganic cation include a lithium cation, a sodium cation, a potassium cation, a calcium cation, and a magnesium cation. Among these, a sodium cation, a lithium cation, or a potassium cation is preferable, and a sodium cation is more preferable.

As the iodonium cation, a cationic moiety of an electron-accepting polymerization initiator described later can be used. Specific examples are shown in the following structural formulae. In the following structural formulae, Me represents a methyl group. The iodonium cation is not limited to the following specific examples.

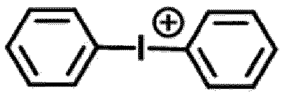
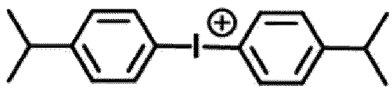
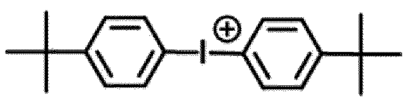
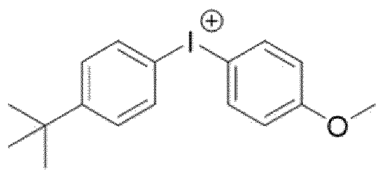
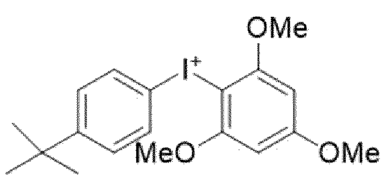
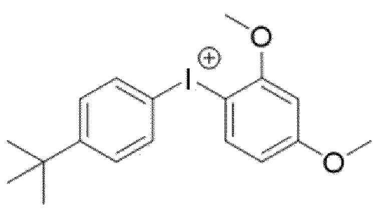

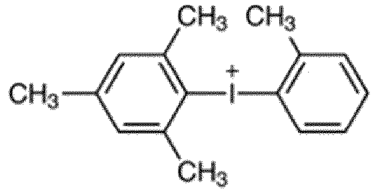

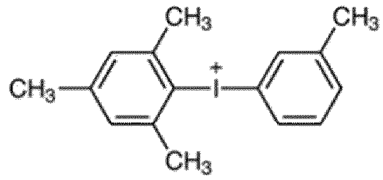

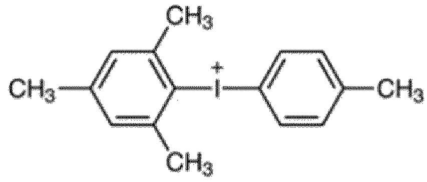

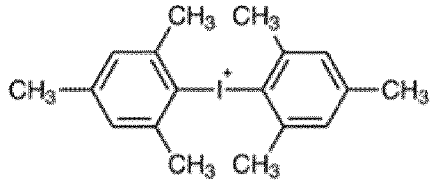

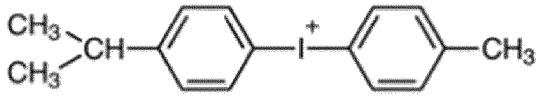

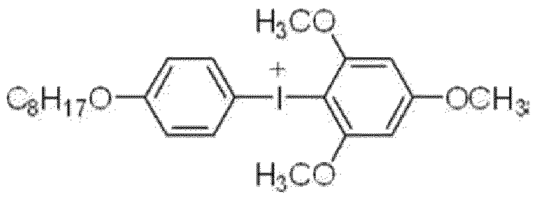

As the infrared-absorbing colorant cation, a cationic moiety of an infrared absorber described later can be used. Specific examples are shown in the following structural formulae. In the following structural formulae, Me represents a methyl group, and Bu represents a butyl group. The infrared absorbing colorant cation is not limited to the following specific examples.

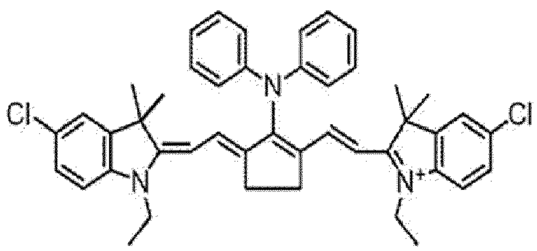

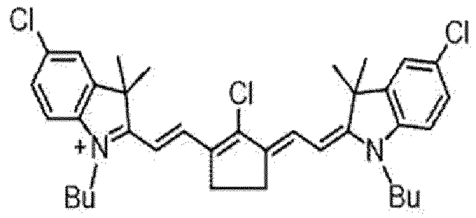

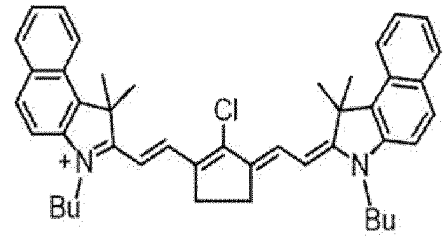

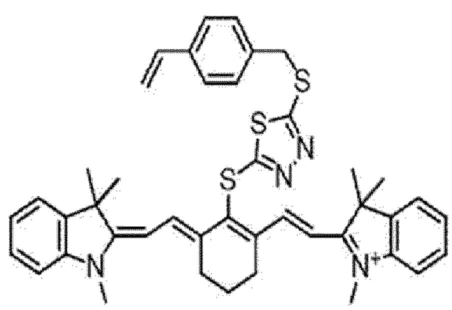

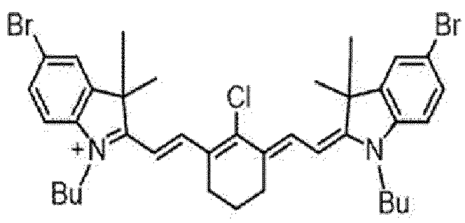

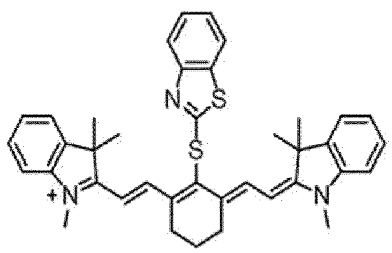

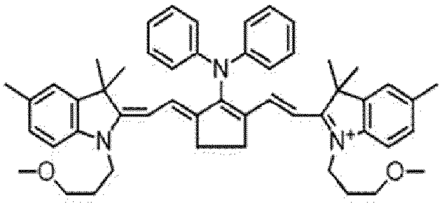

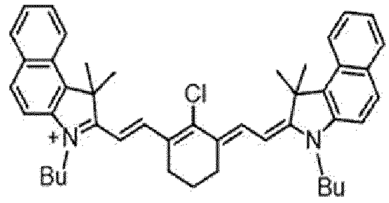

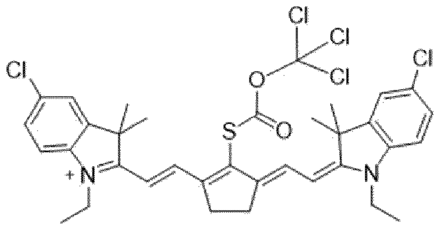

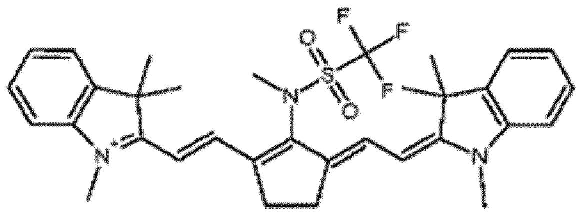

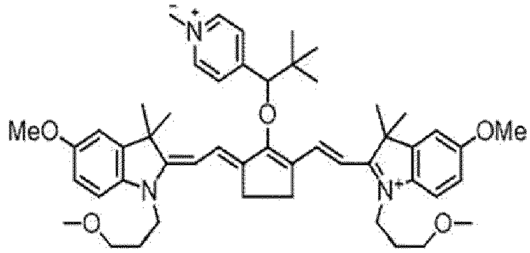

From the viewpoint of chemical resistance and printing durability, the highest occupied molecular orbital (HOMO) of the borate compound is preferably -6.0 eV or more, more preferably -5.95 eV or more, and still more preferably -5.93 eV or more.

The upper limit of HOMO is preferably -5.00 eV or less and more preferably -5.40 eV or less. The range of the HOMO is still more preferably -5.93 eV to -5.70 eV.

In the present disclosure, the highest occupied molecular orbital (HOMO) and the lowest unoccupied molecular orbital (LUMO) are calculated by the following methods.

First, the counteranion in the compound as a calculation object is ignored.

The structural optimization is carried out by DFT (B3LYP/6-31G(d)) using quantum chemical calculation software Gaussian 09.

The molecular orbital (MO) energy is calculated by DFT (B3LYP/6-31+G(d,p)/CPCM (solvent = methanol)) using the structure obtained by the structural optimization.

By the following formula, the MO energy Ebare (unit: hartree) obtained by the above-described MO energy calculation is converted into Escaled (unit: eV) used as the HOMO value and the LUMO value in the present disclosure.

$$\text{Escaled} = 0.823168 \times 27.2114 \times \text{Ebare} - 1.07634$$

27.2114 is simply a coefficient for converting hartree into eV, and 0.823168 and - 1.07634 are adjustment coefficients. These are determined such that the calculated HOMO and LUMO of the compound as a calculation object match the measured values.

Preferred specific examples of the borate compound represented by Formula (B1) are shown below, but the present invention is not limited thereto. In the following structural formulae, Me represents a methyl group.

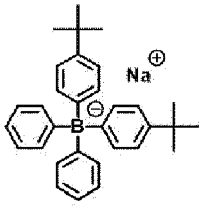
B-1

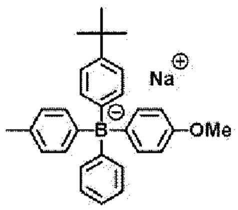
B-2

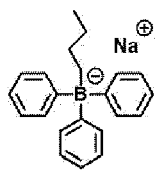
B-3

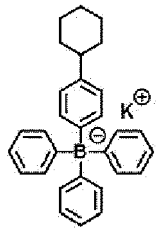
B-4

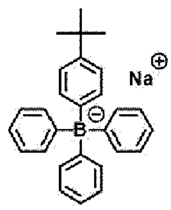
B-5

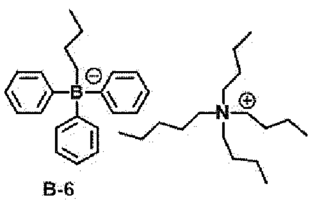
B-6

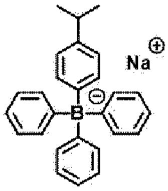
B-7

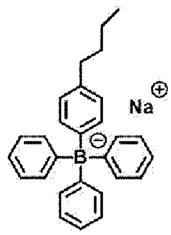
B-8

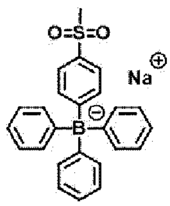
B-9

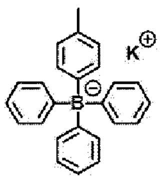
B-10

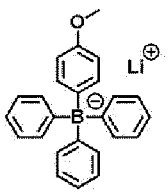
B-11

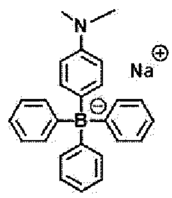
B-12

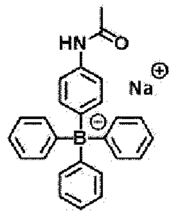
B-13

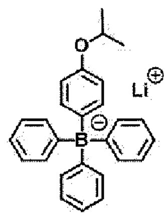
B-14

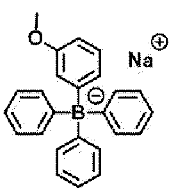
B-15

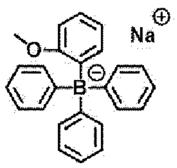
B-16

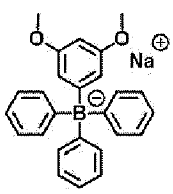
B-17

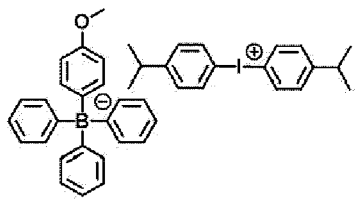
B-18

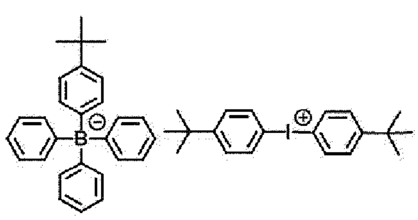
B-19

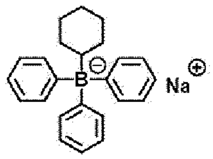
B-20

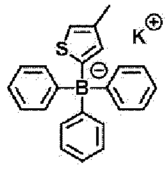
B-21

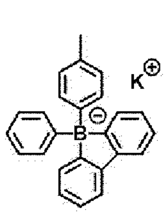
B-22

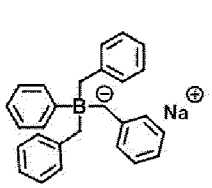
B-23

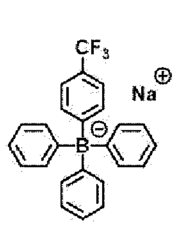
B-24

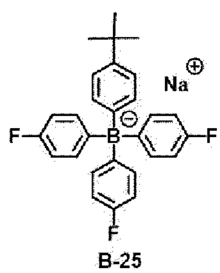
B-25

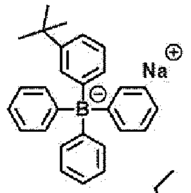

B-26

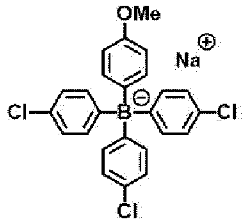

B-27

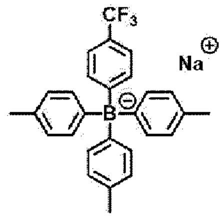

B-28

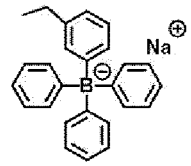

B-29

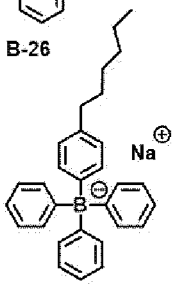

B-30

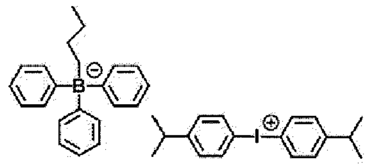

B-31

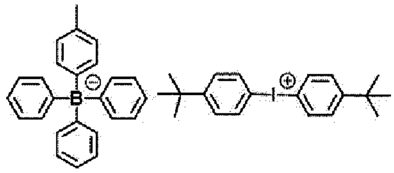

B-32

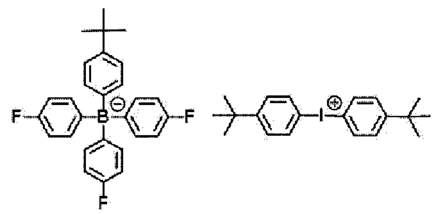

B-33

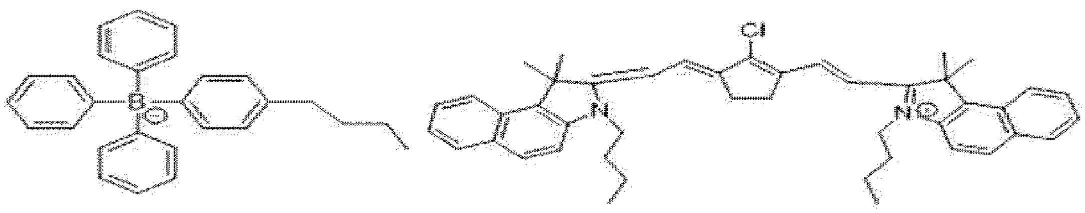

B-34

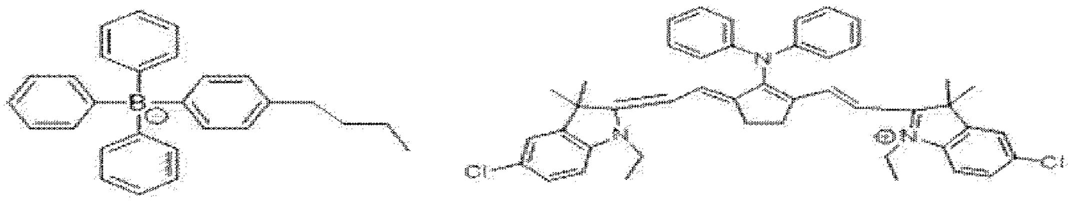

B-35

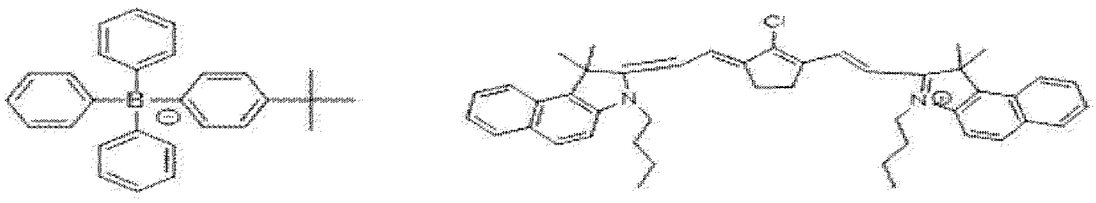

B-36

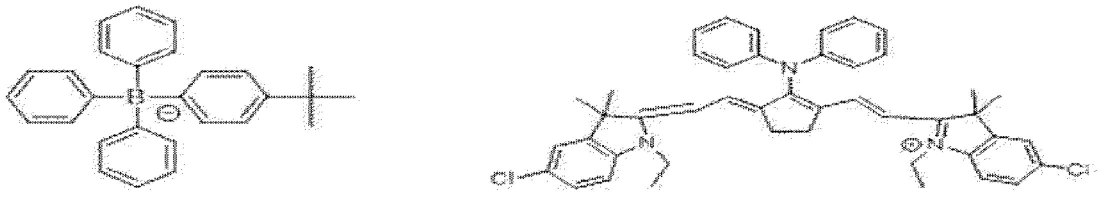

B-37

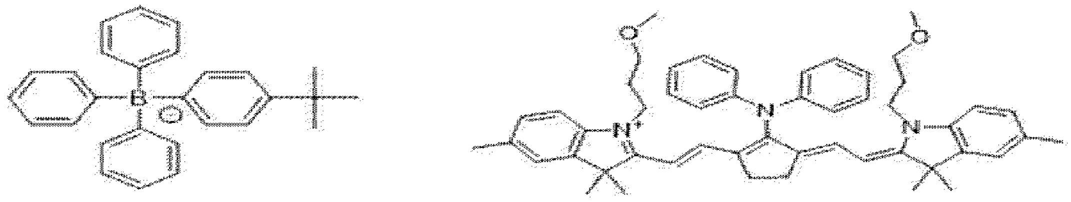

B-38

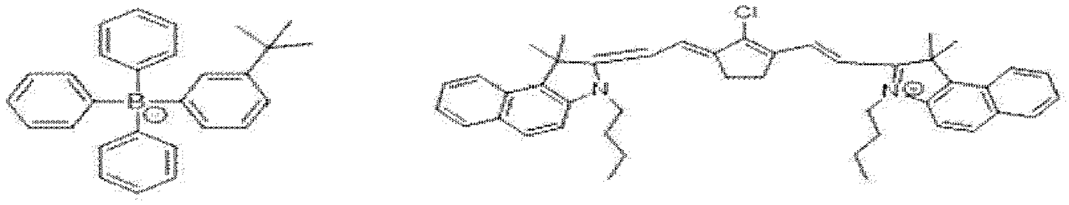

B-39

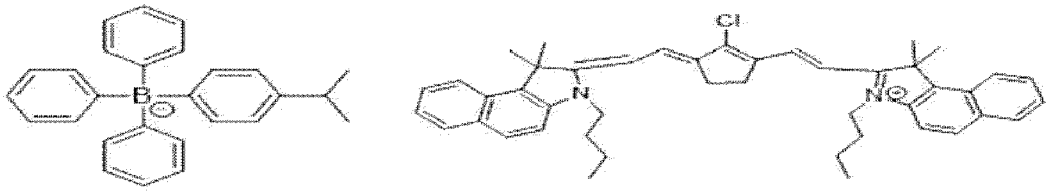

B-40

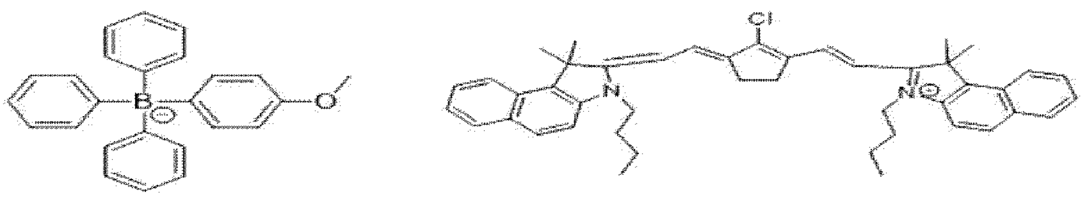

B-41

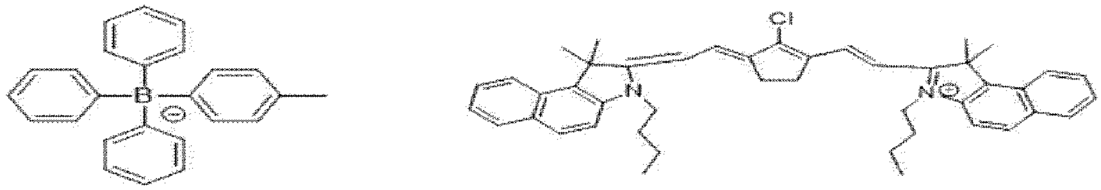

B-42

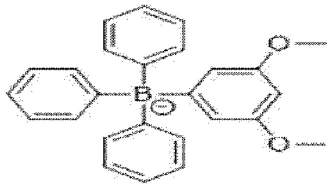
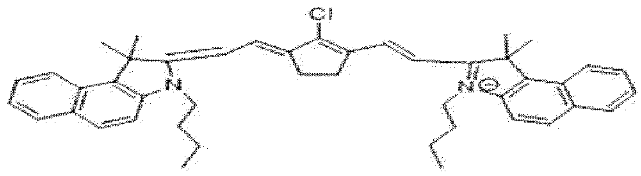

B-43

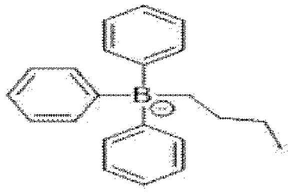
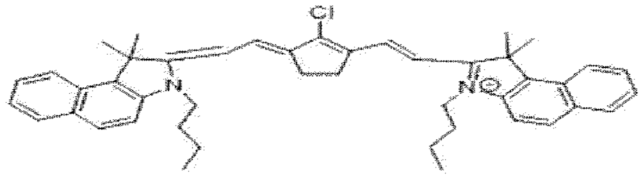

B-44

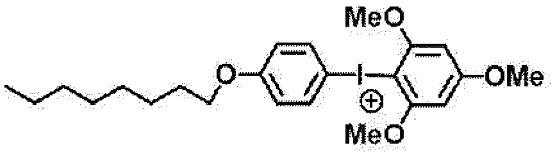
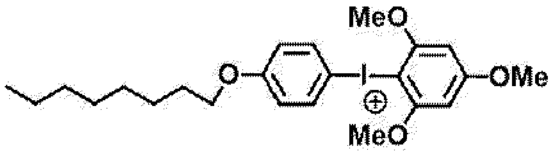

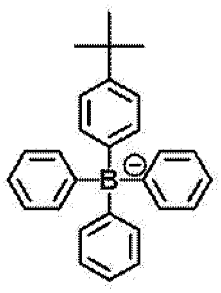
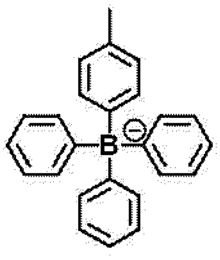

B-45 B-46

The image-recording layer may contain only one kind of borate compound, or may contain two or more kinds thereof.

From the viewpoint of the color formability, the temporal color formability after exposure, the developability, and the property of suppressing the temporal development defect in the obtained lithographic printing plate, the content of the borate compound is preferably in a range of 0.01% by mass to 30% by mass, more preferably in a range of 0.05% by mass to 25% by mass, and still more preferably in a range of 0.1% by mass to 20% by mass with respect to the total mass of the image recording layer, as the weight of the anion portion of the borate compound.

Infrared absorber other than compound represented by Formula (1)

The image-recording layer may contain an infrared absorber (hereinafter, also simply referred to as an "infrared absorber") other than the compound represented by Formula (1).

The infrared absorber is not particularly limited, and examples thereof include pigments and dyes.

As the dye that is used as the infrared absorber, it is possible to use commercially available dyes and known dyes described in publications, for example, "Dye Handbooks" (edited by the Society of Synthetic Organic Chemistry, Japan, 1970). Specific examples thereof include dyes such as an azo dye, a metal complex azo dye, a pyrazolone azo dye, a naphthoquinone dye, an anthraquinone dye, a phthalocyanine dye, a carbonium dye, a quinoneimine dye, a methine dye, a cyanine dye, a squarylium colorant, a pyrylium salt, and a metal thiolate complex.

Among these dyes, for example, a cyanine colorant, a squarylium colorant, a pyrylium salt, a nickel thiolate complex, and an indolenine cyanine colorant are particularly preferable. Furthermore, for example, a cyanine colorant or an indolenine cyanine colorant is preferable. Among these, a cyanine colorant is particularly preferable.

The infrared absorber is preferably a cationic polymethine colorant having an oxygen or nitrogen atom at the meso-position. Preferred examples of the cationic polymethine colorant include a cyanine colorant, a pyrylium colorant, a

thiopyrylium colorant, an azulenium colorant, and the like. From the viewpoint of ease of availability, solubility in a solvent during an introduction reaction, and the like, a cyanine colorant is preferable.

Specific examples of the cyanine colorant include the compounds described in paragraphs "0017" to "0019" of JP2001-133969A and the compounds described in paragraphs "0016" to "0021" of JP2002-023360A and paragraphs "0012" to "0037" of JP2002-040638A. As the cyanine colorant, for example, the compounds described in paragraphs "0034" to "0041" of JP2002-278057A and paragraphs "0080" to "0086" of JP2008-195018A are preferable, and the compounds described in paragraphs "0035" to "0043" of JP2007-90850A and the compounds described in paragraphs "0105" to "0113" of JP2012-206495A are particularly preferable.

In addition, the compounds described in paragraphs "0008" and "0009" of JP1993-5005A (JP-H05-5005A) and paragraphs "0022" to "0025" of JP2001-222101A can also be preferably used.

As pigments, the compounds described in paragraphs "0072" to "0076" of JP2008-195018A are preferable.

In addition, the infrared absorber may include an infrared absorber (decomposition-type infrared absorber) that decomposes by exposure to infrared, other than the compound represented by Formula (1).

Presumably, in a case where a decomposition-type infrared absorber is used as the infrared absorber, the infrared absorber or a decomposition product thereof may promote polymerization, and the decomposition product of the infrared absorber and the polymerizable compound may interact with each other, which may result in excellent printing durability.

The decomposition-type infrared absorber is preferably an infrared absorber that performs a function of forming color by absorbing infrared and decomposing by exposure to infrared.

Hereinafter, a color-forming compound formed as a result of infrared absorption and decomposition of the decomposition-type infrared absorber by exposure to infrared will be also called "color forming substance of the decomposition-type infrared absorber".

In addition, it is preferable that the decomposition-type infrared absorber have a function of absorbing infrared by exposure to infrared and converting the absorbed infrared into heat.

The decomposition-type infrared absorber may be an infrared absorber that decomposes by absorbing at least a part of light in the infrared wavelength region (wavelength of 750 nm to 1 mm). The decomposition-type infrared absorber is preferably an infrared absorber having a maximal absorption wavelength in a wavelength region of 750 nm to 1,400 nm, and more preferably an infrared absorber having a maximal absorption wavelength in a wavelength region of 760 nm to 900 nm.

More specifically, the decomposition-type infrared absorber is preferably a compound that decomposes upon the exposure to infrared and generates a compound having maximal absorption wavelength in a wavelength region of 500 nm to 600 nm.

The decomposition-type infrared absorber is preferably an infrared absorber that decomposes by either or both of heat and electron migration resulting from exposure to infrared, and more preferably an infrared absorber that decomposes by electron migration resulting from exposure to infrared. "Decomposes by electron migration" mentioned herein means that electrons excited to the lowest unoccupied molecular orbital (LUMO) from the highest occupied molecular orbital (HOMO) of the decomposition-type infrared absorber by exposure to infrared rays move to electron accepting groups (groups having potential close to LUMO) in a molecule by means of intramolecular electron migration and thus result in decomposition.

As the infrared absorber and the infrared absorber that decomposes by exposure to infrared, those described in WO2020/262692A can also be suitably used.

As the infrared absorber that decomposes by exposure to infrared, those described in JP2008-544322A or WO2016/027886A can also be suitably used.

In addition, as the cyanine colorant which is a decomposition-type infrared absorber, the infrared absorbing compounds described in WO2019/219560A can be suitably used.

The infrared absorber may be used alone or in combination of two or more kinds thereof.

The total content of the infrared absorber in the image-recording layer with respect to the total mass of the image-recording layer is preferably 0.1% by mass to 10.0% by mass and more preferably 0.5% by mass to 5.0% by mass.

Relationship between borate compound (electron-donating polymerization initiator), electron-accepting polymerization initiator, and compound represented by Formula (1) or infrared absorber

The image-recording layer in the present disclosure preferably contains a borate compound and an electron-accepting polymerization initiator, and more preferably contains a borate compound, an electron-accepting polymerization initiator, and an infrared absorber.

In a case where the image-recording layer contains a borate compound, an electron-accepting polymerization initiator, and a compound represented by Formula (1) or an infrared absorber, it is preferable that HOMO of the borate compound is -6.0 eV or more and LUMO of the electron-accepting polymerization initiator is -3.0 eV or less.

More preferred aspects of the HOMO of the borate compound and the LUMO of the electron-accepting polymerization initiator are as described above.

In the image-recording layer in the present disclosure, it is presumed that energy transfer is carried out between the borate compound, at least one of the compound represented by Formula (1), and the infrared absorber, and the electron-accepting polymerization initiator, for example, as described in the following chemical formulae.

Accordingly, it is considered that in a case where HOMO of the borate compound is - 6.0 eV or more and LUMO of the electron-accepting polymerization initiator is -3.0 eV or less, radicals may be more efficiently generated, which may facilitate further improvement of chemical resistance and printing durability.

From the viewpoint of printing durability and chemical resistance, the HOMO value of at least one HOMO-borate compound of the compound represented by Formula (1) or the infrared absorber is preferably 1.0 eV or less, more preferably 0.70 eV or less, and particularly preferably 0.60 eV or less. In addition, from the same viewpoint, the HOMO value of at least one of the compound represented by Formula (1) or the infrared absorber of the HOMO-borate compound is preferably -0.200 eV or more and more preferably -0.100 eV or more.

The negative value means that the HOMO of the borate compound is higher than the HOMO of at least one of the compound represented by Formula (1) or the infrared absorber.

In addition, from the viewpoints of printing durability and chemical resistance, the LUMO value of at least one of the compound represented by Formula (1) of the electron-accepting polymerization initiator or the infrared absorber is preferably 1.00 eV or less and more preferably 0.700 eV or less.

In addition, from the same viewpoint, the LUMO value of at least one of the compound represented by Formula (1) of the electron-accepting polymerization initiator or the infrared absorber is preferably -0.200 eV or more and more preferably -0.100 eV or more.

In addition, from the same viewpoint, the LUMO value of at least one of the compound represented by Formula (1) of the electron-accepting polymerization initiator or the infrared absorber is preferably 1.00 eV to -0.200 eV and more preferably 0.700 eV to -0.100 eV.

The negative value means that at least one of LUMO of the compound represented by Formula (1) or the infrared absorber is higher than LUMO of the electron-accepting polymerization initiator.

Color forming agent

The image-recording layer preferably contains a color forming agent, and more preferably contains an acid color forming agent. "Color forming agent" used in the present disclosure means a compound that forms or removes color by a stimulus such as light or acid and thus changes the color of the image-recording layer. Furthermore, "acid color forming agent" means a compound that forms or removes color by being heated in a state of accepting an electron accepting compound (for example, a proton of an acid or the like) and thus changes the color of the image-recording layer. The acid color forming agent is particularly preferably a colorless compound which has a partial skeleton such as lactone, lactam, sultone, spiropyran, an ester, or an amide and allows such a partial skeleton to rapidly open the ring or to be cleaved when coming into contact with an electron-accepting compound.

Examples of such an acid color forming agent include the acid color forming agents described in paragraphs "0229" to "0236" of WO2020/262685A.

Particularly, from the viewpoint of color formability, the color forming agent used in the present disclosure is preferably at least one type of compound selected from the group consisting of a spiropyran compound, a spirooxazine compound, a spirolactone compound, and a spirolactam compound.

From the viewpoint of visibility, the hue of the colorant after color forming is preferably green, blue, or black.

From the viewpoint of color formability and visibility of exposed portions, the acid color forming agent is preferably a leuco colorant.

The leuco colorant is not particularly limited as long as it has a leuco structure. The leuco colorant preferably has a spiro structure, and more preferably has a spirolactone ring structure.

From the viewpoint of color formability and visibility of exposed portions, the leuco colorant is preferably a leuco colorant having a phthalide structure or a fluoran structure.

Furthermore, from the viewpoint of color formability and visibility of exposed portions, the leuco colorant having a phthalide structure or a fluoran structure is preferably a compound represented by any of Formula (Le-1) to Formula (Le-3), and more preferably a compound represented by Formula (Le-2).

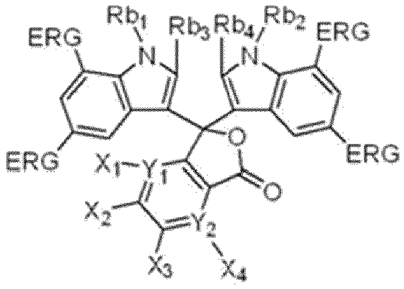

(Le-1)

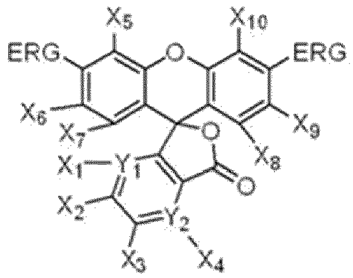

(Le-2)

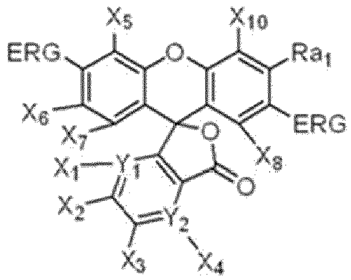

(Le-3)

In Formula (Le-1) to Formula (Le-3), ERG's each independently represent an electron-donating group, $X_1$ to $X_4$ each independently represent a hydrogen atom, a halogen atom, or dialkylanilino group, $X_5$ to $X_{10}$ each independently represent a hydrogen atom, a halogen atom, or a monovalent organic group, $Y_1$ and $Y_2$ each independently represent C or N, $X_1$ does not exist in a case where $Y_1$ is N, $X_4$ does not exist in a case where $Y_2$ is N, $Ra_1$ represents a hydrogen atom, an alkyl group, or an alkoxy group, and $Rb_1$ to $Rb_4$ each independently represent a hydrogen atom, an alkyl group, an aryl group, or a heteroaryl group.

From the viewpoints of color formability and visibility of exposed portions, the electron-donating group represented by ERG in Formula (Le-1) to Formula (Le-3) is preferably an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, an alkoxy group, an aryloxy group, a heteroaryloxy group, or an alkyl group, more preferably an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, an alkoxy group, or an aryloxy group, still more preferably a monoalkyl monoarylamino group, a diarylamino group, a diheteroarylamino group, or a monoaryl monoheteroarylamino group, and particularly preferably a monoalkyl monoarylamino group.

From the viewpoints of color formability and visibility of exposed portions, the electron-donating group represented by ERG is preferably a disubstituted amino group having an aryl group that has a substituent on at least one ortho position or a heteroaryl group that has a substituent on at least one ortho position, more preferably a disubstituted amino group having a phenyl group having a substituent on at least one ortho position and an electron-donating group at a para position, still more preferably an amino group having a phenyl group having a substituent on at least one ortho position and an electron-donating group at a para position, and an aryl group or a heteroaryl group, and particularly preferably an amino group having a phenyl group having a substituent on at least one ortho position and an electron-donating group at a para position, and an aryl group having an electron-donating group or a heteroaryl group having an electron-donating group.

In the present disclosure, in a case where a bonding position of an aryl group or a heteroaryl group with other structures is defined as position 1, the ortho position in the aryl group or heteroaryl group other than a phenyl group is called a bonding position (for example, position 2 or the like) adjacent to the position 1.

Furthermore, from the viewpoints of color formability and visibility of exposed portions, the electron-donating group that the aryl group or heteroaryl group has is preferably an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, an alkoxy group, an aryloxy group, a heteroaryloxy group, or an alkyl group, more preferably an alkoxy group, an aryloxy group, a heteroaryloxy group, or an alkyl group, and particularly preferably an alkoxy group.

From the viewpoints of color formability and visibility of exposed portions, $X_1$ to $X_4$ in Formula (Le-1) to Formula (Le-3) each independently are preferably a hydrogen atom or a chlorine atom, and more preferably each independently represent a hydrogen atom.

From the viewpoints of color formability and visibility of exposed portions, $X_5$ to $X_{10}$ in Formula (Le-2) or Formula (Le-3) each independently are preferably a hydrogen atom, a halogen atom, an alkyl group, an aryl group, an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, a hydroxy group, an alkoxy group, an aryloxy group, a heteroaryloxy group, an acyl group, an alkoxycarbonyl group, an aryloxycarbonyl group, a heteroaryloxycarbonyl group, or a cyano group, more preferably a hydrogen atom, a halogen atom, an alkyl group, an aryl group, an alkoxy group, or an aryloxy group, still more preferably a hydrogen atom, a halogen atom, an alkyl group, or an aryl group, and particularly preferably a hydrogen atom.

From the viewpoints of color formability and visibility of exposed portions, it is preferable that at least one of $Y_1$ or $Y_2$ in Formula (Le-1) to Formula (Le-3) be C, and it is more preferable that both of $Y_1$ and $Y_2$ be C.

From the viewpoints of color formability and visibility of exposed portions, $Ra_1$ in Formula (Le-1) to Formula (Le-3) is preferably an alkyl group or an alkoxy group, more preferably an alkoxy group, and particularly preferably a methoxy group.

From the viewpoints of color formability and visibility of exposed portions, $Rb_1$ to $Rb_4$ in Formula (Le-1) to Formula (Le-3) preferably each independently represent a hydrogen atom or an alkyl group, more preferably each independently represent an alkyl group, and particularly preferably each independently represent a methyl group.

In addition, from the viewpoints of color formability and visibility of exposed portions, the leuco colorant having a phthalide structure or a fluoran structure is more preferably a compound represented by any of Formula (Le-4) to Formula (Le-6), and still more preferably a compound represented by Formula (Le-5).

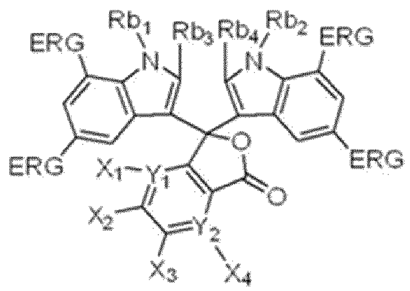

(Le - 4)

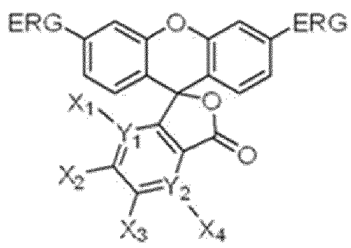

(Le - 5)

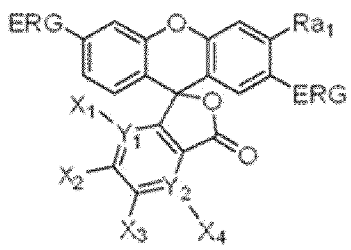

(Le - 6)

In Formula (Le-4) to Formula (Le-6), ERG's each independently represent an electron-donating group, $X_1$ to $X_4$ each independently represent a hydrogen atom, a halogen atom, or a dialkylanilino group, $Y_1$ and $Y_2$ each independently represent C or N, $X_1$ does not exist in a case where $Y_1$ is N, $X_4$ does not exist in a case where $Y_2$ is N, $Ra_1$ represents a hydrogen atom, an alkyl group, or an alkoxy group, and $Rb_1$ to $Rb_4$ each independently represent a hydrogen atom, an alkyl group, an aryl group, or a heteroaryl group.

ERG, $X_1$ to $X_4$, $Y_1$, $Y_2$, $Ra_1$, and $Rb_1$ to $Rb_4$ in Formula (Le-4) to Formula (Le-6) have the same definitions as ERG, $X_1$ to $X_4$, $Y_1$, $Y_2$, $Ra_1$, and $Rb_1$ to $Rb_4$ in Formula (Le-1) to Formula (Le-3) respectively, and preferred aspects thereof are also the same.

Furthermore, from the viewpoints of color formability and visibility of exposed portions, the leuco colorant having a phthalide structure or a fluoran structure is still more preferably a compound represented by any of Formula (Le-7) to Formula (Le-9), and particularly preferably a compound represented by Formula (Le-8).

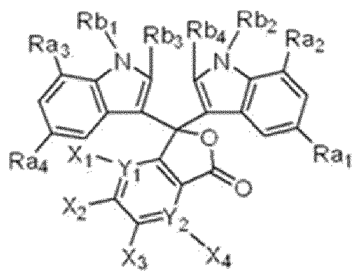

(Le - 7)

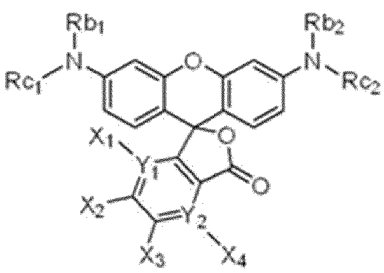

(Le - 8)

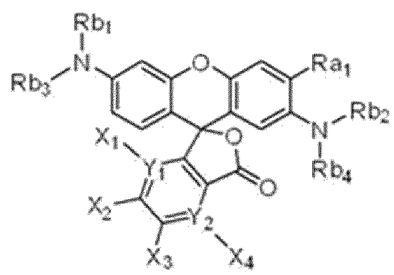

(Le - 9)

In Formula (Le-7) to Formula (Le-9), $X_1$ to $X_4$ each independently represent a hydrogen atom, a halogen atom, or a dialkylanilino group, $Y_1$ and $Y_2$ each independently represent C or N, $X_1$ does not exist in a case where $Y_1$ is N, $X_4$ does not exist in a case where $Y_2$ is N, $Ra_1$ to $Ra_4$ each independently represent a hydrogen atom, an alkyl group, or an alkoxy group, $Rb_1$ to $Rb_4$ each independently represent a hydrogen atom, an alkyl group, an aryl group, or a heteroaryl group, and $Rc_1$ and $Rc_2$ each independently represent an aryl group or a heteroaryl group.

$X_1$ to $X_4$, $Y_1$, and $Y_2$ in Formula (Le-7) to Formula (Le-9) have the same definition as $X_1$ to $X_4$, $Y_1$, and $Y_2$ in Formula (Le-1) to Formula (Le-3) respectively, and preferred aspects thereof are also the same.

From the viewpoints of color formability and visibility of exposed portions, $Ra_1$ to $Ra_4$ in Formula (Le-7) or Formula (Le-9) each independently are preferably an alkyl group or an alkoxy group, more preferably an alkoxy group, and particularly preferably represent a methoxy group.

From the viewpoints of color formability and visibility of exposed portions, $Rb_1$ to $Rb_4$ in Formula (Le-7) to Formula (Le-9) each independently are preferably a hydrogen atom, an alkyl group, or an aryl group substituted with an alkoxy

group, more preferably an alkyl group, and particularly preferably a methyl group.

From the viewpoints of color formability and visibility of exposed portions, $Rc_1$ and $Rc_2$ in Formula (Le-8) each independently are preferably a phenyl group or an alkylphenyl group, and more preferably a phenyl group.

From the viewpoints of color formability and visibility of exposed portions, $Rc_1$ and $Rc_2$ in Formula (Le-8) each independently are preferably an aryl group having a substituent on at least one ortho position or a heteroaryl group having a substituent on at least one ortho position, more preferably an aryl group having a substituent on at least one ortho position, still more preferably a phenyl group having a substituent on at least one ortho position, and particularly preferably a phenyl group having a substituent on at least one ortho position and having an electron-donating group at the para position. Examples of the substituent in $Rc_1$ and $Rc_2$ include substituents that will be described later.

In Formula (Le-8), from the viewpoints of color formability and visibility of exposed portions, it is preferable that $X_1$ to $X_4$ are a hydrogen atom, and $Y_1$ and $Y_2$ are C.

Furthermore, from the viewpoints of color formability and visibility of exposed portions, in Formula (Le-8), $Rb_1$ and $Rb_2$ each independently are preferably an alkyl group or an aryl group substituted with an alkoxy group.

Furthermore, from the viewpoints of color formability and visibility of exposed portions, in Formula (Le-8), $Rb_1$ and $Rb_2$ each independently are preferably an aryl group or a heteroaryl group, more preferably an aryl group, still more preferably an aryl group having an electron-donating group, and particularly preferably a phenyl group having an electron-donating group at the para position.

From the viewpoints of color formability and visibility of exposed portions, the electron-donating group in $Rb_1$, $Rb_2$, $Rc_1$, and $Rc_2$ is preferably an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, an alkoxy group, an aryloxy group, a heteroaryloxy group, or an alkyl group, more preferably an alkoxy group, an aryloxy group, a heteroaryloxy group, or an alkyl group, and particularly preferably an alkoxy group.

In addition, from the viewpoints of the color formability and the visibility of the exposed portion, it is also preferable that the acid color forming agent includes one or more compounds selected from the group consisting of a compound represented by Formula (Le-10) and a compound represented by Formula (Z-4).

That is, it is preferable that the image-recording layer in the lithographic printing plate precursor according to the present disclosure further contains one or more compounds selected from the group consisting of a compound represented by Formula (Le-10) and a compound represented by Formula (Z-4).

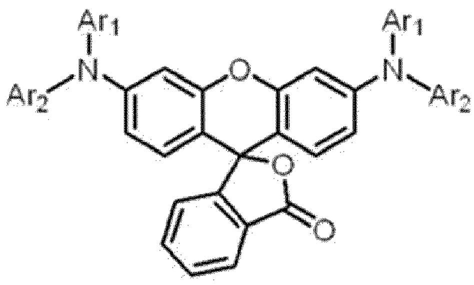

( Le - 10 )

In Formula (Le-10), $Ar_1$'s each independently represent an aryl group or a heteroaryl group, and $Ar_2$'s each independently represent an aryl group having a substituent on at least one ortho position or a heteroaryl group having a substituent on at least one ortho position.

$Ar_1$ in Formula (Le-10) has the same definition as $Rb_1$ and $Rb_2$ in Formula (Le-7) to Formula (Le-9), and preferred aspects thereof are also the same.

$Ar_2$ in Formula (Le-10) has the same definition as $Rc_1$ and $Rc_2$ in Formula (Le-7) to Formula (Le-9), and preferred aspects thereof are also the same.

The alkyl group in Formula (Le-1) to Formula (Le-9) may be linear or branched or may have a ring structure.

The carbon number of the alkyl group in Formula (Le-1) to Formula (Le-9) is preferably 1 to 20, more preferably 1 to 8, still more preferably 1 to 4, and particularly preferably 1 or 2.

The carbon number of the aryl group in Formula (Le-1) to Formula (Le-10) is preferably 6 to 20, more preferably 6 to 10, and particularly preferably 6 to 8.

Specific examples of the aryl group in Formula (Le-1) to Formula (Le-10) include a phenyl group, a naphthyl group, an anthracenyl group, a phenanthrenyl group, and the like which may have a substituent.

Specific examples of the heteroaryl group in Formula (Le-1) to Formula (Le-10) include a furyl group, a pyridyl

group, a pyrimidyl group, a pyrazoyl group, a thiophenyl group, and the like which may have a substituent.

Each of the groups in Formula (Le-1) to Formula (Le-10), such as a monovalent organic group, an alkyl group, an aryl group, a heteroaryl group, a dialkylanilino group, an alkylamino group, and an alkoxy group, may have a substituent. Examples of the substituent include an alkyl group, an aryl group, a heteroaryl group, a halogen atom, an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, a hydroxy group, an alkoxy group, an aryloxy group, a heteroaryloxy group, an acyl group, an alkoxycarbonyl group, an aryloxycarbonyl group, a heteroaryloxycarbonyl group, a cyano group, and the like. In addition, these substituents may be further substituted with these substituents.

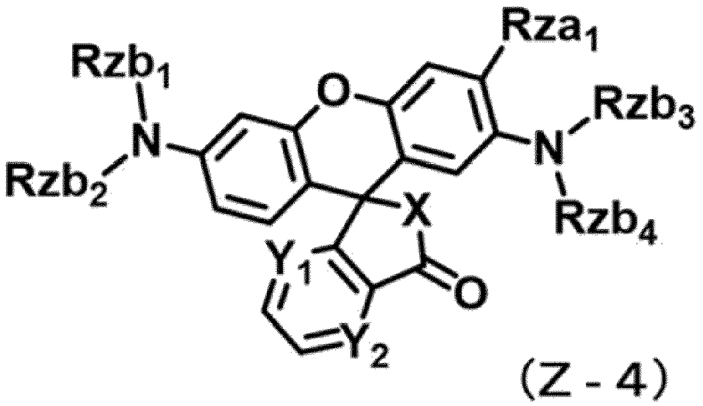

(Z - 4)

In Formula (Z-4), $Rza_1$ represents a hydrogen atom, an alkyl group, or an alkoxy group, $Rzb_1$ to $Rzb_4$ each independently represent a hydrogen atom, an alkyl group, or an aryl group, $Rzb_1$ and $Rzb_2$, and $Rzb_3$ and $Rzb_4$ may be linked to each other to form a ring structure, X represents O or NR, R represents a hydrogen atom, an alkyl group, an aryl group, or a heteroaryl group, and $Y_1$ and $Y_2$ each independently represent CH or N.

$Rza_1$ in Formula (Z-4) is preferably an alkyl group or an alkoxy group.

$Rzb_1$ and $Rzb_2$ in Formula (Z-4) are each independently preferably an alkyl group.

$Rzb_3$ and $Rzb_4$ in Formula (Z-4) each independently represent a hydrogen atom, an alkyl group, or an aryl group, and it is preferable that one of them represents an aryl group.

X in Formula (Z-4) is preferably O, and $Y_1$ and $Y_2$ are preferably CH.

The alkyl group in Formula (Z-4) may be linear or branched or may have a ring structure.

The number of carbon atoms in the alkyl group in Formula (Z-4) is preferably 1 to 20, more preferably 1 to 8, and still more preferably 1 to 5.

The number of carbon atoms in the aryl group in Formula (Z-4) is preferably 6 to 20, more preferably 6 to 10, and particularly preferably 6 to 8.

Each of the groups such as the alkyl group and the aryl group in Formula (Z-4) may have a substituent. Examples of the substituent include an alkyl group, an aryl group, a halogen atom, an amino group, an alkylamino group, an arylamino group, a dialkylamino group, a monoalkyl monoarylamino group, a diarylamino group, a hydroxy group, an alkoxy group, an aryloxy group, an acyl group, an alkoxycarbonyl group, an aryloxycarbonyl group, a cyano group, and the like. In addition, these substituents may be further substituted with these substituents.

From the viewpoint of color formability and visibility of exposed portions, the acid color forming agent preferably includes a compound represented by Formula (Le-11).

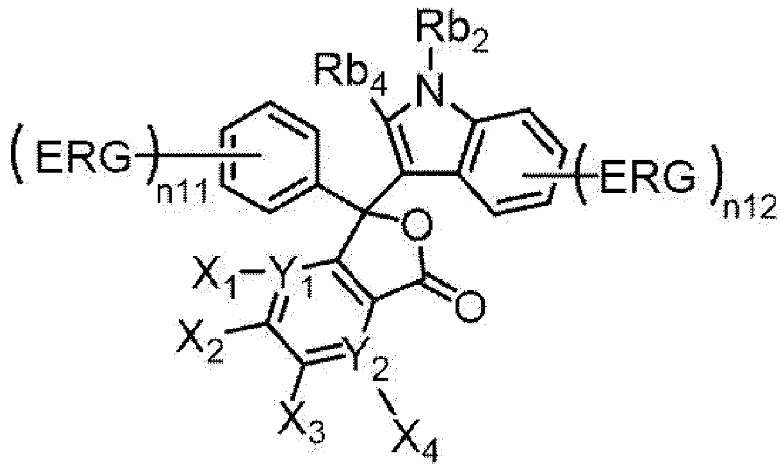

( Le - 11 )

In Formula (Le-11), ERG's each independently represent an electron-donating group, n11 represents an integer of 1 to 5, n12 represents an integer of 0 to 2, $X_1$ to $X_4$ each independently represent a hydrogen atom, a halogen atom, or a dialkylanilino group, $Y_1$ and $Y_2$ each independently represent C or N, $X_1$ is not present in a case where $Y_1$ represents N, $X_4$ is not present in a case where $Y_2$ represents N, and $Rb_2$ and $Rb_4$ each independently represent a hydrogen atom, an alkyl group, an aryl group, or a heteroaryl group.

ERG, $X_1$ to $X_4$, $Y_1$, $Y_2$, $Rb_2$, and $Rb_4$ in Formula (Le-11) each have the same definition as that for ERG, $X_1$ to $X_4$, $Y_1$, $Y_2$, $Rb_2$, and $Rb_4$ in Formulae (Le-1) to (Le-3), and the preferable aspects thereof are also the same as described above.

n 11 in Formula (Le-11) is preferably an integer of 1 to 3, and more preferably 1 or 2.
n12 in Formula (Le-11) is preferably 0 or 1, and more preferably 0.

The alkyl group in Formula (Le-11) may be linear or branched or may have a ring structure.

The carbon number of the alkyl group in Formula (Le-11) is preferably 1 to 20, more preferably 1 to 8, still more preferably 1 to 4, and particularly preferably 1 or 2.

The number of carbon atoms in the aryl group in Formula (Le-11) is preferably 6 to 20, more preferably 6 to 10, and particularly preferably 6 to 8.

Specific examples of the aryl group in Formula (Le-11) include a phenyl group, a naphthyl group, an anthracenyl group, a phenanthrenyl group, and the like which may have a substituent.

Specific examples of the heteroaryl group in Formula (Le-11) include a furyl group, a pyridyl group, a pyrimidyl group, a pyrazoyl group, a thiophenyl group, and the like which may have a substituent.

Each of the groups in Formula (Le-11), such as a monovalent organic group, an alkyl group, an aryl group, a heteroaryl group, a dialkylanilino group, an alkylamino group, and an alkoxy group, may have a substituent. Examples of the substituent include an alkyl group, an aryl group, a heteroaryl group, a halogen atom, an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, a hydroxy group, an alkoxy group, an aryloxy group, a heteroaryloxy group, an acyl group, an alkoxycarbonyl group, an aryloxycarbonyl group, a heteroaryloxycarbonyl group, a cyano group, and the like. In addition, these substituents may be further substituted with these substituents.

In addition, from the viewpoint of color formability and visibility of exposed portions, the acid color forming agent preferably includes a compound represented by any of Formula (3a) or Formula (3b).

As a preferred aspect, the acid color forming agent is preferably a compound represented by any of Formula (3a) or Formula (3b).

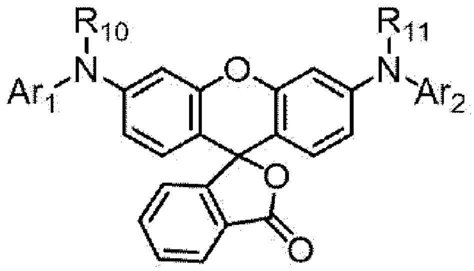

(3a)

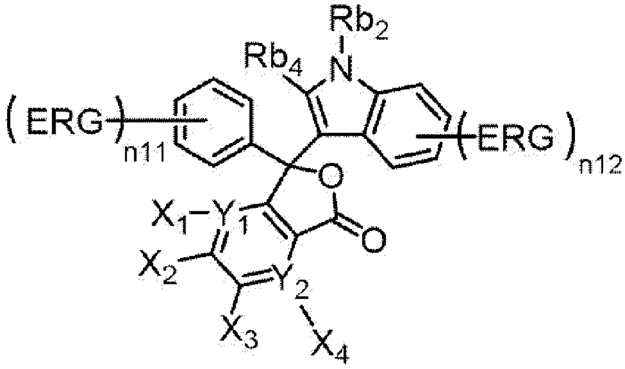

(3b)

In Formula (3a), $Ar_1$ and $Ar_2$ each independently represent an aryl group or a heteroaryl group, and $R_{10}$ and $R_{11}$ each independently represent a hydrogen atom, an alkyl group, an aryl group, or a heteroaryl group.

In Formula (3b), ERG's each independently represent an electron-donating group, n11 represents an integer of 1 to 5, n12 represents an integer of 0 to 2, $X_1$ to $X_4$ each independently represent a hydrogen atom, a halogen atom, or a monovalent organic group, $Y_1$ and $Y_2$ each independently represent C or N, $X_1$ is not present in a case where $Y_1$ is N, $X_4$ is not present in a case where $Y_2$ is N, and $Rb_2$ and $Rb_4$ each independently represent a hydrogen atom, an alkyl group, an aryl group, or a heteroaryl group.

ERG, $R_{12}$, and $R_{13}$ in Formula (3b) each have the same meaning as ERG, $Rb_2$, and $Rb_4$ in Formula (Le-1) to Formula (Le-3), and preferred aspects thereof are also the same. In addition, the bonding position of ERG in Formula (3b) is preferably an ortho position and a para position with respect to the bonding position of the nitrogen atom on the aromatic ring.

The alkyl group in Formula (3a) may be linear or branched or may have a ring structure.

In addition, the number of carbon atoms in the alkyl group in Formula (3a) is preferably 1 to 20, more preferably 1 to 8, still more preferably 1 to 4, and particularly preferably 1 or 2.

The number of carbon atoms in the aryl group in Formula (3a) is preferably 6 to 20, more preferably 6 to 10, and particularly preferably 6 to 8.

Specific examples of the aryl group in Formula (3a) include a phenyl group, a naphthyl group, an anthracenyl group, and a phenanthrenyl group, which may have a substituent.

Specific examples of the heteroaryl group in Formula (3a) include a furyl group, a pyridyl group, a pyrimidyl group, a pyrazoyl group, and a thiophenyl group, which may have a substituent.

ERG in Formula (3b) has the same meaning as ERG in Formula (Le-1) to Formula (Le-3), and the same applies to the preferred aspect thereof.

n11 in Formula (3b) is preferably an integer of 1 to 3, and more preferably 1 or 2.
n12 in Formula (3b) is preferably an integer of 0 to 2, more preferably 0 or 1, and still more preferably 0.

The monovalent organic group in Formula (3b) has the same meaning as the monovalent organic group as $X_5$ to $X_{10}$ in Formula (Le-2) and Formula (Le-3), and the same applies to the preferred aspect thereof.

In addition, each of the groups such as the monovalent organic group, the alkyl group, the aryl group, and the heteroaryl group in Formula (3a) and Formula (3b) may have a substituent. Examples of the substituent include an alkyl group, an aryl group, a heteroaryl group, a halogen atom, an amino group, an alkylamino group, an arylamino group, a heteroarylamino group, a dialkylamino group, a monoalkyl monoarylamino group, a monoalkyl monoheteroarylamino group, a diarylamino group, a diheteroarylamino group, a monoaryl monoheteroarylamino group, a hydroxy group, an alkoxy group, an aryloxy group, a heteroaryloxy group, an acyl group, an alkoxycarbonyl group, an aryloxycarbonyl group, a heteroaryloxycarbonyl group, a cyano group, and the like. In addition, these substituents may be further substituted with these substituents.

In addition, examples of the leuco colorant having the above-described phthalide structure or fluoran structure, which is suitably used, include the acid color forming agent described in paragraphs "0160" to "0168" of WO2023/032681A.

Furthermore, suitable examples of the acid color forming agent also include the following compounds.

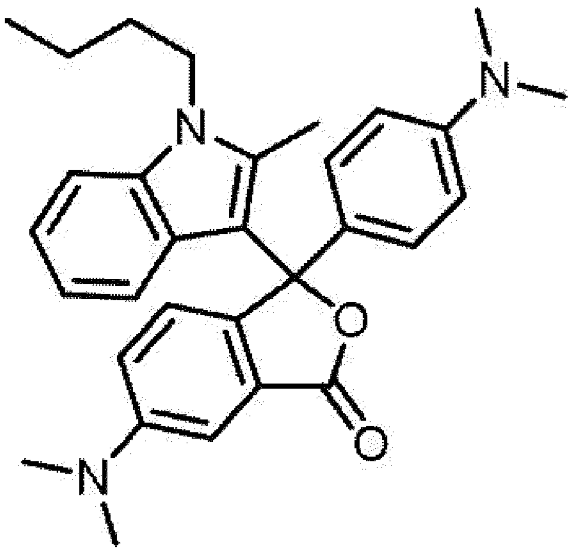

As the color forming agent, a commercially available product can be used, and examples thereof include ETAC, RED500, RED520, CVL, S-205, BLACK305, BLACK400, BLACK100, BLACK500, H-7001, GREEN300, NIRBLACK78, BLUE220, H-3035, BLUE203, ATP, H-1046, and H-2114 (all manufactured by Fukui Yamada Chemical Co., Ltd.), ORANGE-DCF, Vermilion-DCF, PINK-DCF, RED-DCF, BLMB, CVL, GREEN-DCF, and TH-107 (all manufactured by HODOGAYA CHEMICAL CO.,LTD.), ODB, ODB-2, ODB-4, ODB-250, ODB-BlackXV, Blue-63, Blue-502, GN-169, GN-2, Green-118, Red-40, and Red-8 (all manufactured by Yamamoto Chemicals Inc.), and Crystal Violet Lactone (manufactured by Tokyo Chemical Industry Co., Ltd.), and the like. Among these commercially available products, ETAC, S-205, BLACK305, BLACK400, BLACK100, BLACK500, H-7001, GREEN300, NIRBLACK78, H-3035, ATP, H-1046, H-2114, GREEN-DCF, Blue-63, GN-169, and crystal violet lactone are preferable because these form a film having excellent visible light absorbance.

Each of these color forming agents may be used alone. Alternatively, two or more components can be used in combination.

The content of the color forming agent with respect to the total mass of the image-recording layer is preferably 0.5% by mass to 10% by mass, and more preferably 1% by mass to 5% by mass.

Polymerizable compound

The polymerizable compound may be, for example, a radically polymerizable compound or a cationically polymerizable compound. As the polymerizable compound, an addition polymerizable compound having at least one ethylenically unsaturated bond (ethylenically unsaturated compound) is preferable. The ethylenically unsaturated compound is preferably a compound having at least one ethylenically unsaturated bond on a terminal, and more preferably a compound having two or more ethylenically unsaturated bonds on a terminal. The polymerizable compound can have a chemical form such as a monomer or a prepolymer, that is, a dimer, a trimer, an oligomer, a mixture of these, or the like.

From the viewpoint of printing durability, the polymerizable compound preferably has 5 or more functionalities, more preferably has 7 or more functionalities, and still more preferably has 11 or more functionalities. The "functionality" indicates a group (functional group) that contributes to polymerization, and an ethylenically unsaturated bond is preferable. For example, the term "4 or more functionalities" indicates that the number of groups contributing to polymerization is 4 or more.

The polymerizable compound is not particularly limited, but usually has 20 or less functionalities.

From the viewpoint of printing durability, the polymerizable compound is preferably an ethylenically unsaturated compound having 5 or more functionalities, more preferably an ethylenically unsaturated compound having 7 or more functionalities, and still more preferably an ethylenically unsaturated compound having 11 or more functionalities.

The molecular weight of the polymerizable compound (the weight-average molecular weight in a case where the compound has molecular weight distribution) is not particularly limited, but the molecular weight thereof is preferably smaller from the viewpoint of on-press developability and the molecular weight thereof is preferably larger from the viewpoint of printing durability.

The molecular weight of the polymerizable compound is preferably less than 15,000. In addition, the molecular weight thereof is preferably 100 or greater.

From the viewpoints of achieving both on-press developability and printing durability, the molecular weight of the polymerizable compound is preferably 100 or more and less than 15,000, more preferably 500 or more and less than 13,000, and still more preferably 1,000 or more and less than 10,000.

As a preferred aspect, the image-recording layer preferably contains a polymerizable compound having 7 or more functionalities and having a molecular weight of less than 15,000, as the polymerizable compound.

As a preferred aspect, the image-recording layer preferably contains a polymerizable compound having 11 or more functionalities and having a molecular weight of less than 15,000, as the polymerizable compound.

Examples of the monomer include unsaturated carboxylic acids (for example, acrylic acid, methacrylic acid, itaconic acid, crotonic acid, isocrotonic acid, and maleic acid), and esters and amides thereof. It is preferable to use esters of an unsaturated carboxylic acid and a polyhydric alcohol compound, or amides of an unsaturated carboxylic acid and a polyvalent amine compound. In addition, products of an addition reactant of unsaturated carboxylic acid esters or amides having a nucleophilic substituent such as a hydroxy group, an amino group, or a mercapto group and monofunctional or polyfunctional isocyanates or epoxies, a dehydrocondensation reactant of the aforementioned unsaturated carboxylic acid esters or amides and a monofunctional or polyfunctional carboxylic acid, and the like are also suitably used. Furthermore, an addition reactant of unsaturated carboxylic acid esters or amides having an electrophilic substituent such as an isocyanate group or an epoxy group and monofunctional or polyfunctional alcohols, amines, or thiols, and a substitution reactant of unsaturated carboxylic acid esters or amides having a leaving substituent such as a halogen atom or a tosyloxy group and monofunctional or polyfunctional alcohols, amines, or thiols are also suitable. In addition, as another example, it is also possible to use a group of compounds obtained by substituting the unsaturated carboxylic acid with an unsaturated phosphonic acid, styrene, vinyl ether, or the like. These compounds are described in JP2006-508380A, JP2002-287344A, JP2008-256850A, JP2001-342222A, JP1997-179296A (JP-H09-179296A), JP1997-179297A (JP-H09-179297A), JP1997-179298A (JP-H09-179298A), JP2004-294935A, JP2006-243493A, JP2002-275129A, JP2003-64130A, JP2003-280187A, JP1998-333321A (JP-H10-333321A), and the like.

Specific examples of monomers of esters of polyhydric alcohol compounds and unsaturated carboxylic acids include acrylic acid esters such as ethylene glycol diacrylate, 1,3-butanediol diacrylate, tetramethylene glycol diacrylate, propylene glycol diacrylate, trimethylolpropane triacrylate, hexanediol diacrylate, tetraethylene glycol diacrylate, pentaerythritol tetraacrylate, sorbitol triacrylate, isocyanuric acid ethylene oxide (EO)-modified triacrylate, and polyester acrylate oligomers, and the like. Examples of the methacrylic acid ester include tetramethylene glycol dimethacrylate, neopentyl glycol dimethacrylate, trimethylolpropane trimethacrylate, ethylene glycol dimethacrylate, pentaerythritol trimethacrylate, bis[p-(3-methacryloxy-2-hydroxypropoxy)phenyl]dimethylmethane, bis[p-(methacryloxyethoxy)phenyl] dimethylmethane, and the like. In addition, specific examples of monomers of amides of polyvalent amine compounds and unsaturated carboxylic acids include methylene bisacrylamide, methylene bismethacrylamide, 1,6-hexamethylene bisacrylamide, 1,6-hexamethylene bismethacrylamide, diethylenetriamine trisacrylamide, xylylene bisacrylamide, xylylene bismethacrylamide, and the like.

In addition, urethane-based addition polymerizable compounds manufactured using an addition reaction between an isocyanate and a hydroxy group are also suitable, and specific examples thereof include vinyl urethane compounds having two or more polymerizable vinyl groups in one molecule obtained by adding vinyl monomers containing a hydroxy group represented by the following Formula (M) to a polyisocyanate compound having two or more isocyanate groups in one molecule which is described in, for example, JP1973-41708B (JP-S48-41708B).

$$CH_2=C(R^{M4})COOCH_2CH(R^{M5})OH \qquad (M)$$

In Formula (M), $R^{M4}$ and $R^{M5}$ each independently represent a hydrogen atom or a methyl group.

Furthermore, urethane acrylates described in JP1976-37193A (JP-S51-37193A), JP1990-32293B (JP-H02-32293B), JP1990-16765B (JP-H02-16765B), JP2003-344997A, and JP2006-65210A; urethane compounds having an ethylene oxide-based skeleton described in JP1983-49860B (JP-S58-49860B), JP1981-17654B (JP-S56-17654B), JP1987-39417B (JP-S62-39417B), JP1987-39418B (JP-S62-39418B), JP2000-250211A, and JP2007-94138A; and urethane compounds having a hydrophilic group described in US7153632B, JP1996-505958A (JP-H08-505958A), JP2007-293221A, and JP2007-293223A are also suitable.

Details of how to use the polymerizable compound, such as the structure of the compound, whether the compound is used alone or used in combination with other compounds, and the amount of the compound to be added, can be randomly set in consideration of the final use of the lithographic printing plate precursor or the like.

The content of the polymerizable compound is preferably in a range of 1% by mass to 90% by mass, more preferably in a range of 5% by mass to 70% by mass, and still more preferably in a range of 10% by mass to 50% by mass with respect to the total mass of the image recording layer.

Hydrogen donating compound

The image-recording layer preferably contains a hydrogen donating compound.

The hydrogen donating compound is a compound having at least one group selected from the group consisting of -OH, -NH-, -$SO_2$-NH-, -$SO_2$-OH, -CO-NH-, and -CO-OH in a molecule and having a molecular weight of less than 3,000, and is preferably a phenolic compound.

In addition, the hydrogen donating compound is a compound different from the polymerization initiator, the polymerizable compound, and the color forming agent.

A group selected from the group consisting of -OH, -NH-, -$SO_2$-NH-, -$SO_2$-OH, -CO-NH-, and -COOH (hereinafter, also simply referred to as a "hydrogen donating group") is a group capable of donating a hydrogen atom.

"-OH" represents a hydroxy group and is a group that does not bond to "-COOH" (a carboxy group) and "-$SO_2$-".
"-NH-" is a group that does not bond to -$SO_2$- or -CO-.

The hydrogen donating compound is preferably a compound having at least one partial structure represented by Formula (I) in the molecule.

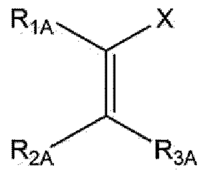

(I)

In Formula (I), X is a group selected from the group consisting of -OH, -NH-, -$SO_2$-NH-, -$SO_2$-OH, -CO-NH-, and -CO-OH.

$R_{1A}$ to $R_{3A}$ each independently have a group having at least one selected from the group consisting of a hydrogen atom, a carbon atom, a halogen atom, and a heteroatom.

At least two of $R_{1A}$ to $R_{3A}$ may be linked to each other to form a ring.

In a case where X is a monovalent group, one or more hydrogen atoms included in $R_{1A}$ to $R_{3A}$ are removed to form a bond.

In a case where X is a divalent group, one or more hydrogen atoms included in $R_{1A}$ to $R_{3A}$ may be further removed to form a bond.

As the hydrogen donating group of X, from the viewpoint of suppressing fading during exposure to a white lamp and ease of familiarity with the image-recording layer, -OH, -NH-, - $SO_2$-NH-, or -CO-NH- is preferable, and -OH or -$SO_2$-NH- is more preferable.

The group (hereinafter, also referred to as a "substituent A") having at least one selected from the group consisting of a hydrogen atom, a carbon atom, a halogen atom, and a heteroatom as $R_{1A}$ to $R_{3A}$ is not particularly limited; however, it is preferably a group having 0 to 30 carbon atoms.

The halogen atom is not particularly limited, and examples thereof include a fluorine atom, a chlorine atom, a bromine atom, and an iodine atom.

The heteroatom is not particularly limited, and examples thereof include a nitrogen atom, an oxygen atom, a sulfur atom, and a phosphorus atom.

The substituent A is not particularly limited, but for example, a hydrogen atom or a hydrocarbon group is preferable. The hydrocarbon group may have a heteroatom or a halogen atom.

At least two of $R_{1A}$ to $R_{3A}$ may be linked to each other to form a ring. The ring to be formed may be a monocyclic ring or a polycyclic ring. Examples of the ring to be formed include an aromatic ring and an aliphatic hydrocarbon ring, and the number of ring member atoms of the ring to be formed is preferably 6 to 14.

In a case where X is a monovalent group (specifically, a group selected from the group consisting of -OH, -$SO_2$-OH, and -CO-OH), one or more hydrogen atoms included in $R_{1A}$ to $R_{3A}$ are removed to form a bonding site.

In a case where X is a divalent group (specifically, a group selected from the group consisting of -NH-, -$SO_2$-NH-, and -CO-NH-), the divalent group can constitute a partial structure in Formula (I), but one or more hydrogen atoms included in $R_{1A}$ to $R_{3A}$ may be further removed to form a bond.

The hydrogen donating compound is preferably a compound having at least one partial structure represented by Formula (I) in the molecule, and may be a compound having at least two or more partial structures represented by Formula (I) in the molecule.

The number of the partial structures represented by Formula (I) in the molecule is not particularly limited, but is preferably 1 to 10 and more preferably 1 to 5.

The hydrogen donating compound is preferably a compound having at least one partial structure represented by Formula (II) in the molecule.

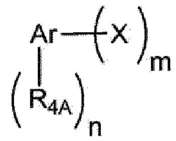 (II)

In Formula (II), Ar represents an aromatic ring group.
X is a group selected from the group consisting of -OH, -NH-, $-SO_2-NH-$, $-SO_2-OH$, - CO-NH-, and -CO-OH.
$R_{4A}$ is a group having at least one selected from the group consisting of a hydrogen atom, a carbon atom, a halogen atom, and a heteroatom.
m represents an integer of 1 to 5. In a case where m represents an integer of 2 or more, a plurality of X's may be the same as or different from each other.
n represents an integer of 0 to 5. In a case where n represents an integer of 2 or more, a plurality of $R_{4A}$'s may be the same as or different from each other. In a case where n is 2 or more, a plurality of $R_{4A}$'s may be linked to each other to form a ring.
In a case where all X's are monovalent groups, one or more hydrogen atoms included in $R_{4A}$ are removed to form a bond.
In a case where at least one X is a divalent group, one or more hydrogen atoms contained in $R_{4A}$ may be further removed to form a bond.
X has the same meaning as X in Formula (I), and the same applies to the preferred range thereof.
Examples of the aromatic ring in the aromatic ring group of Ar include an aromatic hydrocarbon ring and an aromatic heterocyclic ring.
The aromatic hydrocarbon ring may be monocyclic or polycyclic. The number of ring member atoms is preferably 6 to 15 and more preferably 6 to 10.
Examples of the aromatic hydrocarbon ring include a benzene ring, a naphthalene ring, and an anthracene ring. Among these, a benzene ring or a naphthalene ring is preferable, and a benzene ring is more preferable.
The aromatic heterocyclic ring may be monocyclic or polycyclic. The number of ring member atoms is preferably 5 to 15.
Examples of the heterocyclic ring having aromaticity include a furan ring, a thiophene ring, a benzofuran ring, a benzothiophene ring, a dibenzofuran ring, a dibenzothiophene ring, a pyridine ring, an indole ring, a benzodiazole ring, and a carbazole ring.
The group (hereinafter, also referred to as a "substituent A1") having at least one selected from the group consisting of a hydrogen atom, a carbon atom, a halogen atom, and a heteroatom as $R_{4A}$ is not particularly limited, but is preferably a group having 0 to 24 carbon atoms.
The halogen atom is not particularly limited, and examples thereof include a fluorine atom, a chlorine atom, a bromine atom, and an iodine atom.
The heteroatom is not particularly limited, and examples thereof include a nitrogen atom, an oxygen atom, a sulfur atom, and a phosphorus atom.
The substituent A1 is not particularly limited, but for example, a hydrogen atom or a hydrocarbon group is preferable. The hydrocarbon group may have a heteroatom or a halogen atom.
m represents an integer of 1 to 5. m preferably represents an integer of 1 to 3, and more preferably represents 1 or 2.
n represents an integer of 0 to 5. n preferably represents an integer of 0 to 2, and more preferably represents 0 or 1.
In a case where n is 2 or more, a plurality of $R_{4A}$'s may be linked to each other to form a ring.
The ring formed by linking a plurality of $R_{4A}$'s may be a monocyclic ring or a polycyclic ring. Examples of the ring to be formed include an aromatic ring and an aliphatic hydrocarbon ring, and the number of ring member atoms of the ring to be formed is preferably 6 to 10.
In a case where X is a monovalent group (specifically, a group selected from the group consisting of -OH, $-SO_2-OH$, and -CO-OH), one or more hydrogen atoms included in $R_{4A}$ are removed to form a bonding site.
In a case where X is a divalent group (specifically, a group selected from the group consisting of -NH-, $-SO_2-NH-$, and -CO-NH-), the divalent group can constitute a partial structure in Formula (I), but one or more hydrogen atoms contained in $R_{4A}$ may be further removed to form a bonding site.
The hydrogen donating compound is preferably a compound having at least one partial structure represented by Formula (II) in the molecule, and may be a compound having at least two or more partial structures represented by Formula

(II) in the molecule.

The number of the partial structures represented by Formula (I) in the molecule is not particularly limited, but is preferably 1 to 5 and more preferably 1 to 3.

The hydrogen donating compound is preferably a compound having at least one partial structure represented by Formula (III) in the molecule.

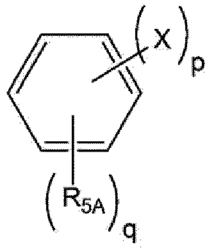

(III)

In Formula (III), X is a group selected from the group consisting of -OH, -NH-, $-SO_2-NH-$, $-SO_2-OH$, -CO-NH-, and -CO-OH.

$R_{5A}$ is a group having at least one selected from the group consisting of a hydrogen atom, a carbon atom, a halogen atom, and a heteroatom.

p represents an integer of 1 to 5. In a case where p represents an integer of 2 or more, a plurality of X's may be the same as or different from each other.

q represents an integer of 0 to 5. In a case where q represents an integer of 2 or more, a plurality of $R_5$'s may be the same or different from each other. In a case where q is 2 or more, a plurality of $R_{5A}$'s may be linked to each other to form a ring.

p + q is 6 or less.

In a case where all X's are monovalent groups, one or more hydrogen atoms included in $R_{5A}$ are removed to form a bond.

In a case where at least one X is a divalent group, one or more hydrogen atoms contained in $R_{5A}$ may be further removed to form a bond.

X has the same meaning as X in Formula (I), and the same applies to the preferred range thereof.

The group (hereinafter, also referred to as a "substituent A2") having at least one selected from the group consisting of a hydrogen atom, a carbon atom, a halogen atom, and a heteroatom as $R_{5A}$ is not particularly limited; however, it is preferably a group having 0 to 24 carbon atoms.

The halogen atom is not particularly limited, and examples thereof include a fluorine atom, a chlorine atom, a bromine atom, and an iodine atom.

The heteroatom is not particularly limited, and examples thereof include a nitrogen atom, an oxygen atom, a sulfur atom, and a phosphorus atom.

The substituent A2 is not particularly limited, but for example, a hydrogen atom or a hydrocarbon group is preferable. The hydrocarbon group may have a heteroatom or a halogen atom.

p represents an integer of 1 to 5. p preferably represents an integer of 1 to 3, and more preferably represents 1 or 2.

q represents an integer of 0 to 5. q preferably represents an integer of 0 to 2, and more preferably represents 0 or 1.

In a case where q is 2 or more, a plurality of $R_{5A}$'s may be linked to each other to form a ring.

The ring formed by linking a plurality of $R_{5A}$'s may be a monocyclic ring or a polycyclic ring. Examples of the ring to be formed include an aromatic ring and an aliphatic hydrocarbon ring, and the number of ring member atoms of the ring to be formed is preferably 6 to 10.

From the viewpoint of suppressing fading during exposure to a white lamp and ease of familiarity with the image-recording layer, the hydrogen donating group in the hydrogen donating compound is preferably a group selected from the group consisting of -OH, -NH-, - $SO_2$-NH-, and -CO-NH-, and more preferably a group selected from the group consisting of - OH and $-SO_2-NH-$.

From the viewpoint of suppressing fading during exposure to a white lamp, the hydrogen donating compound preferably contains at least one hydrogen donating group, more preferably at least two hydrogen donating groups, and still more preferably at least three hydrogen donating groups.

The number of hydrogen donating groups included in the hydrogen donating compound is not particularly limited, but is, for example, preferably 10 or less, more preferably 7 or less, and still more preferably 5 or less.

As a preferred aspect, the hydrogen donating compound is preferably a compound having at least two groups selected from the group consisting of -OH, -NH-, $-SO_2-NH-$, $-SO_2-OH$, -CO-NH-, and -CO-OH in the molecule.

As a preferred aspect, the hydrogen donating compound preferably has at least one group selected from the group consisting of -OH and $-SO_2-NH-$.

As a preferred aspect, the hydrogen donating compound preferably has at least two groups selected from the group consisting of -OH and $-SO_2-NH-$.

The hydrogen donating compound preferably has at least one phenolic hydroxyl group.

The phenolic hydroxyl group represents a hydroxyl group (-OH) directly connected to an aromatic ring (specifically, a benzene ring).

The hydrogen donating compound preferably has two or more phenolic hydroxyl groups.

The hydrogen donating compound preferably has two or more and six or less phenolic hydroxyl groups, and more preferably has two or more and four or less phenolic hydroxyl groups.

"Having two or more phenolic hydroxyl groups" may mean that an aromatic ring bonded to one phenolic hydroxyl group may further bond one or more phenolic hydroxyl groups, or an aromatic ring different from the aromatic ring bonded to one phenolic hydroxyl group may further bond one or more phenolic hydroxyl groups.

The hydrogen donating compound is preferably a compound represented by Formula (IV) or a compound represented by Formula (V).

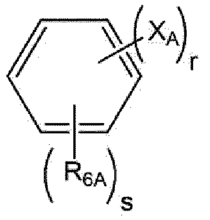

(IV)

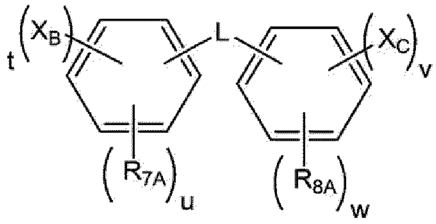

(V)

In Formula (IV), $X_A$ represents -OH.

$R_{6A}$ represents an organic group or a halogen atom.
r represents an integer of 1 to 5.
s represents an integer of 0 to 5. In a case where s represents an integer of 2 or more, a plurality of $R_{6A}$'s may be the same as or different from each other. In a case where s is 2 or more, a plurality of $R_{6A}$'s may be linked to each other to form a ring.

In Formula (V), L represents a single bond or a divalent linking group.

$X_B$ represents -OH.
$R_{7A}$ represents an organic group or a halogen atom.
$X_c$ represents -OH.
$R_{8A}$ represents an organic group or a halogen atom.
t represents an integer of 1 to 5.
u represents an integer of 0 to 5. In a case where u represents an integer of 2 or more, a plurality of $R_{7A}$'s may be the same or different from each other. In a case where u is 2 or more, a plurality of $R_{7A}$'s may be linked to each other to form a ring.
v represents an integer of 1 to 5.
w represents an integer of 0 to 5. In a case where w represents an integer of 2 or more, a plurality of $R_{5A}$'s may be the same or different from each other. In a case where w is 2 or more, a plurality of $R_{8A}$'s may be linked to each other to form a ring.

The organic group of $R_{6A}$, $R_{7A}$, and $R_{8A}$ is not particularly limited, and examples thereof include an alkyl group and an aryl group.

The alkyl group may be linear or branched, and examples thereof include an alkyl group having 1 to 10 carbon atoms.

The aryl group may be monocyclic or polycyclic, and examples thereof include an aryl group having 6 to 20 carbon atoms.

The halogen atom of $R_{6A}$, $R_{7A}$, and $R_{8A}$ is not particularly limited, and examples thereof include a fluorine atom, a

chlorine atom, a bromine atom, and an iodine atom.

r represents an integer of 1 to 5. r preferably represents an integer of 1 to 3, and more preferably represents 1 or 2.
s represents an integer of 0 to 5. s preferably represents an integer of 0 to 3, and more preferably represents 0 or 1.
r + s is preferably 6 or less.

The ring formed by linking a plurality of $R_{6A}$'s may be a monocyclic ring or a polycyclic ring. Examples of the ring to be formed include an aliphatic hydrocarbon ring, and the number of ring member atoms of the ring to be formed is preferably 6 to 10.
The divalent linking group of L is not particularly limited, and examples thereof include an alkylene group, -CO-, $-SO_2-$, -O-, and a combination thereof.
Examples of the alkylene group include a linear or branched alkylene group having 1 to 5 carbon atoms.
The alkylene group may further have a substituent. The substituent is not particularly limited, and examples thereof include an aryl group (preferably having 6 to 10 carbon atoms). The aryl group may further have a substituent, and examples of the further substituent include a hydroxyl group.

t represents an integer of 1 to 5. t preferably represents an integer of 1 to 3, and more preferably represents 1 or 2.
u represents an integer of 0 to 5. u preferably represents an integer of 0 to 3, and more preferably represents 0 or 1.
t + u is preferably 6 or less.

The ring formed by linking a plurality of $R_{7A}$'s may be a monocyclic ring or a polycyclic ring. Examples of the ring to be formed include an aliphatic hydrocarbon ring, and the number of ring member atoms of the ring to be formed is preferably 6 to 10.

v represents an integer of 1 to 5. v preferably represents an integer of 1 to 3, and more preferably represents 1 or 2.
w represents an integer of 0 to 5. w preferably represents an integer of 0 to 3, and more preferably represents 0 or 1.

It is preferable that v + w is 6 or less.
The ring formed by linking a plurality of $R_{8A}$'s may be a monocyclic ring or a polycyclic ring. Examples of the ring to be formed include an aliphatic hydrocarbon ring, and the number of ring member atoms of the ring to be formed is preferably 6 to 10.
The hydrogen donating compound is a low-molecular-weight compound having a molecular weight of less than 3,000, preferably a low-molecular-weight compound having a molecular weight of 1,500 or less, and more preferably a low-molecular-weight compound having a molecular weight of 1,000 or less.
Here, the low-molecular-weight compound in the present disclosure is not a so-called polymer or oligomer obtained by cleaving an unsaturated bond of a compound (so-called polymerizable monomer) having an unsaturated bond while using an initiator and growing a bond in a chain-like manner, but is a compound having a certain molecular weight of 3,000 or less (more preferably 2,000 or less and still more preferably 1,000 or less) (a compound having substantially no molecular weight distribution).
The molecular weight of the hydrogen donating compound is not particularly limited, but is preferably 50 or more, more preferably 100 or more, and still more preferably 200 or more.
In a preferred aspect, from the viewpoint of on-press developability and printing durability, the molecular weight is preferably 50 or more and 3,000 or less, more preferably 100 or more and 1,500 or less, and still more preferably 200 or more and 1,000 or less.
Specific examples of the hydrogen donating compound are shown below, but the present disclosure is not limited thereto.

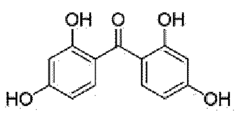
HD-1

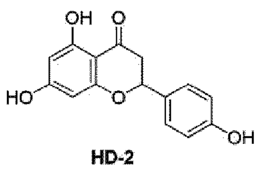
HD-2

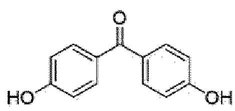
HD-3

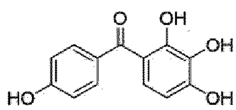
HD-4

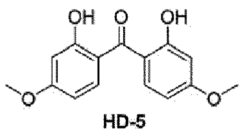
HD-5

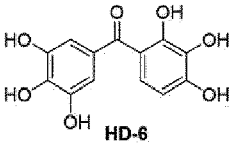
HD-6

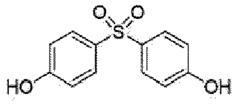
HD-7

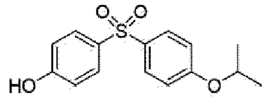
HD-8

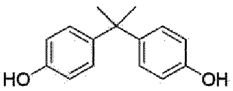
HD-9

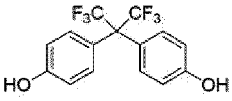
HD-10

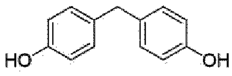
HD-11

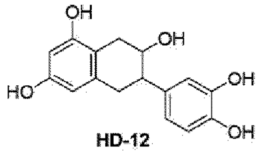
HD-12

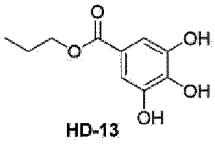
HD-13

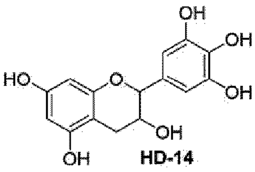
HD-14

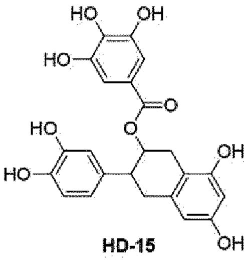
HD-15

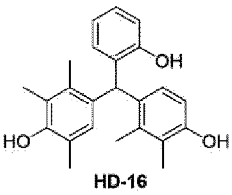
HD-16

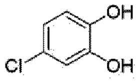
HD-17

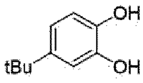
HD-18

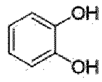
HD-19

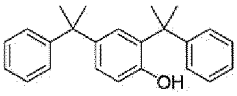
HD-20

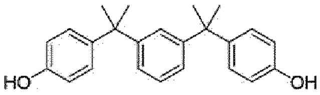
HD-21

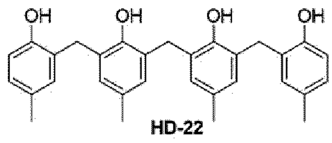
HD-22

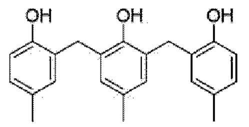
HD-23

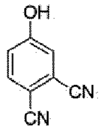
HD-24

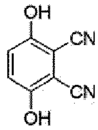
HD-25

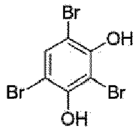
HD-26

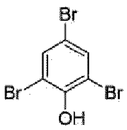
HD-27

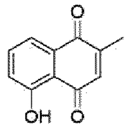
HD-28

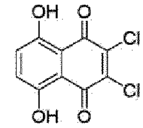
HD-29

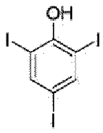
HD-30

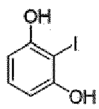
HD-31

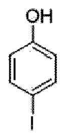
HD-32

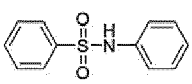
HD-33
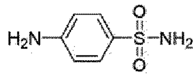
HD-34
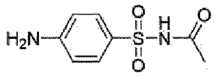
HD-35
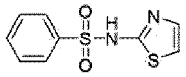
HD-36

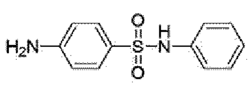
HD-37
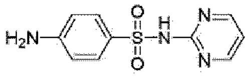
HD-38
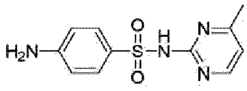
HD-39
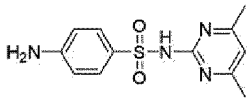
HD-40

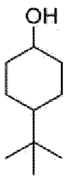
HD-41
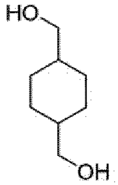
HD-42
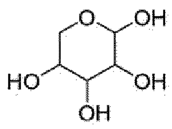
HD-43
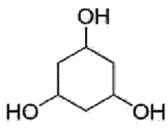
HD-44
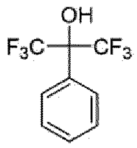
HD-45

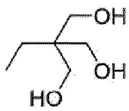
HD-46
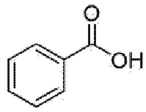
HD-47
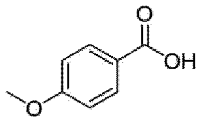
HD-48
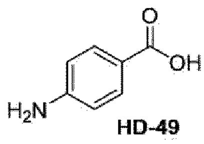
HD-49

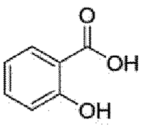
HD-51
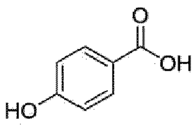
HD-52
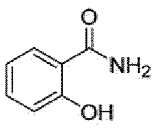
HD-53
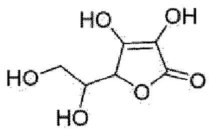
HD-58

The hydrogen donating compound may be used alone or in combination of two or more kinds thereof.

The content of the hydrogen donating compound with respect to the total mass of the image-recording layer is preferably 0.5% by mass to 30% by mass, more preferably 1% by mass to 20% by mass, and still more preferably 1% by mass to 10% by mass.

Binder polymer

As the binder polymer, a polymer having film forming properties is preferable, and preferred examples thereof include a (meth)acrylic resin, a polyvinyl acetal resin, a polyurethane resin, and the like.

As the binder polymer used in the image-recording layer, a binder polymer having an alkylene oxide chain is preferable.

In addition, examples of the binder polymer include the polymers described in paragraphs "0161" to "0173" of JP2023-20769A.

The molecular weight of the binder polymer that is a polystyrene-equivalent weight-average molecular weight (Mw) determined by GPC is preferably 2,000 or more, more preferably 5,000 or more, and still more preferably 10,000 to 300,000.

As necessary, a hydrophilic polymer such as polyacrylic acid or polyvinyl alcohol described in JP2008-195018A can be used in combination. In addition, a lipophilic polymer and a hydrophilic polymer can be used in combination.

One binder polymer may be used alone, or two or more binder polymers may be used in combination.

The content of the binder polymer is preferably 1% by mass to 90% by mass, and more preferably 5% by mass to 80% by mass with respect to the total mass of the image recording layer.

Polymer particles

It is preferable that the image-recording layer contain polymer particles. The polymer particles contribute to the

improvement of on-press developability. The polymer particles are preferably polymer particles that can convert the image-recording layer into hydrophobic when heat is applied thereto. The polymer particles are preferably at least one kind of particles selected from hydrophobic thermoplastic polymer particles, thermal reactive polymer particles, polymer particles having a polymerizable group, microcapsules encapsulating a hydrophobic compound, and a microgel (crosslinked polymer particles).

Examples of the polymer particles include polymer particles described in paragraphs "0177" to "0192" of JP2023-20769A and polymer particles described in paragraphs "0135" to "0172" of JP2023-179624A.

The average particle diameter of the polymer particles is preferably in a range of 0.01 μm to 3.0 μm, more preferably in a range of 0.05 μm to 2.0 μm, and particularly preferably in a range of 0.10 μm to 1.0 μm. In a case where the average particle diameter is in this range, excellent resolution and temporal stability are obtained.

One kind of polymer particles may be used alone, or two or more kinds of polymer particles may be used in combination.

The content of the polymer particles is preferably in a range of 5% by mass to 90% by mass, more preferably in a range of 5% by mass to 80% by mass, and still more preferably in a range of 10% by mass to 75% by mass with respect to the total mass of the image recording layer.

Chain transfer agent

The chain transfer agent contributes to the improvement of printing durability of the lithographic printing plate prepared from the lithographic printing plate precursor.

As the chain transfer agent, a thiol compound is preferable, a thiol having 7 or more carbon atoms is more preferable from the viewpoint of boiling point (low volatility), and a compound having a mercapto group on an aromatic ring (aromatic thiol compound) is still more preferable. The thiol compound is preferably a monofunctional thiol compound.

Specifically, suitable examples of the chain transfer agent include those described in WO2020/262692A.

One chain transfer agent may be used alone, or two or more chain transfer agents may be used in combination.

The content of the chain transfer agent with respect to the total mass of the image-recording layer is preferably 0.01% by mass to 50% by mass, more preferably 0.05% by mass to 40% by mass, and still more preferably 0.1% by mass to 30% by mass.

Sensitization agent

The oil sensitization agent contributes to the improvement of ink receptivity (hereinafter, also simply called "receptivity") of the lithographic printing plate prepared from the lithographic printing plate precursor. Examples of the oil sensitization agent include a phosphonium compound, a nitrogen-containing low-molecular-weight compound, an ammonium group-containing polymer, and the like. Particularly, in a case where the lithographic printing plate precursor has a protective layer containing an inorganic lamellar compound, these compounds function as a surface coating agent for the inorganic lamellar compound and can inhibit the receptivity deterioration caused in the middle of printing by the inorganic lamellar compound.

As the oil sensitization agent, it is preferable to use a phosphonium compound, a nitrogen-containing low-molecular-weight compound, and an ammonium group-containing polymer in combination, and it is more preferable to use a phosphonium compound, quaternary ammonium salts, and an ammonium group-containing polymer in combination.

As the oil sensitizing agent, those described in paragraphs "0151" to "0155" of WO2020/137919A can be used.

The content of the oil sensitizing agent is preferably in a range of 0.01% by mass to 30% by mass, more preferably in a range of 0.1% by mass to 15% by mass, and still more preferably in a range of 1% by mass to 10% by mass with respect to the total mass of the image recording layer.

Other components

As other components, a surfactant, a printing-out agent, a polymerization inhibitor, a higher fatty acid derivative, a plasticizer, inorganic particles, an inorganic lamellar compound, and the like can be contained in the image-recording layer. Specifically, the above components described in paragraphs "0114" to "0159" of JP2008-284817A can be used.

The content of the non-particle-shaped polymer compound having a weight-average molecular weight of more than 15,000 is preferably 5% by mass or less and more preferably 4% by mass or less with respect to the total mass of the image-recording layer.

Formation of image-recording layer

The image-recording layer can be formed, for example, by preparing a coating liquid by appropriately dispersing

or dissolving the necessary components described above in a known solvent, performing coating with the coating liquid by a known method such as bar coating, and drying the coating liquid, as described in paragraphs "0142" and "0143" of JP2008-195018A. The coating amount (solid content) of the image-recording layer after coating and drying varies with uses. However, from the viewpoint of obtaining excellent sensitivity and excellent film characteristics of the image-recording layer, the coating amount is preferably about 0.3 to 3.0 g/$m^2$.

From the viewpoint of imparting on-press developability, the image-recording layer preferably has water solubility or water-dispersibility. Here, the "water soluble" means that 0.1 g or more of compound is dissolved in 100 g of water at 20°C, and the "water-dispersible" means that the compound is uniformly dispersed in water at 20°C.

Support

As the support, an aluminum support is preferable. The aluminum plate used in the aluminum support consists of a dimensionally stable metal containing aluminum as a main component, that is, aluminum or an aluminum alloy. It is preferable that the aluminum plate is selected from a pure aluminum plate and an alloy that contains aluminum as a main component and traces of foreign elements.

The foreign elements contained in the aluminum alloy include silicon, iron, manganese, copper, magnesium, chromium, zinc, bismuth, nickel, titanium, and the like. The content of the foreign elements in the alloy is 10% by mass or less. As the aluminum plate, a pure aluminum plate is suitable. However, the aluminum plate may be an alloy that contains traces of foreign elements, because it is difficult to manufacture perfectly pure aluminum by smelting technologies. The composition of the aluminum plate used for the aluminum support is not specified, and it is possible to appropriately use aluminum plates known in the related art, for example, JIS A 1050, JIS A 1100, JIS A 3103, and JIS A 3005, and the like.

The thickness of the support (preferably, the aluminum plate) is preferably about 0.1 mm to 0.6 mm.

Anodic oxide film

The support preferably has an anodic oxide film.

The anodic oxide film means an anodic oxide film (preferably an anodic aluminum oxide film) which is formed on the surface of a support (preferably an aluminum plate) by an anodization treatment and has supermicropores (also called micropores). The micropores extend from the surface of the anodic oxide film on the side opposite to the support along the thickness direction (support side, depth direction).

From the viewpoints of tone reproducibility, printing durability, and stain resistance, the average diameter (average opening diameter) of the micropores within the surface of the anodic oxide film is preferably 7 nm to 150 nm, more preferably 10 nm to 100 nm, still more preferably 10 nm to 60 nm, particularly preferably 15 nm to 60 nm, and most preferably 18 nm to 40 nm.

The depth of the micropores is preferably 10 nm to 3,000 nm, more preferably 10 nm to 2,000 nm, and still more preferably 10 nm to 1,000 nm.

Usually, the micropores have a substantially straight tubular shape (substantially cylindrical shape) in which the diameter of the micropores substantially does not change in the depth direction (thickness direction). The micropores may also have a conical shape that continues to taper in the depth direction (thickness direction). In addition, the diameter of the micropores may have a shape that discontinuously tapers along the depth direction (thickness direction).

Examples of the micropores having a shape that discontinuously tapers in the depth direction (thickness direction) include micropores each configured with a large diameter portion that extends along the depth direction from the surface of the anodic oxide film and a small diameter portion that is in communication with the bottom portion of the large diameter portion and extends along the depth direction from the communicate position.

Specifically, the micropores are preferable which are each configured with a large diameter portion that extends 10 nm to 1,000 nm in the depth direction from the surface of the anodic oxide film and a small diameter portion that is in communication with the bottom portion of the large diameter portion and further extends 20 to 2,000 nm in the depth direction from the communicate position.

The depth of the micropores is not particularly limited, but from the viewpoint of achieving both on-press developability and printing durability, the depth of the micropores is preferably 0.01 $\mu$m to 1 $\mu$m, more preferably 0.05 $\mu$m to 0.6 $\mu$m, and still more preferably 0.07 $\mu$m to 0.25 $\mu$m. The depth of the micropores means a distance in a depth direction from the surface of the coating film of the micropores to the deepest portion of the bottom portion of the micropores.

The density of the micropores on the surface of the coating film is not particularly limited, but from the viewpoint of achieving both on-press developability and printing durability, is preferably 200 pores/$\mu m^2$ to 2,000 pores/$\mu m^2$ and more preferably 400 pores/$\mu m^2$ to 1,500 pores/$\mu m^2$.

The density of the micropores is a value obtained by observing the surface of the coating film with a field emission scanning electron microscope (FE-SEM) at a magnification of 150,000 times, by randomly selecting a measurement region of 400 nm $\times$ 600 nm in four images obtained by observing different four locations, by measuring the number of

micropores present in the measurement region, by calculating the number of micropores per area of the measurement region for each image, and by arithmetically averaging the calculated values.

From the viewpoint of achieving both on-press developability and printing durability, the opening ratio due to the micropores on the surface of the coating film is preferably in a range of 10% to 90% and more preferably in a range of 30% to 85%.

The opening ratio is a value obtained by converting, into a percentage, a value obtained by multiplying an average area of the opening portion by the large-diameter pores of the micropores on the surface of the coating film, which is calculated by dividing the average diameter of the large-diameter pores of the micropores on the surface of the coating film by 2, by the density (number density) of the micropores on the surface of the coating film.

Hereinafter, the large diameter portion and the small diameter portion will be specifically described.

Large diameter portion

From the viewpoints of tone reproducibility, printing durability, and stain resistance, the average diameter (average opening diameter) of the large diameter portion within the surface of the anodic oxide film is preferably 7 nm to 150 nm, more preferably 10 nm to 100 nm, still more preferably 15 nm to 100 nm, particularly preferably 15 nm to 60 nm, and most preferably 18 nm to 40 nm.

The average diameter of the large diameter portion is calculated by a method of observing the surface of the anodic oxide film with a field emission scanning electron microscope (FE-SEM) at 150,000X magnification (N = 4), by randomly selecting 50 micropores (large diameter portions) existing in a range of 400 nm × 600 nm in the obtained 4 images, by measuring the sizes (diameters), and by calculating the arithmetic mean.

In a case where the shape of the large diameter portion is not circular, the equivalent circle diameter is used. "Equivalent circular diameter" is a diameter determined on an assumption that the opening portion is in the form of a circle having the same projected area as the projected area of the opening portion.

The bottom portion of the large diameter portion is preferably in a position at a depth of 70 nm to 1,000 nm (hereinafter, also called depth A) from the surface of the anodic oxide film. That is, the large diameter portion is preferably a pore portion extending to a position at a depth of 70 nm to 1,000 nm from the surface of the anodic oxide film in the depth direction (thickness direction). Particularly, from the viewpoint of further improving the effect of the manufacturing method of a lithographic printing plate precursor, the depth A is more preferably 90 nm to 850 nm, still more preferably 90 nm to 800 nm, and particularly preferably 90 nm to 600 nm.

The depth is a value obtained by taking a photograph (150,000X magnification) of a cross section of the anodic oxide film, measuring the depths of 25 or more large diameter portions, and calculating the arithmetic mean thereof.

The shape of the large diameter portion is not particularly limited. Examples of the shape of the large diameter portion include a substantially straight tubular shape (substantially cylindrical shape) and a conical shape that tapers along the depth direction (thickness direction). Among these, a substantially straight tubular shape is preferable. The shape of the bottom portion of the large diameter portion is not particularly limited, and may be a curved (convex) or planar shape.

The inner diameter of the large diameter portion is not particularly limited, but is preferably as large as the diameter of the opening portion or smaller than the diameter of the opening portion. There may be a difference of about 1 nm to 10 nm between the inner diameter of the large diameter portion and the diameter of the opening portion.

Small diameter portion

The small diameter portion is a pore portion that is in communication with the bottom portion of the large diameter portion and further extends from the communicate position in the depth direction (thickness direction). Generally, one small diameter portion is in communication with one large diameter portion. However, two or more small diameter portions may be in communication with the bottom portion of one large diameter portion.

The average diameter of the small diameter portion at the communicate position is preferably 15 nm or less, more preferably 13 nm or less, still more preferably 11 nm or less, and particularly preferably 10 nm or less. The lower limit thereof is not particularly limited, but is preferably 5 nm.

The average diameter of the small diameter portion is obtained by observing the surface of the anodic oxide film with FE-SEM at 150,000X magnification (N = 4), by randomly selecting 50 micropores (small diameter portion) existing in a range of 400 nm × 600 nm in the obtained 4 images, by measuring the sizes (diameters), and by calculating the arithmetic mean of the diameters. In a case where the large diameter portion is deep, as necessary, the upper portion of the anodic oxide film (region where the large diameter portion is located) may be cut (for example, cut by using argon gas), then the surface of the anodic oxide film may be observed with FE-SEM described above, and the average diameter of the small diameter portion may be determined.

In a case where the shape of the small diameter portion is not circular, the equivalent circle diameter is used. "Equivalent circular diameter" is a diameter determined on an assumption that the opening portion is in the form of a circle

having the same projected area as the projected area of the opening portion.

The bottom portion of the small diameter portion is preferably in a position 20 nm to 2,000 nm distant from the communicate position (corresponding to the depth A described above) with the large diameter portion in the depth direction. In other words, the small diameter portion is a pore portion that extends further from the communicate position with the large diameter portion in the depth direction (thickness direction), and the depth of the small diameter portion is preferably 20 nm to 2,000 nm, more preferably 100 nm to 1,500 nm, and particularly preferably 200 nm to 1,000 nm.

The depth is a value obtained by taking a photograph (150,000X magnification) of a cross section of the anodic oxide film, measuring the depths of 25 or more small diameter portions, and calculating the arithmetic mean thereof.

The shape of the small diameter portion is not particularly limited. Examples of the shape of the small diameter portion include a substantially straight tubular shape (substantially cylindrical shape) and a conical shape that tapers along the depth direction. Among these, a substantially straight tubular shape is preferable. In addition, the shape of the bottom portion of the small diameter portion is not particularly limited, and may be a curved (convex) or planar shape.

The inner diameter of the small diameter portion is not particularly limited, and may be the same as the diameter at the communicate position, or may be smaller or larger than the diameter at the communicate position. Generally, there may be a difference of about 1 nm to 10 nm between the inner diameter of the small diameter portion and the diameter of the opening portion.

The ratio of the average diameter of the large diameter portion within the surface of the anodic oxide film to the average diameter of the small diameter portion at the communicate position, (average diameter of large diameter portion within surface of anodic oxide film)/(average diameter of small diameter portion at communicate position) is preferably 1.1 to 13, and more preferably 2.5 to 6.5.

The ratio of the depth of the large diameter portion to the depth of the small diameter portion, (depth of large diameter portion)/(depth of small diameter portion) is preferably 0.005 to 50, and more preferably 0.025 to 40.

The micropores have a substantially straight tubular shape (substantially cylindrical shape) in which the diameter of the micropores substantially does not change in the depth direction (thickness direction). The micropores may also have a conical shape that continues to widen in the depth direction (thickness direction). The micropores may have a shape that discontinuously widens along the depth direction (thickness direction).

Examples of the micropores having a shape that discontinuously widens in the depth direction (thickness direction) include micropores each configured with a small diameter portion that extends along the depth direction from the surface of the anodic oxide film and a large diameter portion that is in communication with the bottom portion of the small diameter portion and extends along the depth direction from the communicate position.

Specifically, the micropores are preferable which are each configured with a small diameter portion that extends 10 nm to 1,000 nm in the depth direction from the surface of the anodic oxide film and a large diameter portion that is in communication with the bottom portion of the small diameter portion and further extends 20 nm to 2,000 nm in the depth direction from the communicate position.

In addition, the small diameter portion and the large diameter portion shown below are also suitably used.

The average diameter (average opening diameter) of the small diameter portion within the surface of the anodic oxide film is not particularly limited, but is preferably 35 nm or less, more preferably 25 nm or less, and particularly preferably 20 nm or less.

The lower limit thereof is not particularly limited, but is preferably 15 nm.

The average diameter of the small diameter portion is calculated by a method of observing the surface of the anodic oxide film with a field emission scanning electron microscope (FE-SEM) at 150,000X magnification (N = 4), by randomly selecting 50 micropores (small diameter portions) existing in a range of 400 nm × 600 nm in the obtained 4 images, by measuring the sizes (diameters), and by calculating the arithmetic mean of the diameters.

In a case where the shape of the small diameter portion is not circular, the equivalent circle diameter is used. "Equivalent circular diameter" is a diameter determined on an assumption that the opening portion is in the form of a circle having the same projected area as the projected area of the opening portion.

The bottom portion of the small diameter portion is preferably in a position at a depth of 70 nm to 1,000 nm (hereinafter, also called depth A') from the surface of the anodic oxide film. That is, the small diameter portion is preferably a pore portion extending 70 nm to 1,000 nm in the depth direction (thickness direction) from the surface of the anodic oxide film.

The depth is a value obtained by taking a photograph (150,000X magnification) of a cross section of the anodic oxide film, measuring the depths of 25 or more small diameter portions, and calculating the arithmetic mean thereof.

The shape of the small diameter portion is not particularly limited. Examples of the shape of the small diameter portion include a substantially straight tubular shape (substantially cylindrical shape) and a conical shape that widens along the depth direction (thickness direction). Among these, a substantially straight tubular shape is preferable. In addition, the shape of the bottom portion of the small diameter portion is not particularly limited, and may be a curved (convex) or planar shape.

The inner diameter of the small diameter portion is not particularly limited, but is preferably as large as the

diameter of the opening portion or smaller than the diameter of the opening portion. There may be a difference of about 1 nm to 10 nm between the inner diameter of the small diameter portion and the diameter of the opening portion.

The large diameter portion is a pore portion that is in communication with the bottom portion of the small diameter portion and further extends from the communicate position in the depth direction (thickness direction). Generally, one large diameter portion may be in communication with the bottom portion of two or more small diameter portions.

The average diameter of the large diameter portion at the communicate position is preferably 20 nm to 400 nm, more preferably 40 nm to 300 nm, still more preferably 50 nm to 200 nm, and particularly preferably 50 nm to 100 nm.

The average diameter of the large diameter portion is obtained by observing the surface of the anodic oxide film with FE-SEM at 150,000X magnification (N = 4), by randomly selecting 50 micropores (large diameter portion) existing in a range of 400 nm × 600 nm in the obtained 4 images, by measuring the sizes (diameters), and by calculating the arithmetic mean of the diameters. In a case where the small diameter portion is deep, as necessary, the upper portion of the anodic oxide film (region where the small diameter portion is located) may be cut (for example, cut by using argon gas), then the surface of the anodic oxide film may be observed with FE-SEM described above, and the average diameter of the large diameter portion may be determined.

In a case where the shape of the large diameter portion is not circular, the equivalent circle diameter is used. "Equivalent circular diameter" is a diameter determined on an assumption that the opening portion is in the form of a circle having the same projected area as the projected area of the opening portion.

The bottom portion of the large diameter portion is preferably in a position 20 nm to 2,000 nm distant from the communicate position (corresponding to the depth A' described above) with the small diameter portion in the depth direction. In other words, the large diameter portion is a pore portion that extends further from the communicate position with the small diameter portion in the depth direction (thickness direction), and the depth of the large diameter portion is preferably 20 nm to 2,000 nm, more preferably 100 nm to 1,500 nm, and particularly preferably 200 nm to 1,000 nm.

The depth is a value obtained by taking a photograph (150,000X magnification) of a cross section of the anodic oxide film, measuring the depths of 25 or more large diameter portions, and calculating the arithmetic mean thereof.

The shape of the large diameter portion is not particularly limited. Examples of the shape of the large diameter portion include a substantially straight tubular shape (substantially cylindrical shape) and a conical shape that tapers along the depth direction. Among these, a substantially straight tubular shape is preferable. The shape of the bottom portion of the large diameter portion is not particularly limited, and may be a curved (convex) or planar shape.

The inner diameter of the large diameter portion is not particularly limited, and may be the same as the diameter at the communicate position, or may be smaller or larger than the diameter at the communicate position. Generally, there may be a difference of about 1 nm to 10 nm between the inner diameter of the large diameter portion and the diameter of the opening portion.

It is preferable that the support has an anodic oxide film, and the anodic oxide film has, in order from a surface of the anodic oxide film in a depth direction, an upper layer having micropores with an average diameter of 20 to 100 nm and a thickness of 30 to 500 nm, a middle layer having micropores with an average diameter of 1/2 to 5 times the average diameter of the micropores in the upper layer and a thickness of 100 to 300 nm, and a lower layer having micropores with an average diameter of 15 nm or less and a thickness of 300 to 2000 nm.

Si atomic weight

From the viewpoint of printing durability, in the lithographic printing plate precursor according to the present disclosure, an average value of Si atomic amounts (hereinafter, also referred to as "specific Si atomic amount") calculated by measuring a circular region having a diameter of 30 mm in a surface of the anodic oxide film of the support on the image recording layer side by fluorescence X-ray analysis is preferably 0.008 mg to 0.14 mg.

From the viewpoint of printing durability, it is preferable that the lithographic printing plate precursor according to the embodiment of the present disclosure has a specific structure in which micropores formed in the anodic oxide film of the support have a depth of 0.05 μm to 0.5 μm from the surface of the film, and the average diameter d1 of the large diameter portions in the surface of the film is 0.015 μm to 0.1 μm, the specific structure consisting of the large diameter portions and the small diameter portions in which the small diameter portions communicate with the bottom portions of the large diameter portions.

In the above-described aspect, on the surface of the anodic oxide film in which the micropores having the specific structure are formed, there is a possibility that not only the image-recording layer is simply stacked on the surface of the appearance, but also a part of the image-recording layer enters the inside of each micropore. Therefore, in the anodic oxide film in which the micropores having the specific structure are formed on the surface, the area in which the image-recording layer is substantially in contact with the surface of the support is different from that in the anodic oxide film in which the micropores are not formed on the surface, and thus it is considered that the appropriate range of the Si amount with respect to the apparent surface is different.

The specific Si atomic amount is obtained by performing the fluorescence X-ray analysis on a circular region

having a diameter of 30 mm on the surface of the coating film, measuring the intensity of the Kα ray of the Si element, and then quantifying the Si atomic amount present on the surface of the coating film using a calibration curve. Here, the "average value of Si atomic weights" means a numerical value obtained by selecting three or more circular regions that do not overlap each other on the surface of the anodic oxide film on the image-recording layer side, obtaining the Si atomic weight for each region, and arithmetically averaging the obtained Si atomic weights of each of the regions.

Details of the method for measuring the specific Si atom amount by the fluorescence X-ray analysis will be described in Examples described later.

From the viewpoint of the balance between on-press developability, ink removability, and printing durability, the specific Si atom amount per circular region having a diameter of 30 mm on the surface of the anodic oxide film is preferably 0.08 mg to 0.14 mg, more preferably 0.010 mg to 0.080 mg, and still more preferably 0.011 mg to 0.060 mg.

Examples of the method of adjusting the specific Si atom amount on the surface of the anodic oxide film within the above-described range in the support of the lithographic printing plate precursor include a method of adjusting the specific Si atom amount by changing any one of the concentration of the silicate, the temperature of the treatment liquid, or the treatment time in the silicate treatment which will be described later and which is performed on the formed anodic oxide film after the anodic oxide film having the micropores is formed on the aluminum plate.

In an on-press development type lithographic printing plate precursor, from the viewpoint of improving image visibility, a support is useful in which the surface of an anodic oxide film (surface on which an image-recording layer is to be formed) has high lightness.

Usually, in a printing step using a lithographic printing plate, before the printing plate is mounted on a printer, the plate is inspected to check whether an image is recorded as intended. For the on-press development type lithographic printing plate precursor, it is required to check the image at the stage where the image is exposed. Therefore, a means generating a so-called printed image in an image exposed portion is used.

Examples of a method of quantitatively evaluating the visibility of an image area (image visibility) of the on-press development type lithographic printing plate precursor having undergone exposure of an image include a method of measuring the lightness of an image exposed portion and the lightness of a non-exposed portion and calculating the difference therebetween. As the lightness, a value of lightness L* in the CIEL*a*b* color system can be used. The lightness can be measured using a color difference meter (Spectro Eye, manufactured by X-Rite, Incorporated.). The larger the difference between the measured lightness of the image exposed portion and the measured lightness of the non-exposed portion is, the higher the visibility of the image area is.

It has been revealed that the larger the value of lightness L* of the surface of the anodic oxide film in the CIEL*a*b* color system is, the more effective it is to increase the difference between the lightness of the image exposed portion and the lightness of the non-exposed portion. That is, the value of the lightness L* is preferably 60 to 100.

As necessary, the support having an anodic oxide film may have a backcoat layer containing the organic polymer compound described in JP1993-45885A (JP-H5-45885A), the alkoxy compound of silicon described in JP1994-35174A (JP-H6-35174A), or the like, on a surface opposite to the side where a constitutional layer containing a hydroxy acid compound having two or more hydroxyl groups is formed.

Manufacturing of aluminum support having anodic oxide film

The manufacturing method of an aluminum support having an anodic oxide film, which is an example of support, will be described.

The aluminum support having an anodic oxide film can be manufactured using known methods. The manufacturing method of the aluminum support having an anodic oxide film is not particularly limited. Examples of preferred embodiments of the manufacturing method of the aluminum support having an anodic oxide film include a method including a step of performing a roughening treatment on an aluminum plate (roughening treatment step), a step of anodizing the aluminum plate having undergone the roughening treatment (anodization treatment step), and a step of bringing the aluminum plate having an anodic oxide film obtained by the anodization treatment step into contact with an aqueous acid solution or an aqueous alkali solution to enlarge the diameter of micropores in the anodic oxide film (pore widening treatment step).

Hereinafter, each step will be described in detail.

Roughening treatment step

The roughening treatment step is a step of performing a roughening treatment including an electrochemical roughening treatment on the surface of the aluminum plate. The roughening treatment step is preferably performed before the anodization treatment step which will be described later. However, in a case where the surface of the aluminum plate already has a preferable shape, the roughening treatment step may be omitted.

As the roughening treatment, only an electrochemical roughening treatment may be performed, or an electrochemical roughening treatment may be performed in combination with at least either a mechanical roughening treatment or a chemical roughening treatment.

In a case where the mechanical roughening treatment and the electrochemical roughening treatment are combined, it is preferable to perform the electrochemical roughening treatment after the mechanical roughening treatment.

The electrochemical roughening treatment is preferably performed in an aqueous solution of nitric acid or hydrochloric acid.

Generally, the mechanical roughening treatment is performed such that the aluminum plate has a surface roughness Ra: 0.35 to 1.0 μm.

The conditions of the mechanical roughening treatment are not particularly limited. For example, the mechanical roughening treatment can be performed according to the method described in JP1975-40047B (JP-S50-40047B). The mechanical roughening treatment can be performed by a brush graining treatment using a pumice stone suspension or by a transfer method.

The chemical roughening treatment is also not particularly limited, and can be performed according to known methods.

After the mechanical roughening treatment, it is preferable to perform the following chemical etching treatment.

By the chemical etching treatment performed after the mechanical roughening treatment, the edge portion of surface irregularities of the aluminum plate smoothed, such that ink clotting that may occur during printing is prevented, the antifouling property of the lithographic printing plate is improved, and unnecessary substances such as abrasive particles remaining on the surface are removed.

As the chemical etching treatment, etching with an acid and etching with an alkali are known. One of the examples of particularly efficient etching methods is a chemical etching treatment using an alkaline solution (hereinafter, also called "alkaline etching treatment").

The alkaline agent used in the alkaline solution is not particularly limited. Suitable examples thereof include caustic soda, caustic potash, sodium metasilicate, sodium carbonate, sodium aluminate, sodium gluconate, and the like.

The alkaline solution may contain aluminum ions. The concentration of the alkaline agent in the alkaline solution is preferably 0.01% by mass or more and more preferably 3% by mass or more. In addition, the concentration is preferably 30% by mass or less and more preferably 25% by mass or less.

The temperature of the alkaline solution is preferably equal to or more than room temperature and more preferably 30°C or more. In addition, the temperature is preferably 80°C or less and more preferably 75°C or less.

The etching amount is preferably 0.01 $g/m^2$ or more, and more preferably 0.05 $g/m^2$ or more. In addition, the etching amount is preferably 30 $g/m^2$ or less, and more preferably 20 $g/m^2$ or less.

The treatment time preferably is in a range of 2 seconds to 5 minutes depending on the etching amount. In view of improving productivity, the treatment time is more preferably 2 seconds to 10 seconds.

In a case where the alkaline etching treatment is performed after the mechanical roughening treatment, in order to remove products generated by the alkaline etching treatment, the chemical etching treatment by using a low-temperature acidic solution (hereinafter, also called "desmutting treatment") is preferably performed.

The acid used in the acidic solution is not particularly limited, and examples thereof include sulfuric acid, nitric acid, and hydrochloric acid. The concentration of the acidic solution is preferably 1% by mass to 50% by mass. The temperature of the acidic solution is preferably 20°C to 80°C. In a case where the concentration and temperature of the acidic solution are in this range, the lithographic printing plate using an aluminum support becomes more resistant to dot-like stain.

Examples of preferred aspects of the roughening treatment step are as below.

Aspect SA

An aspect in which the following treatments (1) to (8) are performed in this order.

(1) Chemical etching treatment using alkaline aqueous solution (first alkaline etching treatment)
(2) Chemical etching treatment using acidic aqueous solution (first desmutting treatment)
(3) Electrochemical roughening treatment using aqueous solution containing nitric acid as main component (first electrochemical roughening treatment)
(4) Chemical etching treatment using alkaline aqueous solution (second alkaline etching treatment)
(5) Chemical etching treatment using acidic aqueous solution (second desmutting treatment)
(6) Electrochemical roughening treatment using aqueous solution containing hydrochloric acid as main component (second electrochemical roughening treatment)
(7) Chemical etching treatment using alkaline aqueous solution (third alkaline etching treatment)
(8) Chemical etching treatment using acidic aqueous solution (third desmutting treatment)

Aspect SB

An aspect in which the following treatments (11) to (15) are performed in this order.

(11) Chemical etching treatment using alkaline aqueous solution (fourth alkaline etching treatment)
(12) Chemical etching treatment using acidic aqueous solution (fourth desmutting treatment)
(13) Electrochemical roughening treatment using aqueous solution containing hydrochloric acid as main component (third electrochemical roughening treatment)
(14) Chemical etching treatment using alkaline aqueous solution (fifth alkaline etching treatment)
(15) Chemical etching treatment using acidic aqueous solution (fifth desmutting treatment)

As necessary, a mechanical roughening treatment may be performed before the treatment (1) of the aspect SA or before the treatment (11) of the aspect SB.

The amount of the aluminum plate dissolved by the first alkaline etching treatment and the fourth alkaline etching treatment is preferably 0.5 $g/m^2$ to 30 $g/m^2$ and more preferably 1.0 $g/m^2$ to 20 $g/m^2$.

Examples of the aqueous solution containing nitric acid as a main component used in the first electrochemical roughening treatment of the aspect SA include aqueous solutions used in the electrochemical roughening treatment using direct current or alternating current. Examples thereof include an aqueous solution obtained by adding aluminum nitrate, sodium nitrate, ammonium nitrate, or the like to a 1 g/L to 100 g/L aqueous nitric acid solution.

Examples of the aqueous solution containing hydrochloric acid as a main component used in the second electrochemical roughening treatment of the aspect SA and in the third electrochemical roughening treatment of the aspect SB include aqueous solutions used in the electrochemical roughening treatment using direct current or alternating current. Examples thereof include an aqueous solution obtained by adding 0 g/L to 30 g/L of sulfuric acid to a 1 g/L to 100 g/L aqueous hydrochloric acid solution. Nitrate ions such as aluminum nitrate, sodium nitrate, or ammonium nitrate; or chloride ions such as aluminum chloride, sodium chloride, or ammonium chloride may be further added to the aqueous solution.

As the waveform of an alternating current power source for the electrochemical roughening treatment, a sine wave, a square wave, a trapezoidal wave, a triangular wave, or the like can be used. The frequency is preferably 0.1 Hz to 250 Hz.

Fig. 1 is a graph showing an example of an alternating waveform current waveform diagram used for an electrochemical roughening treatment.

In Fig. 1, ta represents an anodic reaction time, tc represents a cathodic reaction time, tp represents the time taken for current to reach a peak from 0, Ia represents the peak current on the anodic cycle side, and Ic represents the peak current on the cathodic cycle side. For a trapezoidal wave, the time tp taken for current to reach a peak from 0 is preferably 1 msec to 10 msec. Regarding the conditions of one cycle of alternating current used for the electrochemical roughening treatment, a ratio tc/ta of the cathodic reaction time tc of the aluminum plate and the anodic reaction time ta of the aluminum plate is preferably within a range of 1 to 20, a ratio Qc/Qa of an electricity quantity Qc during the cathodic reaction to an electricity quantity Qa during the anodic reaction of the aluminum plate is preferably within a range of 0.3 to 20, and the anodic reaction time ta is preferably within a range of 5 msec to 1,000 msec. The peak current density value of the trapezoidal wave is preferably 10 to 200 $A/dm^2$ at both the anodic cycle side Ia and the cathodic cycle side Ic of the current. Ic/Ia is preferably 0.3 to 20. At a point in time when the electrochemical roughening treatment has ended, the total electricity quantity that participates in the anodic reaction of the aluminum plate is preferably 25 $C/dm^2$ to 1,000 $C/dm^2$.

The electrochemical roughening treatment using alternating current can be performed using the device shown in Fig. 2.

Fig. 2 is a lateral view showing an example of a radial cell in an electrochemical roughening treatment using alternating current.

In Fig. 2, 50 represents a main electrolytic cell, 51 represents an alternating current power source, 52 represents a radial drum roller, 53a and 53b represent main poles, 54 represents an electrolytic solution supply port, 55 represents an electrolytic solution, 56 represents a slit, 57 represents an electrolytic liquid channel, 58 represents an auxiliary anode, 60 represents an auxiliary anode cell, and W represents an aluminum plate. In a case where two or more electrolytic cells are used, the electrolysis conditions may be the same as or different from each other.

The aluminum plate W is wound around the radial drum roller 52 immersed and disposed in the main electrolytic cell 50. While being transported, the aluminum plate W is electrolyzed by the main poles 53a and 53b connected to the alternating current power source 51. From the electrolytic solution supply port 54, the electrolytic solution 55 is supplied to the electrolytic liquid channel 57 between the radial drum roller 52 and the main poles 53a and 53b through the slit 56. The aluminum plate W treated in the main electrolytic cell 50 is then electrolyzed in the auxiliary anode cell 60. In the auxiliary anode cell 60, the auxiliary anode 58 is disposed to face the aluminum plate W. The electrolytic solution 55 is supplied to flow in the space between the auxiliary anode 58 and the aluminum plate W.

In view of easily manufacturing a predetermined lithographic printing plate precursor, the amount of the aluminum plate dissolved by the second alkaline etching treatment is preferably 1.0 g/m$^2$ to 20 g/m$^2$ and more preferably 2.0 g/m$^2$ to 10 g/m$^2$.

In view of easily manufacturing a predetermined lithographic printing plate precursor, the amount of the aluminum plate dissolved by the third alkaline etching treatment and the fifth alkaline etching treatment is preferably 0.01 g/m$^2$ to 0.8 g/m$^2$ and more preferably 0.05 g/m$^2$ to 0.3 g/m$^2$.

In the chemical etching treatment (first to fifth desmutting treatments) using an acidic aqueous solution, an acidic aqueous solution containing phosphoric acid, nitric acid, sulfuric acid, chromic acid, hydrochloric acid, or a mixed acid consisting of two or more of these acids is suitably used.

The concentration of the acid in the acidic aqueous solution is preferably 0.5% by mass to 60% by mass.

Anodization treatment step

The anodization treatment step is a step of performing an anodization treatment on the aluminum plate having undergone the roughening treatment such that an aluminum oxide film is formed on the surface of the aluminum plate. By the anodization treatment, an anodic oxide film of aluminum, having micropores is formed on the surface of the aluminum plate.

The anodization treatment can be performed according to the method known in the field of the related art, by appropriately setting manufacturing conditions in consideration of the desired micropore shape.

In the anodization treatment step, an aqueous solution of sulfuric acid, phosphoric acid, oxalic acid, or the like can be mainly used as an electrolytic solution. In some cases, an aqueous solution or a non-aqueous solution of chromic acid, sulfamic acid, benzenesulfonic acid, or the like or an aqueous solution or a non-aqueous solution containing two or more acids among the above acids can also be used. In a case where direct current or alternating current is applied to the aluminum plate in the electrolytic solution, an anodic oxide film can be formed on the surface of the aluminum plate. The electrolytic solution may contain aluminum ions.

The content of the aluminum ions is not particularly limited, and is preferably 1 to 10 g/L.

The conditions of the anodization treatment are appropriately set depending on the electrolytic solution used. Generally, the concentration of the electrolytic solution of 1% by mass to 80% by mass (preferably 5% by mass to 20% by mass), the liquid temperature of 5°C to 70°C (preferably 10°C to 60°C), the current density of 0.5 A/dm$^2$ to 60 A/dm$^2$ (preferably 5 A/dm$^2$ to 50 A/dm$^2$), the voltage of 1 V to 100 V (preferably 5 V to 50 V), and the electrolysis time of 1 second to 100 seconds (preferably 5 seconds to 60 seconds) are appropriate ranges.

One of the preferred examples of the anodization treatment is the anodization method described in British Patent 1,412,768, which is performed in a sulfuric acid at a high current density.

The anodization treatment can also be performed multiple times. It is possible to change one or more of conditions, such as the type, concentration, and liquid temperature of the electrolytic solution used in each anodization treatment, the current density, the voltage, and the electrolysis time. In a case where the anodization treatment is performed twice, sometime the firstly performed anodization treatment is called first anodization treatment, and the secondly performed anodization treatment is called second anodization treatment. Performing the first anodization treatment and the second anodization treatment makes it possible to form anodic oxide films having different shapes and to provide a lithographic printing plate precursor having excellent printing performance.

Furthermore, it is also possible to perform the following pore widening treatment after the anodization treatment and then perform the anodization treatment again. In this case, the first anodization treatment, the pore widening treatment, and the second anodization treatment are performed.

Using the method of performing the first anodization treatment, the pore widening treatment, and the second anodization treatment makes it possible to form micropores each configured with a large diameter portion that extends from the surface of the anodic oxide film along the depth direction and a small diameter portion that is in communication with the bottom portion of the large diameter portion and extends from the communicate position along the depth direction.

Pore widening treatment step

The pore widening treatment step is a treatment of enlarging the diameter of micropores (pore diameter) present in the anodic oxide film formed by the anodization treatment step (pore diameter enlarging treatment). By the pore widening treatment, the diameter of the micropores is enlarged, and an anodic oxide film having micropores having a larger average diameter is formed.

The pore widening treatment can be carried out by bringing the aluminum plate obtained by the anodization treatment step into contact with an aqueous acid solution or an alkaline aqueous solution. The contact method is not particularly limited, and examples thereof include a dipping method and a spraying method. Among these, a dipping method is preferable.

In a case where an alkaline aqueous solution is used in the pore widening treatment step, it is preferable to use at least one alkaline aqueous solution selected from the group consisting of sodium hydroxide, potassium hydroxide, and lithium hydroxide. The concentration of the aqueous alkali solution is preferably 0.1% by mass to 5% by mass. As an appropriate treatment method, the pH of the aqueous alkali solution is adjusted to 11 to 13, and the aluminum plate is brought into contact with the aqueous alkali solution for 1 second to 300 seconds (preferably 1 second to 50 seconds) under the condition of 10°C to 70°C (preferably 20°C to 50°C). At this time, the alkaline treatment liquid may contain a metal salt of a polyvalent weak acid such as carbonate, borate, or phosphate.

In a case where an aqueous acid solution is used in the pore widening treatment step, it is preferable to use an aqueous solution of an inorganic acid such as sulfuric acid, phosphoric acid, nitric acid, or hydrochloric acid, or a mixture of these. The concentration of the aqueous acid solution is preferably 1% by mass to 80% by mass and more preferably 5% by mass to 50% by mass. It is appropriate that the aluminum plate is brought into contact with the acid aqueous solution for 1 second to 300 seconds (preferably in a range of 1 second to 150 seconds) under the condition that the liquid temperature of the acid aqueous solution is set to be in a range of 5°C to 70°C (preferably in a range of 10°C to 60°C).

The aqueous alkali solution or the aqueous acid solution may contain aluminum ions. The content of the aluminum ions is not particularly limited, but is preferably 1 g/L to 10 g/L.

The manufacturing method of the aluminum support having an anodic oxide film may include a hydrophilic treatment step of performing a hydrophilic treatment after the pore widening treatment step described above. As the hydrophilic treatment, it is possible to use the known method described in paragraphs "0109" to "0114" of JP2005-254638A.

The hydrophilic treatment is preferably performed by a method of immersing the aluminum plate in an aqueous solution of an alkali metal silicate such as sodium silicate or potassium silicate, a method of coating the aluminum plate with a hydrophilic vinyl polymer or a hydrophilic compound to form a hydrophilic undercoat layer, or the like.

The hydrophilic treatment using an aqueous solution of an alkali metal silicate such as sodium silicate or potassium silicate can be performed according to the method and procedure described in US2714066A and US3181461A.

Undercoat layer

The on-press development type lithographic printing plate precursor according to the present disclosure can include an undercoat layer (also referred to as an interlayer) between the image-recording layer and the support.

The undercoat layer enhances the adhesion between the support and the image-recording layer in an exposed portion, and enables the image-recording layer to be easily peeled from the support in a non-exposed portion. Therefore, the undercoat layer contributes to the improvement of developability without deteriorating printing durability. In addition, in the case of exposure to an infrared laser, the undercoat layer functions as a heat insulating layer and thus brings about an effect of preventing sensitivity reduction resulting from the diffusion of heat generated by exposure to the support.

Examples of compounds that are used in the undercoat layer include polymers having absorptive groups that can be absorbed onto the surface of the support and hydrophilic groups. In order to improve adhesiveness to the image-recording layer, it is preferable that polymers having absorptive groups and hydrophilic groups further have crosslinkable groups. The compounds that are used in the undercoat layer may be low-molecular-weight compounds or polymers. As necessary, as the compounds that are used in the undercoat layer, two or more compounds may be used by being mixed together.

In a case where the compound used in the undercoat layer is a polymer, a copolymer of a monomer having an absorptive group, a monomer having a hydrophilic group, and a monomer having a crosslinkable group is preferable.

As the absorptive group that can be absorbed onto the surface of the support, a phenolic hydroxy group, a carboxy group, $-PO_3H_2$, $-OPO_3H_2$, $-CONHSO_2-$, $-SO_2NHSO_2-$, and - $COCH_2COCH_3$ are preferable. As the hydrophilic groups, a sulfo group or salts thereof and salts of a carboxy group are preferable. As the crosslinkable groups, an acryloyl group, a methacryloyl group, an acrylamide group, a methacrylamide group, an allyl group, and the like are preferable.

The polymer may have a crosslinkable group introduced by the formation of a salt of a polar substituent of the polymer and a compound that has a substituent having charge opposite to that of the polar substituent and an ethylenically unsaturated bond, or may be further copolymerized with monomers other than the monomers described above and preferably with hydrophilic monomers.

Specifically, for example, silane coupling agents having addition polymerizable ethylenic double bond reactive groups described in JP1998-282679A (JP-H10-282679A) and phosphorus compounds having ethylenic double bond reactive groups described in JP1990-304441A (JP-H02-304441A) are suitable. The low-molecular-weight compounds or polymer compounds having crosslinkable groups (preferably ethylenically unsaturated bond groups), functional groups that interact with the surface of the support, and hydrophilic groups described in JP2005-238816A, JP2005-125749A, JP2006-239867A, and JP2006-215263A are also preferably used.

For example, the high-molecular-weight polymers having absorptive groups that can be adsorbed onto the

surface of the support, hydrophilic groups, and crosslinkable groups described in JP2005-125749A and JP2006-188038A are more preferable.

The content of ethylenically unsaturated bonding group in the polymer used in the undercoat layer is preferably 0.1 mmol to 10.0 mmol per gram of the polymer, and more preferably 0.2 mmol to 5.5 mmol per gram of the polymer.

The weight-average molecular weight (Mw) of the polymer used in the undercoat layer is preferably 5,000 or more and more preferably 10,000 to 300,000.

In order to prevent contamination with aging, the undercoat layer may contain, in addition to the compounds for the undercoat layer described above, a chelating agent, a secondary or tertiary amine, a polymerization inhibitor, a compound having an amino group or a functional group capable of inhibiting polymerization and a group that interacts with the surface of the support (for example, 1,4-diazabicyclo[2.2.2]octane (DABCO), 2,3,5,6-tetrahydroxy-p-quinone, chloranil, sulfophthalic acid, hydroxyethyl ethylenediaminetriacetic acid, dihydroxyethyl ethylenediaminediacetic acid, hydroxyethyl iminodiacetic acid, and the like), and the like.

The undercoat layer can be formed by coating by a known method, followed by drying. The coating amount (solid content) of the undercoat layer after drying is preferably 0.1 to 100 mg/$m^2$, and more preferably 1 to 30 mg/$m^2$.

Protective layer

The on-press development type lithographic printing plate precursor according to the present disclosure may or may not have a protective layer on the image-recording layer.

The protective layer has a function of suppressing the reaction inhibiting image formation by blocking oxygen, a function of preventing the damage of the image-recording layer, and a function of preventing ablation during exposure to high-illuminance lasers.

The protective layer having such characteristics is described, for example, in US3458311A and JP1980-49729B (JP-S55-49729B). As polymers with low oxygen permeability that are used in the protective layer, any of water-soluble polymers and water-insoluble polymers can be appropriately selected and used. If necessary, two or more such polymers can be used by being mixed together. Specific examples thereof include a polyvinyl alcohol resin (including polyvinyl alcohol and modified polyvinyl alcohol), polyvinylpyrrolidone, a water-soluble cellulose derivative, poly (meth)acrylonitrile, and the like.

As the polyvinyl alcohol, a polyvinyl alcohol having a saponification degree of 50% or more is suitable. The saponification degree of the polyvinyl alcohol is preferably 60% or more, more preferably 70% or more, and still more preferably 85% or more. The upper limit of the saponification degree is not particularly limited. The saponification degree may be 100% or less.

The saponification degree can be measured according to the method described in JIS K 6726:1994.

As the modified polyvinyl alcohol, acid-modified polyvinyl alcohol having a carboxy group or a sulfo group is preferably used. Specific examples thereof include the modified polyvinyl alcohols described in JP2005-250216A and JP2006-259137A.

Among water-soluble polymers, a polyvinyl alcohol resin is preferable.

In order to improve oxygen barrier properties, it is preferable that the protective layer contain an inorganic lamellar compound. The inorganic lamellar compound refers to particles in the form of a thin flat plate, and examples thereof include mica groups such as natural mica and synthetic mica, talc represented by Formula $3MgO\cdot 4SiO\cdot H_2O$, taeniolite, montmorillonite, saponite, hectorite, zirconium phosphate, and the like.

As the inorganic lamellar compound, a mica compound is preferably used. Examples of the mica compound include mica groups such as natural mica and synthetic mica represented by Formula: $A(B, C)_{2-5}D_4O_{10}(OH, F, O)_2$ [here, A represents any of K, Na, and Ca, B and C represent any of Fe (II), Fe (III), Mn, Al, Mg, and V, and D represents Si or Al.].

In the mica group, examples of the natural mica include muscovite, soda mica, phlogopite, biotite, and lepidolite. Examples of synthetic mica include non-swelling mica such as fluorophlogopite $KMg_3(AlSi_3O_{10})F_2$, potassium tetrasilic mica $KMg_{2.5}(Si_4O_{10})F_2$, and, Na tetrasilylic mica $NaMg_{2.5}(Si_4O_{10})F_2$, swelling mica such as Na or Li taeniolite (Na, Li) $Mg_2Li(Si_4O_{10})F_2$, montmorillonite-based Na or Li hectorite $(Na, Li)_{1/8}Mg_{2/5}Li_{1/8}(Si_4O_{10})F_2$, and the like. Furthermore, synthetic smectite is also useful.

Among the mica compounds, fluorine-based swelling mica is particularly useful. That is, swelling synthetic mica has a laminated structure consisting of unit crystal lattice layers having a thickness in a range of approximately 10 to 15Å, and metal atoms in lattices are more actively substituted than in any other clay minerals. As a result, positive charges are deficient in the lattice layers, and positive ions such as $Li^+$, $Na^+$, $Ca^{2+}$, and $Mg^{2+}$ are adsorbed between the layers in order to compensate for the deficiency. Positive ions interposed between the layers are referred to as exchangeable positive ions and are exchangeable with various positive ions. Particularly, in a case where the positive ions between the layers are $Li^+$ and $Na^+$, the ionic radii are small, and thus the bonds between lamellar crystal lattices are weak, and mica is significantly swollen by water. In a case where shearing is applied in this state, mica easily cleavages and forms a stable sol in water. Swelling synthetic mica is particularly preferably used because it clearly exhibits such a tendency.

From the viewpoint of diffusion control, regarding the shapes of the mica compounds, the thickness is preferably thin, and the planar size is preferably large as long as the smoothness and actinic ray-transmitting property of coated surfaces are not impaired. As a result, the aspect ratio is preferably 20 or more, more preferably 100 or more, and particularly preferably 200 or more. The aspect ratio is the ratio of the major diameter to the thickness of a particle, and it can be measured, for example, from a projection view obtained from the microphotograph of the particle. As the aspect ratio increases, the obtained effect is stronger.

Regarding the particle diameter of the mica compound, the average major diameter thereof is preferably 0.3 μm to 20 μm, more preferably 0.5 μm to 10 μm, and particularly preferably 1 μm to 5 μm. The average thickness of the particles is preferably 0.1 μm or less, more preferably 0.05 μm or less, and particularly preferably 0.01 μm or less. Specifically, for example, as a preferable aspect of swelling synthetic mica which is a representative compound, the thickness thereof is in a range of about 1 nm to 50 nm and the surface size (major diameter) is in a range of about 1 μm to 20 μm.

The content of the inorganic lamellar compound with respect to the total mass of the protective layer is preferably 0% by mass to 60% by mass and more preferably 3% by mass to 50% by mass. Even in a case where two or more inorganic lamellar compounds are used in combination, the total amount of the inorganic lamellar compounds preferable equals the content described above. In a case where the content is within the above range, the oxygen barrier property is improved, and excellent sensitivity is obtained. In addition, the deterioration of receptivity can be prevented.

The content of the inorganic lamellar compound is preferably 0.01 $mg/m^2$ to 100 $mg/m^2$, more preferably 5 $mg/m^2$ to 80 $mg/m^2$, and particularly preferably 5 $mg/m^2$ to 50 $mg/m^2$.

The protective layer may contain known additives such as a plasticizer for imparting flexibility, a surfactant for improving coating properties, and inorganic fine particles for controlling surface sliding properties. In addition, the oil sensitizing agent described above regarding the image-recording layer may be contained in the protective layer.

The protective layer can be formed by performing coating by a known method, followed by drying. The coating amount (solid content) of the protective layer after drying is preferably 0.01 $g/m^2$ to 10 $g/m^2$, more preferably 0.02 $g/m^2$ to 3 $g/m^2$, and particularly preferably 0.02 $g/m^2$ to 1 $g/m^2$.

The on-press development type lithographic printing plate precursor according to the present disclosure may have a bevel shape at an end portion.

Fig. 3 is a schematic view showing a cross-sectional shape of an end part of a lithographic printing plate precursor.

In Fig. 3, a lithographic printing plate precursor 1 has a bevel 2 at an end part thereof. A distance X between the upper edge of an edge surface 1c of the lithographic printing plate precursor 1 (boundary point between the bevel 2 and the edge surface 1c) and an intersection between an extension line of the edge surface 1c and an extension line of an image-recording layer surface (protective layer surface in a case where a protective layer is formed) 1a is called "bevel amount X". A distance Y between a point at which the image-recording layer surface 1a of the lithographic printing plate precursor 1 begins to b beveled and the aforementioned intersection is called "bevel width Y".

In the bevel shape of the end part, the bevel amount X is preferably 25 μm or more, more preferably 35 μm or more, and still more preferably 40 μm or more. From the viewpoint of preventing on-press developability deterioration resulting from the deterioration of surface condition of the end part, the upper limit of the bevel amount X is preferably 150 μm. In a case where on-press developability deteriorates, ink adheres to the residual image-recording layer, which sometimes leads to the occurrence of edge contamination. In a case where the bevel amount X is too small, the ink having adhered to the end part is likely to be transferred to a blanket, which sometimes leads to the occurrence of edge contamination. In a case where the bevel amount X is in a range of 25 μm to 150 μm, and the bevel width Y is small, the occurrence of cracks at the end part increases, and the printing ink is accumulated in the cracks, which sometimes leads to edge contamination. From such a viewpoint, the bevel width Y is preferably in a range of 70 μm to 300 μm and more preferably in a range of 80 μm to 250 μm. It should be noted that the ranges of the bevel amount and bevel width are not involved in the edge shape of a support surface 1b of the lithographic printing plate precursor 1.

Usually, at the end part of the lithographic printing plate precursor 1, bevels occur in a boundary B between the image-recording layer and the support and in the support surface 1b as in the image-recording layer surface 1a.

The end part having the bevel shape can be formed, for example, by adjusting the cutting conditions of the lithographic printing plate precursor.

Specifically, the beveled end part can be formed by adjusting the gap between an upper cutting blade and a lower cutting blade in a slitter device used for cutting the lithographic printing plate precursor, intrusion amounts of the blades, tip angle of the blades, and the like.

Fig. 4 is a conceptual view showing an example of a cutting portion of a slitter device. In the slitter device, a pair of upper and lower cutting blades 10 and 20 are vertically disposed. The cutting blades 10 and 20 consist of round blades on a disk. Upper cutting blades 10a and 10b are coaxially supported by a rotary shaft 11, and lower cutting blades 20a and 20b are coaxially supported by a rotary shaft 21. The upper cutting blades 10a and 10b and the lower cutting blades 20a and 20b rotate in opposite directions. A lithographic printing plate precursor 30 is passed between the upper cutting blades 10a and 10b and the lower cutting blades 20a and 20b and is cut in a predetermined width. By adjusting the gap between the upper cutting blade 10a and the lower cutting blade 20a and the gap between the upper cutting blade 10b and the lower

cutting blade 20b of the cutting portion of the slitter device, it is possible to form an end part having a bevel shape.

In the on-press development type lithographic printing plate precursor according to the embodiment of the present disclosure, the arithmetic mean height Sa of the outermost layer surface on the side opposite to the side where the image-recording layer is provided is preferably in a range of 0.3 μm to 20 μm. Here, the side opposite to the side where the image-recording layer is provided means the side opposite to the side where the image-recording layer is provided with reference to the support.

In the on-press development type lithographic printing plate precursor according to the present disclosure, it is preferable that an arithmetic average height Sa of an outermost layer surface on a side where the image-recording layer is provided is in a range of 0.3 μm to 20 μm. Here, the side where the image-recording layer is provided means the side where the image-recording layer is provided with reference to the support.

By providing the outermost layer surface having such characteristics, even in a case of being stacked in a form in which a interleaving paper is not interleaved between precursors (also referred to as elimination of interleaving paper), the on-press development type lithographic printing plate precursor according to the embodiment of the present disclosure is excellent in properties such as a property of preventing multiple plates from being fed in a step of taking out a precursor from a stack, a property of preventing scratches caused by a projection provided on the surface of the outermost layer of the precursor, and a property of preventing development delay caused by a projection provided on the surface of the outermost layer of the precursor.

In a case where the outermost layer surface on the side opposite to the side where the image-recording layer is provided has a backcoat layer on the opposite side, the outermost layer surface is the surface of the backcoat layer. In a case where the outermost layer surface has no layer on the opposite side, the outermost layer surface is the support surface.

For example, in a case where protrusions that will be described later are formed, the backcoat layer may be the outermost layer of the lithographic printing plate precursor, and a plurality of protrusions containing a polymer compound may be on the backcoat layer. Alternatively, the support may be the outermost layer, and a plurality of protrusions containing a polymer compound may be on the support.

In a case where an image-recording layer or a protective layer is the outermost layer, the outermost layer surface on the side where the image-recording layer is provided is the surface of the image-recording layer or the surface of the protective layer.

For example, in a case where protrusions that will be described later are formed, the image-recording layer or the protective layer may be the outermost layer of the lithographic printing plate precursor, and a plurality of protrusions containing a polymer compound may be on the image-recording layer or the protective layer.

The arithmetic average height Sa of the outermost layer surface on the side opposite to the side where the image recording layer is provided is more preferably in a range of 0.5 μm to 10 μm and still more preferably in a range of 0.5 μm to 7 μm.

The arithmetic average height Sa of the outermost layer surface on the side where the image recording layer is provided is more preferably in a range of 0.5 μm to 10 μm and still more preferably in a range of 0.5 μm to 7 μm.

The arithmetic mean height Sa of the outermost layer surface is measured according to the method described in ISO 25178. Specifically, the arithmetic mean height Sa is obtained by using MICROMAP MM3200-M100 (manufactured by Mitsubishi Chemical Systems, Inc.), selecting three or more sites from the same sample, performing the measurement, and averaging the obtained values. Regarding the measurement range, a range of 500 μm × 500 μm randomly selected from the sample surface is measured.

In order that the condition where the arithmetic mean height Sa of the outermost layer surface is 0.3 μm to 20 μm is satisfied, it is preferable that the outermost layer be in the form having irregularities.

Specifically, examples of aspects thereof include an aspect in which the outermost layer contains particles having an average particle diameter of 0.5 μm to 20 μm (aspect 1), and an aspect in which a plurality of protrusions containing a polymer compound as a main component is on the outermost layer (aspect 2). Here, the main component indicates a component whose content ratio (% by mass) is the highest.

In the aspect 1, the particles having an average particle diameter of 0.5 μm to 20 μm are not particularly limited, but are preferably at least one kind of particles selected from organic resin particles and inorganic particles.

As the particles, the particles described in paragraphs "0150" to "0166" of WO2022-138880A can be used.

In the aspect 1, the average particle diameter of the particles is preferably 0.5 μm to 10 μm and more preferably 0.5 μm to 5 μm.

The average particle diameter of particles means a volume average particle diameter. The volume average particle diameter is measured with a laser diffraction/scattering-type particle size distribution analyzer. Specifically, the measurement is performed using, for example, a particle size distribution measuring device "MICROTRAC MT-3300II" (manufactured by Nikkiso Co., Ltd.).

For other particles, unless otherwise specified, the average particle diameter is measured by the above measuring method.

In the aspect 1, an in-plane density of particles having an average particle diameter of 0.5 to 20 μm is preferably 10,000 particles/mm$^2$ or less. The in-plane density is more preferably 100 to 5,000 particles/mm$^2$, and still more preferably 100 to 3,000 particles/mm$^2$.

The in-plane density can be checked by observing the surface of the lithographic printing plate precursor with a scanning electron microscope (SEM). Specifically, five sites within the surface of the lithographic printing plate precursor re observed with a scanning electron microscope (SEM), the number of particles is counted and converted into the number of particles per area (mm$^2$) of the visual field of observation, and the average value thereof is calculated. In this way, the in-plane density can be determined.

It is preferable that the outermost layer on the side opposite to the side where the image-recording layer is provided contain a binder, in addition to the particles having an average particle diameter of 0.5 to 20 μm.

The binder preferably contains at least one compound selected from the group consisting of a novolac or resol resin such as a phenolformaldehyde resin, an m-cresol formaldehyde resin, a p-cresol formaldehyde resin, an m-/p-mixed cresol formaldehyde resin, or a phenol/cresol (which may be any of m-, p-, or m-/p-mixed) mixed formaldehyde resin, pyrogallol, an acetone resin, an epoxy resin, a saturated copolymerized polyester resin, a phenoxy resin, a polyvinyl acetal resin, a vinylidene chloride copolymer resin, polybutene, polybutadiene, polyamide, an unsaturated copolymerized polyester resin, polyurethane, polyurea, polyimide, polysiloxane, polycarbonate, an epoxy resin, chlorinated polyethylene, an aldehyde condensation resin of alkylphenol, polyvinyl chloride, polyvinylidene chloride, polystyrene, polyacrylate, a carboxyvinyl polymer, an acrylic resin copolymer resin, hydroxycellulose, hydroxymethylcellulose, polyvinyl alcohol, polyvinylpyrrolidone, cellulose acetate, methylcellulose, and carboxymethylcellulose. In order to prevent concerns on dissolution in dampening water during on-press development, a water-insoluble resin is preferable.

In addition, the binder preferably contains at least one compound selected from the group consisting of polyurethane, an acrylic resin, polystyrene, and polyethylene.

Furthermore, in the aspect 1, it is preferable that the aforementioned particles and the binder each independently contain at least one compound selected from the group consisting of polyurethane, an acrylic resin, polystyrene, and polyethylene.

The outermost layer on the side opposite to the side where the image-recording layer is provided may contain other components in addition to the particles and the binder. Examples of those other components include known additives, such as a surfactant.

The thickness of the outermost layer on the side opposite to the side where the image-recording layer is provided is preferably in a range of 0.5 to 10 μm, more preferably in a range of 0.5 to 5 μm, and still more preferably in a range of 0.5 to 3 μm.

The polymer compound configuring the plurality of protrusions containing a polymer compound as a main component in the aspect 2 preferably contains at least one polymer compound selected from the group consisting of a novolac or resol resin such as a phenolformaldehyde resin, an m-cresol formaldehyde resin, a p-cresol formaldehyde resin, an m-/p-mixed cresol formaldehyde resin, or a phenol/cresol (which may be any of m-, p-, or m/p-mixed) mixed formaldehyde resin, pyrogallol, an acetone resin, an epoxy resin, a saturated copolymerized polyester resin, a phenoxy resin, a polyvinyl acetal resin, a vinylidene chloride copolymer resin, polybutene, polybutadiene, polyamide, an unsaturated copolymerized polyester resin, polyurethane, polyurea, polyimide, polysiloxane, polycarbonate, an epoxy resin, chlorinated polyethylene, an aldehyde condensation resin of alkylphenol, polyvinyl chloride, polyvinylidene chloride, polystyrene, polyacrylate, a carboxyvinyl polymer, an acrylic resin copolymer resin, hydroxycellulose, hydroxymethylcellulose, polyvinyl alcohol, polyvinylpyrrolidone, cellulose acetate, methylcellulose, and carboxymethylcellulose.

Among these, from the viewpoint of causing excellent developability to be exhibited even in a case where the detached protrusions move to the image-recording layer, a watersoluble polymer is more preferable. Specific examples thereof include polyacrylate, a carboxyvinyl polymer, an acrylic resin copolymer resin, hydroxycellulose, hydroxymethyl cellulose, polyvinyl alcohol, modified polyvinyl alcohol, polyvinylpyrrolidone, cellulose acetate, methylcellulose, carboxymethyl cellulose, and the like.

As the modified polyvinyl alcohol, acid-modified polyvinyl alcohol having a carboxy group or a sulfo group is preferably used. Specifically, the modified polyvinyl alcohols described in JP2005-250216A and JP2006-259137A are suitable.

The shape and height of the protrusions are not particularly limited, but the arithmetic mean height Sa is preferably 0.3 to 20 μm.

The method of forming protrusions in a stripe pattern (stripe coating film) is not particularly limited. It is possible to easily form such protrusions by performing coating with a composition containing at least one component selected from the group consisting of particles and a polymer compound, by at least one method selected from the group consisting of a bar coating method, an inkjet printing method, a gravure printing method, a screen printing method, a spray coating method, and a slot die coating method.

The method of forming protrusions in a dot pattern (dot coating film) is not particularly limited. It is possible to easily form such protrusions by performing coating with a composition containing at least one component selected from the

group consisting of particles and a polymer compound, by at least one method selected from the group consisting of a spray coating method, an ink jet printing method, and a screen printing method.

The method of forming protrusions in a broken line pattern (broken line coating film) is not particularly limited. It is possible to easily form such protrusions by performing coating with a composition containing at least one component selected from the group consisting of particles and a polymer compound, by at least one method selected from the group consisting of an ink jet printing method and a screen printing method.

Examples of the binder contained in the outermost layer in the aspect 2 include the same polymer compound as the polymer compound contained in the protrusions, and preferred aspects thereof are also identical.

**In** the aspect 2, from the viewpoint of preventing the detachment of the protrusions, it is preferable that the binder contained in the outermost layer and the polymer compound contained in the protrusions contain the same type of resin. The "same type of resin" means that the types of resins, such as polyurethane, an acrylic resin, polystyrene, and polyethylene, are identical, and it is not necessary for all the constitutional units in the resins to be the same as each other.

The lithographic printing plate precursor according to the present disclosure may have other layers in addition to those mentioned above.

Known layers can be adopted as those other layers without particular limitations. For example, as necessary, a backcoat layer may be provided on a surface of the support that is opposite to the image-recording layer side.

Method of preparing lithographic printing plate and lithographic printing method

It is possible to prepare a lithographic printing plate by performing image exposure and a development treatment on the lithographic printing plate precursor according to the present disclosure.

The method of preparing a lithographic printing plate according to the present disclosure preferably includes a step of exposing the on-press development type lithographic printing plate precursor according to the present disclosure in the shape of an image (hereinafter, this step will be also called "exposure step") and a step of removing the image-recording layer in a non-image area by supplying at least one material selected from the group consisting of a printing ink and dampening water on a printer (hereinafter, this step will be also called "on-press development step").

The lithographic printing method according to the present disclosure preferably includes a step of exposing the on-press development type lithographic printing plate precursor according to the present disclosure in the shape of an image (exposure step), a step of removing the image-recording layer in a non-image area on a printer by supplying at least one material selected from the group consisting of a printing ink and dampening water such that a lithographic printing plate is prepared (on-press development step), and a step of performing printing using the obtained lithographic printing plate (printing step).

Hereinafter, regarding the method of preparing a lithographic printing plate according to the present disclosure and the lithographic printing method according to the present disclosure, preferred aspects of each step will be described in order. Note that the lithographic printing plate precursor according to the present disclosure can also be developed using a developer.

Hereinafter, the exposure step and the on-press development step in the method of preparing a lithographic printing plate will be described. The exposure step in the method of preparing a lithographic printing plate according to the present disclosure is the same step as the exposure step in the lithographic printing method according to the present disclosure. Furthermore, the on-press development step in the method of preparing a lithographic printing plate according to the present disclosure is the same step as the on-press development step in the lithographic printing method according to the present disclosure.

Exposure step

The method of preparing a lithographic printing plate according to the present disclosure preferably includes an exposure step of exposing the lithographic printing plate precursor according to the present disclosure in the shape of an image such that an exposed portion and a non-exposed portion are formed. The lithographic printing plate precursor according to the present disclosure is preferably exposed to a laser through a transparent original picture having a linear image, a halftone dot image, or the like or exposed in the shape of an image by laser light scanning according to digital data, or the like.

The wavelength of a light source to be used is preferably 750 nm to 1,400 nm. As the light source having a wavelength of 750 nm to 1,400 nm, a solid-state laser or a semiconductor laser that radiates infrared rays is suitable. In a case where an infrared laser is used, the output is preferably 100 mW or higher, the exposure time per pixel is preferably 20 microseconds or less, and the amount of irradiation energy is preferably 10 $mJ/cm^2$ to 300 $mJ/cm^2$. **In** addition, in order to shorten the exposure time, a multibeam laser device is preferably used. The exposure mechanism may be any of an inner surface drum method, an external surface drum method, a flat head method, or the like.

The image exposure can be carried out by a common method using a platesetter or the like. In the case of on-press development, image exposure may be carried out on a printer after the lithographic printing plate precursor is mounted on the printer.

On-press development step

The method of preparing a lithographic printing plate according to the present disclosure preferably includes an on-press development step of removing the image-recording layer in a non-image area by supplying at least one selected from the group consisting of printing ink and dampening water on a printer.

Hereinafter, the on-press development method will be described.

On-press development method

In the on-press development method, the lithographic printing plate precursor having undergone image exposure is preferably supplied with an oil-based ink and an aqueous component on a printer such that the image-recording layer in a non-image area is removed and a lithographic printing plate is prepared.

That is, in a case where the lithographic printing plate precursor is subjected to image exposure and then mounted as it is on a printer without being subjected to any development treatment, or the lithographic printing plate precursor is mounted on a printer and then subjected to image exposure on the printer, and then an oil-based ink and an aqueous component are supplied to perform printing, at the initial stage in the middle of printing, in a non-image area, by either or both of the supplied oil-based ink and the aqueous component, a non-cured image-recording layer is dissolved or dispersed to remove, and the hydrophilic surface is exposed in the non-image area. Meanwhile, the image-recording layer cured by exposure forms an oil-based ink-receiving area having a lipophilic surface in the exposed portion. What is supplied first to the precursor surface may be any of the oil-based ink or the aqueous component. However, in view of preventing the plate from being contaminated by the components of the image-recording layer from which aqueous components are removed, it is preferable that the oil-based ink be supplied first. In the manner described above, the lithographic printing plate precursor is subjected to on-press development on a printer and used as it is for printing a number of sheets. As the oil-based ink and the aqueous component, ordinary printing ink and ordinary dampening water for lithographic printing are suitably used.

As the laser used for performing image exposure on the lithographic printing plate precursor according to the present disclosure, a light source having a wavelength of 300 nm to 450 nm or 750 nm to 1,400 nm is preferably used. A light source of 300 nm to 450 nm is preferable for a lithographic printing plate precursor including an image-recording layer containing sensitizing dye having maximum absorption in such a wavelength range. As the light source of 750 nm to 1,400 nm, those described above are preferably used. As the light source of 300 nm to 450 nm, a semiconductor laser is suitable.

Printing step

The lithographic printing method according to the present disclosure includes a printing step of printing a recording medium by supplying a printing ink to the lithographic printing plate.

The printing ink is not particularly limited, and various known inks can be used as desired. In addition, preferred examples of the printing ink include oil-based ink or ultraviolet-curable ink (UV ink).

In the printing step, as necessary, dampening water may be supplied.

Furthermore, the printing step may be successively carried out after the on-press development step without stopping the printer.

The recording medium is not particularly limited, and a known recording medium can be used as desired.

In the method of preparing a lithographic printing plate from the lithographic printing plate precursor according to the present disclosure and in the lithographic printing method according to the present disclosure, as necessary, the entire surface of the lithographic printing plate precursor may be heated before exposure, in the middle of exposure, or during a period of time from exposure to development. In a case where the lithographic printing plate precursor is heated as above, an image-forming reaction in the image-recording layer is accelerated, which can result in advantages such as improvement of sensitivity and printing durability, stabilization of sensitivity, and the like. Heating before development is preferably carried out under a mild condition of 150°C or lower. In a case where this aspect is adopted, it is possible to prevent problems such as curing of a non-image area. For heating after development, it is preferable to use an extremely severe condition which is preferably in a range of 100°C to 500°C. In a case where this aspect is adopted, a sufficient image-strengthening action is obtained, and it is possible to inhibit problems such as the deterioration of the support or the thermal decomposition of the image area.

Compound represented by Formula (1A)

The compound according to the present disclosure is a compound represented by Formula (1A).

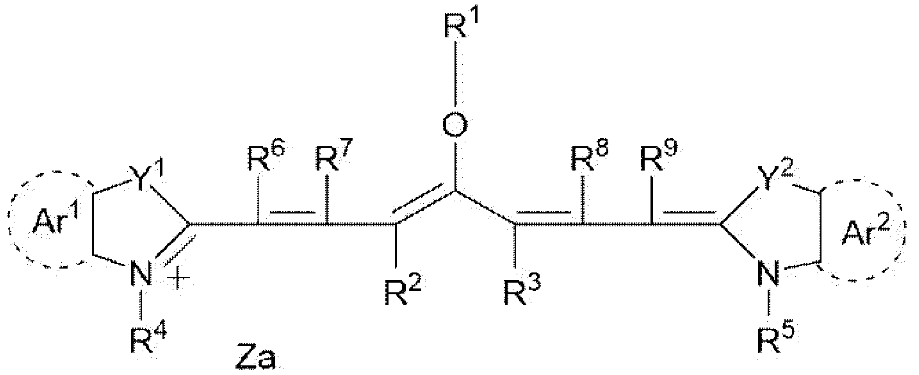

(1A)

In Formula (1A), $R^1$ represents a group represented by Formula (2A) or Formula (3A), $R^2$ and $R^3$ each independently represent a hydrogen atom or an alkyl group, $R^2$ and $R^3$ may be linked to each other to form a ring, $Ar^1$ and $Ar^2$ each independently represent a benzene ring or a naphthalene ring, $Y^1$ and $Y^2$ each independently represent an oxygen atom, a sulfur atom, - $NR^0$-, or a dialkylmethylene group, $R^4$ and $R^5$ each independently represent an alkyl group, $R^6$ to $R^9$ each independently represent a hydrogen atom or an alkyl group, $R^0$ represents a hydrogen atom, an alkyl group, or an aryl group, and Za represents a counterion that neutralizes a charge.

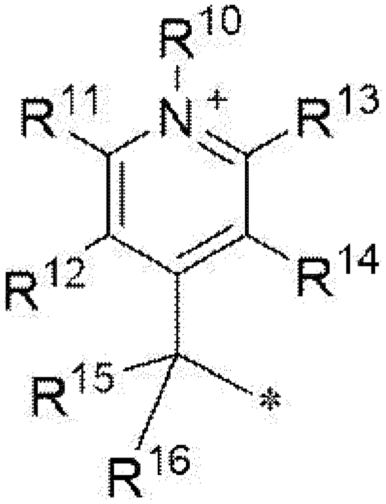

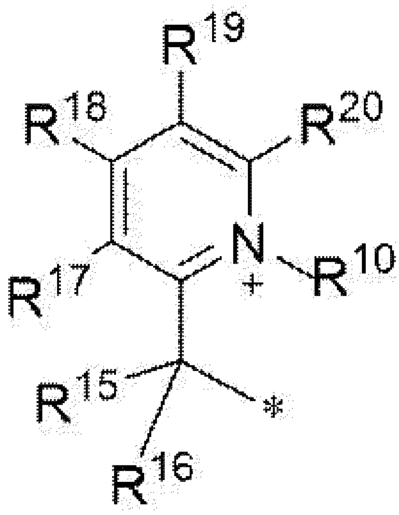

(2A) (3A)

In Formulae (2A) and (3A), $R^{10}$ represents a group having one or more substituents on an aromatic ring of a benzyl group, $R^{11}$ to $R^{14}$ and $R^{17}$ to $R^{20}$ each independently represent a hydrogen atom, an alkyl group, an aryl group, a hydroxy group, an alkoxy group, or a halogen atom, $R^{15}$ and $R^{16}$ each independently represent a hydrogen atom, an alkyl group, or an aryl group, at least two of $R^{11}$ to $R^{20}$ may be bonded to each other to form a ring structure, and * represents a single bond that is bonded to an oxygen atom in Formula (1A).

All of the compounds represented by Formula (1A) are novel compounds, and can be suitably used as a decomposition-type infrared absorber.

The details and preferred aspects of the compound represented by Formula (1A) as a novel compound are the same as the matters described in the description of the compound represented by Formula (1) described above, except for the matters shown below.

From the viewpoint of visibility, printing durability, on-press developability, and on-press developability after aging, $R^{10}$ in Formula (1A) is preferably a benzyl group (substituted benzyl group) having one or more substituents, and

more preferably a benzyl group having an alkyl group on a benzene ring.

In addition, from the viewpoint of visibility, printing durability, on-press developability, and on-press developability after aging, $R^{10}$ in Formula (1A) is preferably the group represented by Formula 4 described above.

From the viewpoint of visibility, printing durability, on-press developability, and on-press developability after aging, the compound according to the present disclosure is preferably a compound represented by Formula (7) or Formula (8) described above, and particularly preferably a compound represented by Formula (9) described above.

However, $Ar^3$ and $Ar^4$ in Formulae (7) to (9) are an aryl group having one or more substituents on an aromatic ring.

Examples

Hereinafter, the present disclosure will be described in detail with reference to examples, but the present disclosure is not limited thereto. In the present examples, unless otherwise specified, "%" and "part" mean "% by mass" and "part by mass" respectively. Unless otherwise described, the molecular weight of a high-molecular-weight compound is a weight-average molecular weight (Mw), and the ratio of repeating constitutional units of a polymer compound is expressed as molar percentage. In addition, the weight-average molecular weight (Mw) is a polystyrene-equivalent molecular weight measured by gel permeation chromatography (GPC).

Examples 1 to 137 and Comparative Examples 1 to 5

The undercoat layer described in Table 1 or Table 2 was formed on the support described in Table 1 or Table 2, the image-recording layer A was formed on the undercoat layer, and the overcoat layer A was formed on the image-recording layer A, thereby obtaining each lithographic printing plate precursor. The details of the method of forming each layer will be described later.

Examples 138 to 256 and Comparative Examples 6 to 10

The undercoat layer described in Table 3 or Table 4 was formed on the support described in Table 3 or Table 4, the image recording layer B was formed on the undercoat layer, the overcoat layer B was formed on the image recording layer B, and then the backcoat layer B was formed on the surface of the support opposite to the printing surface, thereby obtaining each lithographic printing plate precursor.

The details of the method of forming each layer will be described later.

Preparation of support 1

The following treatment was performed on an aluminum plate (aluminum alloy plate) having a thickness of 0.3 mm and a material 1S to produce a support 1. A water washing treatment was performed between all the treatment steps. After the water washing treatment, water was drained using a nip roller.

Alkaline etching treatment (1)

An aqueous solution of caustic soda having a caustic soda concentration of 26% by mass and an aluminum ion concentration of 6.5% by mass was sprayed onto the aluminum plate at a temperature of 70°C, thereby performing an etching treatment. Then, water washing was performed by means of spraying. The amount of dissolved aluminum within the surface to be subjected to the electrochemical roughening treatment later was 5 $g/m^2$.

Desmutting treatment (1) using acidic aqueous solution

Next, a desmutting treatment was performed using an acidic aqueous solution. Specifically, the desmutting treatment was performed for 3 seconds by spraying the acidic aqueous solution onto the aluminum plate. As the acidic aqueous solution used for the desmutting treatment, an aqueous solution containing 150 g/L of sulfuric acid was used. The liquid temperature was 30°C.

Hydrochloric acid electrolytic treatment

Next, a hydrochloric acid electrolytic treatment was performed using an alternating current with an electrolytic solution having a hydrochloric acid concentration of 13 g/L, an aluminum ion concentration of 15 g/L, and a sulfuric acid concentration of 1.0 g/L. The liquid temperature of the electrolytic solution was 25°C. The aluminum ion concentration was adjusted by adding aluminum chloride.

The waveform of the alternating current was a sine wave in which the positive and negative waveforms were symmetrical, the frequency was 50 Hz, the ratio between the anodic reaction time and the cathodic reaction time in one cycle of the alternating current was 1: 1, and the current density was 35 $A/dm^2$ in terms of the peak current value of the alternating current waveform. Further, the sum of the electricity quantity for the anodic reaction of the aluminum plate was 320 $C/dm^2$, and the electrolytic treatment was performed four times at energization intervals of 2.5 seconds for each electric quantity of 80 $C/dm^2$. A carbon electrode was used as the counter electrode of the aluminum plate. Then, a water washing treatment was performed.

Alkaline etching treatment (2)

An aqueous solution of caustic soda having a caustic soda concentration of 5% by mass and an aluminum ion concentration of 0.5% by mass was sprayed onto the aluminum plate after the hydrochloric acid electrolytic treatment at a temperature of 45°C to perform an etching treatment. The amount of aluminum dissolved in the surface subjected to the hydrochloric acid electrolytic treatment was 0.1 $g/m^2$. Then, a water washing treatment was performed.

Desmutting treatment (2) using acidic aqueous solution

Next, a desmutting treatment was performed using an acidic aqueous solution. Specifically, the desmutting treatment was performed for 3 seconds by spraying the acidic aqueous solution onto the aluminum plate. As the acidic aqueous solution used for the desmutting treatment, an aqueous solution having a sulfuric acid concentration of 170 g/L and an aluminum ion concentration of 5 g/L was used. The liquid temperature was 35°C.

First-stage anodization treatment

By using the anodization device for direct current electrolysis having the structure shown in Fig. 5, a first-stage anodization treatment was performed. By using a 170 g/L aqueous sulfuric acid solution as an electrolytic solution, an anodization treatment was performed under the conditions of a liquid temperature of 40°C and a current density of 20 $A/dm^2$, thereby forming an anodic oxide film having a film amount of 0.3 $g/m^2$.

In the anodization treatment device 610 shown in Fig. 5, an aluminum plate 616 is transported as indicated by the arrow in Fig. 5. In a power supply tank 612 storing an electrolytic solution 618, the aluminum plate 616 is positively (+) charged by a power supply electrode 620. Further, the aluminum plate 616 is transported upward by a roller 622 in the power supply tank 612, redirected downward by a nip roller 624, transported toward an electrolytic treatment tank 614 in which an electrolytic solution 626 is stored, and redirected to the horizontal direction by a roller 628. Next, the aluminum plate 616 is negatively (-) charged by an electrolysis electrode 630 so that an anodic oxide film is formed on the surface thereof, and the aluminum plate 616 coming out of the electrolytic treatment tank 614 is transported to the next step. In the anodization treatment device 610, direction changing means is formed of the roller 622, the nip roller 624, and the roller 628. The aluminum plate 616 is transported in a mountain shape and an inverted U shape by the roller 622, the nip roller 624, and the roller 628 in an inter-tank portion between the power supply tank 612 and the electrolytic treatment tank 614. The power supply electrode 620 and the electrolysis electrode 630 are connected to a direct current power source 634. A tank wall 632 is disposed between the power supply tank 612 and the electrolytic treatment tank 614.

Pore widening treatment

The aluminum plate having undergone the anodization treatment was immersed in an aqueous solution of caustic soda having a caustic soda concentration of 5% by mass and an aluminum ion concentration of 0.5% by mass for 5 seconds at 40°C, thereby performing a pore widening treatment. Then, water washing was performed by means of spraying.

Second-stage anodization treatment

By using the anodization device for direct current electrolysis having the structure shown in Fig. 5, a second-stage anodization treatment was performed. By using a 170 g/L aqueous sulfuric acid solution as an electrolytic solution, an anodization treatment was performed under the conditions of a liquid temperature of 50°C and a current density of 13 $A/dm^2$, thereby forming an anodic oxide film having a film amount of 3.5 $g/m^2$.

Silicate treatment

The aluminum plate having undergone the second anodization treatment was immersed in a No. 3 sodium

silicate aqueous solution having a concentration of 5% by mass and a liquid temperature of 40°C for 12 seconds, thereby obtaining a support 1. The amount of Si deposited was 8 mg/$m^2$. The average diameter of the micropores was 30 nm.

Preparation of support 2

The silicate treatment conditions for the method of producing the support 1 were changed to immersion in a 5% by mass No.3 sodium silicate aqueous solution at a liquid temperature of 65°C for 12 seconds, thereby obtaining a support 2. In the other steps, the treatment was performed by the same method and procedure as the method of producing the support 1. The amount of Si deposited to the support 2 was 21 mg/$m^2$.

Preparation of support 3

The following treatments (J-a) to (J-m) were performed on an aluminum plate (aluminum alloy plate) made of a material 1S having a thickness of 0.3 mm, thereby manufacturing a support 1. A water washing treatment was performed between all the treatment steps. After the water washing treatment, water was drained using a nip roller.

(J-a) Mechanical roughening treatment (brush graining method)

A pumice suspension (specific gravity: 1.1 g/$cm^3$) as an abrasive slurry was supplied to the surface of an aluminum plate, and in this state, a mechanical roughening treatment is performed using a rotating bundled brush.

In the mechanical roughening treatment, an abrasive having a median diameter (μm) of 30 μm and 4 brushes were used, and the rotation speed (rpm) of the brushes was set to 250 rpm. The bundled brush was made of 6·10 nylon and consisted of bristles having a diameter of 0.3 mm and a length of 50 mm. The brush was prepared by making holes in a φ 300 mm stainless steel cylinder and densely implanting bristles therein. The distance between two support rollers (φ 200 mm) under the bundled brush was 300 mm. The bundled brush was pressed until the load of the drive motor for rotating the brush was 10 kW higher than the load applied before the bundled brush was pressed on the aluminum plate. The rotation direction of the brush was the same as the moving direction of the aluminum plate.

(J-b) Alkaline etching treatment

An aqueous solution of caustic soda having a caustic soda concentration of 26% by mass and an aluminum ion concentration of 6.5% by mass was sprayed onto an aluminum plate at a temperature of 70°C to perform an etching treatment. The amount of dissolved aluminum within the surface to be subjected to the electrochemical roughening treatment later was 10 g/$m^2$.

(J-c) Desmutting treatment using acidic aqueous solution

The desmutting treatment was performed by spraying the waste liquid of nitric acid used in the subsequent step of the electrochemical roughening treatment at a liquid temperature of 35°C onto the aluminum plate for 3 seconds.

(J-d) Electrochemical roughening treatment using aqueous nitric acid solution

The electrochemical roughening treatment was continuously performed using an alternating current voltage of 60 Hz. An electrolytic solution at a liquid temperature of 35°C was used which was prepared by adding aluminum nitrate to 10.4 g/L aqueous nitric acid solution such that the aluminum ion concentration was adjusted to 4.5 g/L. By using an alternating current power source having the waveform shown in Fig. 1, alternating current having a trapezoidal rectangular waveform, and a carbon electrode as a counter electrode, an electrochemical roughening treatment was performed under the conditions of a time tp taken for the current value to reach the peak from zero of 0.8 msec and the duty ratio of 1:1. As an auxiliary anode, ferrite was used. The electrolytic cell shown in Fig. 2 was used. The current density was 30 A/$dm^2$ in terms of the peak value of current, and 5% of the current coming from the power source was allowed to flow into the auxiliary anode. The electricity quantity (C/$dm^2$) was 185 C/$dm^2$, which is the total quantity of electricity used during the anodic reaction of the aluminum plate.

(J-e) Alkaline etching treatment

An aqueous solution of caustic soda having a caustic soda concentration of 27% by mass and an aluminum ion concentration of 2.5% by mass was sprayed onto the aluminum plate at a temperature of 50°C to perform an etching treatment. The amount of dissolved aluminum was 3.5 g/$m^2$.

(J-f) Desmutting treatment using acidic aqueous solution

As the acidic aqueous solution, an aqueous solution having a sulfuric acid concentration of 170 g/L and an aluminum ion concentration of 5 g/L at a liquid temperature of 30°C was sprayed on the aluminum plate for 3 seconds to perform the desmutting treatment.

(J-g) Electrochemical roughening treatment using hydrochloric acid aqueous solution

The electrochemical roughening treatment was continuously performed using an alternating current voltage of 60 Hz. An electrolytic solution at a liquid temperature of 35°C was used which was prepared by adding aluminum chloride to 6.2 g/L aqueous hydrochloric acid solution such that the aluminum ion concentration was adjusted to 4.5 g/L. By using an alternating current power source having the waveform shown in Fig. 1, alternating current having a trapezoidal rectangular waveform, and a carbon electrode as a counter electrode, an electrochemical roughening treatment was performed under the conditions of a time tp taken for the current value to reach the peak from zero of 0.8 msec and the duty ratio of 1:1. As an auxiliary anode, ferrite was used. The electrolytic cell shown in Fig. 2 was used. The current density was 25 $A/dm^2$ in terms of the peak value of current, and the electricity quantity ($C/dm^2$) during the hydrochloric acid electrolysis was 63 $C/dm^2$ which is the total electricity quantity used during the anodic reaction of the aluminum plate.

(J-h) Alkaline etching treatment

An aqueous solution of caustic soda having a caustic soda concentration of 5% by mass and an aluminum ion concentration of 0.5% by mass was sprayed onto the aluminum plate at a temperature of 60°C to perform an etching treatment. The amount of dissolved aluminum was 0.2 $g/m^2$.

(J-i) Desmutting treatment using aqueous acidic solution

As the aqueous acidic solution, an aqueous solution of the waste liquid (sulfuric acid concentration of 170 g/L and aluminum ion concentration of 5 g/L) generated in the anodization treatment step at a liquid temperature of 35°C was sprayed onto the aluminum plate for 4 seconds to perform the desmutting treatment.

(J-j) First-stage anodization treatment

The first-stage anodization treatment was performed using the anodization device for direct current electrolysis having the structure shown in Fig. 5. By using a 170 g/L aqueous sulfuric acid solution as an electrolytic solution, an anodization treatment was performed under the conditions of a liquid temperature of 50°C and a current density of 30 $A/dm^2$, thereby forming an anodic oxide film having a film amount of 0.3 $g/m^2$.

(J-k) Pore widening treatment

The aluminum plate which had been subjected to the anodization treatment was immersed in an aqueous solution of caustic soda having a caustic soda concentration of 5% by mass and an aluminum ion concentration of 0.5% by mass at 40°C for 5 seconds, thereby performing the pore widening treatment.

(J-l) Second-stage anodization treatment

The second-stage anodization treatment was performed by direct current electrolysis using the anodization device having the structure shown in Fig. 5. By using a 170 g/L aqueous sulfuric acid solution as an electrolytic solution, an anodization treatment was performed under the conditions of a liquid temperature of 50°C and a current density of 13 $A/dm^2$, thereby forming an anodic oxide film having a film amount of 2.6 $g/m^2$.

(J-m) Silicate treatment

In order to ensure the hydrophilicity of the non-image area, the aluminum plate was immersed in a 2.5% by mass of No. 3 sodium silicate aqueous solution at 50°C for 7 seconds to perform a silicate treatment. The amount of Si deposited was 9 $mg/m^2$. The average diameter of the micropores was 31 nm.

Preparation of support 4

The following treatments (F-a) to (F-g) were performed on an aluminum plate (aluminum alloy plate) made of a material 1S having a thickness of 0.3 mm, thereby preparing a support 4. A water washing treatment was performed between all the treatment steps. After the water washing treatment, water was drained using a nip roller.

(F-a) Alkaline etching treatment

An aqueous solution of caustic soda having a caustic soda concentration of 26% by mass and an aluminum ion concentration of 6.5% by mass was sprayed onto an aluminum plate at a temperature of 70°C to perform an etching treatment. The amount of dissolved aluminum within the surface to be subjected to the electrochemical roughening treatment later was 5 $g/m^2$.

(F-b) Desmutting treatment using acidic aqueous solution

The desmutting treatment was performed by spraying an aqueous solution having a sulfuric acid concentration of 150 g/L at a liquid temperature of 30°C onto the aluminum plate for 3 seconds.

(F-c) Electrochemical roughening treatment

An electrochemical roughening treatment was performed using an alternating current and an electrolytic solution having a hydrochloric acid concentration of 14 g/L, an aluminum ion concentration of 13 g/L, and a sulfuric acid concentration of 3 g/L. The liquid temperature of the electrolytic solution was 30°C. The aluminum ion concentration was adjusted by adding aluminum chloride.

The waveform of the alternating current was a sine wave in which positive and negative waveforms are symmetrical, the frequency was 50 Hz, the ratio of the anodic reaction time and the cathodic reaction time in one cycle of the alternating current was 1: 1, and the current density was 75 $A/dm^2$ in terms of the peak current value of the alternating current waveform. In addition, the quantity of electricity was 450 $C/dm^2$ which was the total quantity of electricity used for the aluminum plate to have an anodic reaction, and the electrolytic treatment was performed 4 times by conducting electricity of 112.5 $C/dm^2$ for 4 seconds at each treatment session. A carbon electrode was used as the counter electrode of the aluminum plate.

(F-d) Alkaline etching treatment

An aqueous solution of caustic soda having a caustic soda concentration of 5% by mass and an aluminum ion concentration of 0.5% by mass was sprayed onto the aluminum plate at a temperature of 45°C to perform an etching treatment. The amount of dissolved aluminum within the surface having undergone the electrochemical roughening treatment was 0.2 $g/m^2$.

(F-e) Desmutting treatment using aqueous acidic solution

As the aqueous acidic solution, an aqueous solution having a sulfuric acid concentration of 170 g/L and an aluminum ion concentration of 5 g/L at a liquid temperature of 35°C was sprayed onto the aluminum plate for 3 seconds to perform a desmutting treatment.

(F-f) First-stage anodization treatment

The first-stage anodization treatment was performed by direct current electrolysis using the anodization device having the structure shown in Fig. 5. By using a 150 g/L aqueous phosphoric acid solution as an electrolytic solution, an anodization treatment was performed under the conditions of a liquid temperature of 35°C and a current density of 4.5 $A/dm^2$, thereby forming an anodic oxide film having a film amount of 1 $g/m^2$.

(F-g) Second-stage anodization treatment

The second-stage anodization treatment was performed by direct current electrolysis using the anodization device having the structure shown in Fig. 5. By using a 170 g/L aqueous sulfuric acid solution as an electrolytic solution, an anodization treatment was performed under the conditions of a liquid temperature of 50°C and a current density of 13 $A/dm^2$, thereby forming an anodic oxide film having a film amount of 2.1 $g/m^2$. Then, water washing was performed by

means of spraying. The average diameter of the micropores in the support 4 was 40 nm.

The value of the brightness L* of the surface of the anodic oxide film of the support 4 was 83.7 in the L*a*b* color system.

Preparation of support 5

A support 5 was prepared according to the manufacturing method of the support of Example 5 of WO2021/67054A.

Formation of undercoat layer 1

The support was coated with an undercoat layer coating liquid (1) having the following composition such that the dry coating amount was 26 mg/$m^2$. In this way, an undercoat layer 1 was formed.

Undercoat layer coating solution (1)

· Compound for undercoat layer (1) (the following structure): 0.013 parts
· Hydroxyethyl iminodiacetic acid: 0.005 parts
· Tetrasodium ethylenediaminetetraacetate: 0.005 parts
· Polyoxyethylene lauryl ether: 0.0003 parts
· Water: 3.15 parts

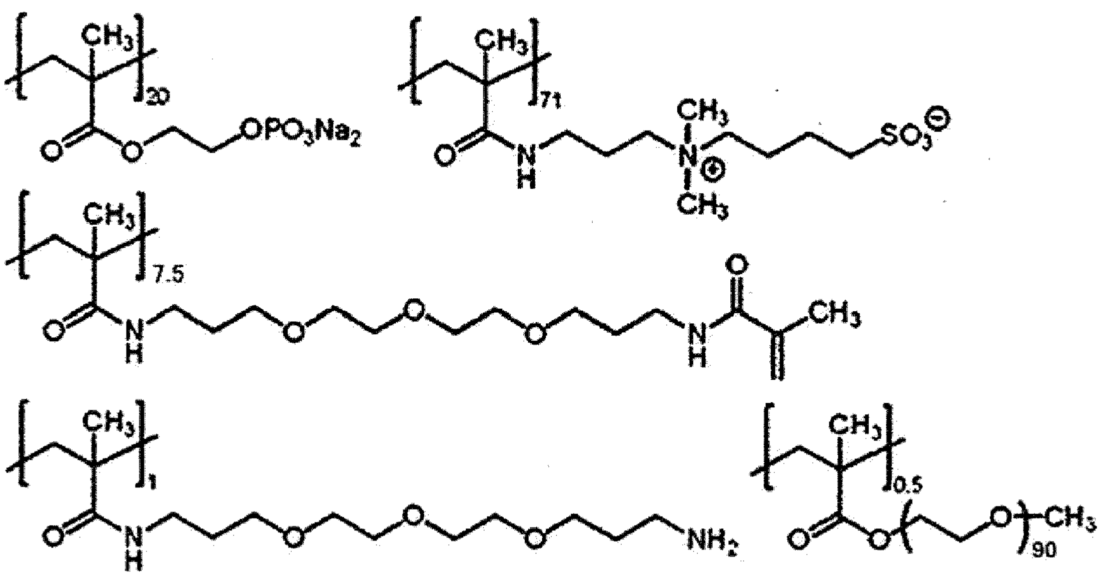

Compound (1) for undercoat layer Mw 100,000

The numerical value at the right lower side of the parentheses of each constitutional unit in the compound (1) for an undercoat layer represents a mass ratio, and the numerical value at the right lower side of the parentheses of the ethyleneoxy unit represents the number of repeating units.

Formation of undercoat layer 2

The support was coated with an undercoat layer coating liquid (2) having the following composition such that the dry coating amount was 0.03 g/$m^2$. In this way, an undercoat layer 2 was formed.

Undercoat layer coating liquid (2)

· Aqueous polyacrylic acid solution (40% by mass) (Jurymer AC-10S, manufactured by TOAGOSEI CO., LTD.): 3.0 parts
· Water: 27.0 parts

Formation of image recording layer A

The undercoat layer was bar-coated with the image recording layer coating solution A having the following composition, and dried in an oven at 110°C for 40 seconds, thereby forming an image recording layer A having a dry weight of 0.9 g/m$^2$. The compounds described in Table 1 were selected such that the dry weight was the described content.

Image recording layer coating liquid A

· 2-Butanone: 5.3155 parts
· 1-Methoxy-2-propanol: 2.8825 parts
· Methanol: 2.3391 parts
· Compound having specific structure (described in Table 1): amount described in Table 1
· Electron-donating polymerization initiator (described in Table 1): amount described in Table 1
· Electron-accepting polymerization initiator (described in Table 1): amount described in Table 1
· Polymerizable compound (described in Table 1): amount described in Table 1
· Infrared absorber (described in Table 1): amount described in Table 1
· Acid color forming agent (described in Table 1): amount described in Table 1
· Hydrogen donating compound (described in Table 1): amount described in Table 1
· Tricresyl phosphate: 0.0125 parts
· Anionic surfactant (A-1): 0.0162 parts
· Microgel liquid: 2.8779 parts
· Fluorine-based surfactant (W-1): 0.0042 parts

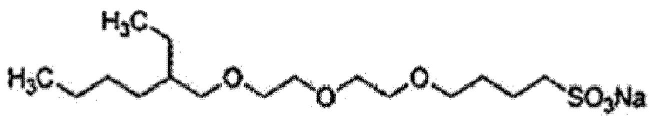
(A-1)

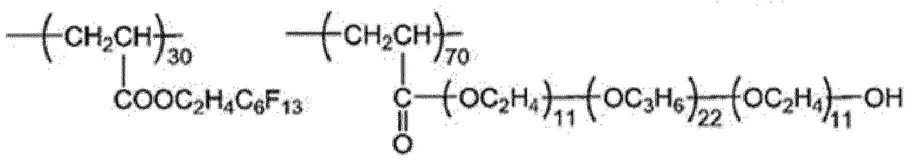
(W-1)

(Mw=13,000)

Formation of image recording layer B

The undercoat layer was bar-coated with an image recording layer coating solution B with the following composition and dried in an oven at 100°C for 60 seconds, thereby forming an image recording layer B having a thickness of 1.0 μm.

Image recording layer coating solution B

· 2-Butanone: 5.6000 parts
· 1-Methoxy-2-propanol (MFG): 3.3000 parts
· Methanol: 1.3000 parts
· Compound having specific structure (described in Table 1): amount described in Table 1
· Electron-donating polymerization initiator (described in Table 1): amount described in Table 1
· Electron-accepting polymerization initiator (described in Table 1): amount described in Table 1
· Polymerizable compound (described in Table 1): amount described in Table 1

· Binder polymer 1: 0.54 (23% MFG solution)
· Infrared absorber (described in Table 1): amount described in Table 1
· Acid color forming agent (described in Table 1): amount described in Table 1
· Hydrogen donating compound (described in Table 1): amount described in Table 1
· Anionic surfactant (A-1): 0.0750 parts
· Polymer particles R-1 (microgel liquid): 2.3256 parts
· Fluorine-based surfactant (W-1): 0.0042 parts

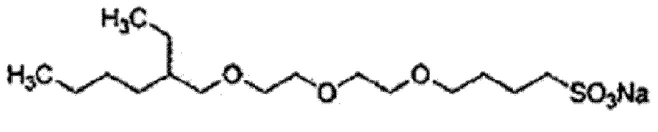
(A-1)

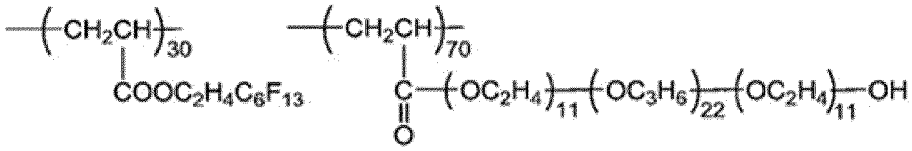
(W-1)

(Mw=13,000)

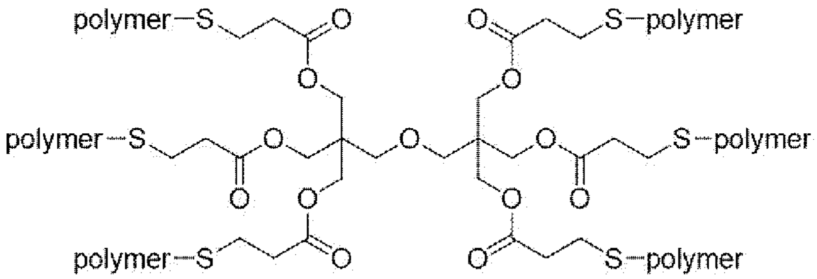

polymer 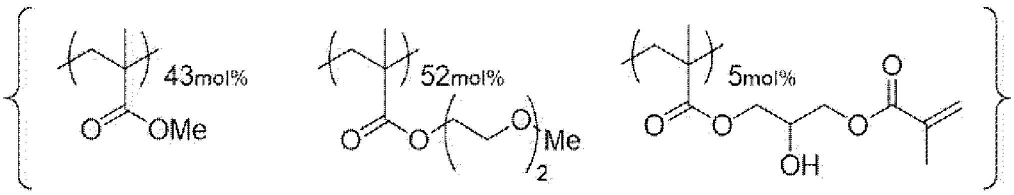

Binder polymer 1

Preparation of binder polymer 1

69.18 g of 1-methoxy-2-propanol was weighed in a three-neck flask and heated to 70°C in 500 mL of a nitrogen stream. A mixed solution consisting of 52.90 g of BLEMMER PME-100 (methoxy diethylene glycol monomethacrylate, manufactured by NOF Corporation), 35.17 g of methyl methacrylate, 6.05 g of methacrylic acid, 0.917 g of hexakis(3-mercaptopropionic acid) dipentaerythritol, 0.809 g of V-601 (2,2'-azobis(isobutyric acid) dimethyl, manufactured by FUJIFILM Wako Pure Chemical Corporation), and 70.10 g of 1-methoxy-2-propanol was added dropwise to this reaction container over 2 hours and 30 minutes. After the dropwise addition, the reaction was continued for another 2 hours. 2 hours later, a mixed solution consisting of 0.081 g of V-601 and 3.32 g of 1-methoxy-2-propanol was added thereto, the temperature was raised to 90°C, and the reaction was continued for 2.5 hours. After the reaction ended, the reaction solution was cooled to room temperature.

97.73 g of 1-methoxy-2-propanol, 0.23 g of 4-hydroxytetramethylpiperidine N-oxide, 6.49 g of glycidyl methacrylate, and 0.90 g of betaine were added to the above-described reaction solution, and the mixture was stirred well and heated at 92°C for 24 hours. After 24 hours, the reaction solution was cooled to room temperature and diluted by adding 78.84 g of 1-methoxy-2-propanol thereto. The binder polymer 1 obtained in this way had a concentration of solid contents of 23% by mass and a weight-average molecular weight of 65,000 measured by GPC and expressed in terms of polystyrene.

Preparation of polymer particles R-1

· Microgel (polymer particles R-1): 2.640 parts
· Distilled water: 2.425 parts

The microgel was prepared by the following method.

Preparation of polyvalent isocyanate compound

0.043 parts of bismuth tris(2-ethylhexanoate) (NEOSTANN U-600, manufactured by Nitto Kasei Co., Ltd.) was added to a suspension solution of 17.78 parts (80 molar equivalents) of isophorone diisocyanate and 7.35 parts (20 molar equivalents) of the following polyvalent phenol compound (1) in ethyl acetate (25.31 parts), and the mixture was stirred. The reaction temperature was set to 50°C at a point in time when heat release subsided, and the solution was stirred for 3 hours, thereby obtaining an ethyl acetate solution of the polyvalent isocyanate compound (1) (50% by mass).

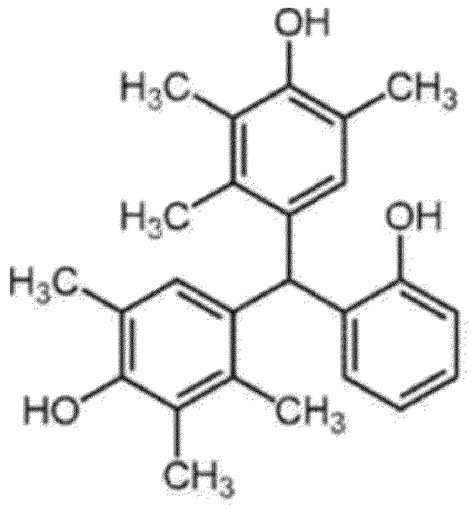

Polyvalent phenol compound (1)

Preparation of microgel

The following oil-phase components and water-phase components were mixed together and emulsified at 12,000 rpm for 10 minutes by using a homogenizer. The obtained emulsion was stirred at 45°C for 4 hours, a 10% by mass aqueous solution of 5.20 g of 1,8-diazabicyclo[5.4.0]undec-7-ene-octylate (U-CAT SA102, manufactured by San-Apro Ltd.) was added thereto, and the solution was stirred at room temperature for 30 minutes and left to stand at 45°C for 24 hours. Distilled water was added thereto such that the concentration of solid contents was adjusted to 20% by mass, thereby obtaining an aqueous dispersion of microgel. The average particle diameter thereof measured by a light scattering method was 0.20 μm.

Oil-phase component

(Component 1) ethyl acetate: 12.0 parts
(Component 2) an adduct obtained by addition of trimethylolpropane (6 molar equivalents), xylene diisocyanate (18 molar equivalents), and polyoxyethylene having one methylated terminal (1 molar equivalent, the number of repeating oxyethylene units: 90) (50% by mass ethyl acetate solution, manufactured by Mitsui Chemicals, Inc.): 3.76 parts
(Component 3) polyvalent isocyanate compound (1) (as 50% by mass ethyl acetate solution): 15.0 parts
(Component 4) 65% by mass ethyl acetate solution of dipentaerythritol pentaacrylate (SR-399, manufactured by Sartomer Company Inc.): 11.54 parts
(Component 5) 10% by mass ethyl acetate solution of sulfonate type surfactant (PIONIN A-41-C, manufactured by

TAKEMOTO OIL & FAT Co., Ltd.): 4.42 parts

Water-phase component

Distilled water: 46.87 parts

Formation of overcoat layer A

The image recording layer was bar-coated with an overcoat layer coating solution A having the following composition and dried in an oven at 120°C for 60 seconds, thereby forming an overcoat layer A having a dry coating amount of 0.1 g/$m^2$.

Overcoat layer coating liquid A

· Inorganic lamellar compound dispersion liquid (1) (below): amount described in Table 1
· Hydrophilic polymer (1) (the following structure, Mw: 30,000): 0.03 parts
· METOLOSE SM04 (manufactured by Shin-Etsu Chemical Co., Ltd.): 0.0600 parts
· RAPISOL A-80 (80% aqueous solution) (manufactured by NOF Corporation): 0.0063 parts
· Water: 2.0 parts

Preparation of inorganic lamellar compound dispersion liquid (1)

Synthetic mica SOMASIF ME-100 manufactured by Co-op Chemical Co., Ltd., 6.4 parts) was added to 193.6 parts of deionized water and was dispersed using a homogenizer until the volume average particle diameter (the laser scattering method) reached 3 μm. The aspect ratio of the obtained dispersed particles was 100 or more.
In the protective layer of Table 1, the "Lamellar compound" represents "synthetic mica SOMASIF ME-100 (manufactured by Co-op Chemical Co., Ltd.)" which is a component of the inorganic layered compound dispersion liquid (1).
In addition, the content of the "inorganic lamellar compound dispersion liquid (1)" was selected such that the "Addition amount" in Table 1 was the content described in the dry weight.

Formation of overcoat layer B

The image recording layer was bar-coated with an overcoat layer coating solution B having the following composition and dried in an oven at 120°C for 60 seconds to form an overcoat layer B having a thickness of 0.20 μm.

Overcoat layer coating solution B

· Inorganic lamellar compound dispersion liquid (1): 2.210 parts
· Polyvinyl alcohol (GOHSERAN L-3266, manufactured by Nippon Synthetic Chemical Industry Co., Ltd., sulfonic acid-modified, saponification degree of 85 mol%): 0.086 parts
· Surfactant (PIONIN A-32-B, manufactured by TAKEMOTO OIL & FAT Co., Ltd., 40% by mass of aqueous solution): 0.014 parts
. Surfactant (SURFYNOL 465 (the following structure), manufactured by Nissin Chemical Co., Ltd.): 0.006 parts
· Phosphoric acid (85 mass% aqueous solution): 0.024 parts
· Sodium phosphate (12-hydrate) (manufactured by Nippon Chemical Industrial Co., Ltd.): 0.090 parts
· Pure water: 5.600 parts

Formation of backcoat layer B

Preparation of backcoat layer coating solution (1)

· BR-605 (acrylic resin) manufactured by Mitsubishi Chemical Corporation: 11.072 parts
· SUMECTON-SEN (flat plate particle) manufactured by Kunimine Industries Co., Ltd.): 0.500 g
· Acrylic particles (ART PEARL J-6PF, manufactured by Negami Chemical Industrial Co., Ltd.): 0.975 parts
· RHEODOL TW-S106V (polyoxyethylene (6) sorbitan monostearate) manufactured by Kao Corporation: 0.250 parts
· 2-Butanone: 74.123 parts
· 1-Methoxy-2-propanol: 8.720 parts
· Methanol: 4.360 parts

The above components were mixed and stirred to prepare a backcoat layer coating solution (1).
After forming the overcoat layer B, the backcoat layer coating liquid (1) having the above composition was bar-coated on the surface of the support on the backcoat layer side (non-printing surface side), and dried at 100°C for 120 seconds, thereby forming a backcoat layer B having a thickness of 1.2 μm.

Visibility evaluation: immediately after exposure

The obtained lithographic printing plate precursor is exposed by Luxel PLATESETTER T-6000III (manufactured by Fujifilm Corporation) equipped with an infrared semiconductor laser with a wavelength of 830 nm such that the exposure amount is 110 mJ/cm$^2$. The exposed image includes a solid image and a non-image area. The exposure was carried out in an environment of 25°C and 50% RH.
Immediately after exposure, the color formation of the lithographic printing plate precursor was measured. The measurement was performed by the specular component excluded (SCE) method by using a spectrocolorimeter CM2600d and operation software CM-S100W manufactured by Konica Minolta, Inc. The color formability is evaluated based on a difference ΔL between the L* value of the exposed portion and the L* value of the unexposed portion using the L* value (brightness) of the L*a*b* color system. It can be said that the larger the value of ΔL, the better the color formability.

Printing durability evaluation

By using Magnus 800 Quantum manufactured by Kodak Japan Ltd. that was equipped with an infrared semiconductor laser, the obtained lithographic printing plate precursor was exposed (equivalent to irradiation energy of 110 mJ/cm$^2$) under the conditions of output of 27 W, an outer drum rotation speed of 450 rpm, and a resolution of 2,400 dots per inch (dpi, 1 inch is equal to 2.54 cm). The exposure image included a solid image and an amplitude modulation screen (AM screen) as a 10% halftone dot chart.
The obtained exposed precursor was mounted on a Kikuban-sized cylinder of a printer SX-74 manufactured by Heidelberger Druckmaschinen AG without being developed. This printer was connected to a 100 L-capacity dampening water circulation tank having a nonwoven fabric filter and a temperature control device. A circulation device was filled with 80 L of dampening water containing 3.5% dampening water S-Z1 (manufactured by FUJIFILM Corporation), UV CORE TYPE-A J ink GE M (manufactured by T&K TOKA CO., LTD.) was used as printing ink, dampening water and ink were supplied by a standard automatic printing start method, and then 1,000 sheets were printed on SHIRAOI paper (ream weight: 48.5 kg, manufactured by NIPPON PAPER INDUSTRIES Co., Ltd.) at a printing speed of 10,000 sheets per hour. Then, printing was performed further. As the number of printing sheets increased, the image area gradually wore out, and thus the ink density on the printed matter decreased. The print durability was evaluated by determining the number of printed sheets, which is designated as the number of finished printed sheets, in a case where the halftone dot area ratio of a 10% AM screen dot on a printed material, which is measured using an eXact spectrophotometer (manufactured by X-Rite), was decreased by 3% compared to the measurement value at the 1,000th print. UV printing durability was evaluated according to the following standard, based on relative printing durability to 100 which represents the printing durability of a lithographic printing plate precursor capable of printing 50,000 sheets. The higher the numerical value, the better the printing durability.

Relative printing durability = (number of printed sheets of target lithographic printing plate precursor/50,000) × 100

Evaluation standards

10: The value of relative printing durability is more than 150.
9: The value of relative printing durability is more than 140 and 150 or less.
8: The value of relative printing durability is more than 130 and 140 or less.
7: The value of relative printing durability is more than 120 and 130 or less.

6: The value of relative printing durability is more than 110 and 120 or less.
5: The value of relative printing durability is more than 100 and 110 or less.
4: The value of relative printing durability is more than 90 and 100 or less.
3: The value of relative printing durability is more than 80 and 90 or less.
2: The value of relative printing durability is more than 70 and 80 or less.
1: The value of relative printing durability is 70 or less.

On-press developability evaluation

By using Magnus 800 Quantum manufactured by Kodak Japan Ltd. that was equipped with an infrared semiconductor laser, the obtained lithographic printing plate precursor was exposed (equivalent to irradiation energy of 110 $mJ/cm^2$) under the conditions of output of 27 W, an outer drum rotation speed of 450 rpm, and a resolution of 2,400 dots per inch (dpi, 1 inch is equal to 2.54 cm). The exposure image included a solid image and an amplitude modulation screen (AM screen) as a 50% halftone dot chart.

The obtained exposed precursor was mounted on a Kikuban-sized cylinder of a printer SX-74 manufactured by Heidelberger Druckmaschinen AG without being developed. This printer was connected to a 100 L-capacity dampening water circulation tank having a nonwoven fabric filter and a temperature control device. A circulation device was filled with 80 L of dampening water containing 3.5% dampening water S-Z1 (manufactured by FUJIFILM Corporation), DIC Fusion-G NEO red N (manufactured by DIC Graphics Corporation) was used as printing ink, dampening water and ink were supplied by a start method of 7 times of dampening and one time of inking, and then 200 sheets were printed on Tokuryo Art paper (ream weight: 76.5 kg, manufactured by MITSUBISHI PAPER MILLS LIMITED.) at a printing speed of 7,000 sheets per hour. During the on-press development described above, the number of printing papers used until no ink was transferred to a non-image area was measured (hereinafter, also called number of sheets of on-press development). It can be said that the smaller the number of sheets of on-press development, the better the on-press developability.

Evaluation standards

7: The number of sheets of on-press development is 20 or less.
6: The number of sheets of on-press development is more than 20 and 25 or less.
5: The number of sheets of on-press development is more than 25 and 30 or less.
4: The number of sheets of on-press development is more than 30 and 35 or less.
3: The number of sheets of on-press development is more than 35 and 40 or less.
2: The number of sheets of on-press development is more than 40 and 45 or less.
1: The number of sheets of on-press development is more than 45.

Evaluation of on-press developability after forced aging

The obtained lithographic printing plate precursor was stored in an environment of 35°C and 80% RH for 3 days before the exposure, and then evaluated in the same manner as the on-press developability.

Evaluation standards

A: The number of printed sheets is increased by an amount equal to or more than, but less than 10%, of the evaluation result in the on-press developability evaluation described above without performing the forced aging.
B: The number of printed sheets is increased by 10% or more and less than 30% as compared with the evaluation result in the on-press developability evaluation described above without performing the forced aging.
C: The number of printed sheets is increased by 30% or more and less than 50% as compared with the evaluation result in the on-press developability evaluation described above without performing the forced aging.
D: The number of printed sheets is increased by 50% or more and less than 100% as compared with the evaluation result in the on-press developability evaluation described above without performing the forced aging.
E: The number of printed sheets is increased by 100% or more as compared with the evaluation result in the on-press developability evaluation described above without performing the forced aging.

The evaluation results are summarized in Table 1 to Table 4.

Table 1

| | Support | Undercoat layer | Image-recording layer A | | | | | | | | | | | | | | Overcoat layer A | | | Visibility | Printing durability | On-press developability | On-press developability after forced aging |
|---|---|---|---|---|---|---|---|---|---|---|---|---|---|---|---|---|---|---|---|---|---|---|---|
| | | | Compound having specific structure | | Electron-donating polymerization initiator | | Electron-accepting polymerization initiator | | Polymerizable compound | | Infrared absorber | | Acid color forming agent | | Hydrogen donor | | No. | Filler | | | | | |
| | No. | No. | Type | Addition amount (mg/m$^2$) | Type | Addition amount (mg/m$^2$) | Type | Addition amount (mg/m$^2$) | Type | Addition amount (mg/m$^2$) | Type | Addition amount (mg/m$^2$) | Type | Addition amount (mg/m$^2$) | Type | Addition amount (mg/m$^2$) | | Name | Addition amount (mg/m$^2$) | | | | |
| Example 1 | 1 | 1 | Dye-1 | 33 | TPB-1 | 30 | I-1 | 25 | M-1 | 250 | - | - | - | - | - | - | 1 | Lamellar compound | 20 | 10.5 | 6 | 5 | A |
| Example 2 | 1 | 1 | Dye-2 | 33 | TPB-1 | 30 | I-1 | 25 | M-1 | 250 | - | - | - | - | - | - | 1 | Lamellar compound | 20 | 11 | 6 | 5 | A |
| Example 3 | 1 | 1 | Dye-3 | 33 | TPB-1 | 30 | I-1 | 25 | M-1 | 250 | - | - | - | - | - | - | 1 | Lamellar compound | 20 | 11.5 | 8 | 6 | A |
| Example 4 | 1 | 1 | Dye-4 | 33 | TPB-1 | 30 | I-1 | 25 | M-1 | 250 | - | - | - | - | - | - | 1 | Lamellar compound | 20 | 12 | 8 | 6 | A |
| Example 5 | 1 | 1 | Dye-5 | 33 | TPB-1 | 30 | I-1 | 25 | M-1 | 250 | - | - | - | - | - | - | 1 | Lamellar compound | 20 | 14 | 8 | 7 | A |
| Example 6 | 1 | 1 | Dye-5 | 15 | TPB-1 | 30 | I-1 | 25 | M-1 | 250 | - | - | - | - | - | - | 1 | Lamellar compound | 20 | 10.5 | 8 | 7 | A |
| Example 7 | 1 | 1 | Dye-5 | 25 | TPB-1 | 30 | I-1 | 25 | M-1 | 250 | - | - | - | - | - | - | 1 | Lamellar compound | 20 | 12 | 8 | 7 | A |
| Example 8 | 1 | 1 | Dye-5 | 45 | TPB-1 | 30 | I-1 | 25 | M-1 | 250 | - | - | - | - | - | - | 1 | Lamellar compound | 20 | 16 | 8 | 7 | A |
| Example 9 | 1 | 1 | Dye-5 | 60 | TPB-1 | 30 | I-1 | 25 | M-1 | 250 | - | - | - | - | - | - | 1 | Lamellar compound | 20 | 17 | 6 | 7 | A |
| Example 10 | 1 | 1 | Dye-5 | 33 | TPB-1 | 30 | I-1 | 25 | M-2 | 250 | - | - | - | - | - | - | 1 | Lamellar compound | 20 | 14 | 7 | 7 | A |
| Example 11 | 1 | 1 | Dye-5 | 33 | TPB-1 | 30 | I-1 | 25 | M-3 | 250 | - | - | - | - | - | - | 1 | Lamellar compound | 20 | 14 | 7 | 7 | A |
| Example 12 | 1 | 1 | Dye-5 | 33 | TPB-1 | 30 | I-1 | 25 | M-4 | 250 | - | - | - | - | - | - | 1 | Lamellar compound | 20 | 14 | 6 | 7 | A |
| Example 13 | 1 | 1 | Dye-5 | 33 | TPB-1 | 10 | I-1 | 25 | M-1 | 250 | - | - | - | - | - | - | 1 | Lamellar compound | 20 | 10.5 | 7 | 7 | A |
| Example 14 | 1 | 1 | Dye-5 | 33 | TPB-1 | 40 | I-1 | 25 | M-1 | 250 | - | - | - | - | - | - | 1 | Lamellar compound | 20 | 15 | 8 | 7 | A |
| Example 15 | 1 | 1 | Dye-5 | 33 | TPB-1 | 50 | I-1 | 25 | M-1 | 250 | - | - | - | - | - | - | 1 | Lamellar compound | 20 | 16 | 7 | 7 | A |
| Example 16 | 1 | 1 | Dye-5 | 33 | TPB-2 | 33 | I-1 | 25 | M-1 | 250 | - | - | - | - | - | - | 1 | Lamellar compound | 20 | 14 | 8 | 7 | A |
| Example 17 | 1 | 1 | Dye-5 | 33 | TPB-2 | 10 | I-1 | 25 | M-1 | 250 | - | - | - | - | - | - | 1 | Lamellar compound | 20 | 10.5 | 7 | 7 | A |
| Example 18 | 1 | 1 | Dye-5 | 33 | TPB-2 | 40 | I-1 | 25 | M-1 | 250 | - | - | - | - | - | - | 1 | Lamellar compound | 20 | 15 | 8 | 7 | A |
| Example 19 | 1 | 1 | Dye-5 | 33 | TPB-2 | 50 | I-1 | 25 | M-1 | 250 | - | - | - | - | - | - | 1 | Lamellar compound | 20 | 16 | 7 | 7 | A |
| Example 20 | 1 | 1 | Dye-5 | 33 | TPB-1 | 30 | I-1 | 0 | M-1 | 250 | - | - | - | - | - | - | 1 | Lamellar compound | 20 | 16 | 6 | 7 | A |
| Example 21 | 1 | 1 | Dye-5 | 33 | TPB-1 | 30 | I-1 | 10 | M-1 | 250 | - | - | - | - | - | - | 1 | Lamellar compound | 20 | 15 | 7 | 7 | A |
| Example 22 | 1 | 1 | Dye-5 | 33 | TPB-1 | 30 | I-1 | 50 | M-1 | 250 | - | - | - | - | - | - | 1 | Lamellar compound | 20 | 12 | 8 | 7 | A |
| Example 23 | 1 | 1 | Dye-5 | 33 | TPB-1 | 30 | I-1 | 80 | M-1 | 250 | - | - | - | - | - | - | 1 | Lamellar compound | 20 | 11 | 8 | 7 | A |
| Example 24 | 1 | 1 | Dye-5 | 33 | TPB-1 | 30 | I-1 | 25 | M-1 | 250 | IR-1 | 5 | - | - | - | - | 1 | Lamellar compound | 20 | 13 | 8 | 7 | A |
| Example 25 | 1 | 1 | Dye-5 | 33 | TPB-1 | 30 | I-1 | 25 | M-1 | 250 | IR-1 | 10 | - | - | - | - | 1 | Lamellar compound | 20 | 12 | 8 | 7 | A |
| Example 26 | 1 | 1 | Dye-5 | 33 | TPB-1 | 30 | I-1 | 25 | M-1 | 250 | IR-1 | 20 | - | - | - | - | 1 | Lamellar compound | 20 | 11 | 8 | 7 | A |
| Example 27 | 1 | 1 | Dye-5 | 33 | TPB-1 | 30 | I-1 | 25 | M-1 | 250 | IR-1 | 30 | - | - | - | - | 1 | Lamellar compound | 20 | 10 | 8 | 7 | A |
| Example 28 | 1 | 1 | Dye-5 | 33 | TPB-1 | 30 | I-1 | 25 | M-1 | 250 | - | - | S-1 | 30 | - | - | 1 | Lamellar compound | 20 | 16 | 7 | 7 | A |
| Example 29 | 1 | 1 | Dye-5 | 33 | TPB-1 | 30 | I-1 | 25 | M-1 | 250 | - | - | S-2 | 30 | - | - | 1 | Lamellar compound | 20 | 16.5 | 7 | 7 | A |
| Example 30 | 1 | 1 | Dye-5 | 33 | TPB-1 | 30 | I-1 | 25 | M-1 | 250 | - | - | S-3 | 30 | - | - | 1 | Lamellar compound | 20 | 15 | 7 | 7 | A |
| Example 31 | 1 | 1 | Dye-5 | 33 | TPB-1 | 30 | I-1 | 25 | M-1 | 250 | - | - | - | - | P-1 | 50 | 1 | Lamellar compound | 20 | 14.5 | 8 | 7 | A |
| Example 32 | 1 | 1 | Dye-5 | 33 | TPB-1 | 30 | I-1 | 25 | M-1 | 250 | - | - | - | - | P-2 | 50 | 1 | Lamellar compound | 20 | 14.5 | 8 | 7 | A |
| Example 33 | 1 | 1 | Dye-5 | 33 | TPB-1 | 30 | I-1 | 25 | M-1 | 250 | - | - | - | - | P-3 | 50 | 1 | Lamellar compound | 20 | 14.5 | 8 | 7 | A |
| Example 34 | 1 | 1 | Dye-5 | 33 | TPB-1 | 30 | I-1 | 25 | M-1 | 250 | - | - | - | - | - | - | 2 | Lamellar compound | 0 | 14 | 6 | 7 | A |
| Example 35 | 1 | 1 | Dye-5 | 33 | TPB-1 | 30 | I-1 | 25 | M-1 | 250 | - | - | - | - | - | - | 3 | Lamellar compound | 50 | 14 | 8 | 7 | A |
| Example 36 | 2 | 1 | Dye-5 | 33 | TPB-1 | 30 | I-1 | 25 | M-1 | 250 | - | - | - | - | - | - | 1 | Lamellar compound | 20 | | | | A |
| Example 37 | 4 | 1 | Dye-5 | 33 | TPB-1 | 30 | I-1 | 25 | M-1 | 250 | - | - | - | - | - | - | 1 | Lamellar compound | 20 | 15 | 8 | 7 | A |
| Example 38 | 5 | 1 | Dye-5 | 33 | TPB-1 | 30 | I-1 | 25 | M-1 | 250 | - | - | - | - | - | - | 1 | Lamellar compound | 20 | 15 | 8 | 7 | A |
| Example 39 | 1 | 1 | Dye-5 | 33 | TPB-1 | 30 | I-1 | 25 | M-1 | 250 | - | - | - | - | - | - | - | - | - | 12.5 | 6 | 7 | A |
| Example 40 | 1 | 2 | Dye-5 | 33 | TPB-1 | 30 | I-2/I-3 | 12/12 | M-2 | 250 | - | - | - | - | - | - | - | - | - | 13 | 7 | 7 | A |
| Example 41 | 1 | 1 | Dye-6 | 33 | TPB-1 | 30 | I-1 | 25 | M-1 | 250 | - | - | - | - | - | - | 1 | Lamellar compound | 20 | 14 | 8 | 7 | A |
| Example 42 | 1 | 1 | Dye-7 | 33 | TPB-1 | 30 | I-1 | 25 | M-1 | 250 | - | - | - | - | - | - | 1 | Lamellar compound | 20 | 14 | 8 | 7 | A |
| Example 43 | 1 | 1 | Dye-8 | 33 | TPB-1 | 30 | I-1 | 25 | M-1 | 250 | - | - | - | - | - | - | 1 | Lamellar compound | 20 | 11 | 7 | 6 | A |
| Example 44 | 1 | 1 | Dye-9 | 33 | TPB-1 | 30 | I-1 | 25 | M-1 | 250 | - | - | - | - | - | - | 1 | Lamellar compound | 20 | 12 | 8 | 5 | A |
| Example 45 | 1 | 1 | Dye-10 | 33 | TPB-1 | 30 | I-1 | 25 | M-1 | 250 | - | - | - | - | - | - | 1 | Lamellar compound | 20 | 14 | 8 | 5 | A |
| Example 46 | 1 | 1 | Dye-11 | 33 | TPB-1 | 30 | I-1 | 25 | M-1 | 250 | - | - | - | - | - | - | 1 | Lamellar compound | 20 | 14 | 8 | 5 | A |
| Example 47 | 1 | 1 | Dye-12 | 33 | TPB-1 | 30 | I-1 | 25 | M-1 | 250 | - | - | - | - | - | - | 1 | Lamellar compound | 20 | 14 | 8 | 7 | A |

| | | | | | | | | | | | | | | | | | | | | | | | |
|---|---|---|---|---|---|---|---|---|---|---|---|---|---|---|---|---|---|---|---|---|---|---|---|
| Example 48 | 1 | 1 | Dye-13 | 33 | TPB-1 | 30 | I-1 | 25 | M-1 | 250 | - | - | - | - | - | - | 1 | Lamellar compound | 20 | 14 | 8 | 5 | A |
| Example 49 | 1 | 1 | Dye-14 | 33 | TPB-1 | 30 | I-1 | 25 | M-1 | 250 | - | - | - | - | - | - | 1 | Lamellar compound | 20 | 14 | 8 | 5 | A |
| Example 50 | 1 | 1 | Dye-15 | 33 | TPB-1 | 30 | I-1 | 25 | M-1 | 250 | - | - | - | - | - | - | 1 | Lamellar compound | 20 | 14 | 8 | 5 | A |
| Example 51 | 1 | 1 | Dye-16 | 33 | TPB-1 | 30 | I-1 | 25 | M-1 | 250 | - | - | - | - | - | - | 1 | Lamellar compound | 20 | 14 | 8 | 5 | A |
| Example 52 | 1 | 1 | Dye-17 | 33 | TPB-1 | 30 | I-1 | 25 | M-1 | 250 | - | - | - | - | - | - | 1 | Lamellar compound | 20 | 14 | 8 | 5 | A |
| Example 53 | 1 | 1 | Dye-18 | 33 | TPB-1 | 30 | I-1 | 25 | M-1 | 250 | - | - | - | - | - | - | 1 | Lamellar compound | 20 | 12 | 7 | 6 | A |
| Example 54 | 1 | 1 | Dye-19 | 33 | TPB-1 | 30 | I-1 | 25 | M-1 | 250 | - | - | - | - | - | - | 1 | Lamellar compound | 20 | 12 | 7 | 6 | A |
| Example 55 | 1 | 1 | Dye-20 | 33 | TPB-1 | 30 | I-1 | 25 | M-1 | 250 | - | - | - | - | - | - | 1 | Lamellar compound | 20 | 12 | 7 | 6 | A |
| Example 56 | 1 | 1 | Dye-21 | 33 | TPB-1 | 30 | I-1 | 25 | M-1 | 250 | - | - | - | - | - | - | 1 | Lamellar compound | 20 | 12 | 7 | 6 | A |
| Example 57 | 1 | 1 | Dye-22 | 33 | TPB-1 | 30 | I-1 | 25 | M-1 | 250 | - | - | - | - | - | - | 1 | Lamellar compound | 20 | 12 | 7 | 6 | A |
| Example 58 | 1 | 1 | Dye-23 | 33 | TPB-1 | 30 | I-1 | 25 | M-1 | 250 | - | - | - | - | - | - | 1 | Lamellar compound | 20 | 11 | 7 | 5 | A |
| Example 59 | 1 | 1 | Dye-24 | 33 | TPB-1 | 30 | I-1 | 25 | M-1 | 250 | - | - | - | - | - | - | 1 | Lamellar compound | 20 | 13 | 7 | 6 | A |
| Example 60 | 1 | 1 | Dye-25 | 33 | TPB-1 | 30 | I-1 | 25 | M-1 | 250 | - | - | - | - | - | - | 1 | Lamellar compound | 20 | 13 | 7 | 6 | A |
| Example 61 | 1 | 1 | Dye-26 | 33 | TPB-1 | 30 | I-1 | 25 | M-1 | 250 | - | - | - | - | - | - | 1 | Lamellar compound | 20 | 13 | 7 | 6 | A |
| Example 62 | 1 | 1 | Dye-27 | 33 | TPB-1 | 30 | I-1 | 25 | M-1 | 250 | - | - | - | - | - | - | 1 | Lamellar compound | 20 | 13 | 7 | 6 | A |
| Example 63 | 1 | 1 | Dye-28 | 33 | TPB-1 | 30 | I-1 | 25 | M-1 | 250 | - | - | - | - | - | - | 1 | Lamellar compound | 20 | 13 | 7 | 6 | A |
| Example 64 | 1 | 1 | Dye-29 | 33 | TPB-1 | 30 | I-1 | 25 | M-1 | 250 | - | - | - | - | - | - | 1 | Lamellar compound | 20 | 16 | 10 | 7 | A |
| Example 65 | 1 | 1 | Dye-29 | 15 | TPB-1 | 30 | I-1 | 25 | M-1 | 250 | - | - | - | - | - | - | 1 | Lamellar compound | 20 | 12.5 | 10 | 7 | A |
| Example 66 | 1 | 1 | Dye-29 | 25 | TPB-1 | 30 | I-1 | 25 | M-1 | 250 | - | - | - | - | - | - | 1 | Lamellar compound | 20 | 14 | 10 | 7 | A |
| Example 67 | 1 | 1 | Dye-29 | 45 | TPB-1 | 30 | I-1 | 25 | M-1 | 250 | - | - | - | - | - | - | 1 | Lamellar compound | 20 | 18 | 10 | 7 | A |
| Example 68 | 1 | 1 | Dye-29 | 60 | TPB-1 | 30 | I-1 | 25 | M-1 | 250 | - | - | - | - | - | - | 1 | Lamellar compound | 20 | 19 | 8 | 7 | A |
| Example 69 | 1 | 1 | Dye-29 | 33 | TPB-1 | 30 | I-1 | 25 | M-2 | 250 | - | - | - | - | - | - | 1 | Lamellar compound | 20 | 16 | 9 | 7 | A |
| Example 70 | 1 | 1 | Dye-29 | 33 | TPB-1 | 30 | I-1 | 25 | M-3 | 250 | - | - | - | - | - | - | 1 | Lamellar compound | 20 | 16 | 9 | 7 | A |
| Example 71 | 1 | 1 | Dye-29 | 33 | TPB-1 | 30 | I-1 | 25 | M-4 | 250 | - | - | - | - | - | - | 1 | Lamellar compound | 20 | 16 | 8 | 7 | A |
| Example 72 | 1 | 1 | Dye-29 | 33 | TPB-1 | 10 | I-1 | 25 | M-1 | 250 | - | - | - | - | - | - | 1 | Lamellar compound | 20 | 12.5 | 9 | 7 | A |
| Example 73 | 1 | 1 | Dye-29 | 33 | TPB-1 | 40 | I-1 | 25 | M-1 | 250 | - | - | - | - | - | - | 1 | Lamellar compound | 20 | 17 | 10 | 7 | A |
| Example 74 | 1 | 1 | Dye-29 | 33 | TPB-1 | 50 | I-1 | 25 | M-1 | 250 | - | - | - | - | - | - | 1 | Lamellar compound | 20 | 18 | 9 | 7 | A |
| Example 75 | 1 | 1 | Dye-29 | 33 | TPB-1 | 33 | I-1 | 25 | M-1 | 250 | - | - | - | - | - | - | 1 | Lamellar compound | 20 | 16 | 10 | 7 | A |
| Example 76 | 1 | 1 | Dye-29 | 33 | TPB-1 | 10 | I-1 | 25 | M-1 | 250 | - | - | - | - | - | - | 1 | Lamellar compound | 20 | 12.5 | 9 | 7 | A |
| Example 77 | 1 | 1 | Dye-29 | 33 | TPB-1 | 40 | I-1 | 25 | M-1 | 250 | - | - | - | - | - | - | 1 | Lamellar compound | 20 | 17 | 10 | 7 | A |
| Example 78 | 1 | 1 | Dye-29 | 33 | TPB-1 | 50 | I-1 | 25 | M-1 | 250 | - | - | - | - | - | - | 1 | Lamellar compound | 20 | 18 | 9 | 7 | A |
| Example 79 | 1 | 1 | Dye-29 | 33 | TPB-1 | 30 | I-1 | 25 | M-1 | 250 | - | - | - | - | - | - | 1 | Lamellar compound | 20 | 16 | 10 | 7 | A |
| Example 80 | 1 | 1 | Dye-29 | 33 | TPB-1 | 30 | I-1 | 25 | M-1 | 250 | - | - | - | - | - | - | 1 | Lamellar compound | 20 | 16 | 10 | 7 | A |

Table 2

| | Support | Undercoat layer | Image-recording layer A | | | | | | | | | | | | | | Overcoat layer A | | | Visibility | Printing durability | On-press developability | On-press developability after forced aging |
|---|---|---|---|---|---|---|---|---|---|---|---|---|---|---|---|---|---|---|---|---|---|---|---|
| | | | Compound having specific structure | | Electron-donating polymerization initiator | | Electron-accepting polymerization initiator | | Polymerizable compound | | Infrared absorber | | Acid color forming agent | | Hydrogen donor | | | Filler | | | | | |
| | No. | No. | Type | Addition amount (mg/m²) | Type | Addition amount (mg/m²) | Type | Addition amount (mg/m²) | Type | Addition amount (mg/m²) | Type | Addition amount (mg/m²) | Type | Addition amount (mg/m²) | Type | Addition amount (mg/m²) | No. | Name | Addition amount (mg/m²) | | | | |
| Example 81 | 1 | 1 | Dye-29 | 33 | TPB-1 | 30 | I-1 | 0 | M-1 | 250 | - | - | - | - | - | - | 1 | Lamellar compound | 20 | 18 | 8 | 7 | A |
| Example 82 | 1 | 1 | Dye-29 | 33 | TPB-1 | 30 | I-1 | 10 | M-1 | 250 | - | - | - | - | - | - | 1 | Lamellar compound | 20 | 17 | 9 | 7 | A |
| Example 83 | 1 | 1 | Dye-29 | 33 | TPB-1 | 30 | I-1 | 50 | M-1 | 250 | - | - | - | - | - | - | 1 | Lamellar compound | 20 | 14 | 10 | 7 | A |
| Example 84 | 1 | 1 | Dye-29 | 33 | TPB-1 | 30 | I-1 | 80 | M-1 | 250 | - | - | - | - | - | - | 1 | Lamellar compound | 20 | 13 | 10 | 7 | A |
| Example 85 | 1 | 1 | Dye-29 | 33 | TPB-1 | 30 | I-1 | 25 | M-1 | 250 | IR-1 | 5 | - | - | - | - | 1 | Lamellar compound | 20 | 15 | 10 | 7 | A |
| Example 86 | 1 | 1 | Dye-29 | 33 | TPB-1 | 30 | I-1 | 25 | M-1 | 250 | IR-1 | 10 | - | - | - | - | 1 | Lamellar compound | 20 | 14 | 10 | 7 | A |
| Example 87 | 1 | 1 | Dye-29 | 33 | TPB-1 | 30 | I-1 | 25 | M-1 | 250 | IR-1 | 20 | - | - | - | - | 1 | Lamellar compound | 20 | 13 | 10 | 7 | A |
| Example 88 | 1 | 1 | Dye-29 | 33 | TPB-1 | 30 | I-1 | 25 | M-1 | 250 | IR-1 | 30 | - | - | - | - | 1 | Lamellar compound | 20 | 12 | 10 | 7 | A |
| Example 89 | 1 | 1 | Dye-29 | 33 | TPB-1 | 30 | I-1 | 25 | M-1 | 250 | - | - | S-1 | 30 | - | - | 1 | Lamellar compound | 20 | 18 | 9 | 7 | A |
| Example 90 | 1 | 1 | Dye-29 | 33 | TPB-1 | 30 | I-1 | 25 | M-1 | 250 | - | - | S-2 | 30 | - | - | 1 | Lamellar compound | 20 | 18.5 | 9 | 7 | A |
| Example 91 | 1 | 1 | Dye-29 | 33 | TPB-1 | 30 | I-1 | 25 | M-1 | 250 | - | - | S-3 | 30 | - | - | 1 | Lamellar compound | 20 | 17 | 9 | 7 | A |
| Example 92 | 1 | 1 | Dye-29 | 33 | TPB-1 | 30 | I-1 | 25 | M-1 | 250 | - | - | - | - | P-1 | 50 | 1 | Lamellar compound | 20 | 16.5 | 10 | 7 | A |
| Example 93 | 1 | 1 | Dye-29 | 33 | TPB-1 | 30 | I-1 | 25 | M-1 | 250 | - | - | - | - | P-2 | 50 | 1 | Lamellar compound | 20 | 16.5 | 10 | 7 | A |
| Example 94 | 1 | 1 | Dye-29 | 33 | TPB-1 | 30 | I-1 | 25 | M-1 | 250 | - | - | - | - | P-3 | 50 | 1 | Lamellar compound | 20 | 16.5 | 10 | 7 | A |
| Example 95 | 1 | 1 | Dye-29 | 33 | TPB-1 | 30 | I-1 | 25 | M-1 | 250 | - | - | - | - | - | - | 2 | Lamellar compound | 0 | 16 | 8 | 7 | A |
| Example 96 | 1 | 1 | Dye-29 | 33 | TPB-1 | 30 | I-1 | 25 | M-1 | 250 | - | - | - | - | - | - | 3 | Lamellar compound | 50 | 16 | 10 | 7 | A |
| Example 97 | 2 | 1 | Dye-29 | 33 | TPB-1 | 30 | I-1 | 25 | M-1 | 250 | - | - | - | - | - | - | 1 | Lamellar compound | 20 | 17 | 10 | 7 | A |
| Example 98 | 4 | 1 | Dye-29 | 33 | TPB-1 | 30 | I-1 | 25 | M-1 | 250 | - | - | - | - | - | - | 1 | Lamellar compound | 20 | 17 | 10 | 7 | A |
| Example 99 | 5 | 1 | Dye-29 | 33 | TPB-1 | 30 | I-1 | 25 | M-1 | 250 | - | - | - | - | - | - | 1 | Lamellar compound | 20 | 17 | 10 | 7 | A |
| Example 100 | 1 | 1 | Dye-29 | 33 | TPB-1 | 30 | I-1 | 25 | M-1 | 250 | - | - | - | - | - | - | - | - | - | 14.5 | 8 | 7 | A |
| Example 101 | 1 | 2 | Dye-29 | 33 | TPB-1 | 30 | I-2/I-3 | 12/12 | M-2 | 250 | - | - | - | - | - | - | - | - | - | 15 | 9 | 7 | A |
| Example 102 | 1 | 1 | Dye-30 | 33 | TPB-1 | 30 | I-1 | 25 | M-1 | 250 | - | - | - | - | - | - | 1 | Lamellar compound | 20 | 14.5 | 10 | 6 | A |
| Example 103 | 1 | 1 | Dye-31 | 33 | TPB-1 | 30 | I-1 | 25 | M-1 | 250 | - | - | - | - | - | - | 1 | Lamellar compound | 20 | 15 | 10 | 6 | A |
| Example 104 | 1 | 1 | Dye-32 | 33 | TPB-1 | 30 | I-1 | 25 | M-1 | 250 | - | - | - | - | - | - | 1 | Lamellar compound | 20 | 13 | 8 | 5 | A |
| Example 105 | 1 | 1 | Dye-33 | 33 | TPB-1 | 30 | I-1 | 25 | M-1 | 250 | - | - | - | - | - | - | 1 | Lamellar compound | 20 | 14 | 9 | 5 | A |
| Example 106 | 1 | 1 | Dye-34 | 33 | TPB-1 | 30 | I-1 | 25 | M-1 | 250 | - | - | - | - | - | - | 1 | Lamellar compound | 20 | 16 | 10 | 5 | A |
| Example 107 | 1 | 1 | Dye-35 | 33 | TPB-1 | 30 | I-1 | 25 | M-1 | 250 | - | - | - | - | - | - | 1 | Lamellar compound | 20 | 16 | 10 | 5 | A |
| Example 108 | 1 | 1 | Dye-36 | 33 | TPB-1 | 30 | I-1 | 25 | M-1 | 250 | - | - | - | - | - | - | 1 | Lamellar compound | 20 | 16 | 10 | 7 | A |
| Example 109 | 1 | 1 | Dye-37 | 33 | TPB-1 | 30 | I-1 | 25 | M-1 | 250 | - | - | - | - | - | - | 1 | Lamellar compound | 20 | 16 | 10 | 5 | A |
| Example 110 | 1 | 1 | Dye-38 | 33 | TPB-1 | 30 | I-1 | 25 | M-1 | 250 | - | - | - | - | - | - | 1 | Lamellar compound | 20 | 16 | 10 | 5 | A |
| Example 111 | 1 | 1 | Dye-39 | 33 | TPB-1 | 30 | I-1 | 25 | M-1 | 250 | - | - | - | - | - | - | 1 | Lamellar compound | 20 | 16 | 10 | 5 | A |
| Example 112 | 1 | 1 | Dye-40 | 33 | TPB-1 | 30 | I-1 | 25 | M-1 | 250 | - | - | - | - | - | - | 1 | Lamellar compound | 20 | 16 | 10 | 5 | A |
| Example 113 | 1 | 1 | Dye-41 | 33 | TPB-1 | 30 | I-1 | 25 | M-1 | 250 | - | - | - | - | - | - | 1 | Lamellar compound | 20 | 16 | 10 | 6 | A |
| Example 114 | 1 | 1 | Dye-42 | 33 | TPB-1 | 30 | I-1 | 25 | M-1 | 250 | - | - | - | - | - | - | 1 | Lamellar compound | 20 | 15 | 9 | 6 | A |
| Example 115 | 1 | 1 | Dye-43 | 33 | TPB-1 | 30 | I-1 | 25 | M-1 | 250 | - | - | - | - | - | - | 1 | Lamellar compound | 20 | 13 | 9 | 6 | A |
| Example 116 | 1 | 1 | Dye-44 | 33 | TPB-1 | 30 | I-1 | 25 | M-1 | 250 | - | - | - | - | - | - | 1 | Lamellar compound | 20 | 13 | 9 | 6 | A |
| Example 117 | 1 | 1 | Dye-45 | 33 | TPB-1 | 30 | I-1 | 25 | M-1 | 250 | - | - | - | - | - | - | 1 | Lamellar compound | 20 | 13 | 9 | 6 | A |
| Example 118 | 1 | 1 | Dye-46 | 33 | TPB-1 | 30 | I-1 | 25 | M-1 | 250 | - | - | - | - | - | - | 1 | Lamellar compound | 20 | 14 | 9 | 6 | A |
| Example 119 | 1 | 1 | Dye-47 | 33 | TPB-1 | 30 | I-1 | 25 | M-1 | 250 | - | - | - | - | - | - | 1 | Lamellar compound | 20 | 14 | 9 | 6 | A |
| Example 120 | 1 | 1 | Dye-48 | 33 | TPB-1 | 30 | I-1 | 25 | M-1 | 250 | - | - | - | - | - | - | 1 | Lamellar compound | 20 | 13 | 9 | 5 | A |
| Example 121 | 1 | 1 | Dye-49 | 33 | TPB-1 | 30 | I-1 | 25 | M-1 | 250 | - | - | - | - | - | - | 1 | Lamellar compound | 20 | 15 | 9 | 6 | A |
| Example 122 | 1 | 1 | Dye-50 | 33 | TPB-1 | 30 | I-1 | 25 | M-1 | 250 | - | - | - | - | - | - | 1 | Lamellar compound | 20 | 15 | 9 | 6 | A |
| Example 123 | 1 | 1 | Dye-51 | 33 | TPB-1 | 30 | I-1 | 25 | M-1 | 250 | - | - | - | - | - | - | 1 | Lamellar compound | 20 | 15 | 9 | 6 | A |
| Example 124 | 1 | 1 | Dye-52 | 33 | TPB-1 | 30 | I-1 | 25 | M-1 | 250 | - | - | - | - | - | - | 1 | Lamellar compound | 20 | 15 | 9 | 6 | A |
| Example 125 | 1 | 1 | Dye-53 | 33 | TPB-1 | 30 | I-1 | 25 | M-1 | 250 | - | - | - | - | - | - | 1 | Lamellar compound | 20 | 16 | 10 | 6 | A |
| Example 126 | 1 | 1 | Dye-54 | 33 | TPB-1 | 30 | I-1 | 25 | M-1 | 250 | - | - | - | - | - | - | 1 | Lamellar compound | 20 | 15 | 10 | 6 | A |
| Example 127 | 1 | 1 | Dye-55 | 33 | TPB-1 | 30 | I-1 | 25 | M-1 | 250 | - | - | - | - | - | - | 1 | Lamellar compound | 20 | 13 | 8 | 6 | A |

| | | | | | | | | | | | | | | | | | | | | | | |
|---|---|---|---|---|---|---|---|---|---|---|---|---|---|---|---|---|---|---|---|---|---|---|
| Example 128 | 1 | 1 | Dye-56 | 33 | TPB-1 | 30 | I-1 | 25 | M-1 | 250 | - | - | - | - | - | - | 1 | Lamellar compound | 20 | 13 | 8 | 6 | A |
| Example 129 | 1 | 1 | Dye-57 | 33 | TPB-1 | 30 | I-1 | 25 | M-1 | 250 | - | - | - | - | - | - | 1 | Lamellar compound | 20 | 13 | 8 | 6 | A |
| Example 130 | 1 | 1 | Dye-58 | 33 | TPB-1 | 30 | I-1 | 25 | M-1 | 250 | - | - | - | - | - | - | 1 | Lamellar compound | 20 | 15 | 10 | 5 | A |
| Example 131 | 1 | 1 | Dye-59 | 33 | TPB-1 | 30 | I-1 | 25 | M-1 | 250 | - | - | - | - | - | - | 1 | Lamellar compound | 20 | 15 | 10 | 5 | A |
| Example 132 | 1 | 1 | Dye-60 | 33 | TPB-1 | 30 | I-1 | 25 | M-1 | 250 | - | - | - | - | - | - | 1 | Lamellar compound | 20 | 15 | 10 | 5 | A |
| Example 133 | 1 | 1 | Dye-61 | 33 | TPB-1 | 30 | I-1 | 25 | M-1 | 250 | - | - | - | - | - | - | 1 | Lamellar compound | 20 | 15 | 10 | 5 | A |
| Example 134 | 1 | 1 | Dye-62 | 33 | TPB-1 | 30 | I-1 | 25 | M-1 | 250 | - | - | - | - | - | - | 1 | Lamellar compound | 20 | 15 | 10 | 5 | A |
| Example 135 | 1 | 1 | Dye-63 | 33 | TPB-1 | 30 | I-1 | 25 | M-1 | 250 | - | - | - | - | - | - | 1 | Lamellar compound | 20 | 14 | 9 | 6 | A |
| Example 136 | 1 | 1 | Dye-64 | 33 | TPB-1 | 30 | I-1 | 25 | M-1 | 250 | - | - | - | - | - | - | 1 | Lamellar compound | 20 | 13 | 8 | 5 | A |
| Example 137 | 1 | 1 | Dye-65 | 33 | TPB-1 | 30 | I-1 | 25 | M-1 | 250 | - | - | - | - | - | - | 1 | Lamellar compound | 20 | 12 | 7 | 5 | A |
| Comparative Example 1 | 1 | 1 | Dye-A | 33 | TPB-1 | 30 | I-1 | 25 | M-1 | 250 | - | - | - | - | - | - | 1 | Lamellar compound | 20 | 10 | 5 | 6 | B |
| Comparative Example 2 | 1 | 1 | Dye-B | 33 | TPB-1 | 30 | I-1 | 25 | M-1 | 250 | - | - | - | - | - | - | 1 | Lamellar compound | 20 | 10 | 5 | 5 | C |
| Comparative Example 3 | 1 | 1 | Dye-C | 33 | TPB-1 | 30 | I-1 | 25 | M-1 | 250 | - | - | - | - | - | - | 1 | Lamellar compound | 20 | 3 | 2 | 5 | C |
| Comparative Example 4 | 1 | 1 | Dye-D | 33 | TPB-1 | 30 | I-2/I-3 | 12/12 | M-2 | 250 | - | - | - | - | - | - | 1 | Lamellar compound | 20 | 3 | 2 | 5 | C |
| Comparative Example 5 | 1 | 1 | Dye-E | 33 | TPB-1 | 30 | I-2/I-3 | 12/12 | M-2 | 250 | - | - | - | - | - | - | 1 | Lamellar compound | 20 | 3 | 2 | 5 | C |

Table 3

| | Support | Undercoat layer | Image-recording layer B | | | | | | | | | | | | | | Overcoat layer B | | | Backcoat layer B | Visibility | Printing durability | On-press developability | On-press developability after forced aging |
|---|---|---|---|---|---|---|---|---|---|---|---|---|---|---|---|---|---|---|---|---|---|---|---|---|
| | | | Compound having specific structure | | Electron-donating polymerization initiator | | Electron-accepting polymerization initiator | | Polymerizable compound | | Infrared absorber | | Acid color forming agent | | Hydrogen donor | | No. | Filler | | | | | | |
| | No. | No. | Type | Addition amount (mg/m²) | Type | Addition amount (mg/m²) | Type | Addition amount (mg/m²) | Type | Addition amount (mg/m²) | Type | Addition amount (mg/m²) | Type | Addition amount (mg/m²) | Type | Addition amount (mg/m²) | | Name | Addition amount (mg/m²) | | | | | |
| Example 138 | 3 | 1 | Dye-1 | 43 | TPB-1 | 30 | I-1 | 12 | M-1 | 500 | - | - | - | - | - | - | 4 | Lamellar compound | 20 | Present | 11 | 6 | 5 | A |
| Example 139 | 3 | 1 | Dye-2 | 43 | TPB-1 | 30 | I-1 | 12 | M-1 | 500 | - | - | - | - | - | - | 4 | Lamellar compound | 20 | Present | 11.5 | 6 | 5 | A |
| Example 140 | 3 | 1 | Dye-3 | 43 | TPB-1 | 30 | I-1 | 12 | M-1 | 500 | - | - | - | - | - | - | 4 | Lamellar compound | 20 | Present | 13.5 | 8 | 6 | A |
| Example 141 | 3 | 1 | Dye-4 | 43 | TPB-1 | 30 | I-1 | 12 | M-1 | 500 | - | - | - | - | - | - | 4 | Lamellar compound | 20 | Present | 14 | 8 | 6 | A |
| Example 142 | 3 | 1 | Dye-5 | 43 | TPB-1 | 30 | I-1 | 12 | M-1 | 500 | - | - | - | - | - | - | 4 | Lamellar compound | 20 | Present | 16 | 8 | 7 | A |
| Example 143 | 3 | 1 | Dye-5 | 15 | TPB-1 | 30 | I-1 | 12 | M-1 | 500 | - | - | - | - | - | - | 4 | Lamellar compound | 20 | Present | 12.5 | 8 | 7 | A |
| Example 144 | 3 | 1 | Dye-5 | 25 | TPB-1 | 30 | I-1 | 12 | M-1 | 500 | - | - | - | - | - | - | 4 | Lamellar compound | 20 | Present | 14 | 8 | 7 | A |
| Example 145 | 3 | 1 | Dye-5 | 35 | TPB-1 | 30 | I-1 | 12 | M-1 | 500 | - | - | - | - | - | - | 4 | Lamellar compound | 20 | Present | 15 | 8 | 7 | A |
| Example 146 | 3 | 1 | Dye-5 | 60 | TPB-1 | 30 | I-1 | 12 | M-1 | 500 | - | - | - | - | - | - | 4 | Lamellar compound | 20 | Present | 19 | 6 | 7 | A |
| Example 147 | 3 | 1 | Dye-5 | 43 | TPB-1 | 30 | I-1 | 12 | M-2 | 500 | - | - | - | - | - | - | 4 | Lamellar compound | 20 | Present | 16 | 7 | 7 | A |
| Example 148 | 3 | 1 | Dye-5 | 43 | TPB-1 | 30 | I-1 | 12 | M-3 | 500 | - | - | - | - | - | - | 4 | Lamellar compound | 20 | Present | 16 | 7 | 7 | A |
| Example 149 | 3 | 1 | Dye-5 | 43 | TPB-1 | 30 | I-1 | 12 | M-4 | 500 | - | - | - | - | - | - | 4 | Lamellar compound | 20 | Present | 16 | 6 | 7 | A |
| Example 150 | 3 | 1 | Dye-5 | 43 | TPB-1 | 10 | I-1 | 12 | M-1 | 500 | - | - | - | - | - | - | 4 | Lamellar compound | 20 | Present | 12.5 | 7 | 7 | A |
| Example 151 | 3 | 1 | Dye-5 | 43 | TPB-1 | 40 | I-1 | 12 | M-1 | 500 | - | - | - | - | - | - | 4 | Lamellar compound | 20 | Present | 17 | 8 | 7 | A |
| Example 152 | 3 | 1 | Dye-5 | 43 | TPB-1 | 50 | I-1 | 12 | M-1 | 500 | - | - | - | - | - | - | 4 | Lamellar compound | 20 | Present | 18 | 7 | 7 | A |
| Example 153 | 3 | 1 | Dye-5 | 43 | TPB-2 | 33 | I-1 | 12 | M-1 | 500 | - | - | - | - | - | - | 4 | Lamellar compound | 20 | Present | 16 | 8 | 7 | A |
| Example 154 | 3 | 1 | Dye-5 | 43 | TPB-2 | 10 | I-1 | 12 | M-1 | 500 | - | - | - | - | - | - | 4 | Lamellar compound | 20 | Present | 12.5 | 7 | 7 | A |
| Example 155 | 3 | 1 | Dye-5 | 43 | TPB-2 | 40 | I-1 | 12 | M-1 | 500 | - | - | - | - | - | - | 4 | Lamellar compound | 20 | Present | 17 | 8 | 7 | A |
| Example 156 | 3 | 1 | Dye-5 | 43 | TPB-2 | 50 | I-1 | 12 | M-1 | 500 | - | - | - | - | - | - | 4 | Lamellar compound | 20 | Present | 18 | 7 | 7 | A |
| Example 157 | 3 | 1 | Dye-5 | 43 | TPB-1 | 30 | I-1 | 0 | M-1 | 500 | - | - | - | - | - | - | 4 | Lamellar compound | 20 | Present | 18 | 6 | 7 | A |
| Example 158 | 3 | 1 | Dye-5 | 43 | TPB-1 | 30 | I-1 | 20 | M-1 | 500 | - | - | - | - | - | - | 4 | Lamellar compound | 20 | Present | 14 | 7 | 7 | A |
| Example 159 | 3 | 1 | Dye-5 | 43 | TPB-1 | 30 | I-1 | 30 | M-1 | 500 | - | - | - | - | - | - | 4 | Lamellar compound | 20 | Present | 13 | 8 | 7 | A |
| Example 160 | 3 | 1 | Dye-5 | 43 | TPB-1 | 30 | I-1 | 12 | M-1 | 500 | IR-1 | 5 | - | - | - | - | 4 | Lamellar compound | 20 | Present | 15 | 8 | 7 | A |
| Example 161 | 3 | 1 | Dye-5 | 43 | TPB-1 | 30 | I-1 | 12 | M-1 | 500 | IR-1 | 10 | - | - | - | - | 4 | Lamellar compound | 20 | Present | 14 | 8 | 7 | A |
| Example 162 | 3 | 1 | Dye-5 | 43 | TPB-1 | 30 | I-1 | 12 | M-1 | 500 | IR-1 | 20 | - | - | - | - | 4 | Lamellar compound | 20 | Present | 13 | 8 | 7 | A |
| Example 163 | 3 | 1 | Dye-5 | 43 | TPB-1 | 30 | I-1 | 12 | M-1 | 500 | IR-1 | 30 | - | - | - | - | 4 | Lamellar compound | 20 | Present | 12 | 8 | 7 | A |
| Example 164 | 3 | 1 | Dye-5 | 43 | TPB-1 | 30 | I-1 | 12 | M-1 | 500 | - | - | S-1 | 30 | - | - | 4 | Lamellar compound | 20 | Present | 18 | 7 | 7 | A |
| Example 165 | 3 | 1 | Dye-5 | 43 | TPB-1 | 30 | I-1 | 12 | M-1 | 500 | - | - | S-2 | 30 | - | - | 4 | Lamellar compound | 20 | Present | 18.5 | 7 | 7 | A |
| Example 166 | 3 | 1 | Dye-5 | 43 | TPB-1 | 30 | I-1 | 12 | M-1 | 500 | - | - | S-3 | 30 | - | - | 4 | Lamellar compound | 20 | Present | 17 | 7 | 7 | A |
| Example 167 | 3 | 1 | Dye-5 | 43 | TPB-1 | 30 | I-1 | 12 | M-1 | 500 | - | - | - | - | P-1 | 50 | 4 | Lamellar compound | 20 | Present | 16.5 | 8 | 7 | A |
| Example 168 | 3 | 1 | Dye-5 | 43 | TPB-1 | 30 | I-1 | 12 | M-1 | 500 | - | - | - | - | P-2 | 50 | 4 | Lamellar compound | 20 | Present | 16.5 | 8 | 7 | A |
| Example 169 | 3 | 1 | Dye-5 | 43 | TPB-1 | 30 | I-1 | 12 | M-1 | 500 | - | - | - | - | P-3 | 50 | 4 | Lamellar compound | 20 | Present | 16.5 | 8 | 7 | A |
| Example 170 | 3 | 1 | Dye-6 | 43 | TPB-1 | 30 | I-1 | 12 | M-1 | 500 | - | - | - | - | - | - | 4 | Lamellar compound | 20 | Present | 16 | 8 | 7 | A |
| Example 171 | 3 | 1 | Dye-7 | 43 | TPB-1 | 30 | I-1 | 12 | M-1 | 500 | - | - | - | - | - | - | 4 | Lamellar compound | 20 | Present | 16 | 8 | 7 | A |
| Example 172 | 3 | 1 | Dye-8 | 43 | TPB-1 | 30 | I-1 | 12 | M-1 | 500 | - | - | - | - | - | - | 4 | Lamellar compound | 20 | Present | 13 | 7 | 6 | A |
| Example 173 | 3 | 1 | Dye-9 | 43 | TPB-1 | 30 | I-1 | 12 | M-1 | 500 | - | - | - | - | - | - | 4 | Lamellar compound | 20 | Present | 14 | 8 | 5 | A |
| Example 174 | 3 | 1 | Dye-10 | 43 | TPB-1 | 30 | I-1 | 12 | M-1 | 500 | - | - | - | - | - | - | 4 | Lamellar compound | 20 | Present | 16 | 8 | 5 | A |
| Example 175 | 3 | 1 | Dye-11 | 43 | TPB-1 | 30 | I-1 | 12 | M-1 | 500 | - | - | - | - | - | - | 4 | Lamellar compound | 20 | Present | 16 | 8 | 5 | A |
| Example 176 | 3 | 1 | Dye-12 | 43 | TPB-1 | 30 | I-1 | 12 | M-1 | 500 | - | - | - | - | - | - | 4 | Lamellar compound | 20 | Present | 16 | 8 | 7 | A |
| Example 177 | 3 | 1 | Dye-13 | 43 | TPB-1 | 30 | I-1 | 12 | M-1 | 500 | - | - | - | - | - | - | 4 | Lamellar compound | 20 | Present | 16 | 8 | 5 | A |
| Example 178 | 3 | 1 | Dye-14 | 43 | TPB-1 | 30 | I-1 | 12 | M-1 | 500 | - | - | - | - | - | - | 4 | Lamellar compound | 20 | Present | 16 | 8 | 5 | A |
| Example 179 | 3 | 1 | Dye-15 | 43 | TPB-1 | 30 | I-1 | 12 | M-1 | 500 | - | - | - | - | - | - | 4 | Lamellar compound | 20 | Present | 16 | 8 | 5 | A |
| Example 180 | 3 | 1 | Dye-16 | 43 | TPB-1 | 30 | I-1 | 12 | M-1 | 500 | - | - | - | - | - | - | 4 | Lamellar compound | 20 | Present | 16 | 8 | 5 | A |

| | | | | | | | | | | | | | | | | | | | | | | | | |
|---|---|---|---|---|---|---|---|---|---|---|---|---|---|---|---|---|---|---|---|---|---|---|---|---|
| Example 181 | 3 | 1 | Dye-17 | 43 | TPB-1 | 30 | I-1 | 12 | M-1 | 500 | - | - | - | - | - | - | 4 | Lamellar compound | 20 | Present | 16 | 8 | 5 | A |
| Example 182 | 3 | 1 | Dye-18 | 43 | TPB-1 | 30 | I-1 | 12 | M-1 | 500 | - | - | - | - | - | - | 4 | Lamellar compound | 20 | Present | 14 | 7 | 6 | A |
| Example 183 | 3 | 1 | Dye-19 | 43 | TPB-1 | 30 | I-1 | 12 | M-1 | 500 | - | - | - | - | - | - | 4 | Lamellar compound | 20 | Present | 14 | 7 | 6 | A |
| Example 184 | 3 | 1 | Dye-20 | 43 | TPB-1 | 30 | I-1 | 12 | M-1 | 500 | - | - | - | - | - | - | 4 | Lamellar compound | 20 | Present | 14 | 7 | 6 | A |
| Example 185 | 3 | 1 | Dye-21 | 43 | TPB-1 | 30 | I-1 | 12 | M-1 | 500 | - | - | - | - | - | - | 4 | Lamellar compound | 20 | Present | 14 | 7 | 6 | A |
| Example 186 | 3 | 1 | Dye-22 | 43 | TPB-1 | 30 | I-1 | 12 | M-1 | 500 | - | - | - | - | - | - | 4 | Lamellar compound | 20 | Present | 14 | 7 | 6 | A |
| Example 187 | 3 | 1 | Dye-23 | 43 | TPB-1 | 30 | I-1 | 12 | M-1 | 500 | - | - | - | - | - | - | 4 | Lamellar compound | 20 | Present | 13 | 7 | 5 | A |
| Example 188 | 3 | 1 | Dye-24 | 43 | TPB-1 | 30 | I-1 | 12 | M-1 | 500 | - | - | - | - | - | - | 4 | Lamellar compound | 20 | Present | 15 | 7 | 6 | A |
| Example 189 | 3 | 1 | Dye-25 | 43 | TPB-1 | 30 | I-1 | 12 | M-1 | 500 | - | - | - | - | - | - | 4 | Lamellar compound | 20 | Present | 15 | 7 | 6 | A |
| Example 190 | 3 | 1 | Dye-26 | 43 | TPB-1 | 30 | I-1 | 12 | M-1 | 500 | - | - | - | - | - | - | 4 | Lamellar compound | 20 | Present | 15 | 7 | 6 | A |
| Example 191 | 3 | 1 | Dye-27 | 43 | TPB-1 | 30 | I-1 | 12 | M-1 | 500 | - | - | - | - | - | - | 4 | Lamellar compound | 20 | Present | 15 | 7 | 6 | A |
| Example 192 | 3 | 1 | Dye-28 | 43 | TPB-1 | 30 | I-1 | 12 | M-1 | 500 | - | - | - | - | - | - | 4 | Lamellar compound | 20 | Present | 15 | 7 | 6 | A |
| Example 193 | 3 | 1 | Dye-29 | 43 | TPB-1 | 30 | I-1 | 12 | M-1 | 500 | - | - | - | - | - | - | 4 | Lamellar compound | 20 | Present | 18 | 10 | 7 | A |
| Example 194 | 3 | 1 | Dye-29 | 15 | TPB-1 | 30 | I-1 | 12 | M-1 | 500 | - | - | - | - | - | - | 4 | Lamellar compound | 20 | Present | 14.5 | 10 | 7 | A |
| Example 195 | 3 | 1 | Dye-29 | 25 | TPB-1 | 30 | I-1 | 12 | M-1 | 500 | - | - | - | - | - | - | 4 | Lamellar compound | 20 | Present | 16 | 10 | 7 | A |
| Example 196 | 3 | 1 | Dye-29 | 35 | TPB-1 | 30 | I-1 | 12 | M-1 | 500 | - | - | - | - | - | - | 4 | Lamellar compound | 20 | Present | 17 | 10 | 7 | A |
| Example 197 | 3 | 1 | Dye-29 | 60 | TPB-1 | 30 | I-1 | 12 | M-1 | 500 | - | - | - | - | - | - | 4 | Lamellar compound | 20 | Present | 21 | 8 | 7 | A |
| Example 198 | 3 | 1 | Dye-29 | 43 | TPB-1 | 30 | I-1 | 12 | M-2 | 500 | - | - | - | - | - | - | 4 | Lamellar compound | 20 | Present | 18 | 9 | 7 | A |
| Example 199 | 3 | 1 | Dye-29 | 43 | TPB-1 | 30 | I-1 | 12 | M-3 | 500 | - | - | - | - | - | - | 4 | Lamellar compound | 20 | Present | 18 | 9 | 7 | A |
| Example 200 | 3 | 1 | Dye-29 | 43 | TPB-1 | 30 | I-1 | 12 | M-4 | 500 | - | - | - | - | - | - | 4 | Lamellar compound | 20 | Present | 18 | 8 | 7 | A |
| Example 201 | 3 | 1 | Dye-29 | 43 | TPB-1 | 10 | I-1 | 12 | M-1 | 500 | - | - | - | - | - | - | 4 | Lamellar compound | 20 | Present | 14.5 | 9 | 7 | A |
| Example 202 | 3 | 1 | Dye-29 | 43 | TPB-1 | 40 | I-1 | 12 | M-1 | 500 | - | - | - | - | - | - | 4 | Lamellar compound | 20 | Present | 19 | 10 | 7 | A |
| Example 203 | 3 | 1 | Dye-29 | 43 | TPB-1 | 50 | I-1 | 12 | M-1 | 500 | - | - | - | - | - | - | 4 | Lamellar compound | 20 | Present | 20 | 9 | 7 | A |
| Example 204 | 3 | 1 | Dye-29 | 43 | TPB-2 | 33 | I-1 | 12 | M-1 | 500 | - | - | - | - | - | - | 4 | Lamellar compound | 20 | Present | 18 | 10 | 7 | A |
| Example 205 | 3 | 1 | Dye-29 | 43 | TPB-2 | 10 | I-1 | 12 | M-1 | 500 | - | - | - | - | - | - | 4 | Lamellar compound | 20 | Present | 14.5 | 9 | 7 | A |
| Example 206 | 3 | 1 | Dye-29 | 43 | TPB-2 | 40 | I-1 | 12 | M-1 | 500 | - | - | - | - | - | - | 4 | Lamellar compound | 20 | Present | 19 | 10 | 7 | A |
| Example 207 | 3 | 1 | Dye-29 | 43 | TPB-2 | 50 | I-1 | 12 | M-1 | 500 | - | - | - | - | - | - | 4 | Lamellar compound | 20 | Present | 20 | 9 | 7 | A |
| Example 208 | 3 | 1 | Dye-29 | 43 | TPB-1 | 30 | I-1 | 0 | M-1 | 500 | - | - | - | - | - | - | 4 | Lamellar compound | 20 | Present | 20 | 8 | 7 | A |
| Example 209 | 3 | 1 | Dye-29 | 43 | TPB-1 | 30 | I-1 | 20 | M-1 | 500 | - | - | - | - | - | - | 4 | Lamellar compound | 20 | Present | 16 | 9 | 7 | A |
| Example 210 | 3 | 1 | Dye-29 | 43 | TPB-1 | 30 | I-1 | 30 | M-1 | 500 | - | - | - | - | - | - | 4 | Lamellar compound | 20 | Present | 15 | 10 | 7 | A |
| Example 211 | 3 | 1 | Dye-29 | 43 | TPB-1 | 30 | I-1 | 12 | M-1 | 500 | IR-1 | 5 | - | - | - | - | 4 | Lamellar compound | 20 | Present | 17 | 10 | 7 | A |
| Example 212 | 3 | 1 | Dye-29 | 43 | TPB-1 | 30 | I-1 | 12 | M-1 | 500 | IR-1 | 10 | - | - | - | - | 4 | Lamellar compound | 20 | Present | 16 | 10 | 7 | A |
| Example 213 | 3 | 1 | Dye-29 | 43 | TPB-1 | 30 | I-1 | 12 | M-1 | 500 | IR-1 | 20 | - | - | - | - | 4 | Lamellar compound | 20 | Present | 15 | 10 | 7 | A |
| Example 214 | 3 | 1 | Dye-29 | 43 | TPB-1 | 30 | I-1 | 12 | M-1 | 500 | IR-1 | 30 | - | - | - | - | 4 | Lamellar compound | 20 | Present | 14 | 10 | 7 | A |
| Example 215 | 3 | 1 | Dye-29 | 43 | TPB-1 | 30 | I-1 | 12 | M-1 | 500 | - | - | S-1 | 30 | - | - | 4 | Lamellar compound | 20 | Present | 20 | 9 | 7 | A |
| Example 216 | 3 | 1 | Dye-29 | 43 | TPB-1 | 30 | I-1 | 12 | M-1 | 500 | - | - | S-2 | 30 | - | - | 4 | Lamellar compound | 20 | Present | 20.5 | 9 | 7 | A |
| Example 217 | 3 | 1 | Dye-29 | 43 | TPB-1 | 30 | I-1 | 12 | M-1 | 500 | - | - | S-3 | 30 | - | - | 4 | Lamellar compound | 20 | Present | 19 | 9 | 7 | A |

Table 4

| | Support | Undercoat layer | Image-recording layer B | | | | | | | | | | | | | | Overcoat layer B | | | Backcoat layer B | Visibility | Printing durability | On-press developability | On-press developability after forced aging |
|---|---|---|---|---|---|---|---|---|---|---|---|---|---|---|---|---|---|---|---|---|---|---|---|---|
| | | | Compound having specific structure | | Electron-donating polymerization initiator | | Electron-accepting polymerization initiator | | Polymerizable compound | | Infrared absorber | | Acid color forming agent | | Hydrogen donor | | | Filler | | | | | | |
| | No. | No. | Type | Addition amount ($mg/m^2$) | Type | Addition amount ($mg/m^2$) | Type | Addition amount ($mg/m^2$) | Type | Addition amount ($mg/m^2$) | Type | Addition amount ($mg/m^2$) | Type | Addition amount ($mg/m^2$) | Type | Addition amount ($mg/m^2$) | No. | Name | Addition amount ($mg/m^2$) | | | | | |
| Example 218 | 3 | 1 | Dye-29 | 43 | TPB-1 | 30 | I-1 | 12 | M-1 | 500 | - | - | - | - | P-1 | 40 | 4 | Lamellar compound | 20 | Present | 18.5 | 10 | 7 | A |
| Example 219 | 3 | 1 | Dye-29 | 43 | TPB-1 | 30 | I-1 | 12 | M-1 | 500 | - | - | - | - | P-2 | 40 | 4 | Lamellar compound | 20 | Present | 18.5 | 10 | 7 | A |
| Example 220 | 3 | 1 | Dye-29 | 43 | TPB-1 | 30 | I-1 | 12 | M-1 | 500 | - | - | - | - | P-3 | 40 | 4 | Lamellar compound | 20 | Present | 18.5 | 10 | 7 | A |
| Example 221 | 3 | 1 | Dye-30 | 43 | TPB-1 | 30 | I-1 | 12 | M-1 | 500 | - | - | - | - | - | - | 4 | Lamellar compound | 20 | Present | 16.5 | 10 | 6 | A |
| Example 222 | 3 | 1 | Dye-31 | 43 | TPB-1 | 30 | I-1 | 12 | M-1 | 500 | - | - | - | - | - | - | 4 | Lamellar compound | 20 | Present | 17 | 10 | 6 | A |
| Example 223 | 3 | 1 | Dye-32 | 43 | TPB-1 | 30 | I-1 | 12 | M-1 | 500 | - | - | - | - | - | - | 4 | Lamellar compound | 20 | Present | 15 | 8 | 5 | A |
| Example 224 | 3 | 1 | Dye-33 | 43 | TPB-1 | 30 | I-1 | 12 | M-1 | 500 | - | - | - | - | - | - | 4 | Lamellar compound | 20 | Present | 16 | 9 | 5 | A |
| Example 225 | 3 | 1 | Dye-34 | 43 | TPB-1 | 30 | I-1 | 12 | M-1 | 500 | - | - | - | - | - | - | 4 | Lamellar compound | 20 | Present | 18 | 10 | 5 | A |
| Example 226 | 3 | 1 | Dye-35 | 43 | TPB-1 | 30 | I-1 | 12 | M-1 | 500 | - | - | - | - | - | - | 4 | Lamellar compound | 20 | Present | 18 | 10 | 5 | A |
| Example 227 | 3 | 1 | Dye-36 | 43 | TPB-1 | 30 | I-1 | 12 | M-1 | 500 | - | - | - | - | - | - | 4 | Lamellar compound | 20 | Present | 18 | 10 | 7 | A |
| Example 228 | 3 | 1 | Dye-37 | 43 | TPB-1 | 30 | I-1 | 12 | M-1 | 500 | - | - | - | - | - | - | 4 | Lamellar compound | 20 | Present | 18 | 10 | 5 | A |
| Example 229 | 3 | 1 | Dye-38 | 43 | TPB-1 | 30 | I-1 | 12 | M-1 | 500 | - | - | - | - | - | - | 4 | Lamellar compound | 20 | Present | 18 | 10 | 5 | A |
| Example 230 | 3 | 1 | Dye-39 | 43 | TPB-1 | 30 | I-1 | 12 | M-1 | 500 | - | - | - | - | - | - | 4 | Lamellar compound | 20 | Present | 18 | 10 | 5 | A |
| Example 231 | 3 | 1 | Dye-40 | 43 | TPB-1 | 30 | I-1 | 12 | M-1 | 500 | - | - | - | - | - | - | 4 | Lamellar compound | 20 | Present | 18 | 10 | 5 | A |
| Example 232 | 3 | 1 | Dye-41 | 43 | TPB-1 | 30 | I-1 | 12 | M-1 | 500 | - | - | - | - | - | - | 4 | Lamellar compound | 20 | Present | 18 | 10 | 6 | A |
| Example 233 | 3 | 1 | Dye-42 | 43 | TPB-1 | 30 | I-1 | 12 | M-1 | 500 | - | - | - | - | - | - | 4 | Lamellar compound | 20 | Present | 17 | 9 | 6 | A |
| Example 234 | 3 | 1 | Dye-43 | 43 | TPB-1 | 30 | I-1 | 12 | M-1 | 500 | - | - | - | - | - | - | 4 | Lamellar compound | 20 | Present | 15 | 9 | 6 | A |
| Example 235 | 3 | 1 | Dye-44 | 43 | TPB-1 | 30 | I-1 | 12 | M-1 | 500 | - | - | - | - | - | - | 4 | Lamellar compound | 20 | Present | 15 | 9 | 6 | A |
| Example 236 | 3 | 1 | Dye-45 | 43 | TPB-1 | 30 | I-1 | 12 | M-1 | 500 | - | - | - | - | - | - | 4 | Lamellar compound | 20 | Present | 15 | 9 | 6 | A |
| Example 237 | 3 | 1 | Dye-46 | 43 | TPB-1 | 30 | I-1 | 12 | M-1 | 500 | - | - | - | - | - | - | 4 | Lamellar compound | 20 | Present | 16 | 9 | 6 | A |
| Example 238 | 3 | 1 | Dye-47 | 43 | TPB-1 | 30 | I-1 | 12 | M-1 | 500 | - | - | - | - | - | - | 4 | Lamellar compound | 20 | Present | 16 | 9 | 6 | A |
| Example 239 | 3 | 1 | Dye-48 | 43 | TPB-1 | 30 | I-1 | 12 | M-1 | 500 | - | - | - | - | - | - | 4 | Lamellar compound | 20 | Present | 15 | 9 | 5 | A |
| Example 240 | 3 | 1 | Dye-49 | 43 | TPB-1 | 30 | I-1 | 12 | M-1 | 500 | - | - | - | - | - | - | 4 | Lamellar compound | 20 | Present | 17 | 9 | 6 | A |
| Example 241 | 3 | 1 | Dye-50 | 43 | TPB-1 | 30 | I-1 | 12 | M-1 | 500 | - | - | - | - | - | - | 4 | Lamellar compound | 20 | Present | 17 | 9 | 6 | A |
| Example 242 | 3 | 1 | Dye-51 | 43 | TPB-1 | 30 | I-1 | 12 | M-1 | 500 | - | - | - | - | - | - | 4 | Lamellar compound | 20 | Present | 17 | 9 | 6 | A |
| Example 243 | 3 | 1 | Dye-52 | 43 | TPB-1 | 30 | I-1 | 12 | M-1 | 500 | - | - | - | - | - | - | 4 | Lamellar compound | 20 | Present | 17 | 9 | 6 | A |
| Example 244 | 3 | 1 | Dye-53 | 43 | TPB-1 | 30 | I-1 | 12 | M-1 | 500 | - | - | - | - | - | - | 4 | Lamellar compound | 20 | Present | 18 | 10 | 6 | A |
| Example 245 | 3 | 1 | Dye-54 | 43 | TPB-1 | 30 | I-1 | 12 | M-1 | 500 | - | - | - | - | - | - | 4 | Lamellar compound | 20 | Present | 17 | 10 | 6 | A |
| Example 246 | 3 | 1 | Dye-55 | 43 | TPB-1 | 30 | I-1 | 12 | M-1 | 500 | - | - | - | - | - | - | 4 | Lamellar compound | 20 | Present | 15 | 8 | 6 | A |
| Example 247 | 3 | 1 | Dye-56 | 43 | TPB-1 | 30 | I-1 | 12 | M-1 | 500 | - | - | - | - | - | - | 4 | Lamellar compound | 20 | Present | 15 | 8 | 6 | A |
| Example 248 | 3 | 1 | Dye-57 | 43 | TPB-1 | 30 | I-1 | 12 | M-1 | 500 | - | - | - | - | - | - | 4 | Lamellar compound | 20 | Present | 15 | 8 | 6 | A |
| Example 249 | 3 | 1 | Dye-58 | 43 | TPB-1 | 30 | I-1 | 12 | M-1 | 500 | - | - | - | - | - | - | 4 | Lamellar compound | 20 | Present | 17 | 10 | 5 | A |
| Example 250 | 3 | 1 | Dye-59 | 43 | TPB-1 | 30 | I-1 | 12 | M-1 | 500 | - | - | - | - | - | - | 4 | Lamellar compound | 20 | Present | 17 | 10 | 5 | A |
| Example 251 | 3 | 1 | Dye-60 | 43 | TPB-1 | 30 | I-1 | 12 | M-1 | 500 | - | - | - | - | - | - | 4 | Lamellar compound | 20 | Present | 17 | 10 | 5 | A |
| Example 252 | 3 | 1 | Dye-61 | 43 | TPB-1 | 30 | I-1 | 12 | M-1 | 500 | - | - | - | - | - | - | 4 | Lamellar compound | 20 | Present | 17 | 10 | 5 | A |
| Example 253 | 3 | 1 | Dye-62 | 43 | TPB-1 | 30 | I-1 | 12 | M-1 | 500 | - | - | - | - | - | - | 4 | Lamellar compound | 20 | Present | 17 | 10 | 5 | A |
| Example 254 | 3 | 1 | Dye-63 | 43 | TPB-1 | 30 | I-1 | 12 | M-1 | 500 | - | - | - | - | - | - | 4 | Lamellar compound | 20 | Present | 16 | 9 | 6 | A |
| Example 255 | 3 | 1 | Dye-64 | 43 | TPB-1 | 30 | I-1 | 12 | M-1 | 500 | - | - | - | - | - | - | 4 | Lamellar compound | 20 | Present | 15 | 8 | 5 | A |
| Example 256 | 3 | 1 | Dye-65 | 43 | TPB-1 | 30 | I-1 | 12 | M-1 | 500 | - | - | - | - | - | - | 4 | Lamellar compound | 20 | Present | 14 | 7 | 5 | A |
| Comparative Example 6 | 3 | 1 | Dye-A | 43 | TPB-1 | 30 | I-1 | 12 | M-1 | 500 | - | - | - | - | - | - | 4 | Lamellar compound | 20 | Present | 11 | 5 | 6 | B |
| Comparative Example 7 | 3 | 1 | Dye-B | 43 | TPB-1 | 30 | I-1 | 12 | M-1 | 500 | - | - | - | - | - | - | 4 | Lamellar compound | 20 | Present | 11 | 5 | 5 | C |
| Comparative Example 8 | 3 | 1 | Dye-C | 43 | TPB-1 | 30 | I-1 | 12 | M-1 | 500 | - | - | - | - | - | - | 4 | Lamellar compound | 20 | Present | 4 | 2 | 5 | C |
| Comparative Example 9 | 3 | 1 | Dye-D | 43 | TPB-1 | 30 | I-2/I-3 | 6/6 | M-2 | 500 | - | - | - | - | - | - | 4 | Lamellar compound | 20 | Present | 4 | 2 | 5 | C |
| Comparative Example 10 | 3 | 1 | Dye-E | 43 | TPB-1 | 30 | I-2/I-3 | 6/6 | M-2 | 500 | - | - | - | - | - | - | 4 | Lamellar compound | 20 | Present | 4 | 2 | 5 | C |

The details of the abbreviations shown in Tables 1 to 4 other than those described in the production methods for the respective layers described above are shown.
Dye-1 to 65: Dye-1 to 65 described above

Dye-A to C: the following compounds

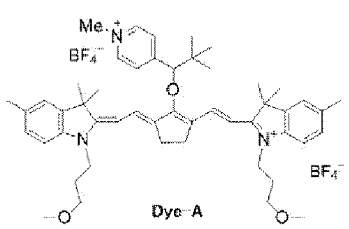
Dye-A

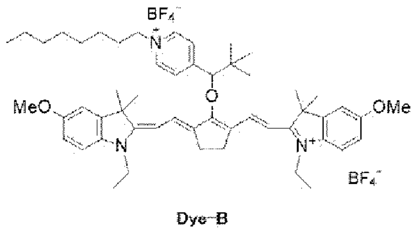
Dye-B

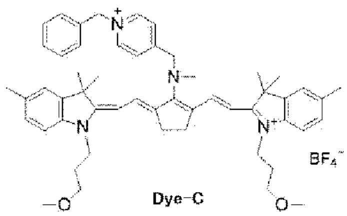
Dye-C

Dye-D and E: the following compounds

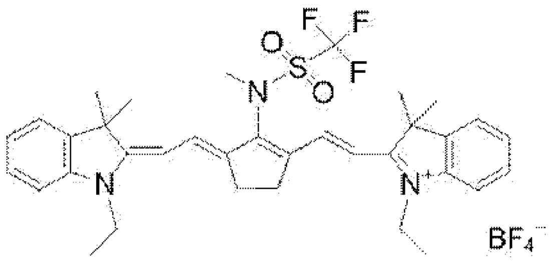
Dye-D

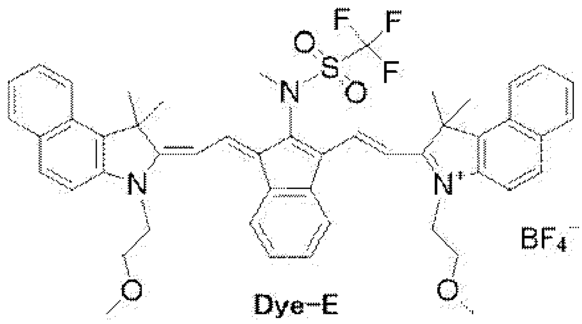
Dye-E

TPB-1 to TPB-4: the following compounds

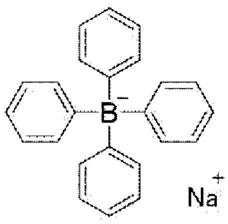
TPB-1

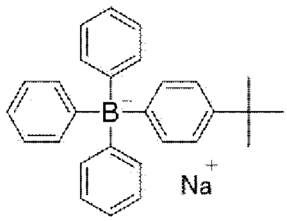
TPB-2

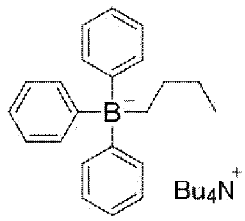
TPB-3

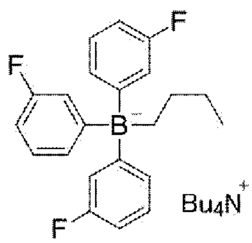
TPB-4

I-1 to I-3: the following compounds

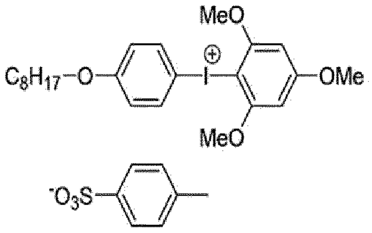

I-1

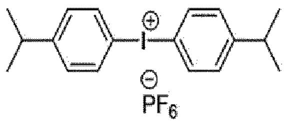

I-2

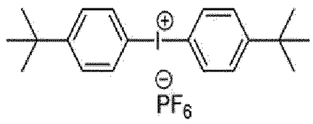

I-3

M-1: prepared by the following method

Method for producing M-1

A mixed solution of TAKENATE D-160N (polyisocyanate trimethylolpropane adduct, manufactured by Mitsui Chemicals, Inc., 4.7 parts), ARONIX M-403 (manufactured by TOAGOSEI CO., LTD., amount yielding the ratio of NCO value of TAKENATE D-160N:hydroxyl number of ARONIX M-403 = 1:1), t-butylbenzoquinone (0.02 parts), and methyl ethyl ketone (11.5 parts) was heated at 65°C. NEOSTAN U-600 (bismuth-based polycondensation catalyst, manufactured by NITTO KASEI CO., LTD., 0.11 parts) was added to the reaction solution, and the reaction solution was heated at 65°C for 4 hours. The reaction solution was cooled to room temperature (25°C), and methyl ethyl ketone was added thereto, thereby synthesizing a urethane acrylate (M-2) solution having a solid content of 50% by mass. By using recycling GPC (instrument: LC908-C60, column: JAIGEL-1H-40 and 2H-40 (manufactured by Japan Analytical Industry Co., Ltd.)) and tetrahydrofuran (THF) as an eluent, molecular weight fractionation of the urethane acrylate solution was performed. The weight-average molecular weight was 14,000.

M-2: A polymerizable compound with a concentration of 80% by weight in a 2-butanone solution, obtained by reacting DESMODUR (registered trademark) N100, hydroxyethyl acrylate, and pentaerythritol acrylate in a molar ratio of 1:1.5:1.5
M-3: Dipentaerythritol hexaacrylate (DPHA)
M-4: Ethoxylated (4) pentaerythritol tetraacrylate (SR494 manufactured by Sartomer Company Inc.)

IR-1: the following compound

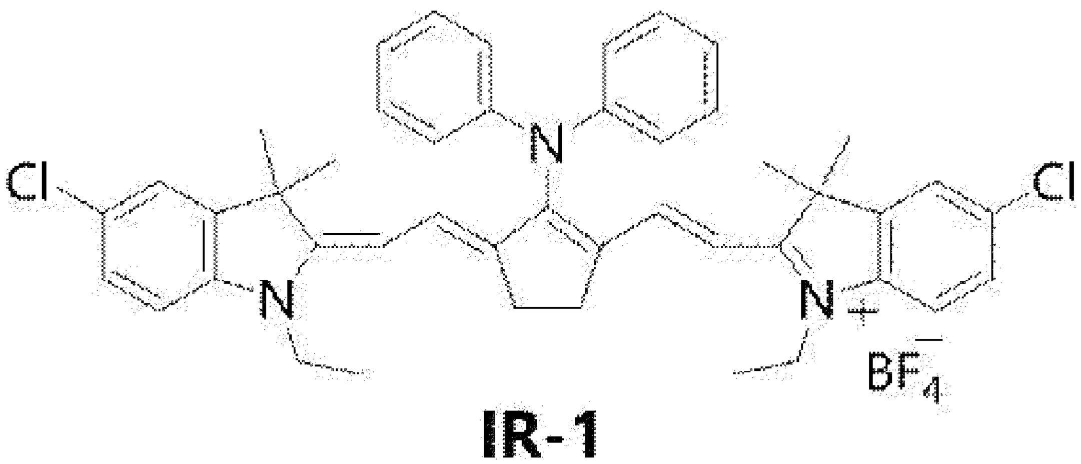

IR-1

S-1 to S-3: the following compounds

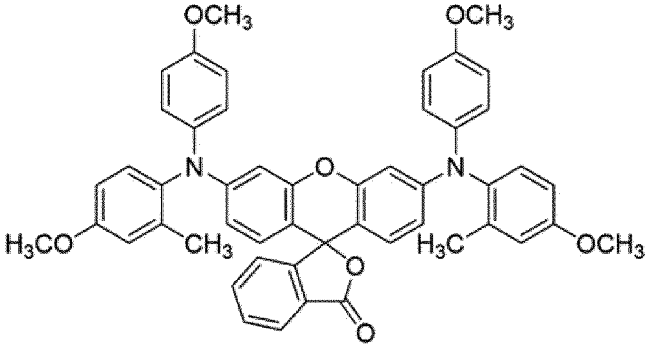

S-1

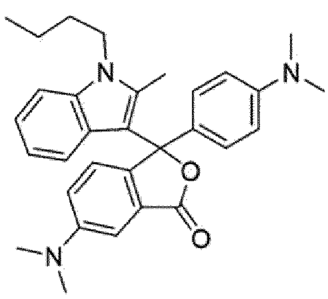

S-2

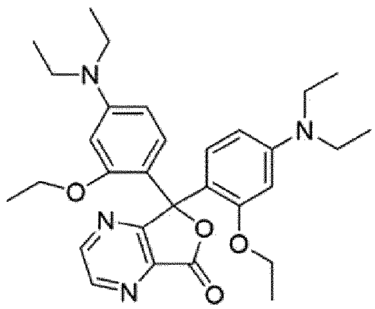

S-3

P-1 to P-3: the following compounds

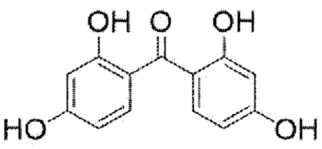

P-1

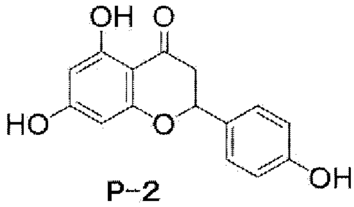

P-2

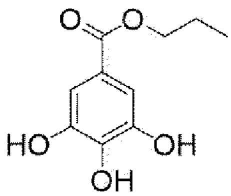

P-3

From the results shown in Tables 1 to 4, it was found that the lithographic printing plate precursors according to Examples had excellent printing durability.

In addition, in Examples 31 to 33, 92 to 94, 167 to 169, and 218 to 220, the visibility of the exposed portion was better even after the storage under a white light after the exposure, compared to a case where the hydrogen donating compound such as a phenol compound was not added.

Example 257

A lithographic printing plate precursor was prepared and evaluated in the same manner as in Example 64, except that the polymer particles R-1 of Example 64 were changed to the polymer particles R-2. The result of the printing durability was that the value of the relative printing durability was increased by 20 as compared with the result of Example 64. Other performances were the same as those in Example 64.

Preparation of polymer particles R-2

Preparation of oil-phase component

A polyfunctional isocyanate compound (PM-200: manufactured by Wanhua Chemical Group Co., Ltd.: 6.66 g, a

50% by mass ethyl acetate solution of "TAKENATE (registered trademark) D-116N (adduct of trimethylolpropane (TMP), m-xylylene diisocyanate (XDI), and polyethylene glycol monomethyl ether (EO90) (following structure)" manufactured by Mitsui Chemicals, Inc.: 5.46 g, a 65% by mass ethyl acetate solution of dipentaerythritol pentaacrylate (SR-399, manufactured by Sartomer Company Inc.): 11.24 g, ethyl acetate: 14.47 g, and PIONIN (registered trademark) A-41-C manufactured by TAKEMOTO OIL & FAT Co., Ltd.: 0.45 g were mixed together and stirred at room temperature (25°C) for 15 minutes, thereby obtaining an oil-phase component.

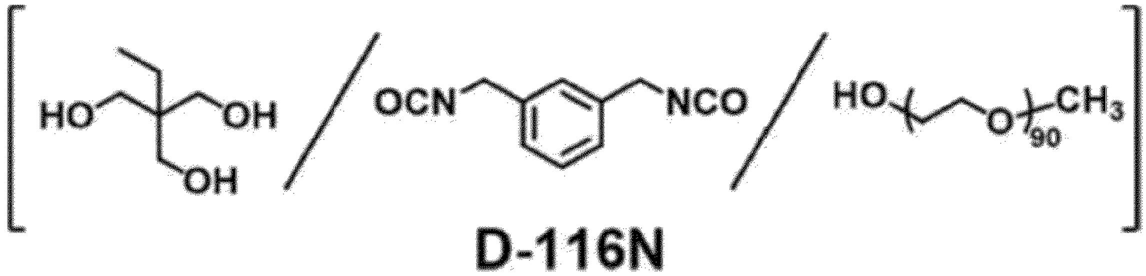

D-116N

Preparation of water-phase component

As a water-phase component, 47.2 g of distilled water was prepared.

Microcapsule forming step

The oil-phase component and the water-phase component were mixed together, and the obtained mixture was emulsified at 12,000 rpm for 16 minutes by using a homogenizer, thereby obtaining an emulsion.

Distilled water (16.8 g) was added to the obtained emulsion, and the obtained liquid was stirred at room temperature for 10 minutes.

After stirring, the liquid was heated to 45°C, and stirred for 4 hours in a state of being kept at 45°C such that ethyl acetate was distilled away from the liquid. Then, a 10% by mass aqueous solution of 5.12 g of 1,8-diazabicyclo[5.4.0]undec-7-ene-octylate (U-CAT SA102, manufactured by San-Apro Ltd.) was added thereto, and the solution was stirred at room temperature for 30 minutes and left to stand at 45°C for 24 hours. Distilled water was added thereto such that the concentration of solid contents was adjusted to 20% by mass, thereby obtaining an aqueous dispersion of polymer particles R-2. R-2 had a volume average particle diameter of 165 nm that was measured using a laser diffraction/scattering-type particle diameter distribution analyzer LA-920 (manufactured by HORIBA, Ltd.).

Example 258

A lithographic printing plate precursor was prepared and evaluated in the same manner as in Example 64, except that the polymer particles R-1 of Example 64 were changed to the polymer particles R-3. The result of the printing durability was that the value of the relative printing durability was decreased by 20 as compared with the result of Example 64. Other performances were the same as those in Example 64.

Preparation of polymer particles R-3

Dispersion unit: the following compound B-1 (n = 45): 10.0 parts, 85.0 parts of distilled water, and 240.0 parts of n-propanol were added to a four-neck flask, and the mixture was heated and stirred at 70°C in a nitrogen atmosphere.

Then, a mixture of the following compound A-1: 20.0 parts, the following compound A-2: 70.0 parts, and 0.7 parts of 2,2'-azobisisobutyronitrile that were mixed together in advance was added dropwise for 2 hours to the four-neck flask.

After the dropwise addition ended, the reaction continued for 5 hours, then 0.5 parts of 2,2'-azobisisobutyronitrile was added thereto, and the solution was heated to 80°C. The reaction was performed for a total of 19 hours by adding 0.4 parts of 2,2'-azobisisobutyronitrile every 6 hours.

The reaction solution was left to cool to room temperature (25°C), thereby obtaining a dispersion (solid content 23%) of polymer particles R-3.

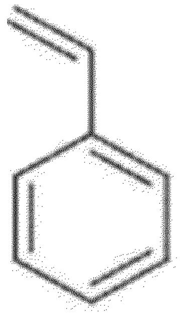

A-1

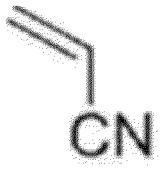

A-2

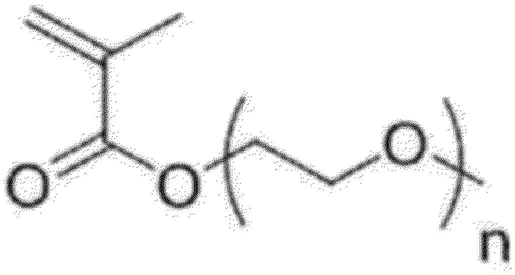

B-1

The polymer particles R-3 had a median diameter of 150 nm and a coefficient of variation of 23%.

Furthermore, the dispersibility of the polymer particles R-3 was checked by the method described above. As a result, the resin particles R-3 were found to be water-dispersible and organic solvent-dispersible particles.

Explanation of References

1: lithographic printing plate precursor
1a: image-recording layer surface
1b: support surface
1c: edge surface
2: bevel
10: cutting blade
10a: upper cutting blade
10b: upper cutting blade
11: rotary shaft
20: cutting blade
20a: lower cutting blade
20b: lower cutting blade
21: rotary shaft
30: lithographic printing plate precursor
50: main electrolytic cell
51: alternating current power source
52: radial drum roller
53a, 53b: main pole
54: electrolytic solution supply port
55: electrolytic solution
56: slit
57: electrolytic liquid channel
58: auxiliary anode
60: auxiliary anode cell
610: anodization treatment device
612: power supply tank
614: electrolytic treatment tank
616: aluminum plate
618, 626: electrolytic solution
620: power supply electrode
622, 628: roller
624: nip roller
630: electrolysis electrode
632: tank wall
634: direct current power source

W: aluminum plate
S: liquid supply direction
Ex: electrolytic solution discharge direction
ta: anodic reaction time
tc: cathodic reaction time
tp: time taken for current to reach peak from 0
Ia: peak current on anodic cycle side
Ic: peak current on cathodic cycle side
AA: current of anodic reaction of aluminum plate
CA: current of cathodic reaction of aluminum plate

## Claims

**1.** An on-press development type lithographic printing plate precursor comprising:

a support; and
an image-recording layer on the support,
wherein the image-recording layer contains a compound represented by Formula (1),

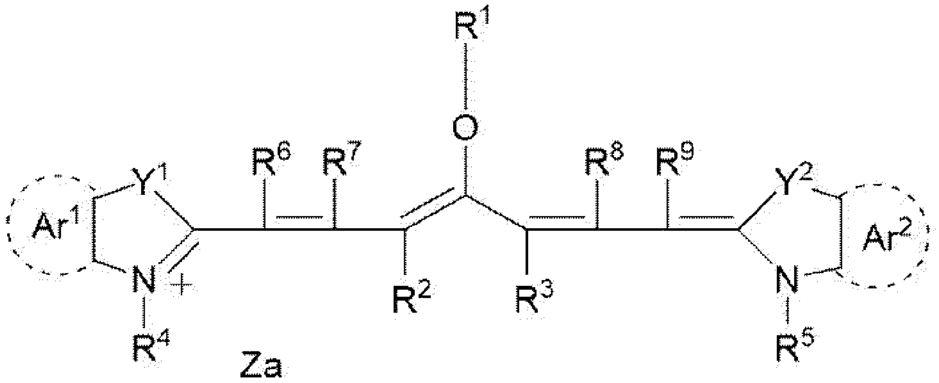

(1)

in Formula (1), $R^1$ represents a group represented by Formula (2) or Formula (3), $R^2$ and $R^3$ each independently represent a hydrogen atom or an alkyl group, $R^2$ and $R^3$ may be linked to each other to form a ring, $Ar^1$ and $Ar^2$ each independently represent a benzene ring or a naphthalene ring, $Y^1$ and $Y^2$ each independently represent an oxygen atom, a sulfur atom, -$NR^0$-, or a dialkylmethylene group, $R^4$ and $R^5$ each independently represent an alkyl group, $R^6$ to $R^9$ each independently represent a hydrogen atom or an alkyl group, $R^0$ represents a hydrogen atom, an alkyl group, or an aryl group, and Za represents a counterion that neutralizes a charge,

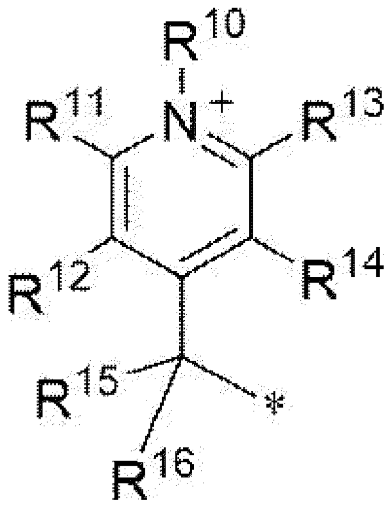

(2)

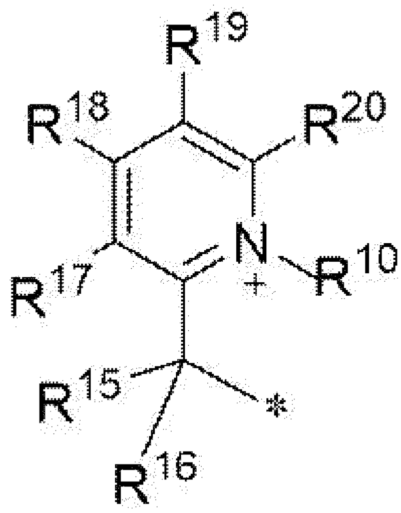

(3)

in Formulae (2) and (3), $R^{10}$ represents a monovalent organic group having an aryl group, $R^{11}$ to $R^{14}$ and $R^{17}$ to $R^{20}$ each independently represent a hydrogen atom, an alkyl group, an aryl group, a hydroxy group, an alkoxy group, or a halogen atom, $R^{15}$ and $R^{16}$ each independently represent a hydrogen atom, an alkyl group, or an aryl group, at least two of $R^{11}$ to $R^{20}$ may be bonded to each other to form a ring structure, and * represents a single bond that is bonded to an oxygen atom in Formula (1).

**2.** The on-press development type lithographic printing plate precursor according to claim 1, wherein $R^{10}$ is a substituted benzyl group.

**3.** The on-press development type lithographic printing plate precursor according to claim 2, wherein $R^{10}$ is a benzyl group having an alkyl group on a benzene ring.

**4.** The on-press development type lithographic printing plate precursor according to any one of claims 1 to 3, wherein $R^{15}$ is a hydrogen atom, and $R^{16}$ is a branched alkyl group.

**5.** The on-press development type lithographic printing plate precursor according to any one of claims 1 to 4, wherein $R^{15}$ is a hydrogen atom, and $R^{16}$ is a t-butyl group.

**6.** The on-press development type lithographic printing plate precursor according to any one of claims 1 to 5, wherein $R^{11}$ to $R^{14}$ and $R^{17}$ to $R^{20}$ are hydrogen atoms.

**7.** The on-press development type lithographic printing plate precursor according to any one of claims 1 to 6, wherein the image-recording layer further contains a borate compound.

**8.** The on-press development type lithographic printing plate precursor according to any one of claims 1 to 7, wherein the image-recording layer further contains a polymerizable compound.

**9.** The on-press development type lithographic printing plate precursor according to any one of claims 1 to 8,

wherein the image-recording layer further contains an onium salt compound, and
wherein the onium salt compound preferably includes an iodonium salt compound.

**10.** The on-press development type lithographic printing plate precursor according to any one of claims 1 to 9, wherein the image-recording layer further contains a phenol compound.

**11.** A method of preparing a lithographic printing plate, comprising:

a step of exposing the on-press development type lithographic printing plate precursor according to any one of claims 1 to 10 in a shape of an image; and
a step of supplying at least one selected from the group consisting of a printing ink and dampening water on a printer to remove an image-recording layer in a non-image area.

**12.** A lithographic printing method comprising:

a step of exposing the on-press development type lithographic printing plate precursor according to any one of claims 1 to 10 in a shape of an image;
a step of supplying at least one selected from the group consisting of a printing ink and dampening water on a printer to remove an image-recording layer in a non-image area and to prepare a lithographic printing plate; and
a step of performing printing using the obtained lithographic printing plate.

**13.** A compound represented by Formula (1A),

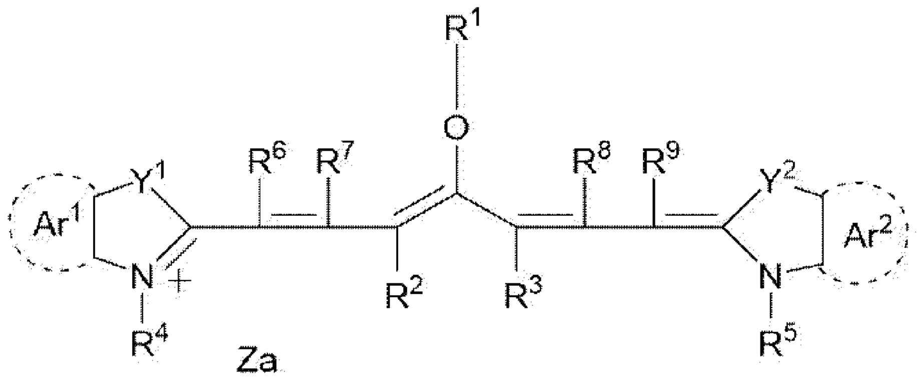

(1A)

in Formula (1A), $R^1$ represents a group represented by Formula (2A) or Formula (3A), $R^2$ and $R^3$ each independently represent a hydrogen atom or an alkyl group, $R^2$ and $R^3$ may be linked to each other to form a ring, $Ar^1$ and $Ar^2$ each independently represent a benzene ring or a naphthalene ring, $Y^1$ and $Y^2$ each independently represent an oxygen atom, a sulfur atom, $-NR^0-$, or a dialkylmethylene group, $R^4$ and $R^5$ each independently represent an alkyl group, $R^6$ to $R^9$ each independently represent a hydrogen atom or an alkyl group, $R^0$ represents a hydrogen atom, an alkyl group, or an aryl group, and Za represents a counterion that neutralizes a charge,

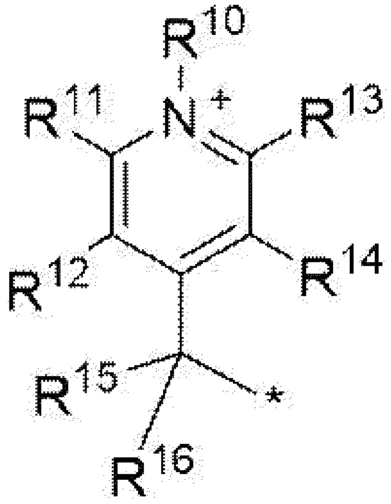

(2A)

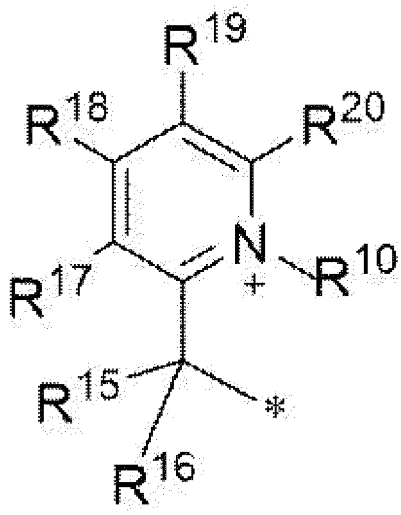

(3A)

in Formulae (2A) and (3A), $R^{10}$ represents a group having one or more substituents on an aromatic ring of a benzyl group, $R^{11}$ to $R^{14}$ and $R^{17}$ to $R^{20}$ each independently represent a hydrogen atom, an alkyl group, an aryl group, a hydroxy group, an alkoxy group, or a halogen atom, $R^{15}$ and $R^{16}$ each independently represent a hydrogen atom, an alkyl group, or an aryl group, at least two of $R^{11}$ to $R^{20}$ may be bonded to each other to form a ring structure, and * represents a single bond that is bonded to an oxygen atom in Formula (1A).

**14.** The compound according to claim 13,

wherein the compound is a compound represented by Formula (7) or Formula (8),

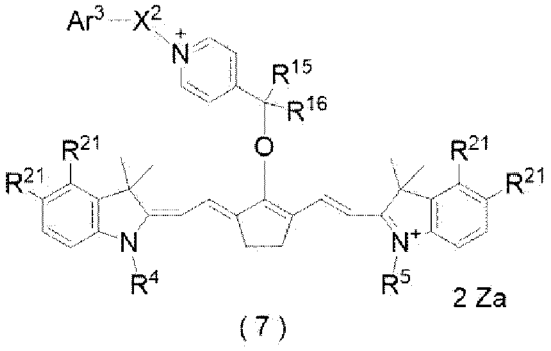

(7)

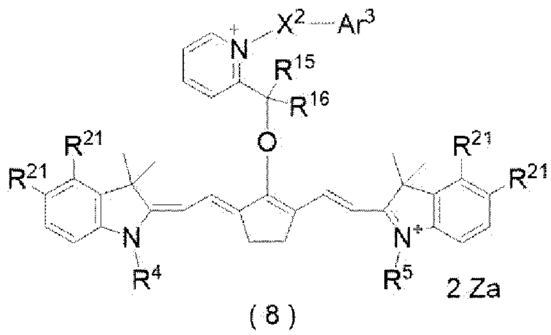

(8)

in Formula (7) and Formula (8), $X^2$ represents a single bond or an alkylene group, $Ar^3$ represents an aryl group having one or more substituents on an aromatic ring, $R^4$ and $R^5$ each independently represent an alkyl group, $R^{15}$ and $R^{16}$ each independently represent a hydrogen atom, an alkyl group, or an aryl group, $R^{15}$ and $R^{16}$ may be bonded to each other to form a ring structure, $R^{21}$'s each independently represent an alkyl group having 1 to 12 carbon atoms, an alkoxy group, an aryloxy group, an amino group, an alkoxythio group, or a halogen atom, $R^{21}$'s may be linked to each other to form a ring, and Za represents a counterion that neutralizes a charge.

**15.** The compound according to claim 13,

wherein the compound is a compound represented by Formula (9),

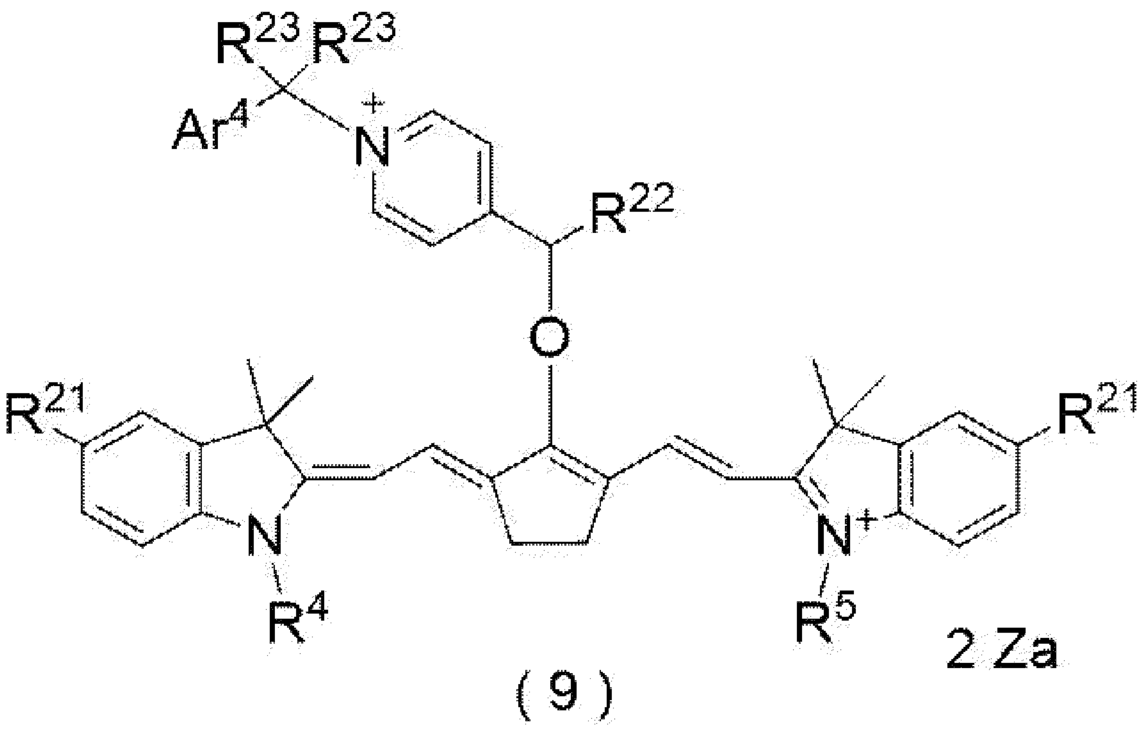

(9)

in Formula (9), $Ar^4$ represents an aryl group having one or more substituents on an aromatic ring, $R^4$ and $R^5$ each independently represent an alkyl group, $R^{21}$'s each independently represent an alkyl group having 1 to 12 carbon atoms, an alkoxy group, an aryloxy group, an amino group, an alkoxythio group, or a halogen atom, $R^{22}$ represents a hydrogen atom, an alkyl group, or an aryl group, $R^{23}$'s each independently represent a hydrogen atom or an alkyl group, and Za represents a counterion that neutralizes charge.

FIG. 1

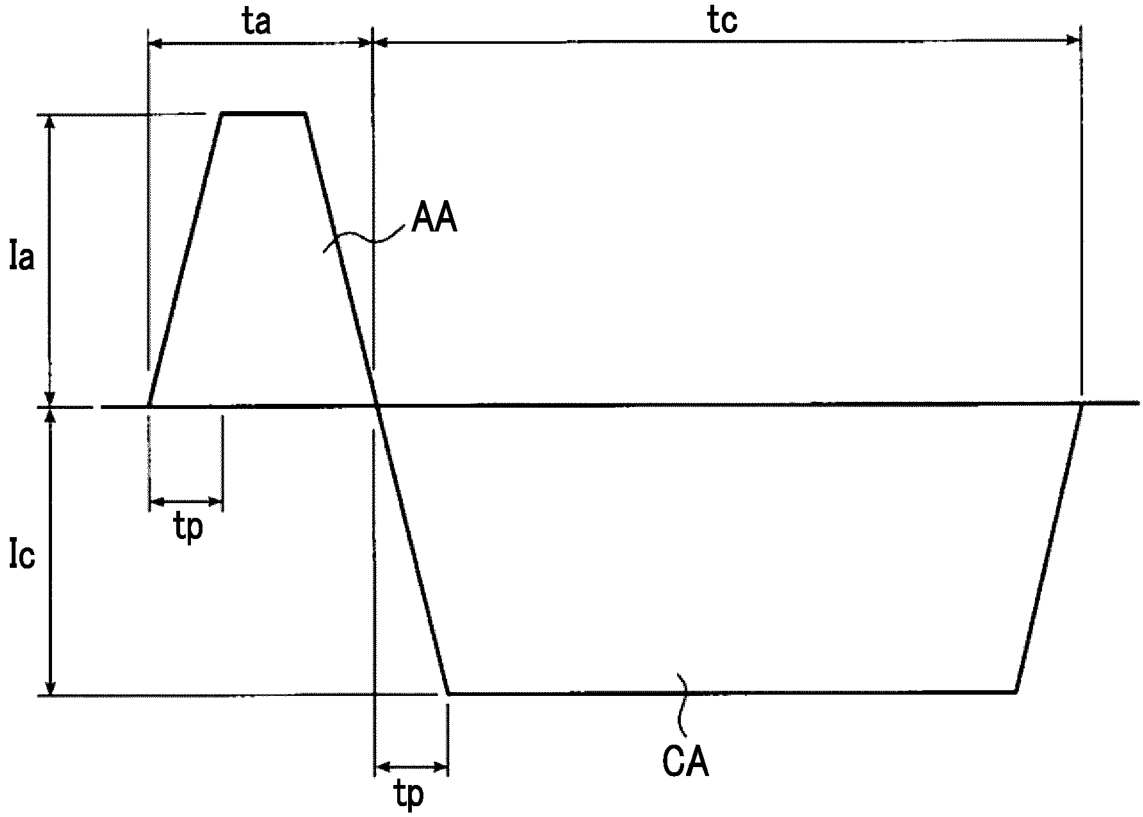

FIG. 2

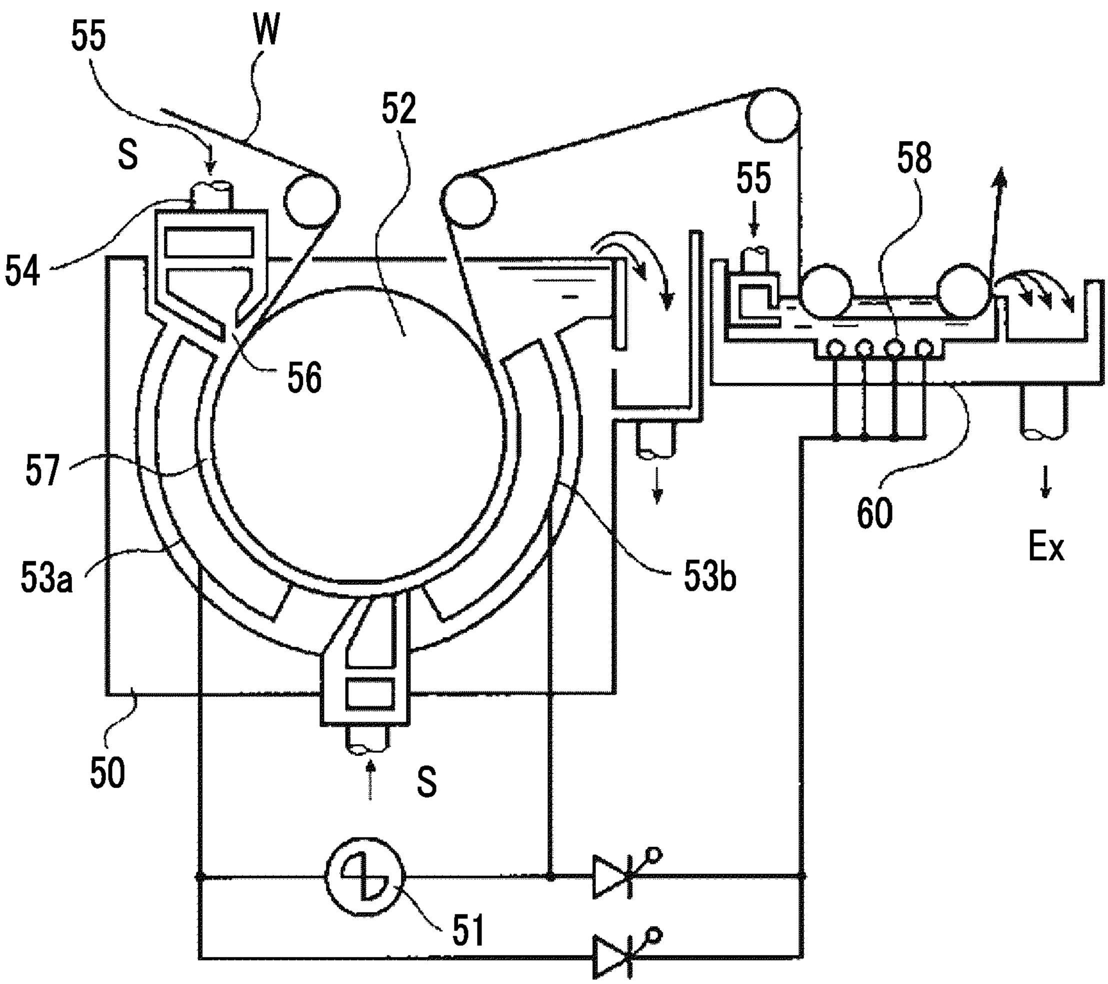

FIG. 3

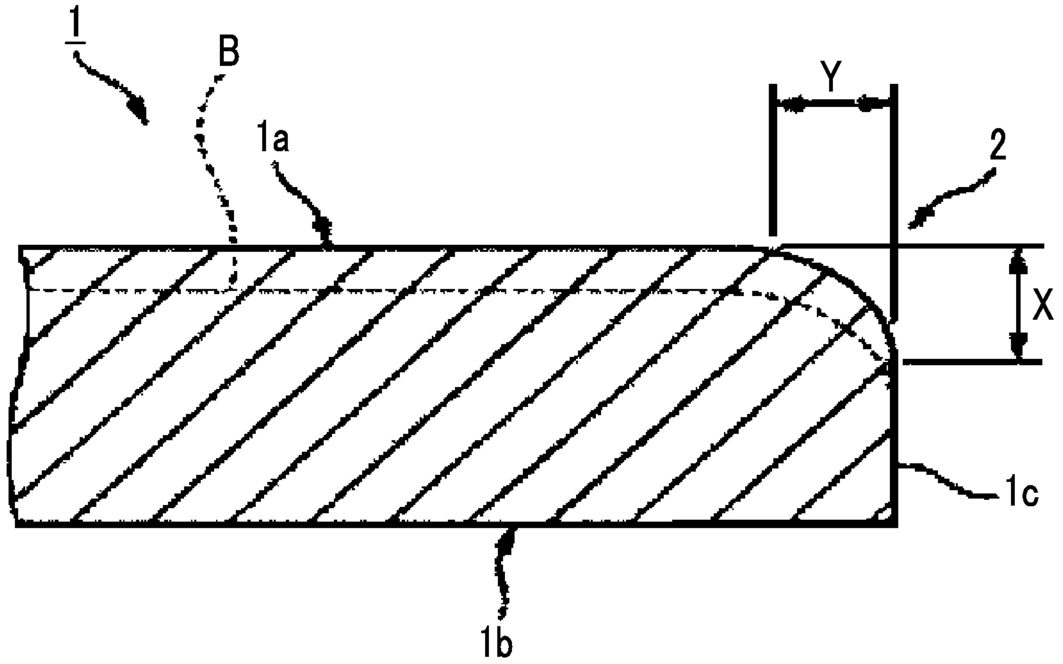


FIG. 4

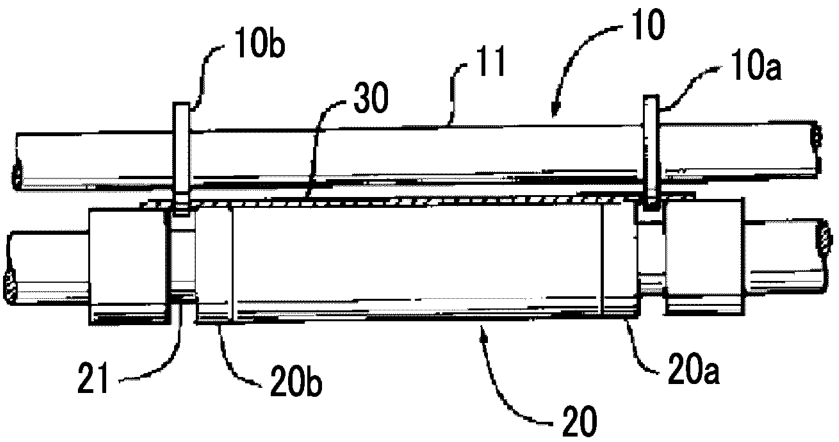

FIG. 5

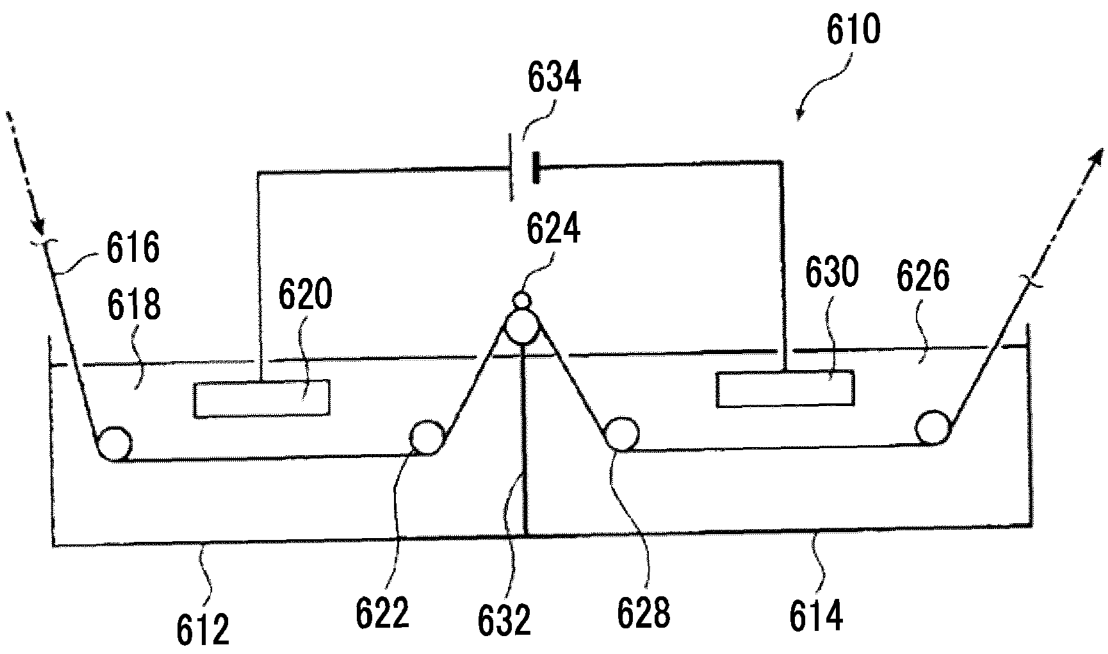

Europäisches Patentamt
European Patent Office
Office européen des brevets

# EUROPEAN SEARCH REPORT

Application Number
EP 25 15 9050

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br><br>A | EP 3 418 332 A1 (FUJIFILM CORP [JP])<br>26 December 2018 (2018-12-26)<br>* the whole document *<br>* especially example 33 using compound A-15 shown on page 22 *<br>----- | 1-12<br><br>13-15 | INV.<br>B41C1/10 |
| X,P | WO 2025/013910 A1 (FUJIFILM CORP [JP])<br>16 January 2025 (2025-01-16)<br>* the whole document *<br>* especially examples 3-44, 3-45 and 3-46, see table 3 on page 220, using compounds IR-13 to IR-15 shown on page 205 *<br>----- | 1,4-9,<br>11,12 | |
| X,P | WO 2025/013922 A1 (FUJIFILM CORP [JP])<br>16 January 2025 (2025-01-16)<br>* the whole document *<br>* especially examples 33 and 34, see paragraph 444, using compounds IR-14 and IR-15 being shown on page 170 *<br>----- | 1,4-9,<br>11,12 | |

TECHNICAL FIELDS SEARCHED (IPC)

B41C

The present search report has been drawn up for all claims

1

| Place of search | Date of completion of the search | Examiner |
|---|---|---|
| Munich | 12 June 2025 | Vogel, Thomas |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.....................................................................................................
& : member of the same patent family, corresponding document

EPO FORM 1503 03.82 (P04C01)

## ANNEX TO THE EUROPEAN SEARCH REPORT ON EUROPEAN PATENT APPLICATION NO. EP 25 15 9050

This annex lists the patent family members relating to the patent documents cited in the above-mentioned European search report. The members are as contained in the European Patent Office EDP file on The European Patent Office is in no way liable for these particulars which are merely given for the purpose of information.

12-06-2025

| Patent document cited in search report | Publication date | Patent family member(s) | Publication date |
|---|---|---|---|
| EP 3418332 A1 | 26-12-2018 | BR 112018016993 A2 | 26-12-2018 |
| | | CN 108699344 A | 23-10-2018 |
| | | EP 3418332 A1 | 26-12-2018 |
| | | JP 6785295 B2 | 18-11-2020 |
| | | JP WO2017141882 A1 | 27-12-2018 |
| | | US 2018356730 A1 | 13-12-2018 |
| | | WO 2017141882 A1 | 24-08-2017 |
| WO 2025013910 A1 | 16-01-2025 | NONE | |
| WO 2025013922 A1 | 16-01-2025 | NONE | |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description

- WO 2017141882 A
- WO 2016027886 A
- JP 2008195018 A
- JP 8108621 A
- JP H8108621 A
- JP 61166544 A
- JP S61166544 A
- JP 2002328465 A
- WO 2020262685 A
- WO 2022019217 A
- JP 2001133969 A
- JP 2002023360 A
- JP 2002040638 A
- JP 2002278057 A
- JP 2007090850 A
- JP 2012206495 A
- JP 1993 A
- JP 5005 A
- JP H055005 A
- JP 2001222101 A
- JP 2008 A
- JP 195018 A
- WO 2020262692 A
- JP 2008544322 A
- WO 2019219560 A
- WO 2023032681 A
- JP 2006508380 A
- JP 2002287344 A
- JP 2008256850 A
- JP 2001342222 A
- JP 9179296 A
- JP H09179296 A
- JP 9179297 A
- JP H09179297 A
- JP 9179298 A
- JP H09179298 A
- JP 2004294935 A
- JP 2006243493 A
- JP 2002275129 A
- JP 2003064130 A
- JP 2003280187 A
- JP 10333321 A
- JP H10333321 A
- JP 48041708 B
- JP S4841708 B
- JP 51037193 A
- JP S5137193 A
- JP 2032293 B
- JP H0232293 B
- JP 2016765 B
- JP H0216765 B
- JP 2003344997 A
- JP 2006065210 A
- JP 58049860 B
- JP S5849860 B
- JP 56017654 B
- JP S5617654 B
- JP 62039417 B
- JP S6239417 B
- JP 62039418 B
- JP S6239418 B
- JP 2000250211 A
- JP 2007094138 A
- US 7153632 B
- JP 8505958 A
- JP H08505958 A
- JP 2007293221 A
- JP 2007293223 A
- JP 2023020769 A
- JP 2023179624 A
- WO 2020137919 A
- JP 2008284817 A
- JP 5045885 A
- JP H545885 A
- JP 6035174 A
- JP H635174 A
- JP 50040047 B
- JP S5040047 B
- GB 1412768 A
- JP 2005254638 A
- US 2714066 A
- US 3181461 A
- JP 10282679 A
- JP H10282679 A
- JP 2304441 A
- JP H02304441 A
- JP 2005238816 A
- JP 2005125749 A
- JP 2006239867 A
- JP 2006215263 A
- JP 2006188038 A
- US 3458311 A
- JP 55049729 B
- JP S5549729 B
- JP 2005250216 A
- JP 2006259137 A
- WO 2022138880 A

- WO 202167054 A

**Non-patent literature cited in the description**


- **NAOKI INAMOTO**. Chemical Seminar 10 Hammett Side-Structure and Reactivity. Maruzen Co., Ltd., June 1983
- Dye Handbooks. 1970